# EUROPEAN PATENT APPLICATION

(11) **EP 3 760 611 A1**
(43) Date of publication of application: **06.01.2021**
(21) Application number: 18907898.3
(22) Date of filing: 21.11.2018
(51) Int. Cl.: C07C 235/76, C07D 207/448, C08G 16/02, G03F 7/11, G03F 7/26

(54) **COMPOUND, RESIN, COMPOSITION AND FILM-FORMING MATERIAL FOR LITHOGRAPHY USING SAME**

(30) Priority: 28.02.2018 JP 2018034327
(71) Applicant: MITSUBISHI GAS CHEMICAL COMPANY, INC., Chiyoda-ku Tokyo 100-8324 (JP)
(72) Inventor: ECHIGO, Masatoshi, Tokyo 100-8324 (JP); MAKINOSHIMA, Takashi, Hiratsuka-shi, Kanagawa 254-0016 (JP)
(74) Representative: Müller-Boré & Partner Patentanwälte PartG mbB
(86) International application number: PCT/JP2018/043019
(87) International publication number: WO 2019/167359

(57) **Abstract**

A compound represented by the following formula (0). 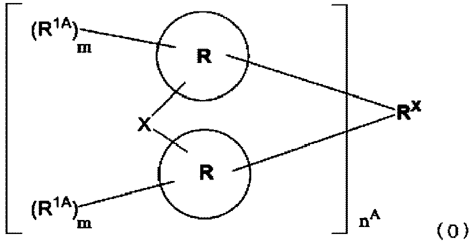 (In the above formula (0),
R^{X} represents a 2n^{A}-valent group having 1 to 70 carbon atoms or a single bond;
each R^{1A} independently represents any of an alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, an alkoxy group having 1 to 30 carbon atoms and optionally having a substituent, a maleamic acid group having 4 to 30 carbon atoms and optionally having a substituent, a maleimide group having 4 to 30 carbon atoms and optionally having a substituent, a halogen atom, a nitro group, an amino group having 0 to 30 carbon atoms and optionally having a substituent, a carboxyl group, a thiol group and a hydroxy group, wherein, when R^{1A} is any of the alkyl group, the aryl group, the crosslinkable group and the alkoxy group, at least one bond selected from the group consisting of an ether bond, a ketone bond and an ester bond is optionally contained, and at least one R^{1A} is any of a maleamic acid group having 4 to 30 carbon atoms and optionally having a substituent and a maleimide group having 4 to 30 carbon atoms and optionally having a substituent;
X represents an oxygen atom or a sulfur atom, or is optionally not present;
each R independently represents any of a benzene ring, a naphthalene ring and an anthracene ring;
each m is independently an integer of 0 to 9, wherein at least one m is an integer of 1 to 9; and
n^{A} is an integer of 1 to 4.)

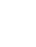

## Description

### Technical Field

The present invention relates to a compound, a resin, a composition, and a film forming material for lithography using the same.

### Background Art

(Poly)maleimide compounds or (poly)maleamic acids are generally known as high heat resistant thermosetting resins, and have been widely used in the fields of molding materials, composite materials, electric and electronic components, and the like. In the field of electric and electronic components, there has been a remarkable development of microelectronics in recent years. In particular, for large computers, high speed transmission of signals is indispensable due to the adoption of multilayer circuit boards and the like. However, when the dielectric constant of the substrate material is large, there will be a delay in signal transmission and this will be an obstacle to high speed transmission. (Poly)maleimide compounds or (poly)maleamic acids are used for an interlayer insulating film of a multilayer wiring structure, but for the reasons above, a (poly)maleimide compound or (poly)maleamic acid that maintains sufficient heat resistance and has a low dielectric constant has been demanded (see, for example, Patent Literatures 1 to 3).

Also, in the production of semiconductor devices, fine processing is practiced by lithography using photoresist materials. In recent years, further miniaturization based on pattern rules has been demanded along with increase in the integration and speed of LSI (large scale integrated circuit). In addition, the light source for lithography used upon forming resist patterns has been shifted to ArF excimer laser (193 nm) having a shorter wavelength from KrF excimer laser (248 nm). The introduction of extreme ultraviolet (EUV, 13.5 nm) is also expected.

However, when the miniaturization of resist patterns proceeds, the problem of resolution or the problem of collapse of resist patterns after development arises. Therefore, resists have been desired to have a thinner film. Nevertheless, if resists merely have a thinner film, it is difficult to obtain the film thicknesses of resist patterns sufficient for substrate processing. Therefore, there has been a need for a process of preparing a resist underlayer film between a resist and a semiconductor substrate to be processed, and imparting functions as a mask for substrate processing to this resist underlayer film in addition to a resist pattern.

Various resist underlayer films for such a process are currently known. For example, in order to achieve a resist underlayer film for lithography having the selectivity of a dry etching rate smaller than that of resists, a resist underlayer film material comprising a polymer having a specific repeat unit has been suggested (see Patent Literature 4). Furthermore, as a material for realizing resist underlayer films for lithography having the selectivity of a dry etching rate smaller than that of semiconductor substrates, a resist underlayer film material comprising a polymer prepared by copolymerizing a repeat unit of an acenaphthylene and a repeat unit having a substituted or unsubstituted hydroxy group has been suggested (see Patent Literature 5).

Meanwhile, as a material having high etching resistance for this kind of resist underlayer film, amorphous carbon underlayer films formed by Chemical Vapour Deposition (CVD) using methane gas, ethane gas, acetylene gas, or the like as a raw material are well known. However, a resist underlayer film material that can form a resist underlayer film by a wet process such as spin coating or screen printing has been demanded from the viewpoint of a process.

The present inventors have proposed an underlayer film forming composition for lithography containing a compound having a specific structure and an organic solvent (see Patent Literature 6) as a material that is excellent in etching resistance, has high heat resistance, and is soluble in a solvent and applicable to a wet process.

### Citation List

### Patent Literature

Patent Literature 1: Japanese Patent Application Laid-Open No. 4-261411
Patent Literature 2: Japanese Patent Application Laid-Open No. 7-215933
Patent Literature 3: Japanese Patent Application Laid-Open No. 2010-18791
Patent Literature 4: Japanese Patent Application Laid-Open No. 2004-271838
Patent Literature 5: Japanese Patent Application Laid-Open No. 2005-250434
Patent Literature 6: International Publication No. WO 2013/024779

### Summary of Invention

### Technical Problem

However, from the viewpoint of developing a new material, the structure forming ability (film forming ability), heat resistance and solubility of the (poly)maleimide compounds or (poly)maleamic acids described in Patent Literatures 1 to 3 are not sufficient, and even further improvement of these properties has been demanded.

On the other hand, a film forming material for lithography with even higher heat resistance has been demanded, while satisfying the solubility in an organic solvent, etching resistance and resist pattern formability at a high level at the same time.

Therefore, an object of the present invention is to provide a novel (poly)imide compound or (poly)amic acid that has excellent structure forming ability (film forming ability), heat resistance and solubility.

### Solution to Problem

The present inventors have, as a result of devoted examinations, found out that a novel (poly)imide compound or (poly)amic acid that has a specific structure can solve the above problems, and have completed the present invention.

More specifically, the present invention is as follows.
[1] A compound represented by the following formula (0). 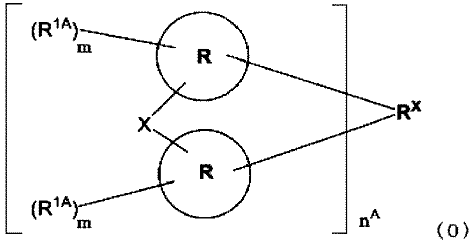 (In the above formula (0),
   R^{X} represents a 2n^{A}-valent group having 1 to 70 carbon atoms or a single bond;
   each R^{1A} independently represents any of an alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, an alkoxy group having 1 to 30 carbon atoms and optionally having a substituent, a maleamic acid group having 4 to 30 carbon atoms and optionally having a substituent, a maleimide group having 4 to 30 carbon atoms and optionally having a substituent, a halogen atom, a nitro group, an amino group having 0 to 30 carbon atoms and optionally having a substituent, a carboxyl group, a thiol group and a hydroxy group, wherein, when R^{1A} is any of the alkyl group, the aryl group, the crosslinkable group and the alkoxy group, at least one bond selected from the group consisting of an ether bond, a ketone bond and an ester bond is optionally contained, and at least one R^{1A} is any of a maleamic acid group having 4 to 30 carbon atoms and optionally having a substituent and a maleimide group having 4 to 30 carbon atoms and optionally having a substituent;
   X represents an oxygen atom or a sulfur atom, or is optionally not present;
   each R independently represents any of a benzene ring, a naphthalene ring and an anthracene ring;
   each m is independently an integer of 0 to 9, wherein at least one m is an integer of 1 to 9; and
   n^{A} is an integer of 1 to 4.)
[2] The compound according to the above [1], wherein the compound represented by the above formula (0) is a compound represented by the following formula (1). 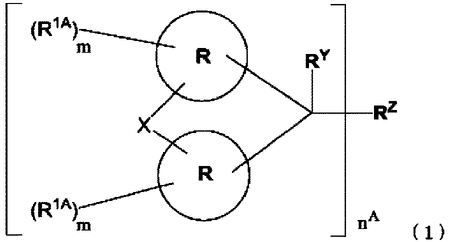 (In the above formula (1),
   R^{Y} represents any of a hydrogen atom, a linear or branched, or cyclic alkyl group having 1 to 30 carbon atoms and optionally having a substituent, and an aryl group having 6 to 30 carbon atoms and optionally having a substituent;
   R^{Z} represents an n^{A}-valent group having 1 to 60 carbon atoms or a single bond, and the total number of carbon atoms in R^{Y} and R^{Z} is 69 or less; and
   X, R, n^{A}, R^{1A} and m are as defined above.)
[3] The compound according to the above [2], wherein the compound represented by the above formula (1) is a compound represented by the following formula (1a-1). 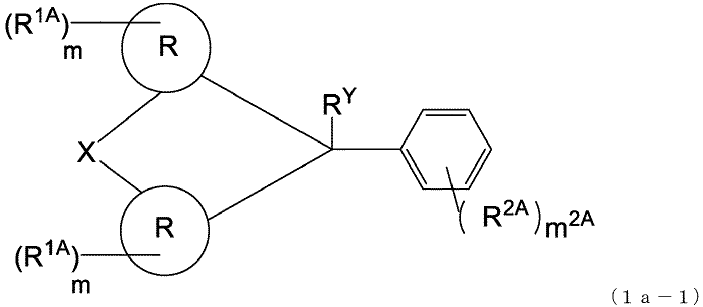 (In the above formula (1a-1), X, R, R^{1A}, m and R^{Y} are as defined above; R^{2A} represents any of a hydrogen atom, a linear or branched, or cyclic alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, and a halogen atom; and m^{2A} represents an integer of 1 to 5.)
[4] The compound according to the above [2], wherein the compound represented by the above formula (1) is a compound represented by the following formula (1b-1). 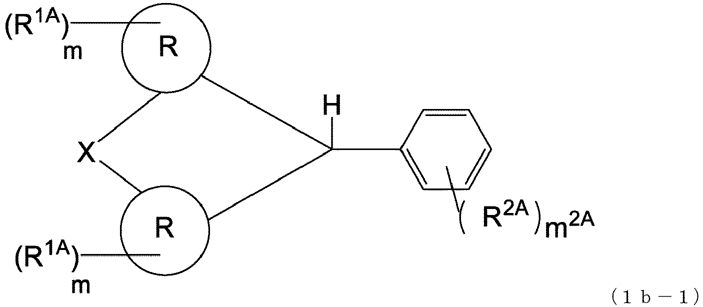 (In the above formula (1b-1), X, R, R^{1A} and m are as defined above; R^{2A} represents any of a hydrogen atom, a linear or branched, or cyclic alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, and a halogen atom; and m^{2A} represents an integer of 1 to 5.)
[5] The compound according to the above [2], wherein the compound represented by the above formula (1) is a compound represented by the following formula (1a-2). 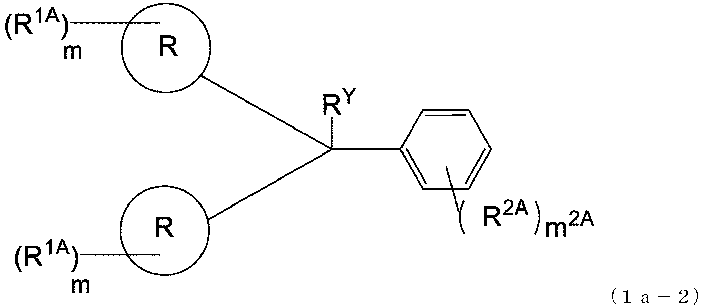 (In the above formula (1a-2), R, R^{1A}, m and R^{Y} are as defined above; R^{2A} represents any of a hydrogen atom, a linear or branched, or cyclic alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, and a halogen atom; and m^{2A} represents an integer of 1 to 5.)
[6] The compound according to the above [2], wherein the compound represented by the above formula (1) is a compound represented by the following formula (1b-2). 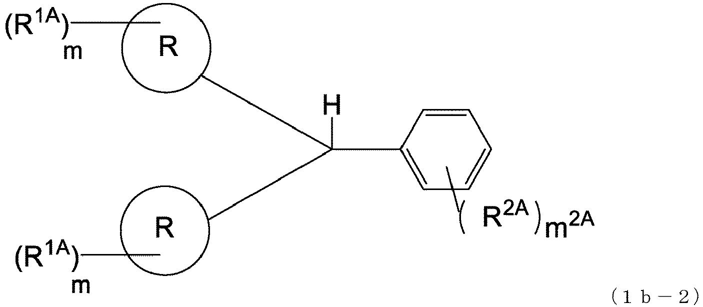 (In the above formula (1b-2), R, R^{1A} and m are as defined above; R^{2A} represents any of a hydrogen atom, a linear or branched, or cyclic alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, and a halogen atom; and m^{2A} represents an integer of 1 to 5.)
[7] The compound according to the above [5], wherein the compound represented by the above formula (1a-2) is a compound represented by the following formula (1a-3). 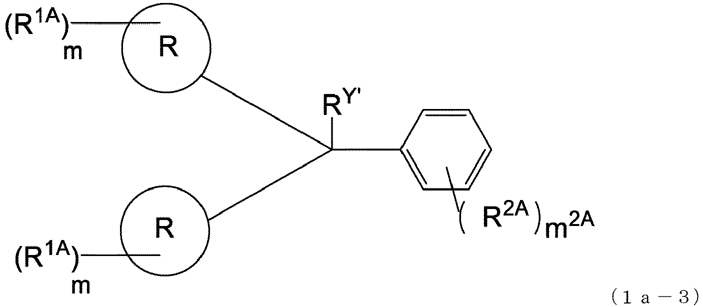 (In the above formula (1a-3), R, R^{1A}, R^{2A}, m and m^{2A} are as defined above; and R^{Y'} represents R^{Y} except for a hydrogen atom.)
[8] The compound according to the above [6], wherein the compound represented by the above formula (1b-2) is a compound represented by the following formula (1b-3). 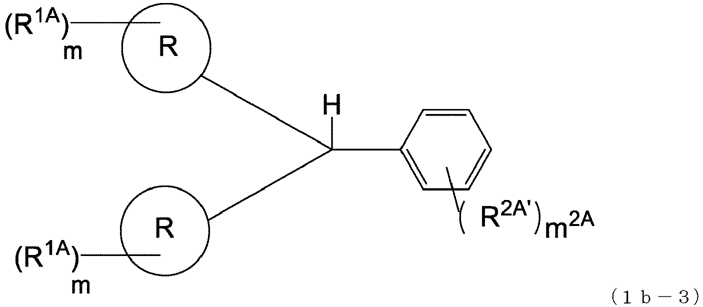 (In the above formula (1b-3), R, R^{1A}, m and m^{2A} are as defined above; and R^{2A'} represents a linear or branched, or cyclic alkyl group having 5 or more carbon atoms and optionally having a substituent.)
[9] The compound according to the above [2], wherein the compound represented by the above formula (1) is a compound represented by the following formula (2) or formula (2'). 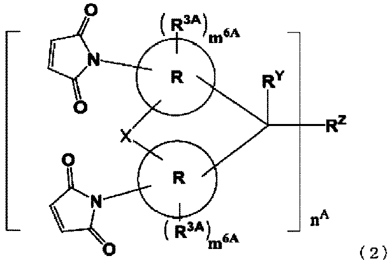 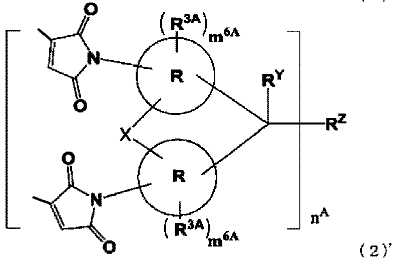 (In the above formula (2) and (2)',
   each R^{3A} independently represents any of a linear or branched, or cyclic alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, an alkoxy group having 1 to 30 carbon atoms and optionally having a substituent, and a halogen atom;
   each m^{6A} is independently an integer of 0 to 5; and
   R^{Y}, R^{Z}, X, R and n^{A} are as defined above.)
[10] The compound according to the above [2], wherein the compound represented by the above formula (1) is a compound represented by the following formula (3) or formula (3'). 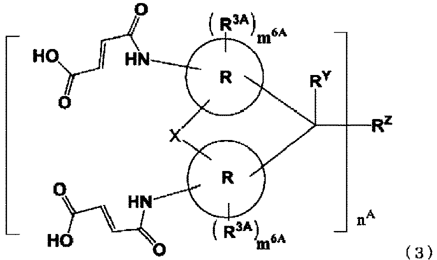 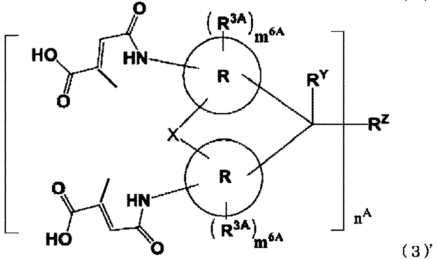 (In the above formula (3) and (3)',
   each R^{3A} is independently any of a linear or branched, or cyclic alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, an alkoxy group having 1 to 30 carbon atoms and optionally having a substituent, and a halogen atom;
   each m^{6A} is independently an integer of 0 to 5; and
   R^{Y}, R^{Z}, X, R and n^{A} are as defined above.)
[11] The compound according to any of the above [1] to [10], wherein X is an oxygen atom in the above formula (0), (1), (2), (2)', (3) or (3)'.
[12] The compound according to the above [2], wherein the compound represented by the above formula (1) is any of compounds represented by the following formulas (MIBiA-1) to (MIBiA-34) and (MABiA-1) to (MABiA-34). 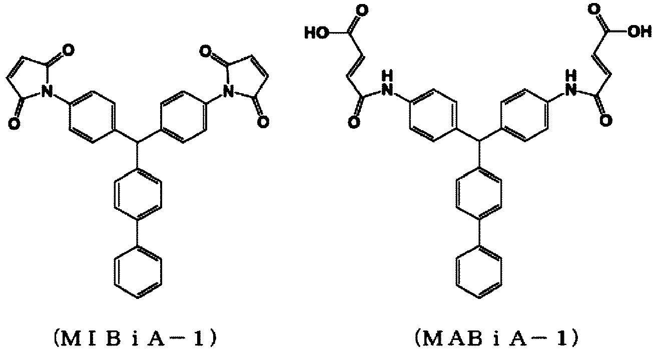 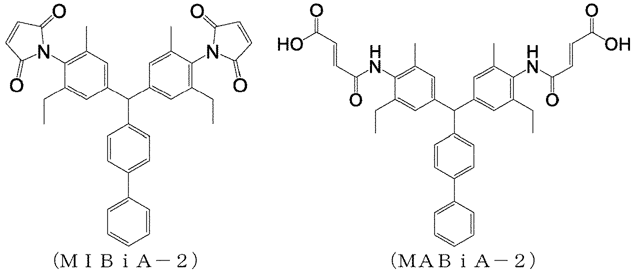 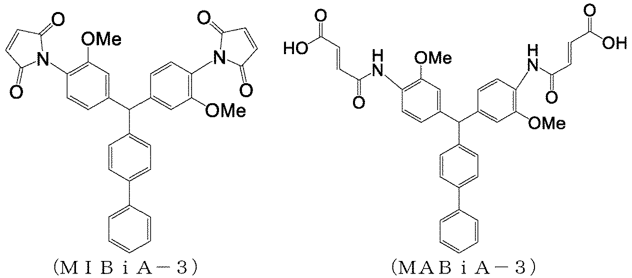 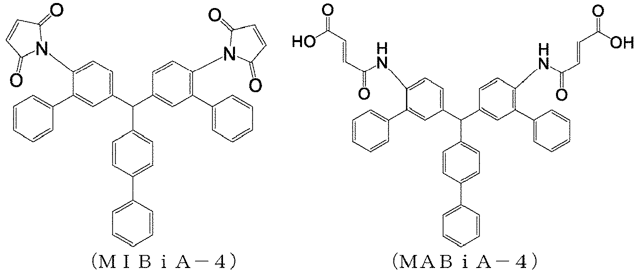 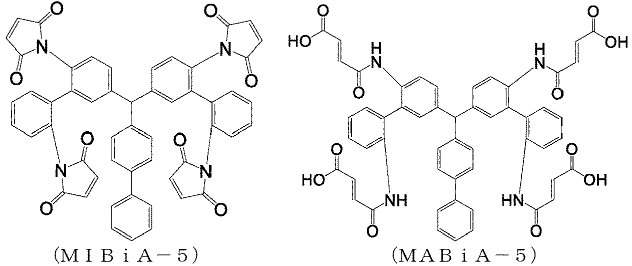 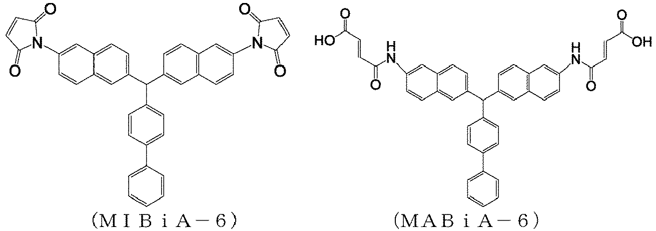 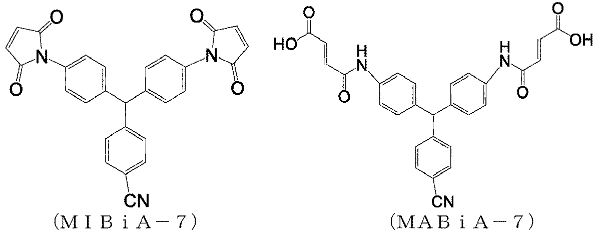 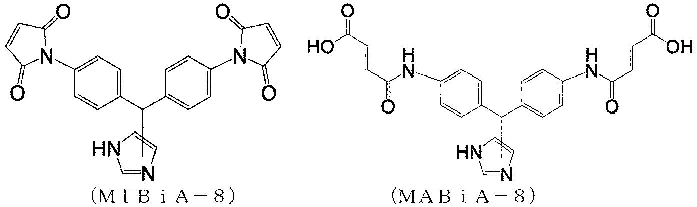 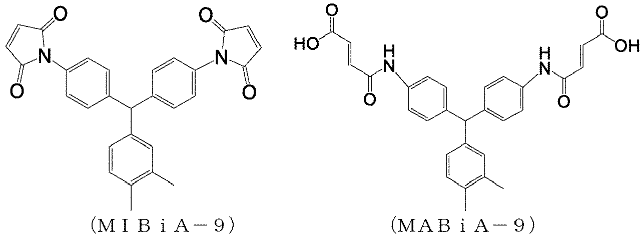 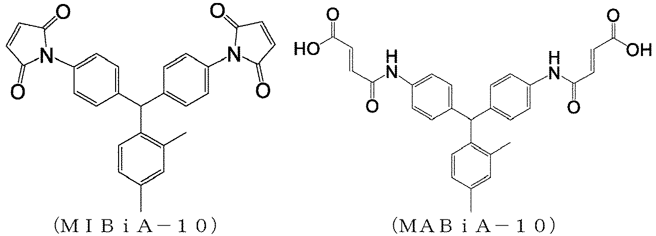 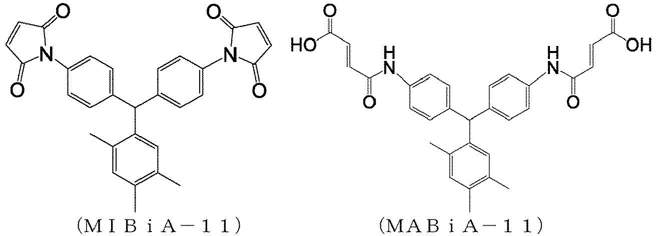 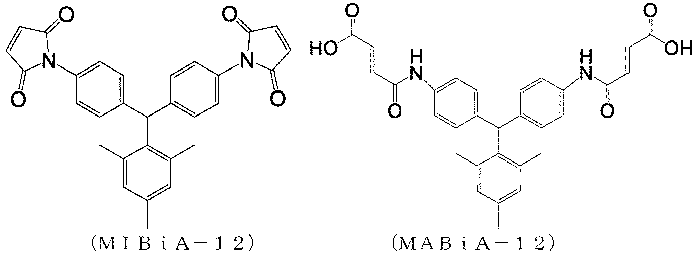 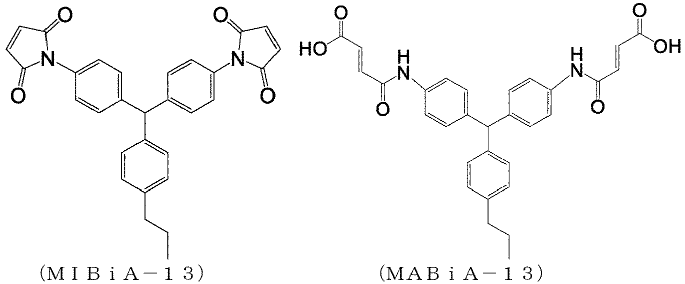 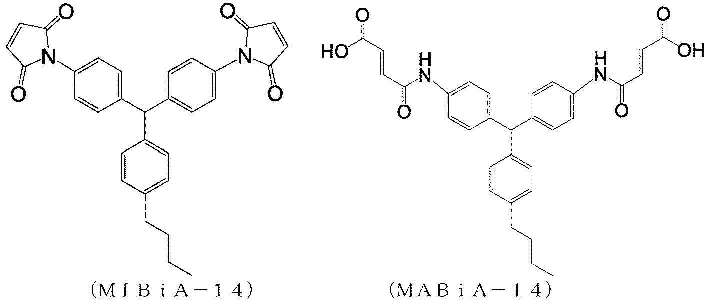 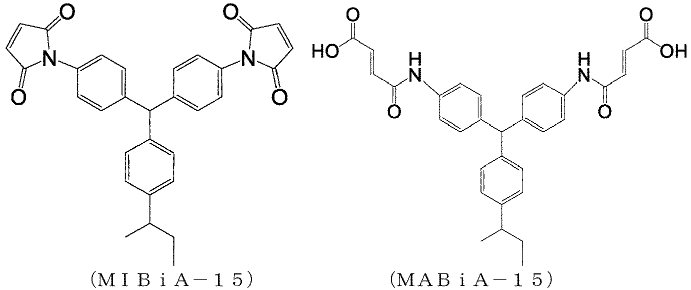 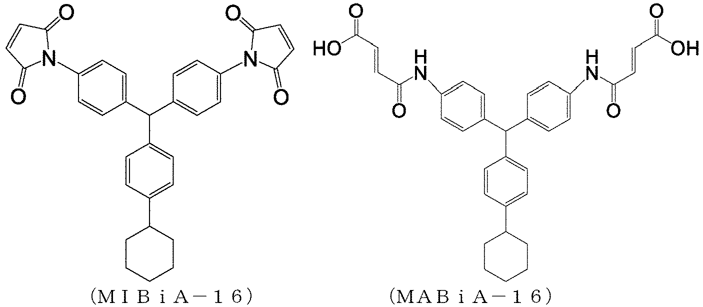 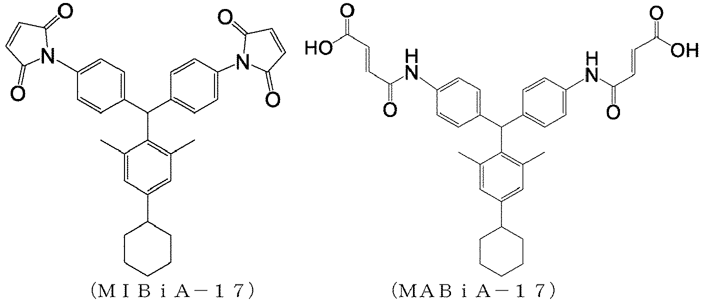 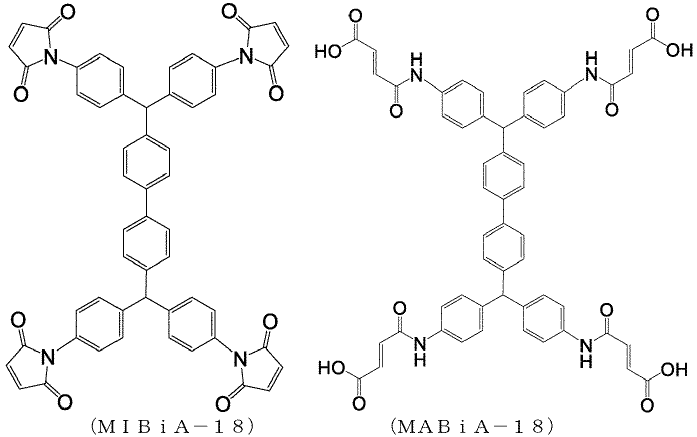 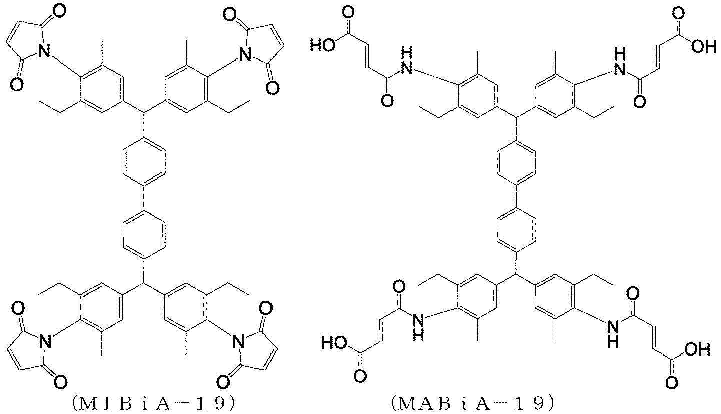 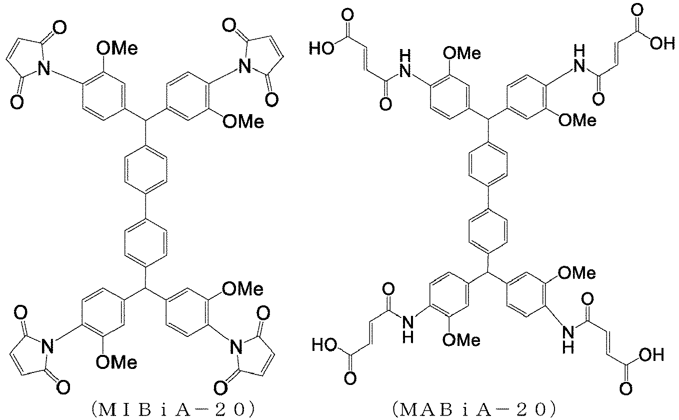 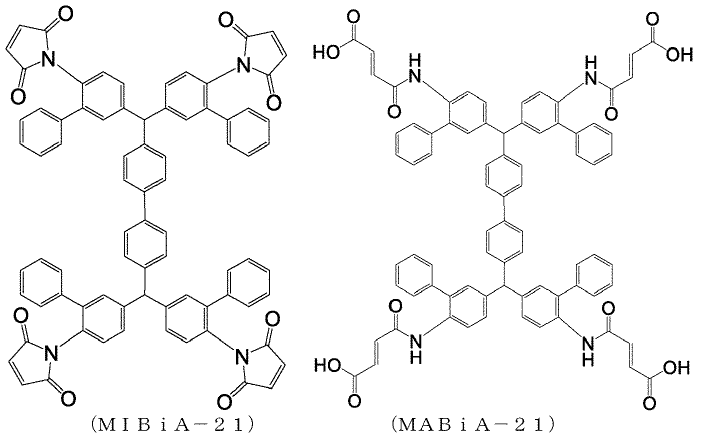 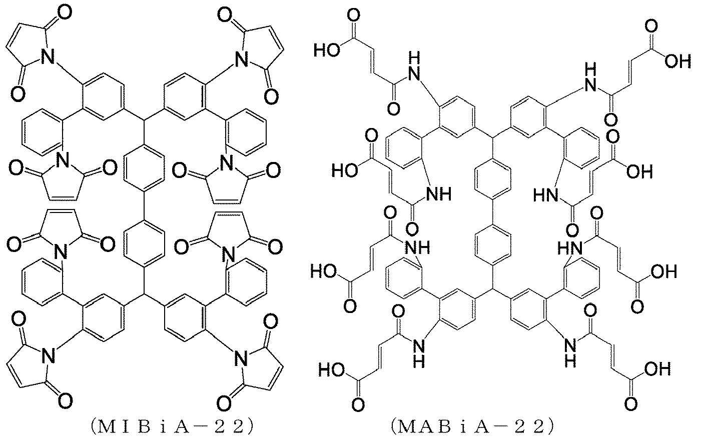 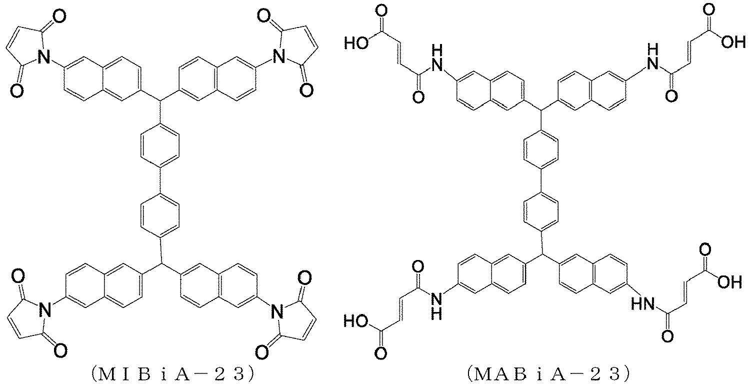 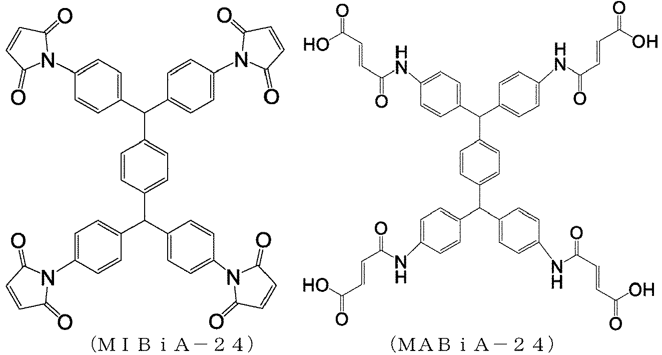 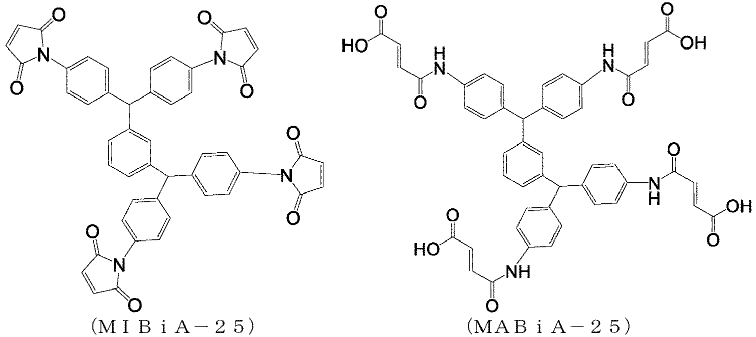 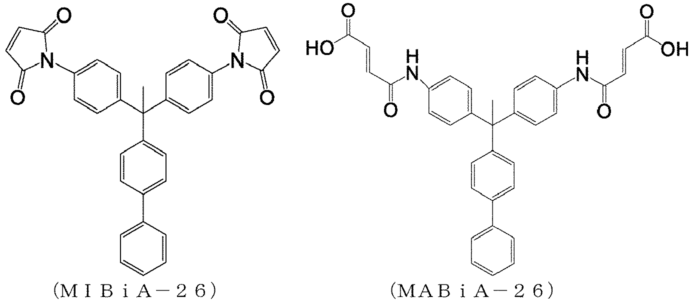 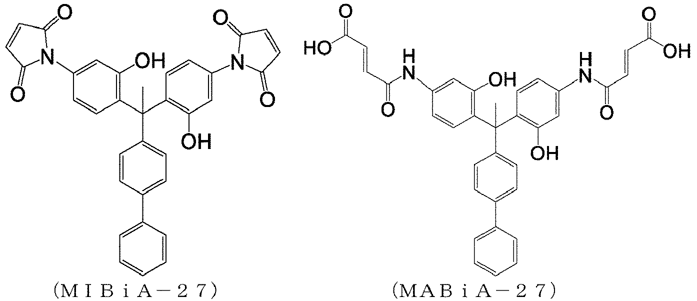 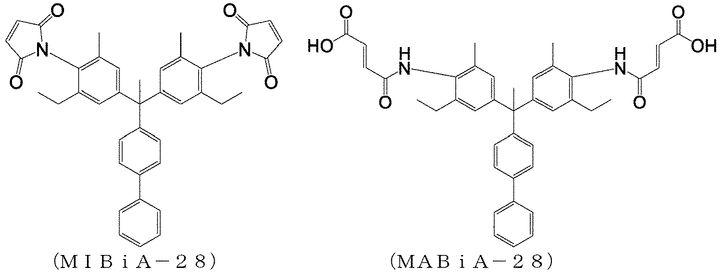 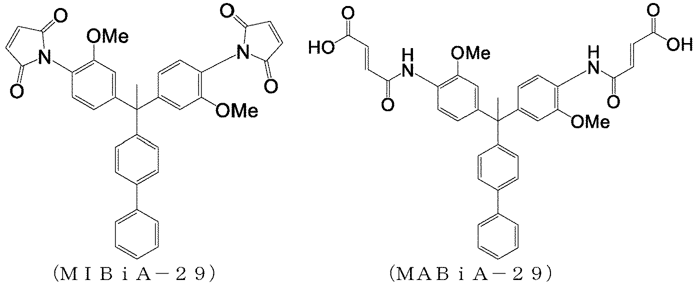 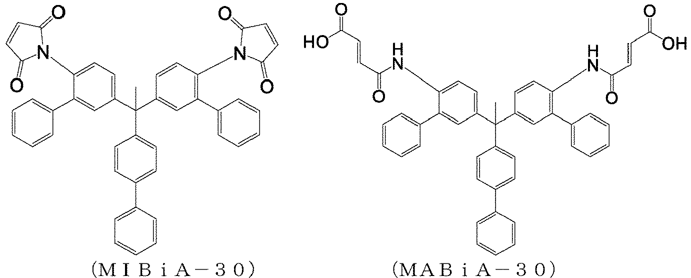 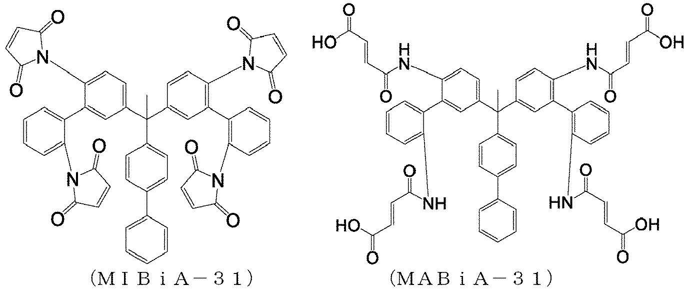 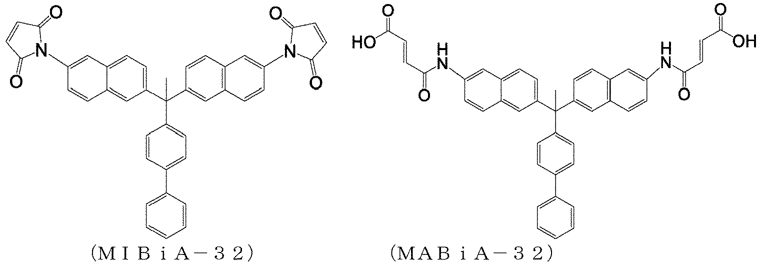 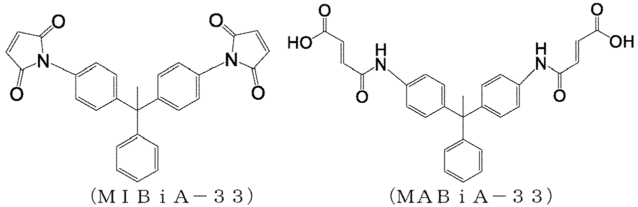 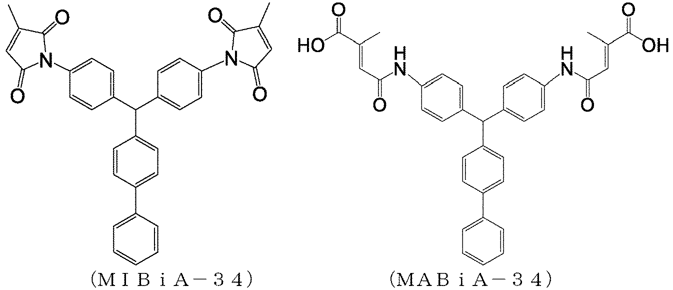
[13] A resin comprising a monomer unit derived from the compound according to any of the above [1] to [12].
[14] The resin according to the above [13], wherein the monomer unit is a unit represented by the following formula (4). 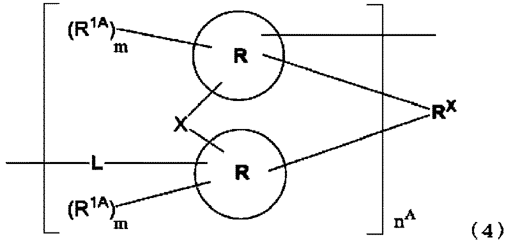 (In the above formula (4),
   L represents a linear or branched linking group having 1 to 30 carbon atoms, or a single bond; and
   R^{X}, R^{1A}, X, R, m and n^{A} are as defined above.)
[15] A composition comprising the compound or the resin according to any of the above [1] to [14].
[16] A cured product obtained by curing the composition according to the above [15].
[17] A film forming material for lithography comprising the compound or the resin according to any of the above [1] to [14] .
[18] The film forming material for lithography according to the above [17], further comprising a crosslinking agent.
[19] The film forming material for lithography according to the above [17] or [18], further comprising a crosslinking promoting agent.
[20] The film forming material for lithography according to any of the above [17] to [19], further comprising a radical polymerization initiator.
[21] A composition for film formation for lithography comprising the film forming material for lithography according to any of the above [17] to [21] and a solvent.
[22] The composition for film formation for lithography according to the above [21], further comprising an acid generating agent.
[23] The composition for film formation for lithography according to the above [21] or [22], wherein the film for lithography is an underlayer film for lithography.
[24] An underlayer film for lithography formed by using the composition for film formation for lithography according to the above [23].
[25] A method for forming a resist pattern, comprising the steps of:
   forming an underlayer film on a substrate by using the composition for film formation for lithography according to the above [23];
   forming at least one photoresist layer on the underlayer film; and
   irradiating a predetermined region of the photoresist layer with radiation for development.
[26] A method for forming a circuit pattern, comprising the steps of:
   forming an underlayer film on a substrate by using the composition for film formation for lithography according to the above [23];
   forming an intermediate layer film on the underlayer film by using a resist intermediate layer film material containing a silicon atom;
   forming at least one photoresist layer on the intermediate layer film;
   irradiating a predetermined region of the photoresist layer with radiation for development, thereby forming a resist pattern;
   etching the intermediate layer film with the resist pattern as a mask;
   etching the underlayer film with the obtained intermediate layer film pattern as an etching mask; and
   etching the substrate with the obtained underlayer film pattern as an etching mask, thereby forming a pattern on the substrate.
[27] A method for purifying a film forming material for lithography, comprising the steps of:
   obtaining an organic phase by dissolving the film forming material for lithography according to any of the above [17] to [20] in a solvent; and
   extracting impurities in the film forming material for lithography by bringing the organic phase into contact with an acidic aqueous solution (a first extraction step),
   wherein
   the solvent used in the step of obtaining the organic phase contains a solvent that does not inadvertently mix with water.

### Advantageous Effects of Invention

According to the present invention, a novel (poly)imide compound or (poly)amic acid that has excellent structure forming ability (film forming ability), heat resistance and solubility can be provided. Furthermore, a useful film forming composition for lithography and film forming material can be provided.

### Description of Embodiments

An embodiment of the present invention (hereinafter, simply referred to as the "present embodiment") will now be described in detail. Note that, in the present embodiment, a "(poly)imide compound" includes a "(poly)maleimide compound" and a "(poly)citraconimide compound", and a "(poly)amic acid" includes a "(poly)maleamic acid" and a "(poly)citraconamic acid". Also, a "(poly)maleimide compound" refers to a monomaleimide compound or a polymaleimide compound. The same also applies to other compounds.

### [Compound represented by formula (0)]

The (poly)imide compound or (poly)amic acid of the present embodiment (hereinafter, also simply referred to as the "compound of the present embodiment") is represented by the following formula (0). The compound of the present embodiment has, for example, excellent structure forming ability (film forming ability), heat resistance and solubility, and can be used usefully as a high heat resistant thermosetting resin and a cured product thereof. In particular, the compound of the present embodiment has excellent structure forming ability (for example, film forming ability), heat resistance and solubility due to high aromatic density and low crystallinity.

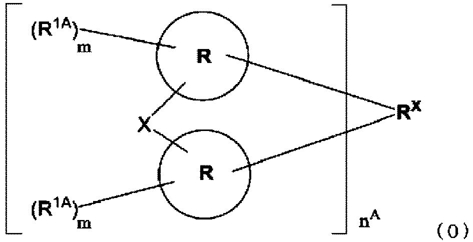

In the above formula (0), R^{X} represents a 2n^{A}-valent group having 1 to 70 carbon atoms or a single bond;
each R^{1A} independently represents any of an alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, an alkoxy group having 1 to 30 carbon atoms and optionally having a substituent, a maleamic acid group having 4 to 30 carbon atoms and optionally having a substituent, a maleimide group having 4 to 30 carbon atoms and optionally having a substituent, a halogen atom, a nitro group, an amino group having 0 to 30 carbon atoms and optionally having a substituent, a carboxyl group, a thiol group and a hydroxy group, wherein, when R^{1A} is any of the alkyl group, the aryl group, the crosslinkable group and the alkoxy group, at least one bond selected from the group consisting of an ether bond, a ketone bond and an ester bond is optionally contained, and at least one R^{1A} is a maleamic acid group having 4 to 30 carbon atoms and optionally having a substituent or a maleimide group having 4 to 30 carbon atoms and optionally having a substituent;
X represents an oxygen atom or a sulfur atom, or is optionally not present;
each R independently represents any of a benzene ring, a naphthalene ring and an anthracene ring;
each m is independently an integer of 0 to 9, wherein at least one m is an integer of 1 to 9; and
n^{A} is an integer of 1 to 4.

In the present specification, an "alkyl group" may be a linear or branched alkyl group, or it may be a cyclic alkyl group. In addition, an "alkoxy group" may also be a linear or branched alkoxy group, or it may be a cyclic alkoxy group.

### (1. R^{X})

In the above formula (0), R^{X} is a 2n^{A}-valent group or a single bond, and is preferably a 2n^{A}-valent group. The number of carbon atoms in the 2n^{A}-valent group is 1 to 70, preferably 3 to 50, and more preferably 6 to 30.

### (1-1. 2n^{A}-valent group)

Examples of the 2n^{A}-valent group include, but not particularly limited to, a hydrocarbon group. Examples of the hydrocarbon group include a linear or branched hydrocarbon group, an alicyclic hydrocarbon group, and a combination of a linear or branched hydrocarbon group and an alicyclic hydrocarbon group. The "alicyclic hydrocarbon group" as used herein may be a hydrocarbon group having a bridged structure in an aliphatic ring, so-called polycyclic bridged alicyclic hydrocarbon group. In addition, the hydrocarbon group may contain any of a double bond, heteroatom and an aromatic group having 6 to 60 carbon atoms.

### (1-1-1. Group represented by formula (0a))

Examples of the 2n^{A}-valent group include a group represented by the following formula (0a).

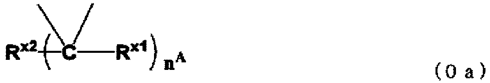

In the above formula (0a), each R^{x1} is independently any of a hydrogen atom, a linear or branched, or cyclic alkyl group having 1 to 30 carbon atoms and optionally having a substituent, and an aryl group (hydrocarbon group) having 6 to 30 carbon atoms and optionally having a substituent, wherein, when R^{X1} is the alkyl group or the aryl group, at least one bond selected from the group consisting of an ether bond, a ketone bond and an ester bond is optionally contained; R^{X2} is any of a hydrogen atom and an n^{A}-valent group having 1 to 60 carbon atoms, and the total number of carbon atoms in R^{X1} and R^{X2} is 69 or less; and n^{A} represents an integer of 1 to 4.

### (1-1-1a. R^{X1})

For R^{X1}, examples of the linear or branched alkyl group can include a linear or branched alkyl group having 1 to 30 carbon atoms, such as a methyl group, an ethyl group, a n-propyl group, an isopropyl group, a n-butyl group, an isobutyl group, a tert-butyl group, a n-pentyl group, a neopentyl group, a tert-pentyl group, a n-hexyl group, a n-heptyl group, a 2,2,4-trimethylpentyl group, a n-octyl group, an isooctyl group, a n-nonyl group, a n-decyl group, a n-undecyl group, a n-dodecyl group, a n-tridecyl group, a n-tetradecyl group, a n-pentadecyl group, a n-hexadecyl group, a n-heptadecyl group, a n-octadecyl group, a n-nonadecyl group, a n-heneicosyl group, a n-tricosyl group, a n-pentacosyl group, a n-heptacosyl group and a n-nonacosyl group. From the viewpoint of achieving the effects of the present invention more effectively and reliably, it is preferably a linear or branched alkyl group having 1 to 20 carbon atoms, and is more preferably a linear or branched alkyl group having 1 to 10 carbon atoms. Examples of the cyclic alkyl group include a monocyclic group (monocyclic cycloalkyl group) and a polycyclic group (polycyclic cycloalkyl group). Examples of the monocyclic group include a monocyclic group having 3 to 30 carbon atoms, such as a cyclopropyl group, a cyclobutyl group, a cyclopentyl group, a cyclohexyl group, a cyclopentyl group, a cycloheptyl group, a cyclooctyl group, a cyclodecyl group and a cycloicosyl group. Examples of the polycyclic group include a multicyclic group having 7 to 30 carbon atoms, such as a dicyclopentyl group, a dicyclohexyl group, a norbornyl group, an adamantyl group, a tricyclodecyl group and a tetracyclododecyl group.

The alkyl group may have a substituent. Examples of the substituent include a halogen atom (for example, a fluorine atom, a chlorine atom and a bromine atom), a nitro group, an amino group optionally having a substituent, a carboxyl group, a thiol group and a hydroxy group. The number of the substituent is not particularly limited, and it may be one or more.

Examples of the aryl group include, but not particularly limited to, an aryl group having 6 to 30 carbon atoms, such as a phenyl group, a naphthyl group (for example, a 1-naphthyl group and a 2-naphthyl group), an anthryl group (for example, a 1-anthryl group) and a phenanthryl group (for example, 1-phenanthryl group). From the viewpoint of achieving the effects of the present invention more effectively and reliably, it is preferably an aryl group having 6 to 10 carbon atoms, such as a phenyl group and a naphthyl group, and is more preferably a phenyl group.

The aryl group may have a substituent. Examples of the substituent include the substituents exemplified as the substituent of the alkyl group in (1-1-1a. R^{X1}) and the alkyl groups exemplified as the alkyl group in (1-1-1a. R^{X1}). The number of the substituent is not particularly limited, and it may be one or more.

### (1-1-1b. R^{X2})

Examples of the n^{A}-valent group having 1 to 60 carbon atoms include a linear or branched alkyl group having 1 to 60 carbon atoms and optionally having a substituent (n^{A} = 1), a linear or branched alkylene group having 1 to 60 carbon atoms and optionally having a substituent (n^{A} = 2), a linear or branched alkyltriyl group having 1 to 60 carbon atoms and optionally having a substituent (n^{A} = 3), a linear or branched alkyltetrayl group having 1 to 60 carbon atoms and optionally having a substituent (n^{A} = 4), an n^{A}-valent aromatic hydrocarbon ring having 6 to 60 carbon atoms and optionally having a substituent, and an n^{A}-valent alicyclic hydrocarbon ring having 3 to 60 carbon atoms and optionally having a substituent.

Examples of the linear or branched alkyl group having 1 to 60 carbon atoms include the alkyl groups exemplified as the alkyl group in (1-1-1a. R^{X1}), and examples of the alkyl group that is preferable from the viewpoint of achieving the effects of the present invention more effectively and reliably also include the alkyl groups exemplified as the preferable alkyl group in (1-1-1a. R^{X1}). The alkyl group may have a substituent, and examples of the substituent include the substituents exemplified as the substituent of the alkyl group in (1-1-1a. R^{X1}).

Examples of the linear or branched alkylene group having 1 to 60 carbon atoms include a group obtained by removing one hydrogen atom from the alkyl groups exemplified as the linear or branched alkyl group having 1 to 60 carbon atoms. The alkylene group may have a substituent, and examples of the substituent include the substituents exemplified as the substituent of the alkyl group in (1-1-1a. R^{X1}).

Examples of the linear or branched alkyltriyl group having 1 to 60 carbon atoms include a group obtained by removing two hydrogen atoms from the alkyl groups exemplified as the linear or branched alkyl group having 1 to 60 carbon atoms. The alkyltriyl group may have a substituent, and examples of the substituent include the substituents exemplified as the substituent of the alkyl group in (1-1-1a. R^{X1}).

Examples of the linear or branched alkyltetrayl group having 1 to 60 carbon atoms include a group obtained by removing three hydrogen atoms from the alkyl groups exemplified as the linear or branched alkyl group having 1 to 60 carbon atoms. The alkyltetrayl group may have a substituent, and examples of the substituent include the substituents exemplified as the substituent of the alkyl group in (1-1-1a. R^{X1}).

Examples of the aromatic hydrocarbon ring include a monocyclic aromatic hydrocarbon ring, a condensed cyclic aromatic hydrocarbon ring, and a ring in which multiple aromatic rings are bonded to each other directly or via a linking group (ring aggregated ring).

The above aromatic hydrocarbon ring may contain a heteroatom (for example, an oxygen atom, a nitrogen atom and a sulfur atom), and may also contain at least one bond selected from the group consisting of an ether bond, a ketone bond and an ester bond.

Examples of the monocyclic aromatic hydrocarbon ring include a benzene ring and a 5-membered or 6-membered aromatic heterocycle containing a heteroatom. Examples of the 5-membered or 6-membered aromatic heterocycle containing a heteroatom include a thiophene ring, a pyridine ring, a furan ring, a thiazole ring, an oxazole ring, a pyrazole ring, a pyrazine ring, a pyrimidine ring, a pyrrole ring, an imidazole ring, a pyridazine ring, an isothiazole ring and an isoxazole ring. Among these monocyclic aromatic hydrocarbon rings, a benzene ring is preferable from the viewpoint of achieving the effects of the present invention more effectively and reliably. These monocyclic aromatic hydrocarbon rings may have a substituent. Examples of the substituent include the substituents exemplified as the substituent of the alkyl group in (1-1-1a. R^{X1}) and the alkyl groups exemplified as the alkyl group in (1-1-1a. R^{X1}). The number of the substituent is not particularly limited, and it may be one or more. The substitution position at which the substituent is bonded to the monocyclic aromatic hydrocarbon ring is not particularly limited, either.

As the monocyclic aromatic hydrocarbon ring, a benzene ring is preferable from the viewpoint of achieving the effects of the present invention more effectively and reliably. When a benzene ring has a substituent, it is preferable that the benzene ring have the substituent at its position 4.

More specifically, examples of the monocyclic aromatic hydrocarbon ring having a substituent include, when n^{A} is 1, (i) a hydroxyphenyl group, (ii) a monoalkylphenyl group, (iii) a dialkylphenyl group, (iv) a trialkylphenyl group and (v) a cyclohexylphenyl group.

Examples of (i) the hydroxyphenyl group include: (i-a) a monohydroxyphenyl group (for example, a 2-hydroxyphenyl group, a 3-hydroxyphenyl group and a 4-hydroxyphenyl group);
(i-b) a dihydroxyphenyl group (for example, a 2,3-dihydroxyphenyl group, a 2,4-dihydroxyphenyl group, a 2,5-dihydroxyphenyl group, a 2,6-dihydroxyphenyl group, a 3,4-dihydroxyphenyl group and a 3,5-dihydroxyphenyl group); and
(i-c) a trihydroxyphenyl group (for example, a 2,3,4-trihydroxyphenyl group, a 2,3,5-trihydroxyphenyl group, a 2,3,6-trihydroxyphenyl group, a 2,4,5-trihydroxyphenyl group and a 2,4,6-trihydroxyphenyl group).

Examples of (ii) the monoalkylphenyl group include: a 2-methylphenyl group, a 3-methylphenyl group, a 4-methylphenyl group, a 2-ethylphenyl group, a 3-ethylphenyl group, a 4-ethylphenyl group, a 2-propylphenyl group, a 3-propylphenyl group, a 4-propylphenyl group, a 2-isopropylphenyl group, a 3-isopropylphenyl group, a 4-isopropylphenyl group, a 2-butylphenyl group, a 3-butylphenyl group and a 4-butylphenyl group.

Examples of (iii) the dialkylphenyl group include: (iii-a) a 2,3-dialkylphenyl group (for example, a 2,3-dimethylphenyl group, a 2,3-diethylphenyl group, a 2,3-dipropylphenyl group, a 2,3-diisopropylphenyl group, a 2-ethyl-3-methylphenyl group and a 3-ethyl-2-methylphenyl group);
(iii-b) a 2,4-dialkylphenyl group (for example, a 2,4-dimethylphenyl group, a 2,4-diethylphenyl group, a 2,4-dipropylphenyl group, a 2,4-diisopropylphenyl group, a 2-ethyl-4-methylphenyl group and a 4-ethyl-2-methylphenyl group); (iii-c) a 2,5-dialkylphenyl group (for example, a 2,5-dimethylphenyl group, a 2,5-diethylphenyl group, a 2,5-dipropylphenyl group, a 2,5-diisopropylphenyl group, a 2-ethyl-5-methylphenyl group and a 5-ethyl-2-methylphenyl group);
(iii-d) a 2,6-dialkylphenyl group (for example, a 2,6-dimethylphenyl group, a 2,6-diethylphenyl group, a 2,6-dipropylphenyl group, a 2,6-diisopropylphenyl group, a 2-ethyl-6-methylphenyl group and a 6-ethyl-2-methylphenyl group);
(iii-e) a 3,4-dialkylphenyl group (for example, a 3,4-dimethylphenyl group, a 3,4-diethylphenyl group, a 3,4-dipropylphenyl group, a 3,4-diisopropylphenyl group, a 3-ethyl-4-methylphenyl group and a 4-ethyl-3-methylphenyl group); and
(iii-f) a 3,5-dialkylphenyl group (for example, a 3,5-dimethylphenyl group, a 3,5-diethylphenyl group, a 3,5-dipropylphenyl group, a 3,5-diisopropylphenyl group, a 3-ethyl-5-methylphenyl group and a 5-ethyl-3-methylphenyl group) .

Examples of (iv) the trialkylphenyl group include: (iv-a) a trimethylphenyl group (for example, a 2,3,4-trimethylphenyl group, a 2,3,5-trimethylphenyl group, a 2,3,6-trimethylphenyl group, a 2,4,5-trimethylphenyl group, a 2,4,6-trimethylphenyl group and a 3,4,5-trimethylphenyl group);
(iv-b) an ethyldimethylphenyl group (for example, a group obtained by substituting one methyl group of the trimethylphenyl group with one ethyl group); (iv-c) a diethylmethylphenyl group (for example, a group obtained by substituting each of two methyl groups of the trimethylphenyl group with an ethyl group); and (iv-d) a triethylphenyl group (for example, a group obtained by substituting each of three methyl groups of the trimethylphenyl group with an ethyl group).

Examples of (v) the cycloalkylphenyl group include a cyclohexylphenyl group such as a 2-cyclohexylphenyl group, a 3-cyclohexylphenyl group and a 4-cyclohexylphenyl group.

Examples of the monocyclic aromatic hydrocarbon ring having a substituent include, when n^{A} is 2, (i) a hydroxyphenylene group obtained by removing one hydrogen atom from a phenyl group of the hydroxyphenyl groups exemplified as the hydroxyphenyl group, (ii) a monoalkylphenylene group obtained by removing one hydrogen atom from a phenyl group of the monoalkylphenyl groups exemplified as the monoalkylphenyl group, (iii) a dialkylphenylene group obtained by removing one hydrogen atom from a phenyl group of the dialkylphenyl groups exemplified as the dialkylphenyl group, (iv) a ditrialkylphenylene group obtained by removing one hydrogen atom from a phenyl group of the trialkylphenyl groups exemplified as the trialkylphenyl group and (v) a cyclohexylphenylene group obtained by removing one hydrogen atom from a phenyl group of the cyclohexylphenyl groups exemplified as the cyclohexylphenyl group.

Examples of the monocyclic aromatic hydrocarbon ring having a substituent include, when n^{A} is 3, (i) a hydroxyphenyltriyl group obtained by removing two hydrogen atoms from a phenyl group of the hydroxyphenyl groups exemplified as the hydroxyphenyl group, (ii) a monoalkylphenyltriyl group obtained by removing two hydrogen atoms from a phenyl group of the monoalkylphenyl groups exemplified as the monoalkylphenyl group, (iii) a dialkylphenyltriyl group obtained by removing two hydrogen atoms from a phenyl group of the dialkylphenyl groups exemplified as the dialkylphenyl group, (iv) a ditrialkylphenyltriyl group obtained by removing two hydrogen atoms from a phenyl group of the trialkylphenyl groups exemplified as the trialkylphenyl group and (v) a cyclohexylphenyltriyl group obtained by removing two hydrogen atoms from a phenyl group of the cyclohexylphenyl groups exemplified as the cyclohexylphenyl group.

Examples of the monocyclic aromatic hydrocarbon ring having a substituent include, when n^{A} is 4, (i) a hydroxyphenyltetrayl group obtained by removing three hydrogen atoms from a phenyl group of the hydroxyphenyl groups exemplified as the hydroxyphenyl group, (ii) a monoalkylphenyltetrayl group obtained by removing three hydrogen atoms from a phenyl group of the monoalkylphenyl groups exemplified as the monoalkylphenyl group, (iii) a dialkylphenyltetrayl group obtained by removing three hydrogen atoms from a phenyl group of the dialkylphenyl groups exemplified as the dialkylphenyl group, (iv) a ditrialkylphenyltetrayl group obtained by removing three hydrogen atoms from a phenyl group of the trialkylphenyl groups exemplified as the trialkylphenyl group and (v) a cyclohexylphenyltetrayl group obtained by removing three hydrogen atoms from a phenyl group of the cyclohexylphenyl groups exemplified as the cyclohexylphenyl group.

Examples of the condensed cyclic aromatic hydrocarbon ring include a condensed bicyclic aromatic hydrocarbon ring (for example, a naphthalene ring and an indene ring), a condensed tricyclic aromatic hydrocarbon ring (for example, an anthracene ring and a phenanthrene ring), a condensed tetracyclic aromatic hydrocarbon ring (for example, a tetracene ring and a pyrene ring), a ring having an acenaphthene skeleton (for example, an acenaphthene ring and a 1-acenaphthene ring), and a ring having a fluorene skeleton (for example, a fluorene ring, a benzofluorene ring such as a 2,3-benzofluorene ring, and a dibenzofluorene ring such as 2,3:6,7-dibenzofluorene ring).

These condensed cyclic aromatic hydrocarbon rings may have a substituent. Examples of the substituent include the substituents exemplified as the substituent of the alkyl group in (1-1-1a. R^{X1}) and the alkyl groups exemplified as the alkyl group in (1-1-1a. R^{X1}). The number of the substituent is not particularly limited, and it may be one or more. The substitution position at which the substituent is bonded to the condensed cyclic aromatic hydrocarbon ring is not particularly limited, either.

Examples of the ring in which multiple aromatic rings are bonded to each other directly (a biarene ring) include a ring in which two benzene rings are bonded to each other directly (a biphenyl ring), a ring in which two naphthalene rings are bonded to each other directly (a binaphthyl ring) and a ring in which a benzene ring and a naphthalene ring are directly bonded (a binaphthylphenyl ring).

The biarene ring may have a substituent. Examples of the substituent include the substituents exemplified as the substituent of the alkyl group in (1-1-1a. R^{X1}) and the alkyl groups exemplified as the alkyl group in (1-1-1a. R^{X1}). The number of the substituent is not particularly limited, and it may be one or more. The substitution position at which the substituent is bonded to the biarene ring is not particularly limited, either.

Examples of the ring in which multiple aromatic hydrocarbon rings are bonded to each other via a linking group (a ring aggregated ring in which rings are bonded to each other via a linking group) include a ring represented by the formula R^{b1}-L^{a}-R^{b2}, where R^{b1} and R^{b2} each represent any of a benzene ring and a naphthalene ring, and L^{a} represents a linking group. In the above formula, examples of L^{a} include a group containing an oxygen atom (for example, an ether group (-O-), a carbonyl group (-CO-) and an oxycarbonyl group (-OCO-)); a group containing a sulfur atom (for example, a thio group (-S-)), an alkylene group (for example, a linear or branched alkylene group such as a methylene group and an ethylene group, and a cycloalkylene group such as a cyclohexylidene group). From the viewpoint of achieving the effects of the present invention more effectively and reliably, it is preferably a group containing an oxygen atom, and is more preferably an ether group.

These ring aggregated rings in which rings are bonded to each other via a linking group may have a substituent. Examples of the substituent include the substituents exemplified as the substituent of the alkyl group in (1-1-1a. R^{X1}) and the alkyl groups exemplified as the alkyl group in (1-1-1a. R^{X1}). The number of the substituent is not particularly limited, and it may be one or more. The substitution position at which the substituent is bonded to the ring aggregated ring in which rings are bonded to each other via a linking group, is not particularly limited, either.

Examples of the alicyclic hydrocarbon ring include a monocyclic aliphatic hydrocarbon ring and a polycyclic aliphatic hydrocarbon ring.

Examples of the monocyclic aliphatic hydrocarbon ring include a cycloalkane ring such as a cyclobutane ring, a cyclopentane ring, a cyclohexane ring and a cycloheptane ring. These monocyclic aliphatic hydrocarbon rings may have a substituent. Examples of the substituent include the substituents exemplified as the substituent of the alkyl group in (1-1-1a. R^{X1}) and the alkyl groups exemplified as the alkyl group in (1-1-1a. R^{X1}). The number of the substituent is not particularly limited, and it may be one or more. The substitution position at which the substituent is bonded to the monocyclic aliphatic hydrocarbon ring is not particularly limited, either.

Examples of the polycyclic aliphatic hydrocarbon ring include a decalin ring, an adamantane ring, a dicyclopentane ring, a norbornane ring, a tricyclodecane ring, and a ring having at least one double bond in these rings (for example, a dicyclopentadiene ring and the like). These polycyclic aliphatic hydrocarbon rings may have a substituent. Examples of the substituent include the substituents exemplified as the substituent of the alkyl group in (1-1-1a. R^{X1}) and the alkyl groups exemplified as the alkyl group in (1-1-1a. R^{X1}). The number of the substituent is not particularly limited, and it may be one or more. The substitution position at which the substituent is bonded to the polycyclic aliphatic hydrocarbon ring is not particularly limited, either.

Examples of the representative R^{X2} include those obtained by removing one hydrogen atom from rings (x1) to (x21) shown below. In the following formulas (x1), (x2) and (x3), R* represents any of a hydrogen atom, a methyl group, an ethyl group, a n-propyl group, a n-butyl group, a cyclohexyl group and a hydroxy group.

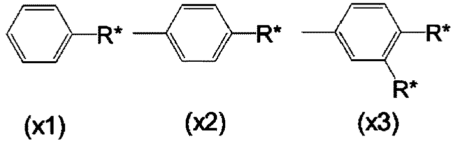

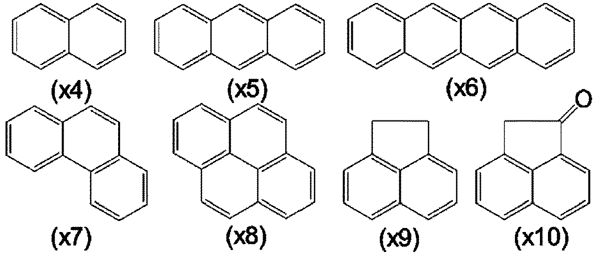

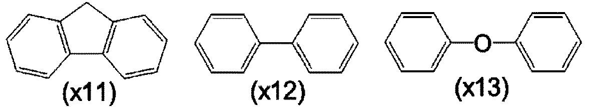

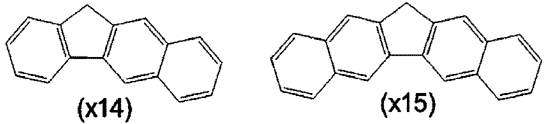

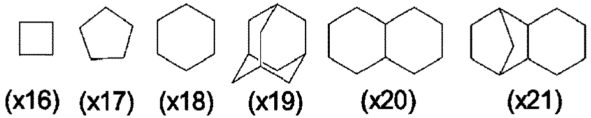

Examples of the representative combination of R^{X1}, R^{X2} and n^{A} in the formula (0a) can include a combination in which R^{X1} is any of a hydrogen atom, a methyl group and a phenyl group; R^{X2} is any of the groups (x1) to (x21); and n^{A} is 1.

In addition, the 2n^{A}-valent group may be a group represented by the following formula (Cy1) when n^{A} = 1, may be a group represented by the following formula (Cy2) when n^{A} = 2, may be a group represented by the following formula (Cy3) when n^{A} = 3, represents a cycloalkanetetrayl group having 3 to 70 carbon atoms when n^{A} = 3, and may be a group represented by the following formula (Cy4) when n^{A} = 4.

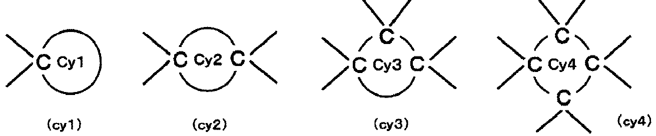

In the above formulas (cy1), (cy2), (cy3) and (cy4), cy1 is a divalent group obtained by removing one hydrogen atom from the cyclic alkyl groups or aryl groups exemplified as R^{x2}, cy2 is a tetravalent group obtained by removing three hydrogen atoms from the cyclic alkyl groups or aryl groups exemplified as R^{x2}, cy3 is a hexavalent group obtained by removing one hydrogen atom from the cyclic alkyl groups or aryl groups exemplified as R^{x2}, and cy4 is a octavalent group obtained by removing one hydrogen atom from the cyclic alkyl groups or aryl groups exemplified as R^{x2}.

More specifically, examples of the group represented by the formulas (cy1) to (cy4) include those obtained by removing 2n^{A} (n^{A} = 1, 2, 3 or 4) hydrogen atoms from (x9), (x10), (x11) and (x14) to (x21).

### (2. R^{1A})

In the formula (0), each R^{1A} independently represents any of an alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, an alkoxy group having 1 to 30 carbon atoms and optionally having a substituent, a maleamic acid group having 4 to 30 carbon atoms and optionally having a substituent, a maleimide group having 4 to 30 carbon atoms and optionally having a substituent, a halogen atom (for example, a fluorine atom, a chlorine atom and a bromine atom), a nitro group, an amino group having 0 to 30 carbon atoms and optionally having a substituent, a carboxyl group, a thiol group and a hydroxy group. When R^{1A} is any of the alkyl group, the aryl group, the crosslinkable group and the alkoxy group, at least one selected from the group consisting of an ether bond, a ketone bond and an ester bond may be contained. However, at least one R^{1A} is a maleamic acid group having 4 to 30 carbon atoms and optionally having a substituent or a maleimide group having 4 to 30 carbon atoms and optionally having a substituent.

Examples of the alkyl group having 1 to 30 carbon atoms and optionally having a substituent include the alkyl groups exemplified as the alkyl group in (1-1-1a. R^{X1}). Among them, from the viewpoint of achieving the effects of the present invention more effectively and reliably, a linear or branched alkyl group having 1 to 6 carbon atoms, such as a methyl group, an ethyl group, a n-propyl group, an isopropyl group, a n-butyl group, an isobutyl group and a tert-butyl group, is preferable, a linear or branched alkyl group having 1 to 3 carbon atoms is more preferable, and a methyl group or an ethyl group is further preferable. In addition, when these alkyl groups have a substituent, from the viewpoint of achieving the effects of the present invention more effectively and reliably, a halogen atom (a fluorine atom, a chlorine atom and a bromine atom) is preferable as the substituent. The alkyl group substituted with a halogen atom is preferably a haloalkyl group having 1 to 6 carbon atoms such as a trifluoromethyl group, a trichloromethyl group and a tetrafluoroethyl group.

Examples of the aryl group having 6 to 30 carbon atoms and optionally having a substituent include the aryl groups exemplified as the aryl group in (1-1-1a. R^{X1}). Among them, from the viewpoint of achieving the effects of the present invention more effectively and reliably, a phenyl group and a naphthyl group (a 1-naphthyl group and a 2-naphthyl group) are preferable, and a phenyl group is more preferable. In addition, when these aryl groups have a substituent, an amino group is preferable as the substituent, and the substitution position at which the amino group is bonded to the aryl group (in particular, a phenyl group) is preferably position 2 or position 4.

The crosslinkable group having 2 to 30 carbon atoms and optionally having a substituent is, for example, a group that is capable of generating a new bond with another compound under certain conditions (for example, conditions with regard to the presence of an acid or base catalyst, heating conditions, conditions for subjecting the crosslinkable group to a radical reaction, conditions for irradiation of visible light or invisible light (for example, ultraviolet ray, infrared ray and the like)), and examples thereof include an allyl group, a (meth)acryloyl group, a vinyl group, an epoxy group, an alkoxymethyl group and a cyanato group.

Examples of the alkoxy group having 1 to 30 carbon atoms and optionally having a substituent include a group represented by -O-R^{a1}, where R^{a1} represents alkyl groups exemplified as the alkyl group in (1-1-1a. R^{X1}). Among them, from the viewpoint of achieving the effects of the present invention more effectively and reliably, a linear or branched alkoxy group having 1 to 6 carbon atoms, such as a methoxy group, an ethoxy group, a n-propoxy group, an isopropoxy group, a n-butoxy group, an isobutoxy group and a tert-butoxy group, is preferable, a linear or branched alkoxy group having 1 to 3 carbon atoms is more preferable, and a methoxy group or an ethoxy group is further preferable.

Examples of the amino group having 0 to 30 carbon atoms and optionally having a substituent include a group represented by (R⁴)₂N-, where each R⁴ independently represents any of a hydrogen atom, a linear or branched, or cyclic alkyl group having 1 to 30 carbon atoms and optionally having a substituent, an aryl group having 6 to 30 carbon atoms and optionally having a substituent, and a crosslinkable group having 2 to 30 carbon atoms and optionally having a substituent, and when R⁴ is any of the alkyl group, the aryl group and the crosslinkable group, at least one bond selected from the group consisting of an ether bond, a ketone bond and an ester bond may be contained. Examples of the linear or branched, or cyclic alkyl group having 1 to 30 carbon atoms can include the alkyl groups exemplified as the alkyl group in (1-1-1a. R^{X1}). Examples of the aryl group having 6 to 30 carbon atoms and optionally having a substituent can include the aryl groups exemplified as the aryl group in (1-1-1a. R^{X1}). Examples of the crosslinkable group having 2 to 30 carbon atoms and optionally having a substituent can include the crosslinkable groups exemplified as the crosslinkable group in (2. R^{1A}) .

As the group represented by the above formula (R⁴)₂N-, from the viewpoint of achieving the effects of the present invention more effectively and reliably, preferable are an amino group; a monosubstituted amino group in which one R⁴ is a hydrogen atom and the other R⁴ is an alkyl group (for example, a linear or branched alkyl group having 1 to 4 carbon atoms such as a methyl group, an ethyl group, a n-propyl group and a n-butyl group) or an alkylcarbonyl group (for example, a linear or branched alkylcarbonyl group having 2 to 5 carbon atoms such as a methylcarbonyl group, an ethylcarbonyl group, a n-propylcarbonyl group and a n-butylcarbonyl group); and a disubstituted amino group in which two R⁴ are each an alkyl group (for example, a linear or branched alkyl group having 1 to 4 carbon atoms such as a methyl group, an ethyl group, a n-propyl group and a n-butyl group) or an alkylcarbonyl group (for example, a linear or branched alkylcarbonyl group having 2 to 5 carbon atoms such as a methylcarbonyl group, an ethylcarbonyl group, a n-propylcarbonyl group and a n-butylcarbonyl group), and more preferable are a an amino group; a monosubstituted amino group in which one R⁴ is a hydrogen atom and the other R⁴ is a methyl group or a methylcarbonyl group; and a disubstituted amino group in which two R⁴ are each a methyl group or a methylcarbonyl group.

At least one R^{1A} is any group of a maleamic acid group having 4 to 30 carbon atoms and optionally having a substituent and a maleimide group having 4 to 30 carbon atoms and optionally having a substituent. The number of the group is not particularly limited as long as it is one or more, but from the viewpoint of achieving the effects of the present invention more effectively and reliably, it is preferably 1 to 3, and is more preferably 1 or 2. When there are a plurality of the groups, for example, each group may be bonded to the same aromatic ring, or may be bonded to different aromatic rings.

### (3. X)

In the formula (0), X represents an oxygen atom or a sulfur atom, or is optionally not present, but from the viewpoint of even further excellent structure forming ability, it is preferably an oxygen atom or a sulfur atom, and is more preferably an oxygen atom. On the other hand, from the viewpoint of solvent solubility, it is preferable that X be not present. Note that a compound represented by the formula (0), wherein X represents an oxygen atom, is represented by the following formula (0-1) . 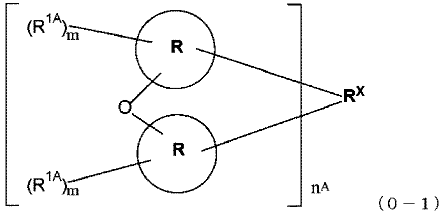 (In the above formula (0-1), R^{X}, R^{1A}, R, m and n^{A} are as defined above.)

### (4. m)

Each m is independently an integer of 0 to 9. However, at least one m is an integer of 1 to 9. From the viewpoint of achieving the effects of the present invention more effectively and reliably, m is preferably an integer of 1 to 5, more preferably an integer of 1 to 3, and still more preferably an integer of 1 or 2.

### (5. n^{A})

n^{A} is an integer of 1 to 4, and from the viewpoint of achieving the effects of the present invention more effectively and reliably, it is preferably an integer of 1 or 2, and is more preferably 1.

### (6. R)

Each R independently represents any of a benzene ring, a naphthalene ring and an anthracene ring. In the above formula (0), when R is a benzene ring, it is shown that the benzene ring is substituted with one or more R^{1A}, and the substitution position of each R^{1A} to the benzene ring is not particularly limited, and the bonding position of X to the benzene ring is not particularly limited, either. In the above formula (0), when R is a naphthalene ring, it is shown that the naphthalene ring is substituted with one or more R^{1A}, and the substitution position of each R^{1A} to the naphthalene ring is not particularly limited, and the bonding position of X to the naphthalene ring is not particularly limited, either. In the above formula (0), when R is an anthracene ring, it is shown that the anthracene ring is substituted with one or more R^{1A}, and the substitution position of each R^{1A} to the anthracene ring is not particularly limited, and the bonding position of X to the anthracene ring is not particularly limited, either. From the viewpoint of achieving the effects of the present invention more effectively and reliably, R is preferably a benzene ring or a naphthalene ring, and is more preferably a benzene ring.

The compound represented by the above formula (0) has, as previously mentioned, high aromatic density and low crystallinity and has excellent structure forming ability (for example, film forming ability), heat resistance and solubility.

Therefore, the compound of the present embodiment can be used usefully as a high heat resistant thermosetting resin and a cured product thereof. The high heat resistant thermosetting resin and a cured product thereof are usefully used in an electrical insulating material, a resin for solder resists, an encapsulation resin for semiconductors, an adhesive for printed circuit boards, an electrical laminate mounted in electric equipment, electronic equipment, industrial equipment, and the like, a matrix resin of prepregs mounted in electric equipment, electronic equipment, industrial equipment, and the like, a buildup laminate material, a resin for fiber-reinforced plastics, a resin for encapsulation of liquid crystal display panels, a coating material, a variety of coating agents, an adhesive, a coating agent for semiconductors, a resin for resists for semiconductor lithography, a resin for underlayer film formation, and an additive agent. When used for these materials, the functional resin and the functional cured product are used in the form of a film or sheet. In addition, the functional resin and the functional cured product are also usefully used in a component or the like, such as a plastic lens (a prism lens, a lenticular lens, a microlens, a Fresnel lens, a viewing angle control lens, a contrast improving lens, etc.), a phase difference film, a film for electromagnetic wave shielding, a prism, an optical fiber, a solder resist for flexible printed wiring, a plating resist, an interlayer insulating film for multilayer printed circuit boards, and a photosensitive optical waveguide.

### [Compound represented by formula (1)]

It is preferable that the compound represented by the formula (0) be a compound represented by the following formula (1) from the viewpoint of even further improvement in solubility in an organic solvent.

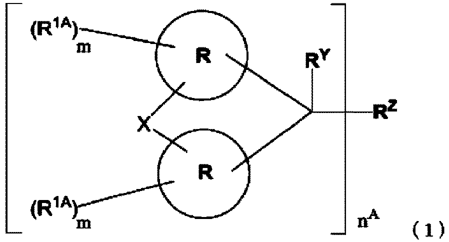

In the above formula (1), R^{Y} is any of a hydrogen atom, a linear or branched, or cyclic alkyl group having 1 to 30 carbon atoms and optionally having a substituent, and an aryl group having 6 to 30 carbon atoms and optionally having a substituent; R^{Z} is an n^{A}-valent group having 1 to 60 carbon atoms or a single bond, and the total number of carbon atoms in R^{Y} and R^{Z} is 69 or less; and X, R, n^{A}, R^{1A} and m are as defined above.

From the viewpoint of further improvement in solubility and heat resistance, the compound represented by the above formula (1) is preferably a compound represented by the following formula (1a-1), and is more preferably a compound represented by the following formula (1b-1).

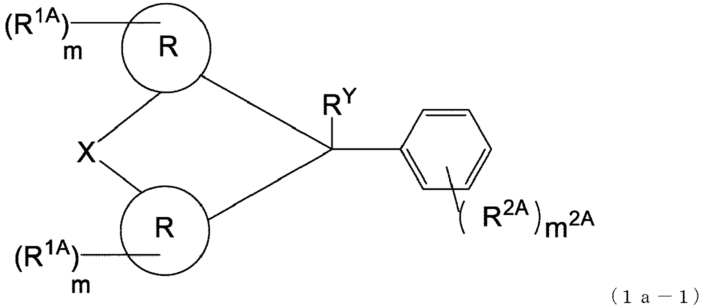

In the above formula (1a-1), X, R, R^{1A}, m and R^{Y} are as defined above; R^{2A} represents any of a hydrogen atom, a linear or branched, or cyclic alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, and a halogen atom (for example, a fluorine atom, a chlorine atom and a bromine atom). In addition, m^{2A} represents an integer of 1 to 5.

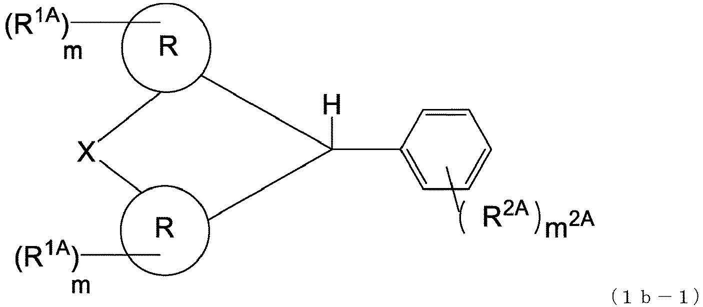

In the above formula (1b-1), X, R, R^{1A}, R^{2A}, m and m^{2A} are as defined above.

From the viewpoint of further improvement in solubility, the compound represented by the above formula (1) is preferably a compound represented by the following formula (1a-2), and is more preferably a compound represented by the following formula (1b-2).

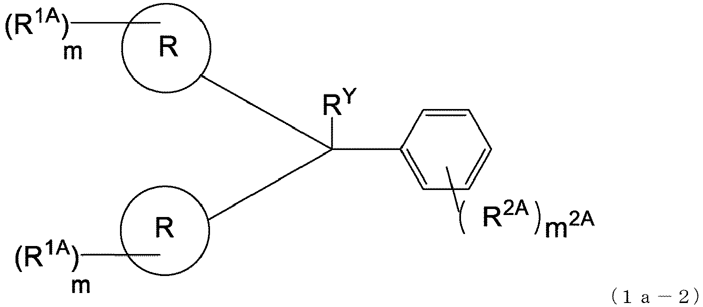

In the above formula (1a-2), R, R^{1A}, m and R^{Y} are as defined above; R^{2A} represents any of a hydrogen atom, a linear or branched, or cyclic alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, and a halogen atom (for example, a fluorine atom, a chlorine atom and a bromine atom). In addition, m^{2A} represents an integer of 1 to 5.

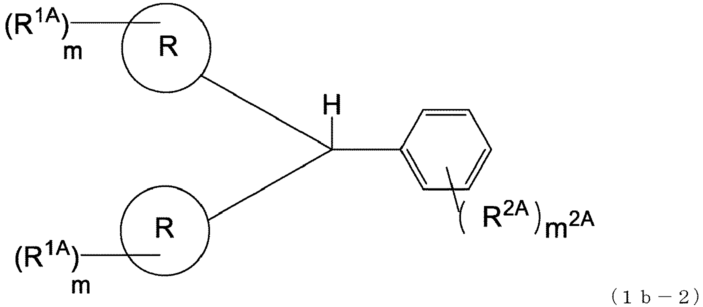

In the above formula (1b-2), X, R, R^{1A}, R^{2A}, m and m^{2A} are as defined above.

### [Compound represented by formula (1a-2)]

It is preferable that the compound represented by the above formula (1a-2) be a compound represented by the following formula (1a-3) from the viewpoint of further improvement in solubility.

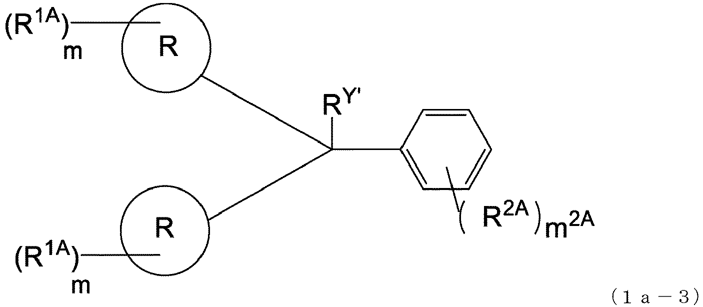

In the above formula (1a-3), R, R^{1A}, R^{2A}, m and m^{2A} are as defined above; and R^{Y'} represents R^{Y} except for a hydrogen atom.

### [Compound represented by formula (1b-2)]

It is preferable that the compound represented by the above formula (1b-2) be a compound represented by the following formula (1b-3) from the viewpoint of further improvement in flowability upon film formation.

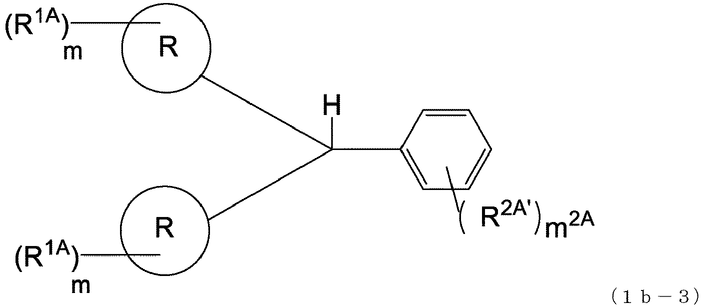

In the above formula (1b-3), R, R^{1A}, m and m^{2A} are as defined above; and R^{2A'} represents a linear or branched, or cyclic alkyl group having 5 or more carbon atoms and optionally having a substituent.

### (1. R^{Y})

R^{Y} represents any of a hydrogen atom, a linear or branched, or cyclic alkyl group having 1 to 30 carbon atoms and optionally having a substituent, and an aryl group having 6 to 30 carbon atoms and optionally having a substituent. Examples of the linear or branched, or cyclic alkyl group having 1 to 30 carbon atoms and optionally having a substituent include the alkyl groups exemplified as the alkyl group in (1-1-1a. R^{X1}), and examples of the alkyl group that is preferable from the viewpoint of achieving the effects of the present invention more effectively and reliably include the alkyl groups exemplified as the preferable alkyl group in (1-1-1a. R^{X1}). Examples of the aryl group having 6 to 30 carbon atoms and optionally having a substituent include the aryl groups exemplified as the aryl group in (1-1-1a. R^{X1}), and examples of the aryl group that is preferable from the viewpoint of achieving the effects of the present invention more effectively and reliably include the aryl groups exemplified as the preferable aryl group in (1-1-1a. R^{X1}). Among them, R^{Y} is preferably a hydrogen atom or an aryl group having 6 to 30 carbon atoms and optionally having a substituent, and is more preferably a hydrogen atom or a phenyl group.

### (2. R^{Z})

It is preferable that R^{Z} be an n^{A}-valent group having 1 to 60 carbon atoms from the viewpoint of achieving the effects of the present invention more effectively and reliably. Examples of the n^{A}-valent group having 1 to 60 carbon atoms include the n^{A}-valent groups exemplified in (1-1-1b. R^{X2}), and examples of the group that is preferable include the n^{A}-valent group exemplified as the preferable n^{A}-valent group in (1-1-1b. R^{X2}).

### [Compound represented by formula (2) or (2)' ((poly)imide compound)]

It is preferable that the compound represented by the formula (1) be a compound represented by the following formula (2) or (2)' from the viewpoint of even further improvement in film forming ability.

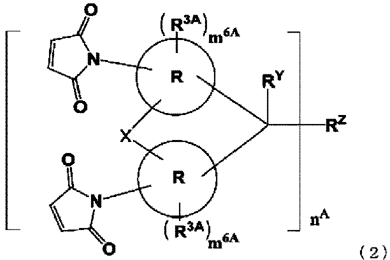

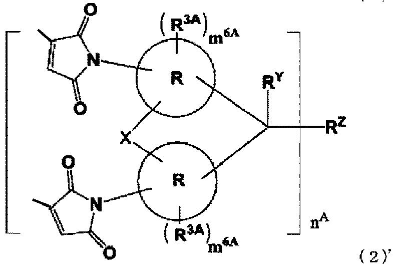

In the formulas (2) and (2)', each R^{3A} is independently any of a linear or branched, or cyclic alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, an alkoxy group having 1 to 30 carbon atoms and optionally having a substituent, and a halogen atom (for example, a fluorine atom, a chlorine atom and a bromine atom); each m^{6A} is independently an integer of 0 to 5; and R^{Y}, R^{Z}, X, R and n^{A} are as defined above.

### (1. R^{3A})

Each R^{3A} independently represents any of a linear or branched, or cyclic alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, an alkoxy group having 1 to 30 carbon atoms and optionally having a substituent, and a halogen atom (for example, a fluorine atom, a chlorine atom and a bromine atom). Examples of the linear or branched, or cyclic alkyl group having 1 to 30 carbon atoms and optionally having a substituent include the alkyl groups exemplified as the alkyl group in (1-1-1a. R^{X1}). Examples of the aryl group having 6 to 30 carbon atoms and optionally having a substituent include the aryl groups exemplified as the aryl group in (1-1-1a. R^{X1}). Examples of the crosslinkable group having 2 to 30 carbon atoms and optionally having a substituent include the crosslinkable groups exemplified as the crosslinkable group in (2. R^{1A}). Examples of the alkoxy group having 1 to 30 carbon atoms and optionally having a substituent include the alkoxy groups exemplified as the alkoxy group in (1-1-1a. R^{X1}).

Among them, from the viewpoint of achieving the effects of the present invention more effectively and reliably, R^{3A} is preferably a linear or branched alkyl group having 1 to 30 carbon atoms and optionally having a substituent or an aryl group having 6 to 30 carbon atoms and optionally having a substituent. It is preferable that the linear or branched alkyl group having 1 to 30 carbon atoms and optionally having a substituent be any of the alkyl groups exemplified as the preferable alkyl group optionally having a substituent in (2. R^{1A}) of [Compound represented by formula (0)]. It is preferable that the aryl group having 6 to 30 carbon atoms and optionally having a substituent be any of the aryl groups exemplified as the preferable aryl group optionally having a substituent in (2. R^{1A}).

### (2. m^{6A})

Each m^{6A} is independently an integer of 0 to 5, and from the viewpoint of achieving the effects of the present invention more effectively and reliably, it is preferably an integer of 0 to 3, and is more preferably an integer of 0 to 2.

### (3. X)

In the compound represented by the formula (2) or (2)', X is an oxygen atom or a sulfur atom, or X is optionally not present.

### [Compound represented by formula (3) or (3)' ((poly)amic acid)]

It is preferable that the compound represented by the formula (1) be a compound represented by the following formula (3) or (3)' from the viewpoint of even further improvement in film forming ability.

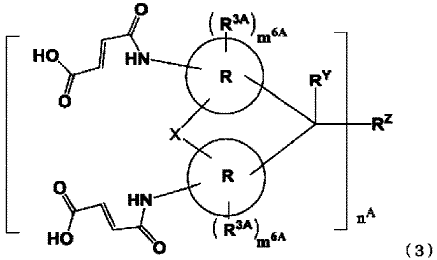

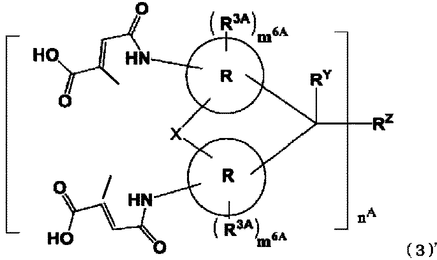

In the formulas (3) and (3)', each R^{3A} is independently any of a linear or branched, or cyclic alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, an alkoxy group having 1 to 30 carbon atoms and optionally having a substituent, and a halogen atom (for example, a fluorine atom, a chlorine atom and a bromine atom); each R^{4A} is independently a hydrogen atom, a linear or branched, or cyclic alkyl group having 1 to 30 carbon atoms and optionally having a substituent, an aryl group having 6 to 30 carbon atoms and optionally having a substituent, and a crosslinkable group having 2 to 30 carbon atoms and optionally having a substituent, wherein, when R^{4A} is any of the alkyl group, the aryl group and the crosslinkable group, at least one bond selected from the group consisting of an ether bond, a ketone bond and an ester bond may be optionally contained; each m^{6A} is independently an integer of 0 to 5; and R^{Y}, R^{Z}, X, R and n^{A} are as defined above.

### (1. R^{3A})

Each R^{3A} independently represents any of a linear or branched, or cyclic alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, an alkoxy group having 1 to 30 carbon atoms and optionally having a substituent, and a halogen atom (for example, a fluorine atom, a chlorine atom and a bromine atom). Examples of the linear or branched, or cyclic alkyl group having 1 to 30 carbon atoms and optionally having a substituent include the alkyl groups exemplified as the alkyl group in (1-1-1a. R^{X1}). Examples of the aryl group having 6 to 30 carbon atoms and optionally having a substituent include the aryl groups exemplified as the aryl group in (1-1-1a. R^{X1}). Examples of the crosslinkable group having 2 to 30 carbon atoms and optionally having a substituent include the crosslinkable groups exemplified as the crosslinkable group in (2. R^{1A}). Examples of the alkoxy group having 1 to 30 carbon atoms and optionally having a substituent include the alkoxy groups exemplified as the alkoxy group in (1-1-1a. R^{X1}).

Among them, from the viewpoint of achieving the effects of the present invention more effectively and reliably, R^{3A} is preferably a linear or branched alkyl group having 1 to 30 carbon atoms and optionally having a substituent or an aryl group having 6 to 30 carbon atoms and optionally having a substituent. It is preferable that the linear or branched alkyl group having 1 to 30 carbon atoms and optionally having a substituent be any of the alkyl groups exemplified as the preferable alkyl group optionally having a substituent in (2. R^{1A}) of [Compound represented by formula (0)]. It is preferable that the aryl group having 6 to 30 carbon atoms and optionally having a substituent be any of the aryl groups exemplified as the preferable aryl group optionally having a substituent in (2. R^{1A}).

### (2. m^{6A})

Each m^{6A} is independently an integer of 0 to 5, and from the viewpoint of achieving the effects of the present invention more effectively and reliably, it is preferably an integer of 0 to 3, and is more preferably an integer of 0 to 2.

### (3. X)

In the compound represented by the formula (3) or (3)', X is an oxygen atom or a sulfur atom, or X is optionally not present.

Examples of the representative (poly)imide compound or (poly)amic acid of the present embodiment include (poly)imide compounds or (poly)amic acids represented by the formulas (p1) to (p72).

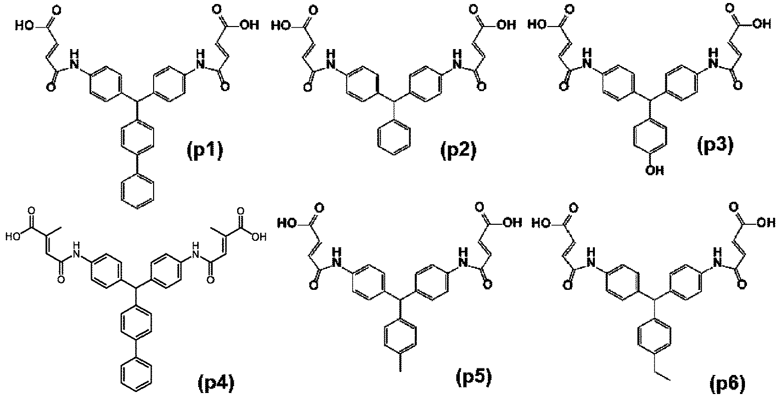

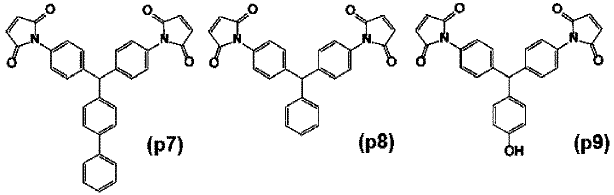

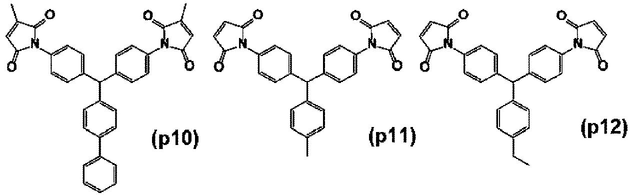

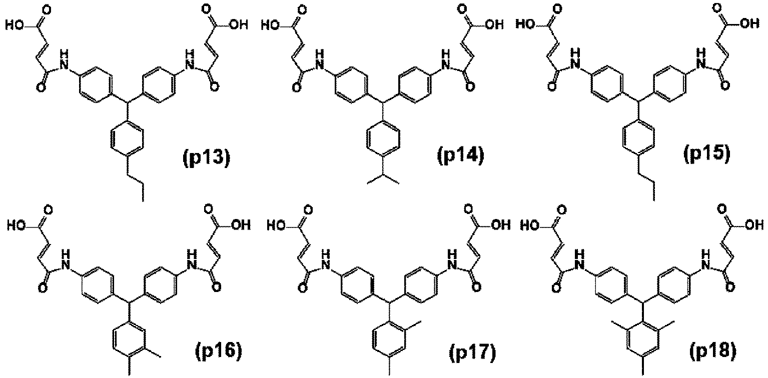

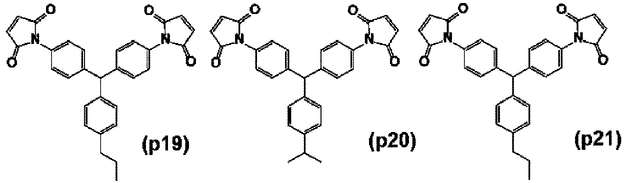

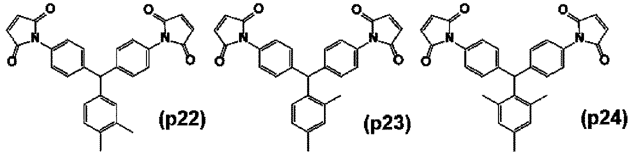

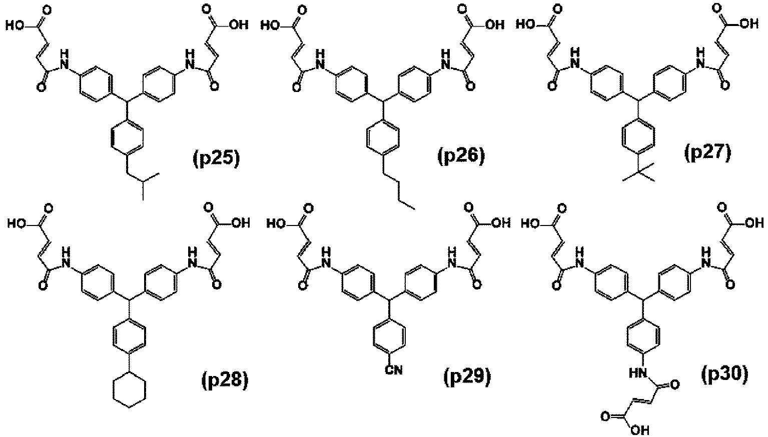

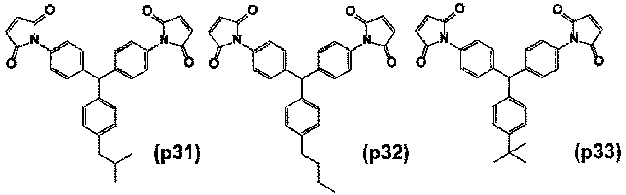

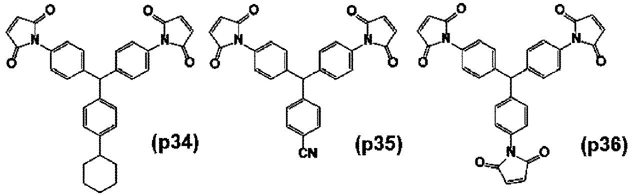

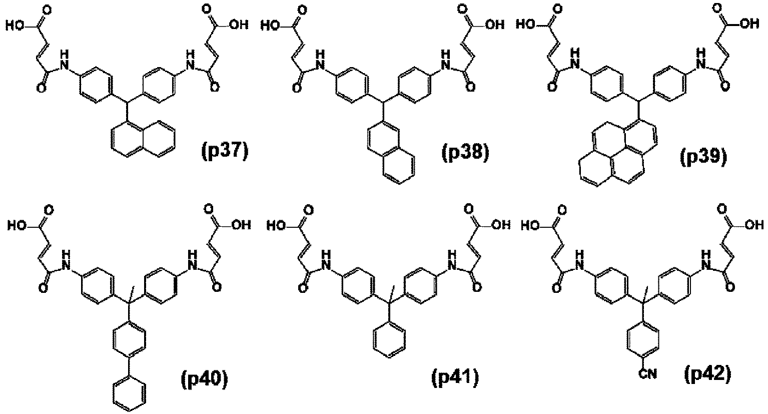

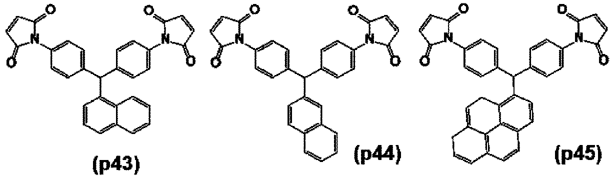

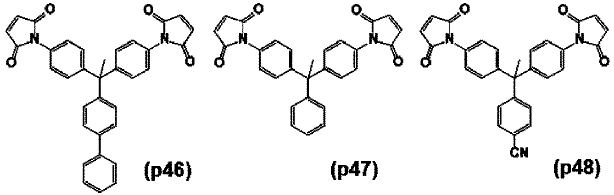

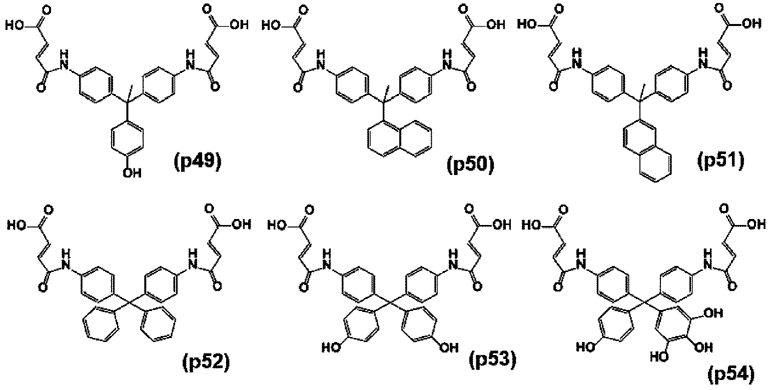

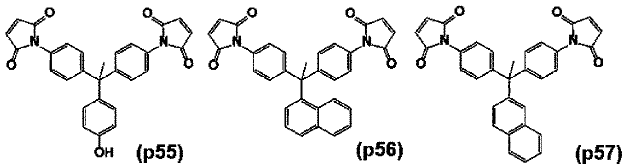

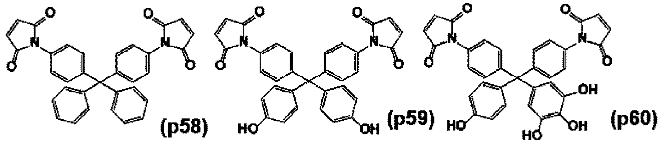

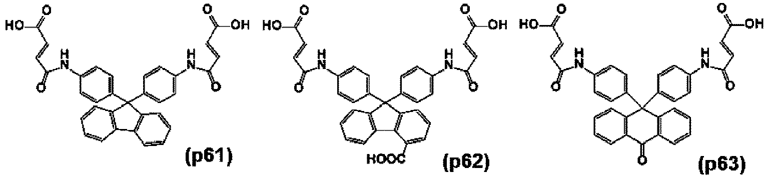

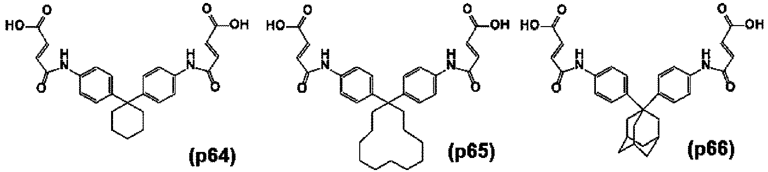

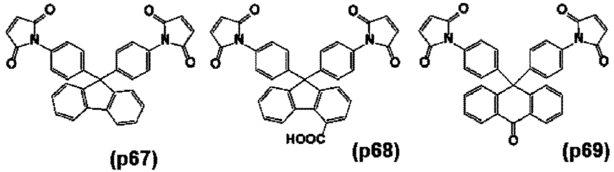

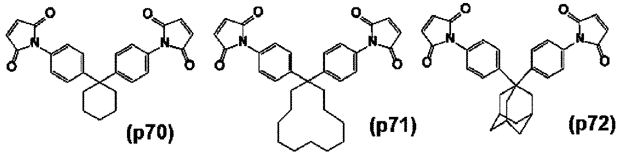

Other examples of the representative (poly)imide compound or (poly)amic acid of the present embodiment include (poly)imide compounds or (poly)amic acids represented by the formulas (p73) to (p504).

Here, in the formula (p73) to the formula (p504), each R^{A} independently represents any of a hydrogen atom, the formula (A1), the formula (A2), the formula (A3) and the formula (A4), and at least one R^{A} is any of the formula (A1), the formula (A2), the formula (A3) and the formula (A4).

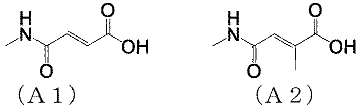

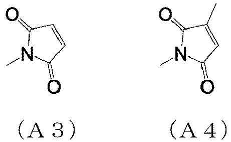

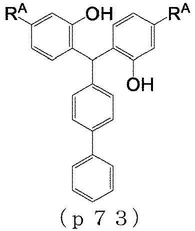

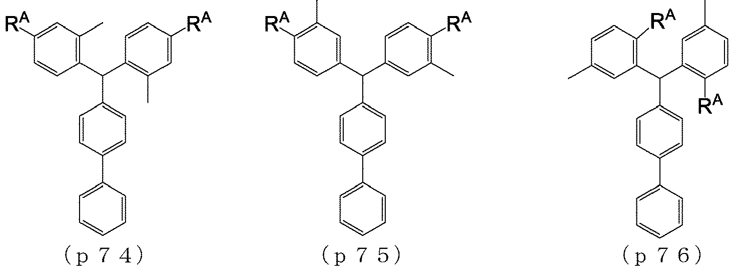

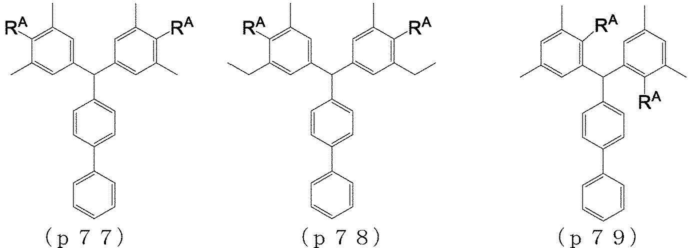

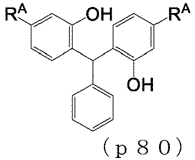

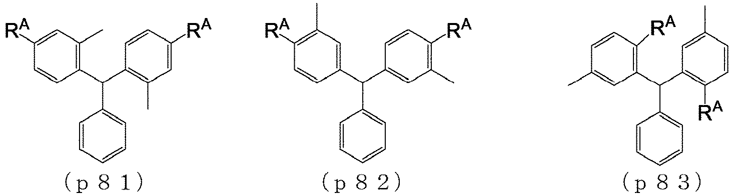

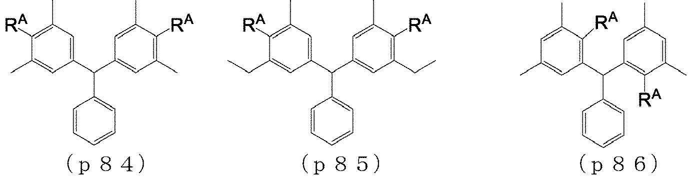

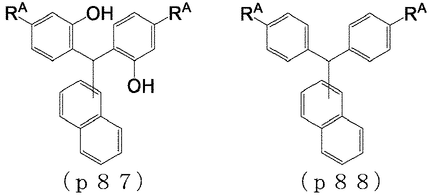

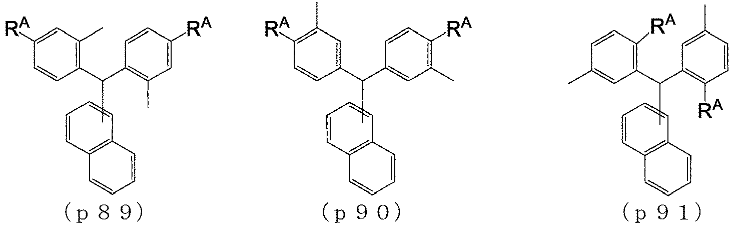

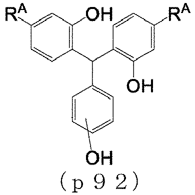

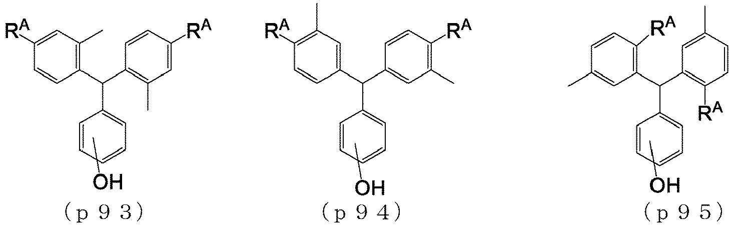

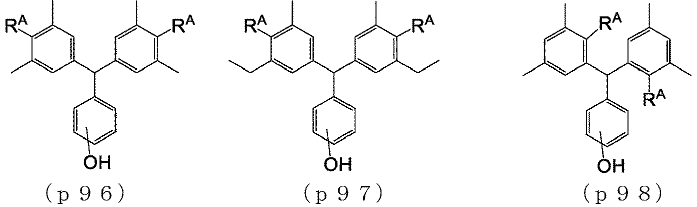

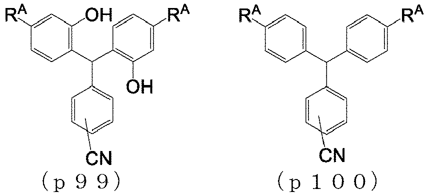

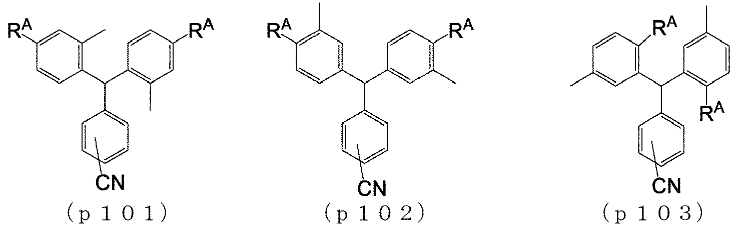

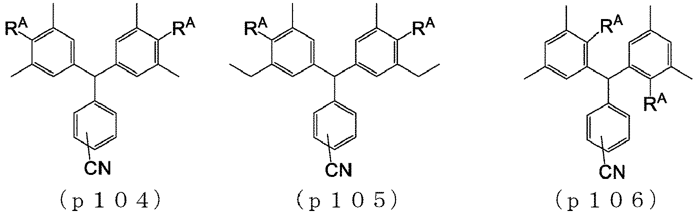

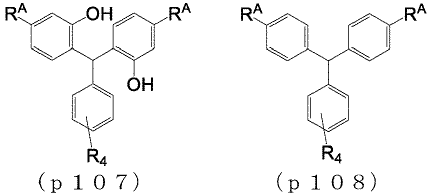

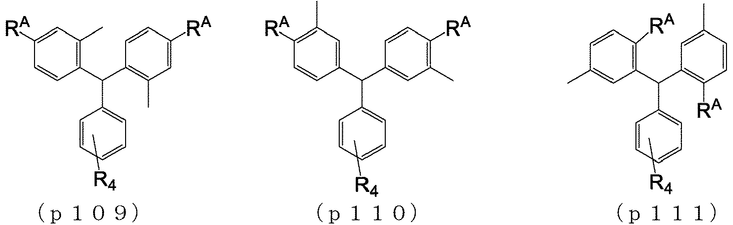

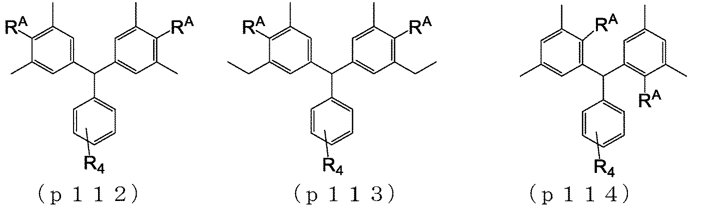

In the above formulas, examples of R₄ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group and a triacontyloxy group, all of which include isomers. For example, a butyl group includes a n-butyl group, an isobutyl group, a sec-butyl group and a tert-butyl group. The number of R₄ may be one or more.

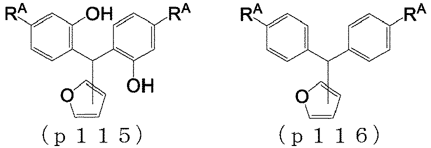

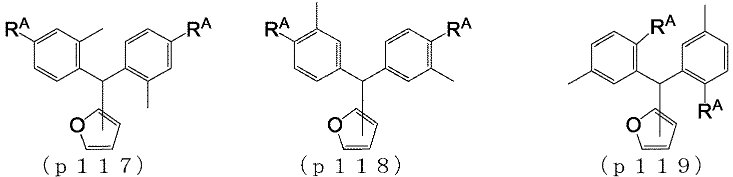

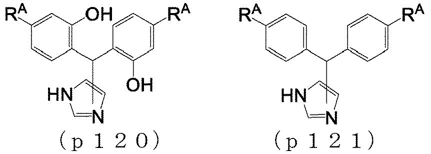

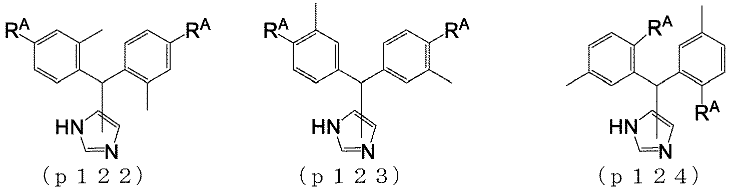

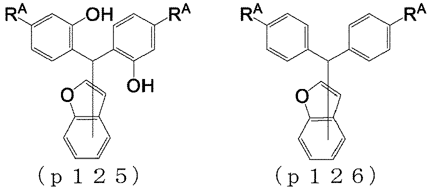

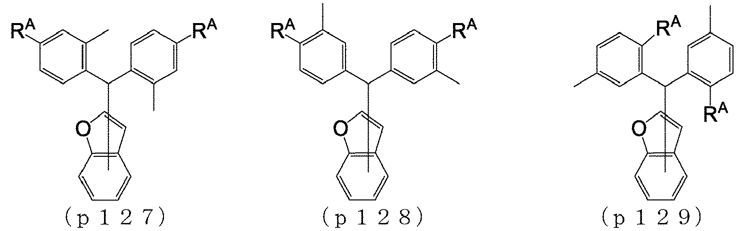

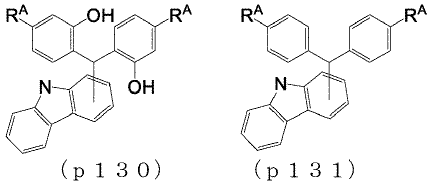

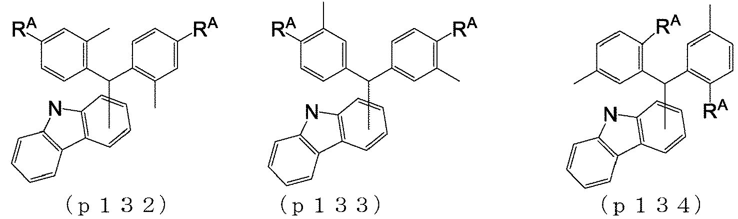

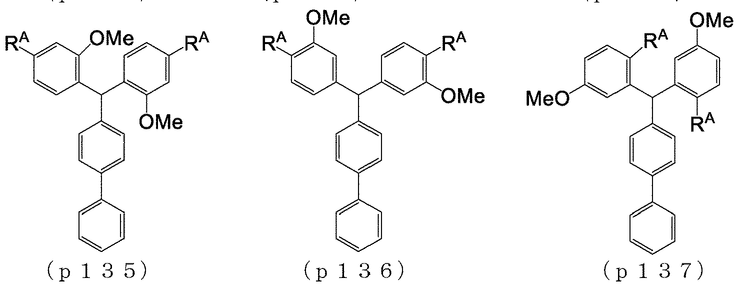

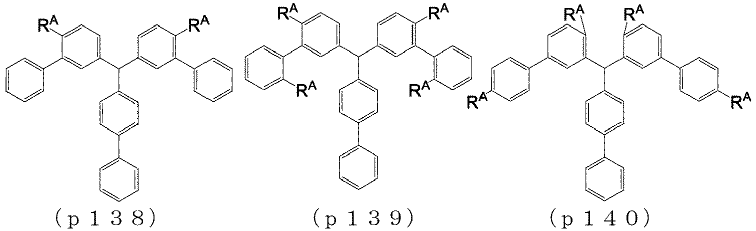

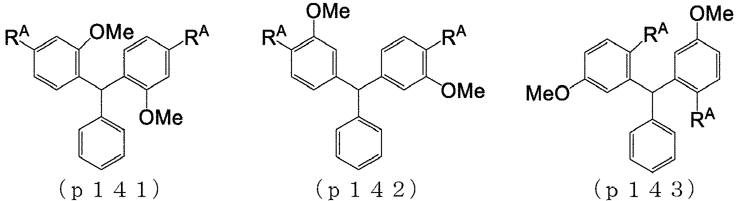

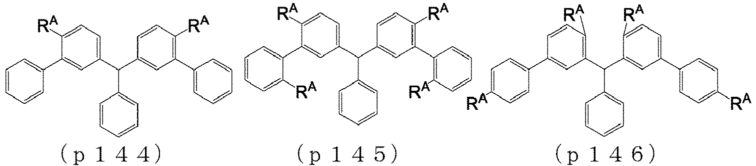

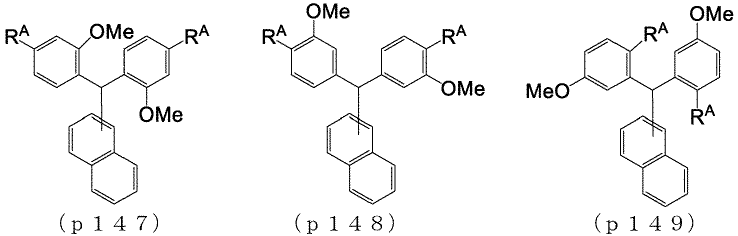

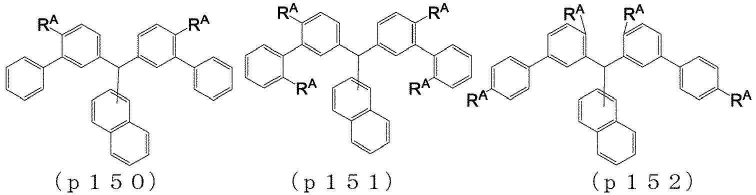

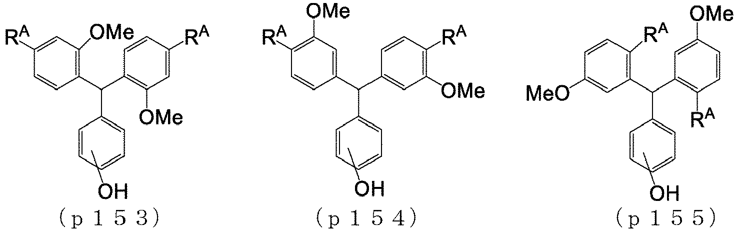

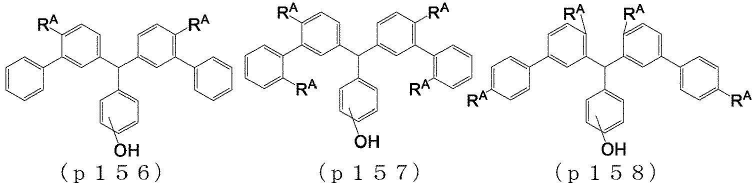

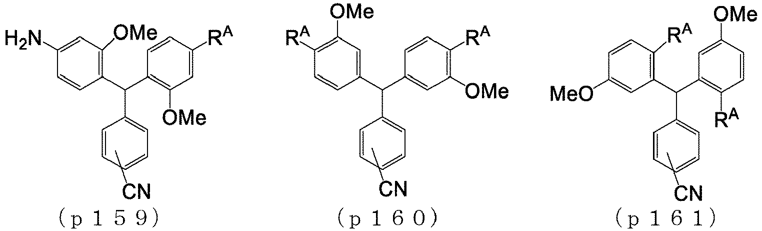

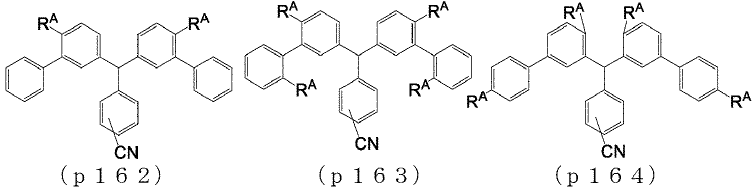

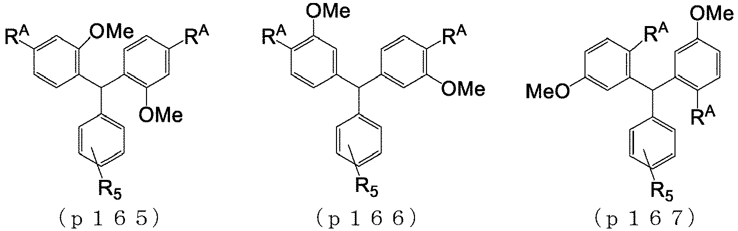

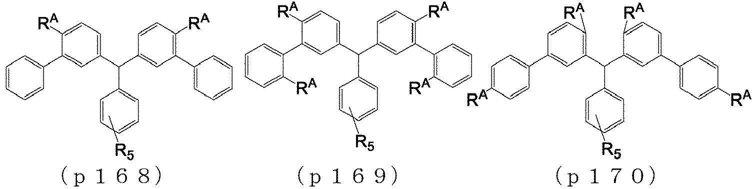

Examples of R₅ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group and a triacontyloxy group, all of which include isomers. For example, a butyl group includes a n-butyl group, an isobutyl group, a sec-butyl group and a tert-butyl group. The number of R₅ may be one or more.

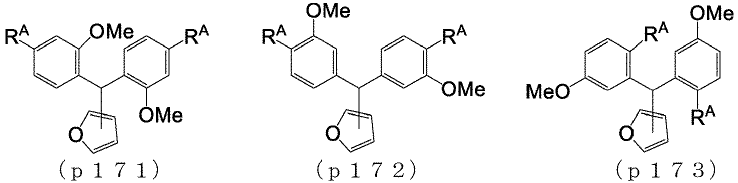

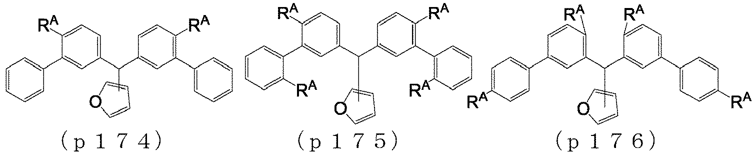

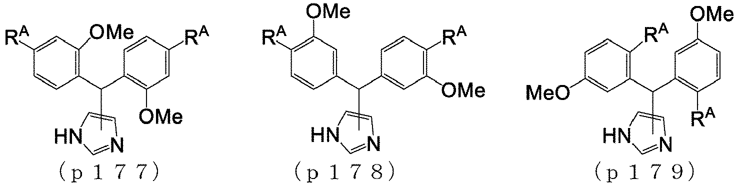

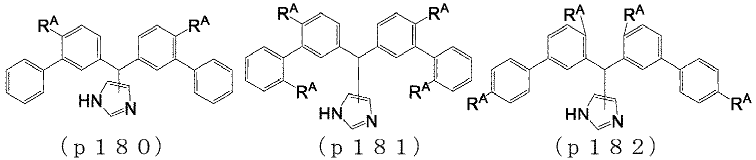

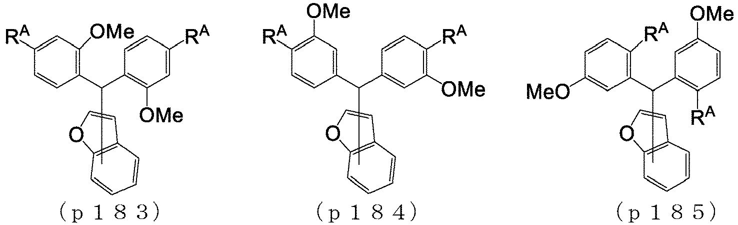

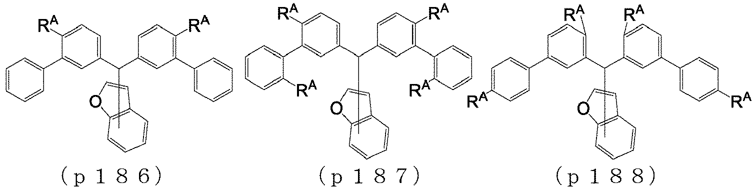

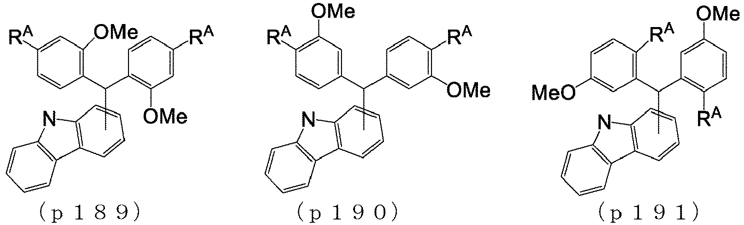

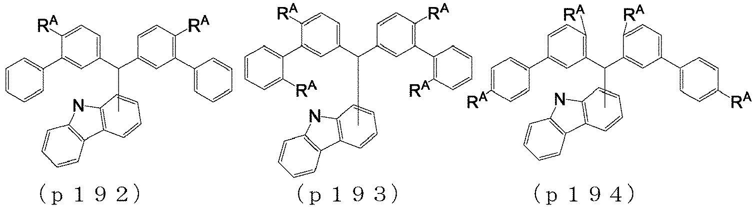

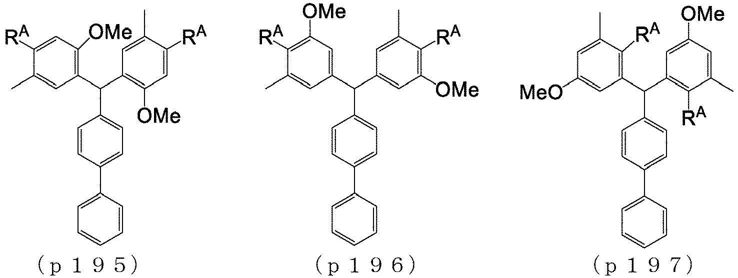

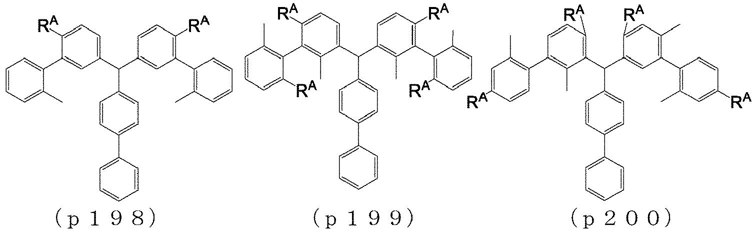

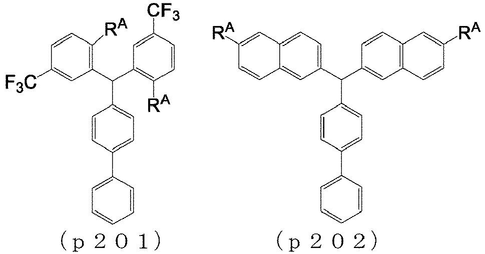

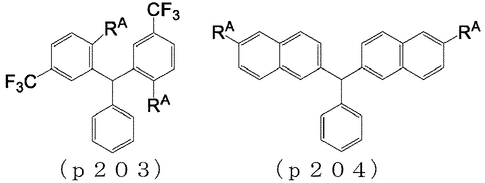

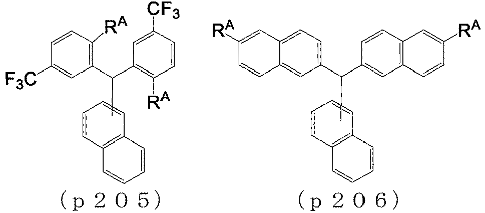

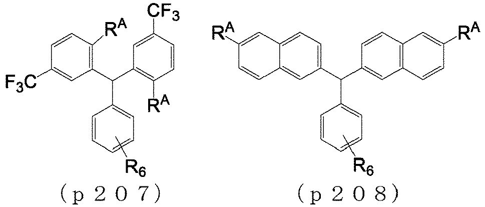

Examples of R₆ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group and a triacontyloxy group, all of which include isomers. For example, a butyl group includes a n-butyl group, an isobutyl group, a sec-butyl group and a tert-butyl group. The number of R₆ may be one or more.

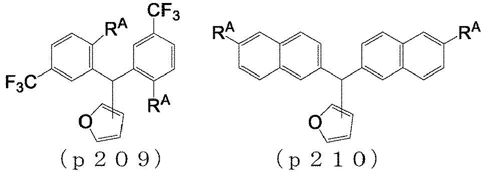

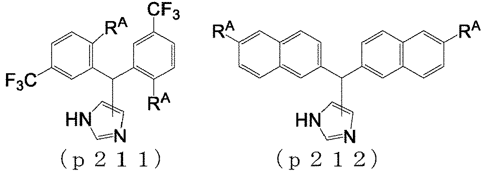

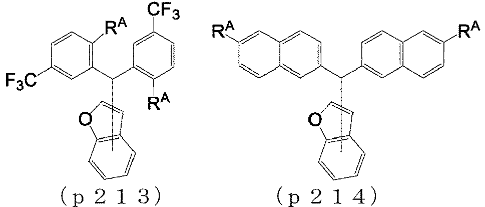

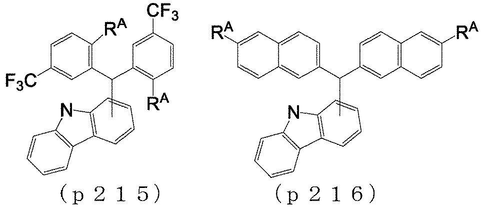

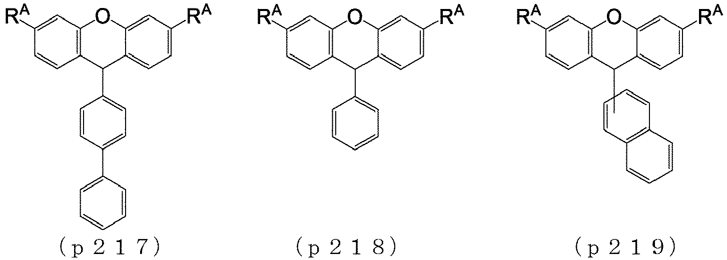

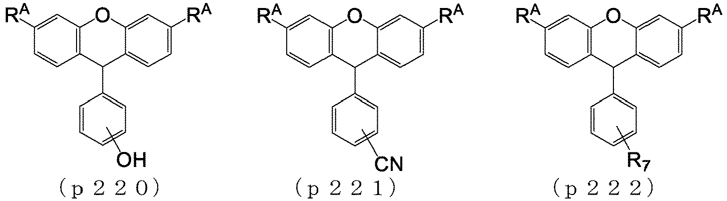

Examples of R₇ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group and a triacontyloxy group, all of which include isomers. For example, a butyl group includes a n-butyl group, an isobutyl group, a sec-butyl group and a tert-butyl group. The number of R₇ may be one or more.

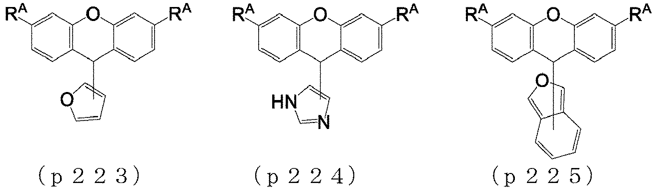

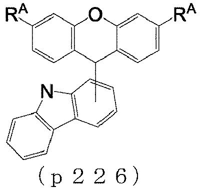

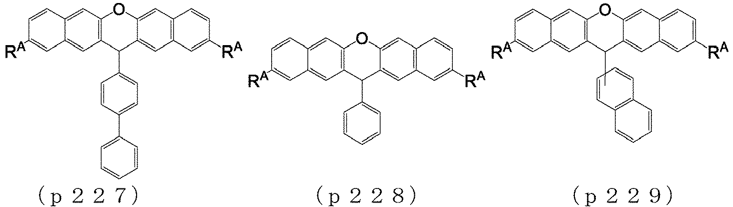

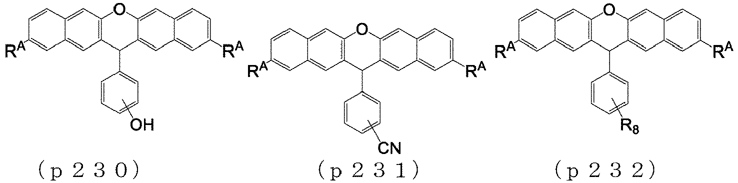

Examples of R₈ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group and a triacontyloxy group, all of which include isomers. For example, a butyl group includes a n-butyl group, an isobutyl group, a sec-butyl group and a tert-butyl group. The number of R₈ may be one or more.

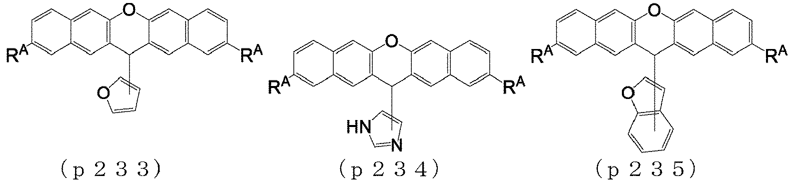

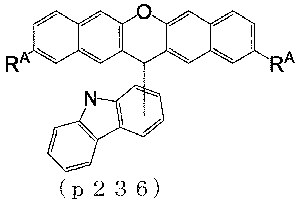

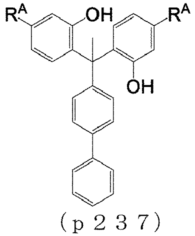

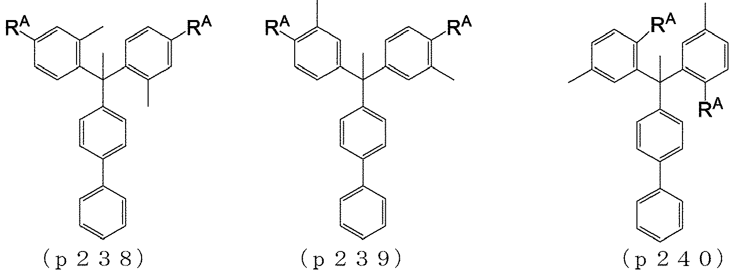

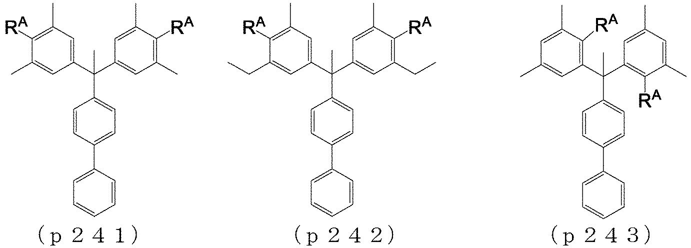

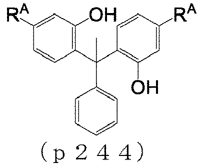

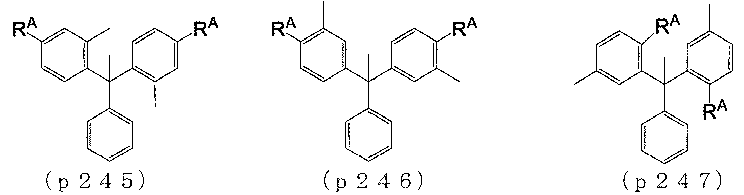

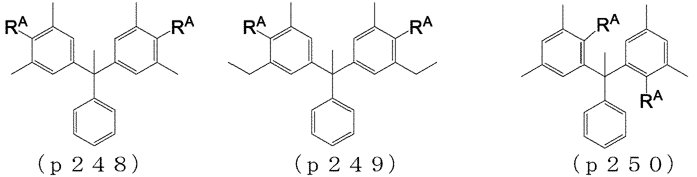

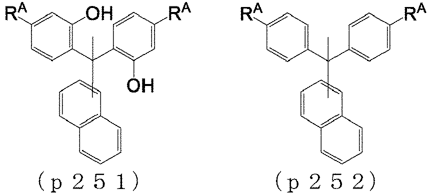

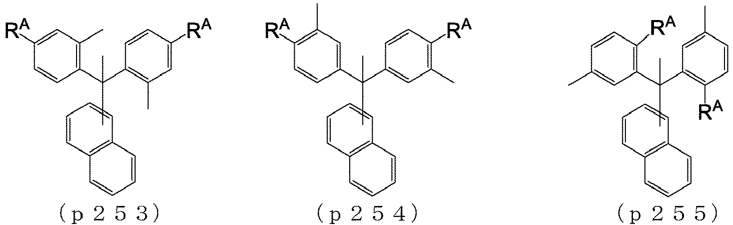

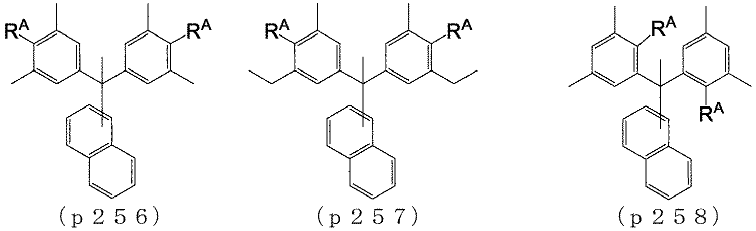

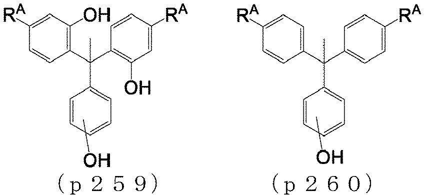

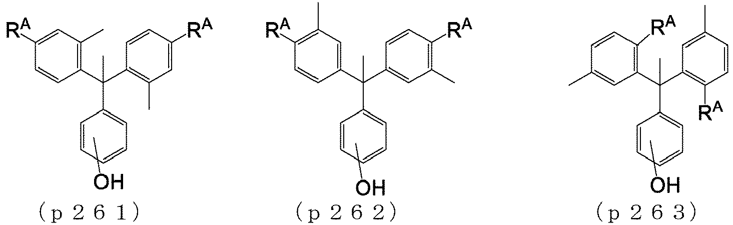

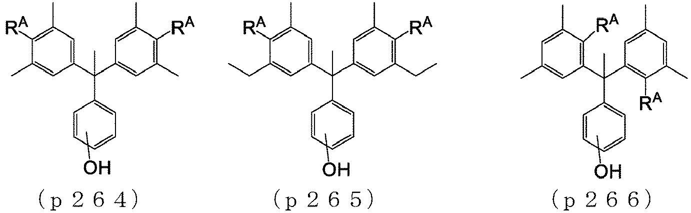

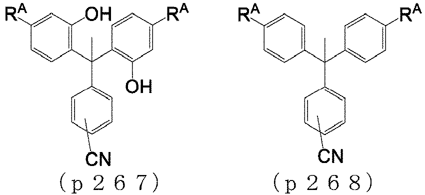

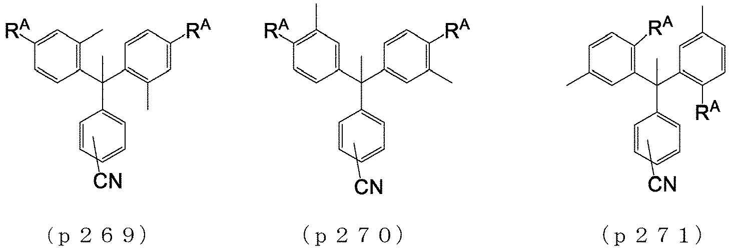

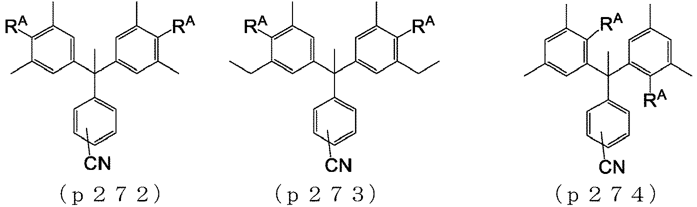

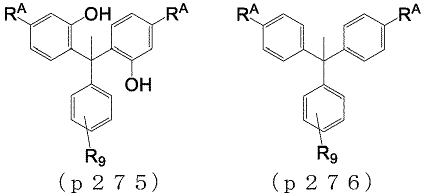

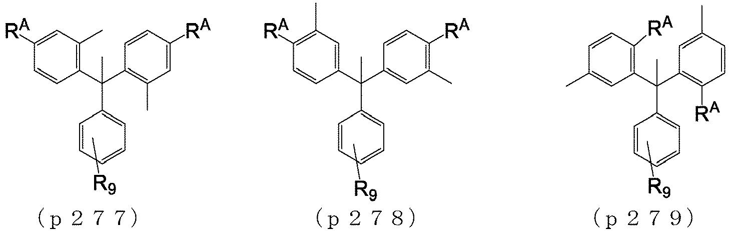

Examples of R₉ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group and a triacontyloxy group, all of which include isomers. For example, a butyl group includes a n-butyl group, an isobutyl group, a sec-butyl group and a tert-butyl group. The number of R₉ may be one or more.

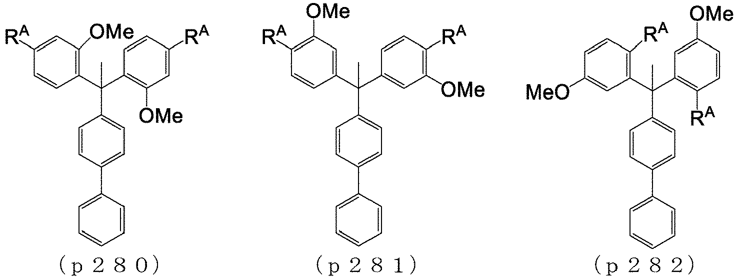

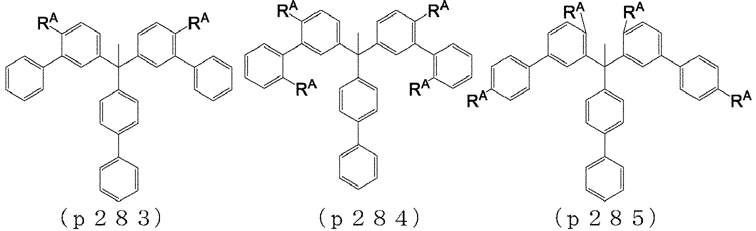

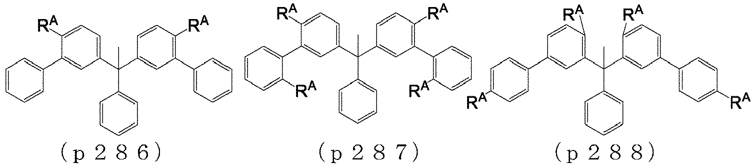

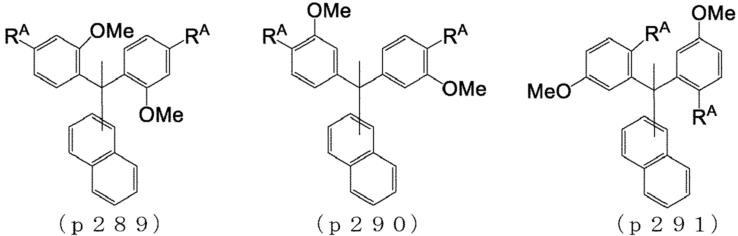

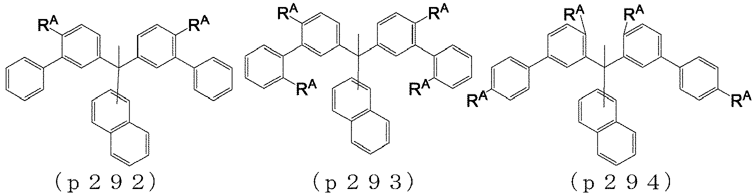

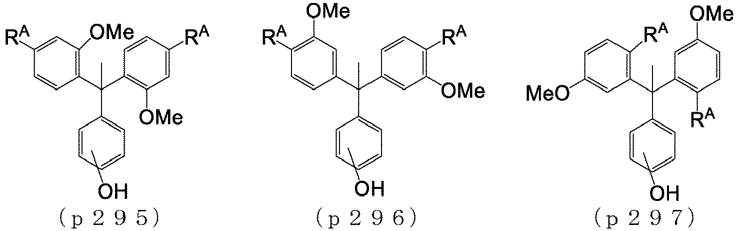

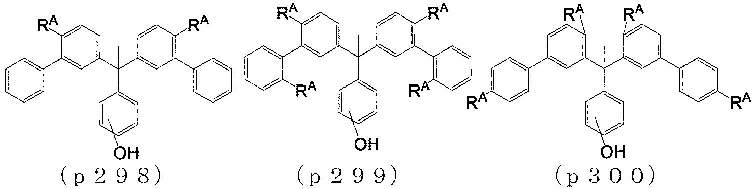

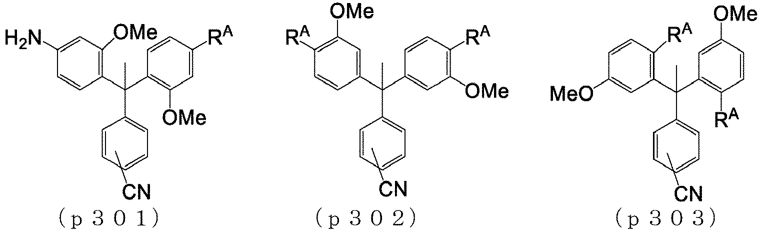

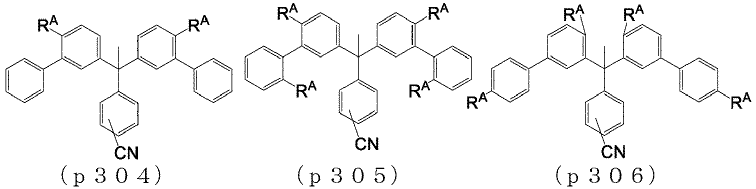

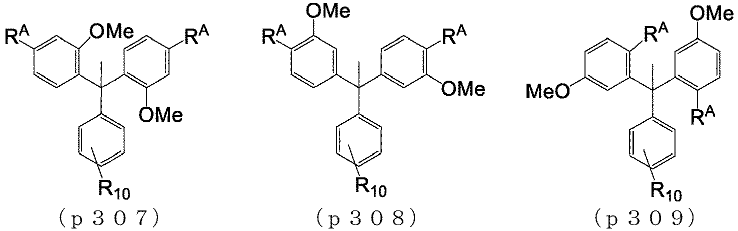

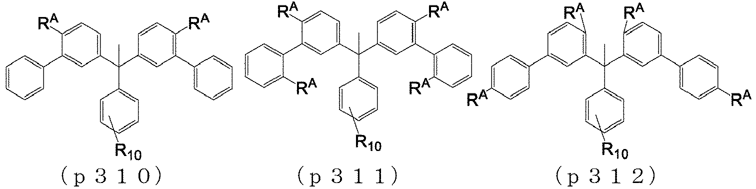

Examples of R₁₀ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group and a triacontyloxy group, all of which include isomers. For example, a butyl group includes a n-butyl group, an isobutyl group, a sec-butyl group and a tert-butyl group. The number of R₁₀ may be one or more.

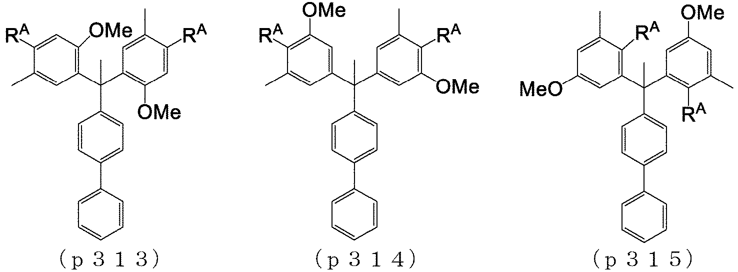

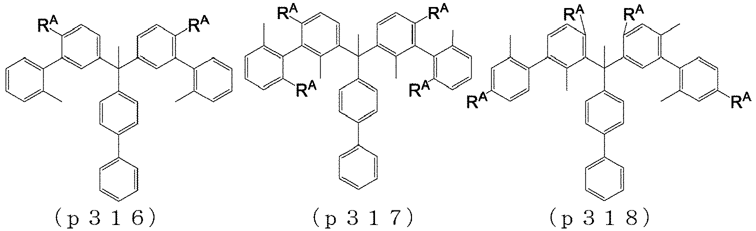

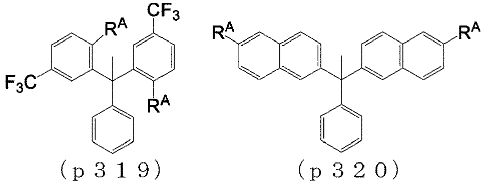

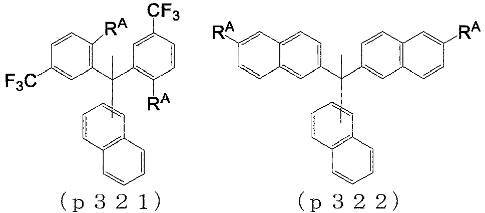

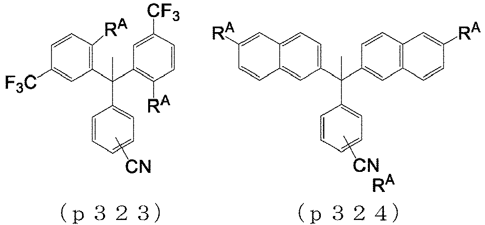

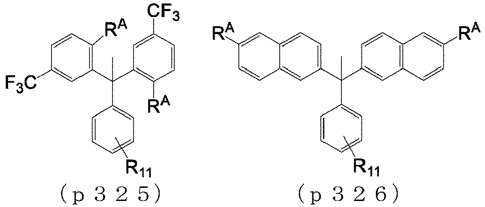

Examples of R₁₁ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group and a triacontyloxy group, all of which include isomers. For example, a butyl group includes a n-butyl group, an isobutyl group, a sec-butyl group and a tert-butyl group. The number of R₁₁ may be one or more.

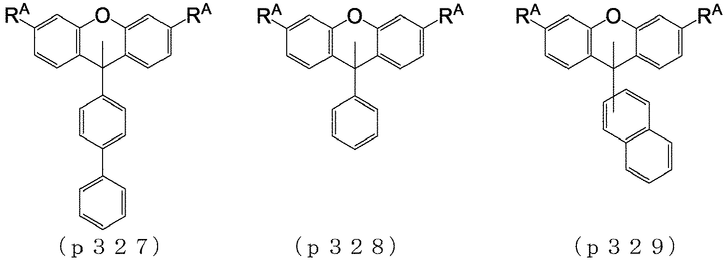

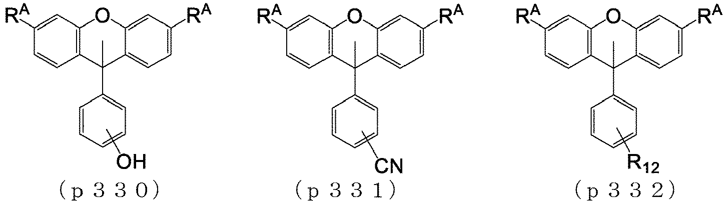

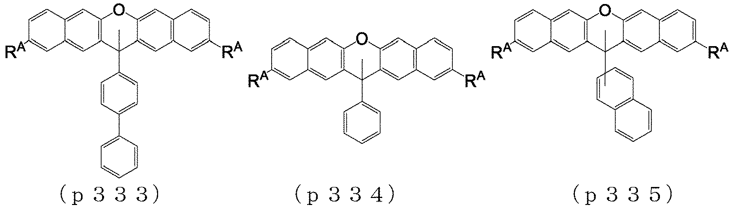

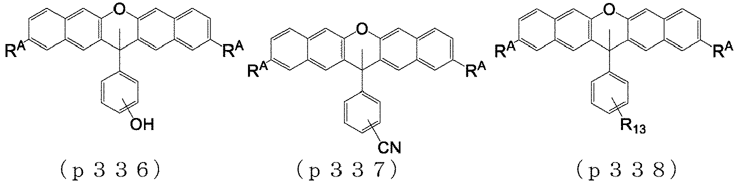

Examples of R₁₂ or R₁₃ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group and a triacontyloxy group, all of which include isomers. For example, a butyl group includes a n-butyl group, an isobutyl group, a sec-butyl group and a tert-butyl group. The number of R₁₂ or R₁₃ may be one or more.

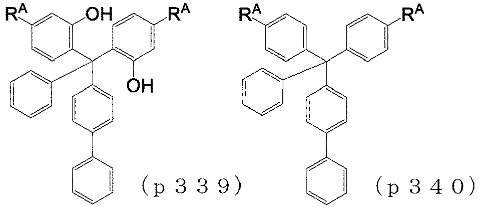

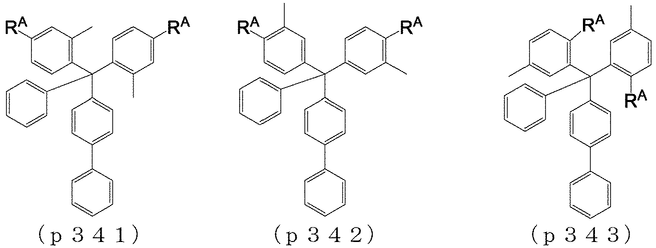

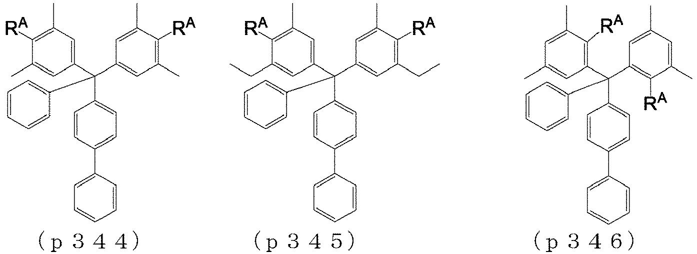

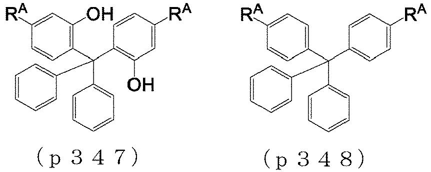

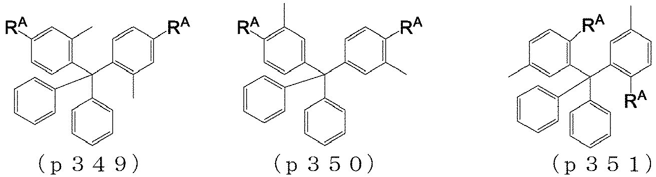

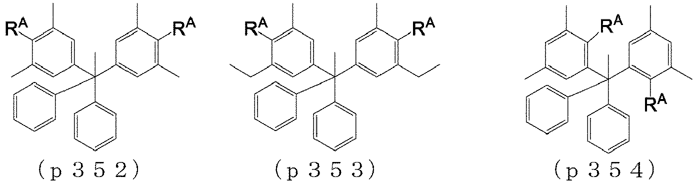

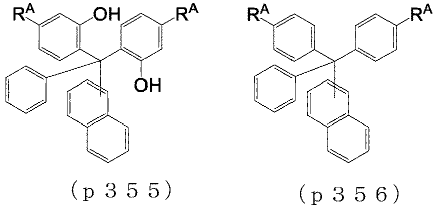

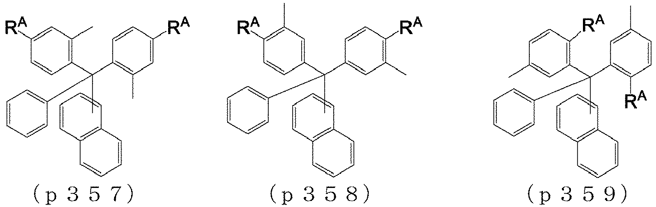

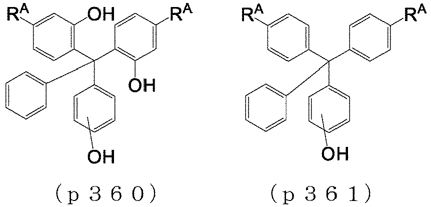

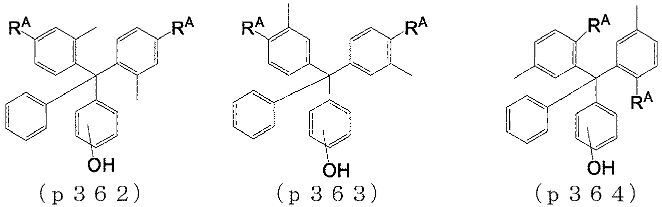

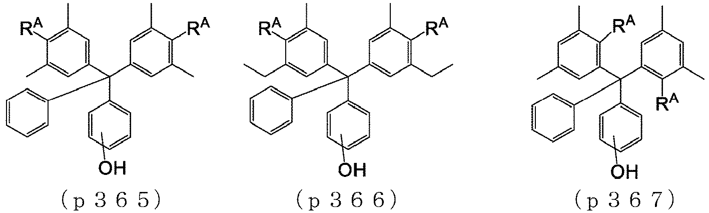

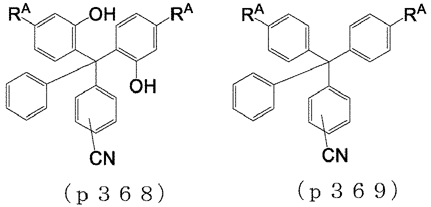

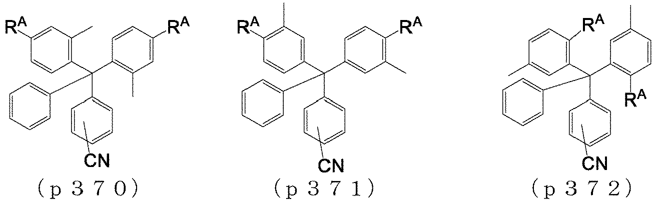

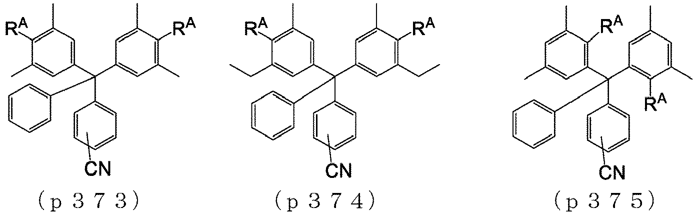

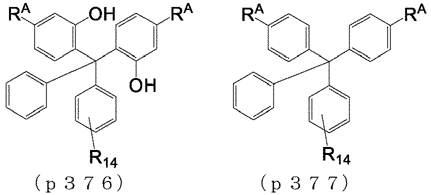

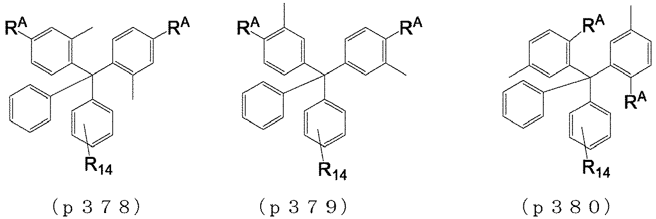

Examples of R₁₄ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group and a triacontyloxy group, all of which include isomers. For example, a butyl group includes a n-butyl group, an isobutyl group, a sec-butyl group and a tert-butyl group. The number of R₁₄ may be one or more.

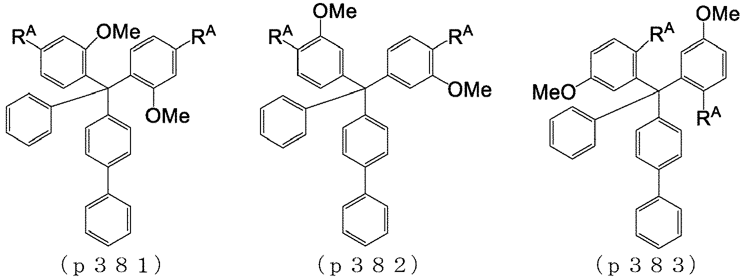

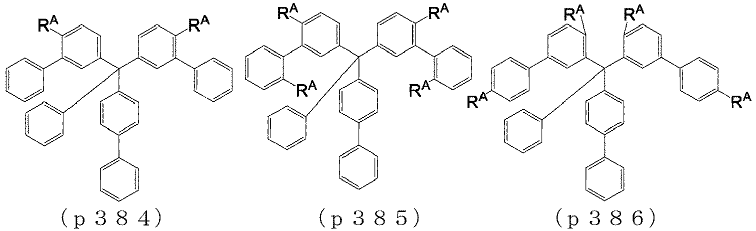

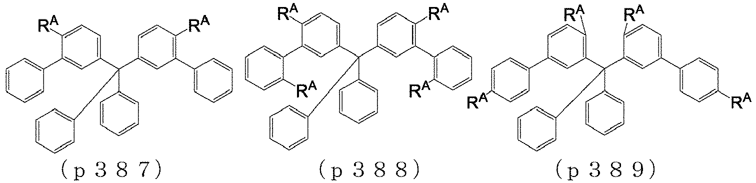

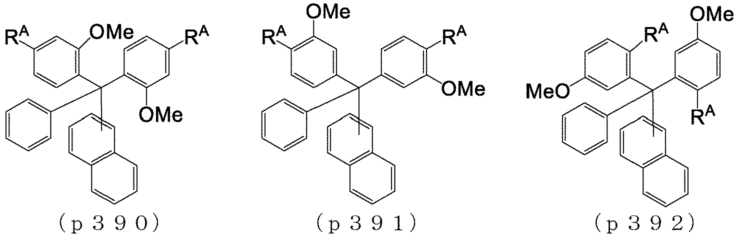

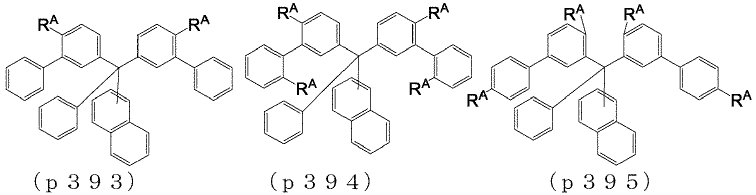

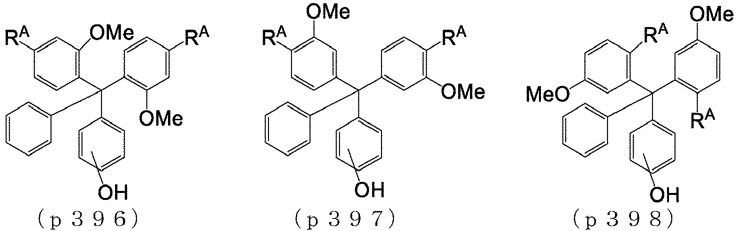

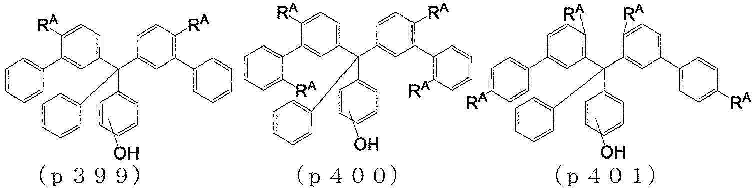

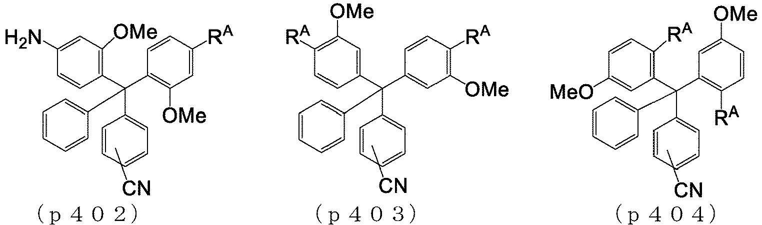

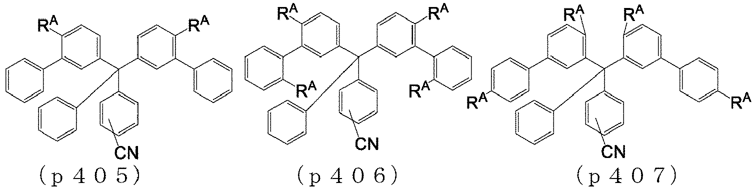

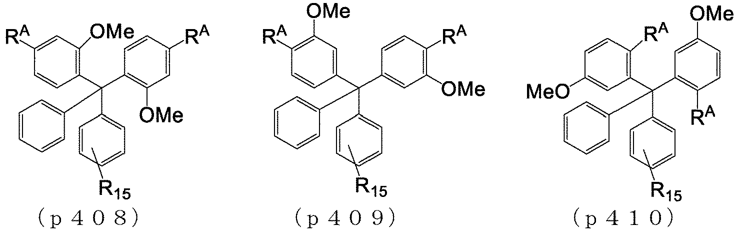

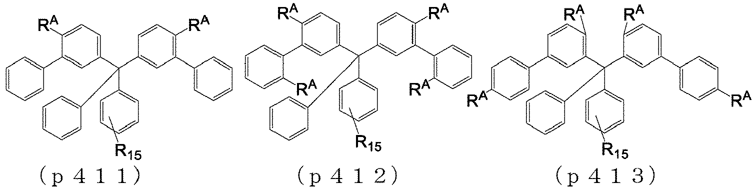

Examples of R₁₅ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group and a triacontyloxy group, all of which include isomers. For example, a butyl group includes a n-butyl group, an isobutyl group, a sec-butyl group and a tert-butyl group. The number of R₁₅ may be one or more.

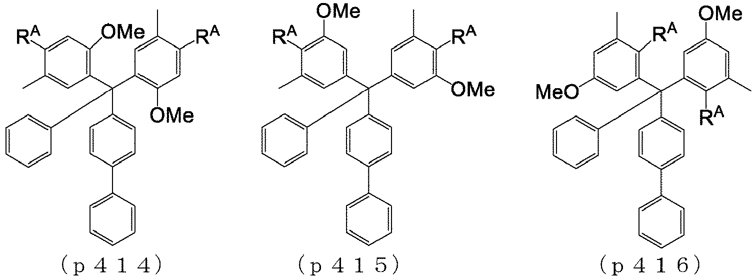

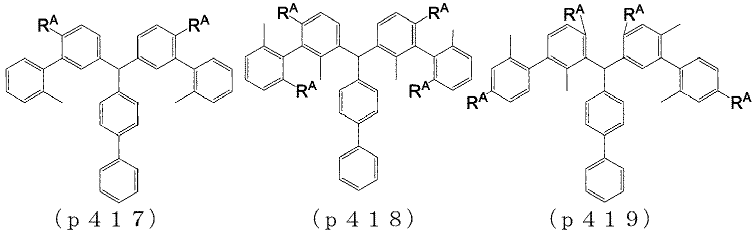

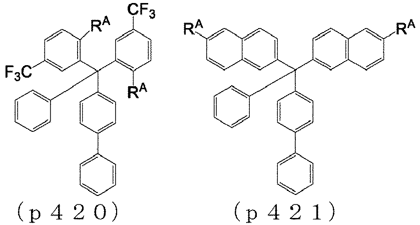

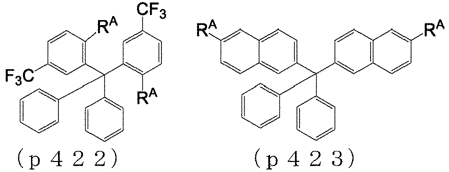

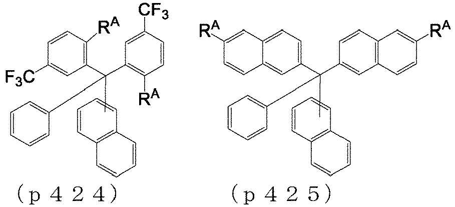

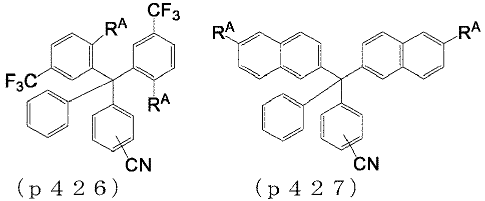

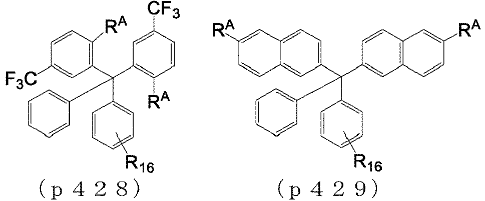

Examples of R₁₆ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group and a triacontyloxy group, all of which include isomers. For example, a butyl group includes a n-butyl group, an isobutyl group, a sec-butyl group and a tert-butyl group. The number of R₁₆ may be one or more.

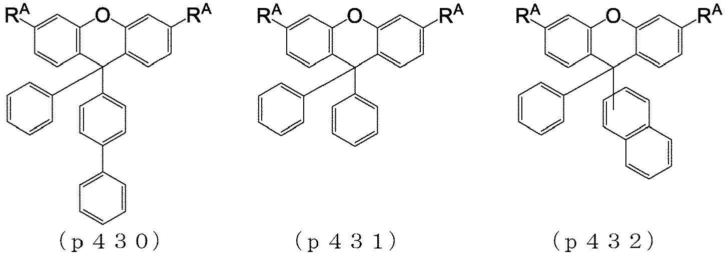

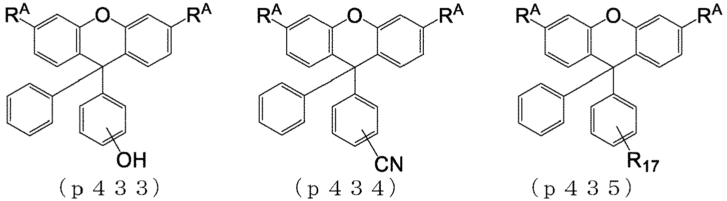

Examples of R₁₇ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group and a triacontyloxy group, all of which include isomers. For example, a butyl group includes a n-butyl group, an isobutyl group, a sec-butyl group and a tert-butyl group. The number of R₁₇ may be one or more.

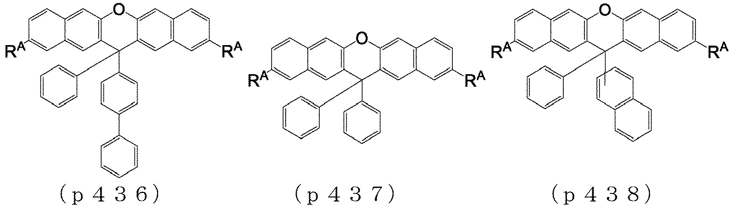

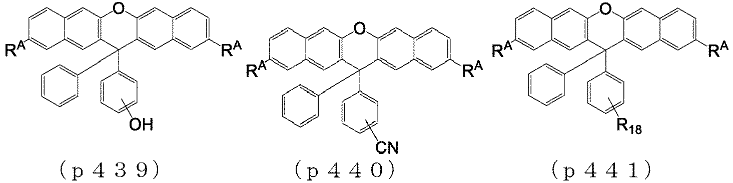

Examples of R₁₈ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group and a triacontyloxy group, all of which include isomers. For example, a butyl group includes a n-butyl group, an isobutyl group, a sec-butyl group and a tert-butyl group. The number of R₁₈ may be one or more.

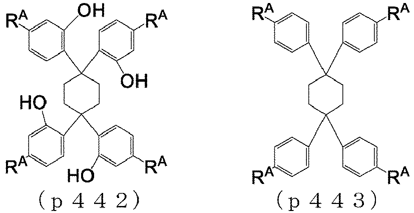

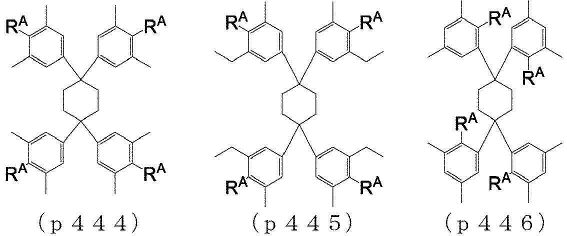

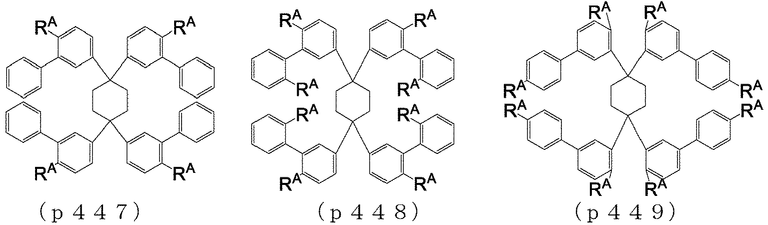

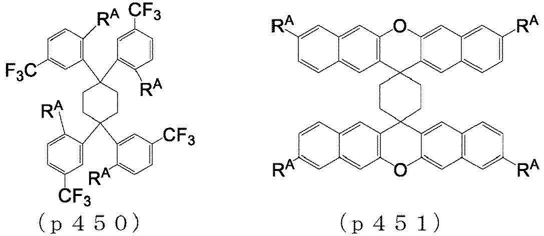

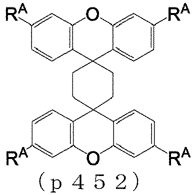

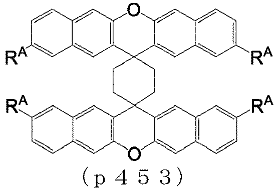

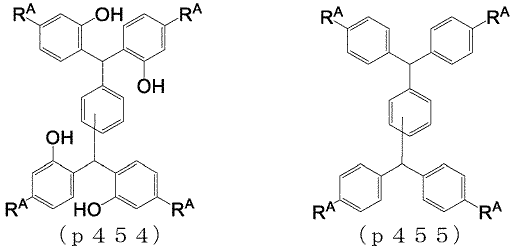

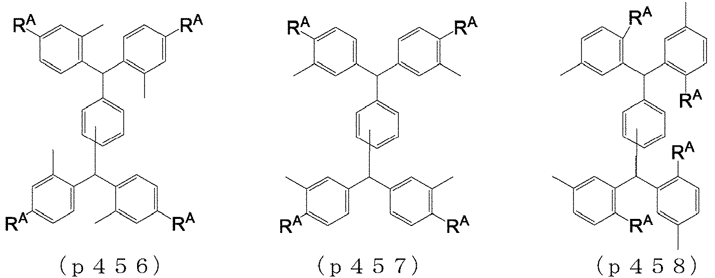

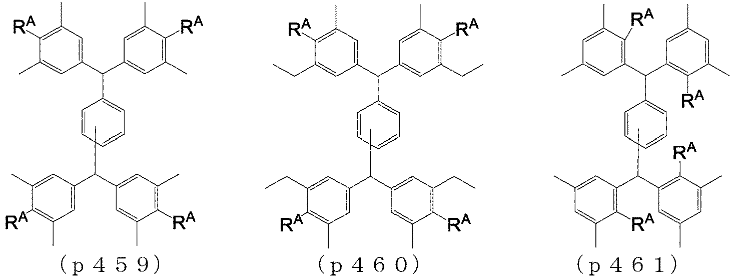

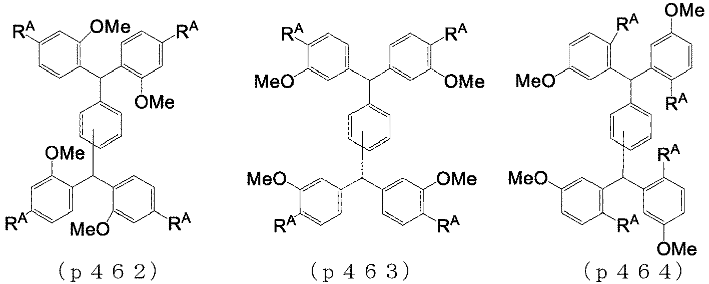

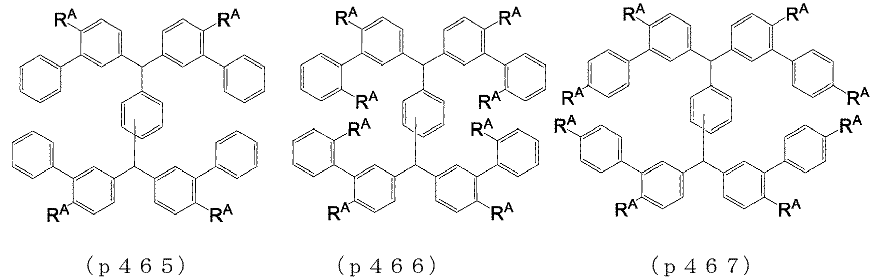

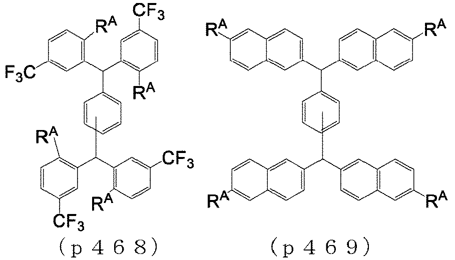

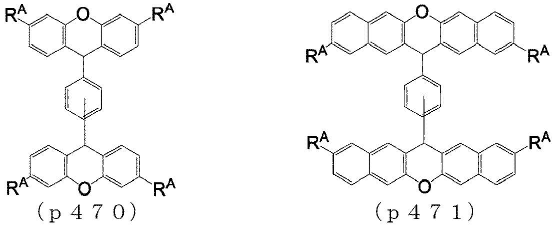

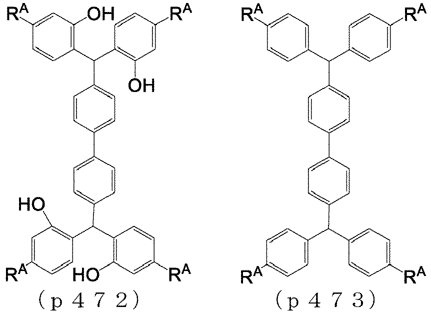

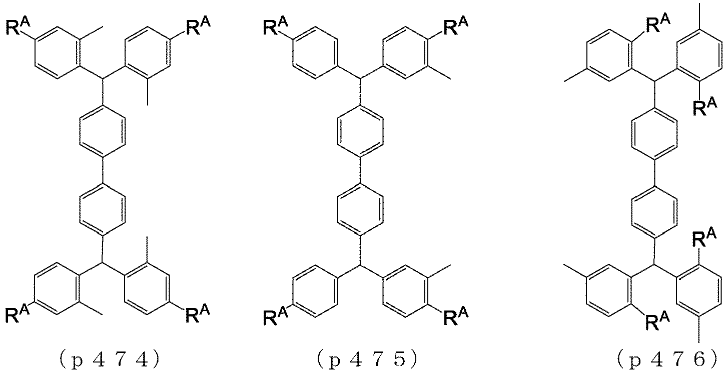

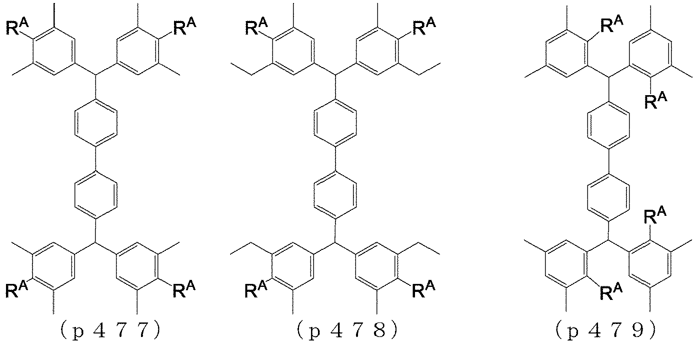

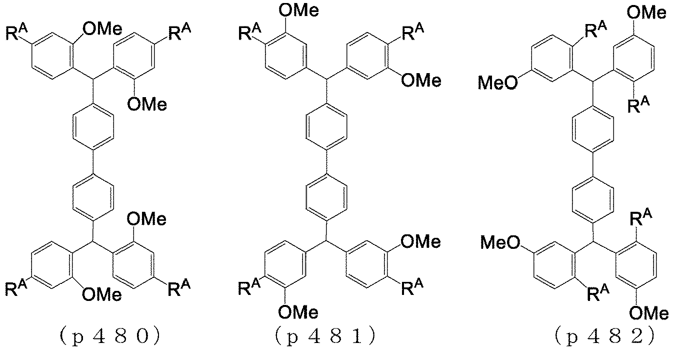

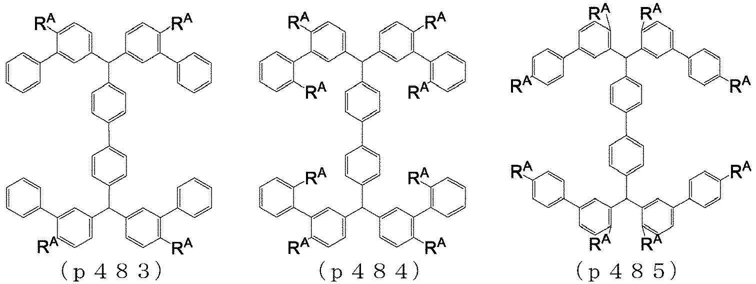

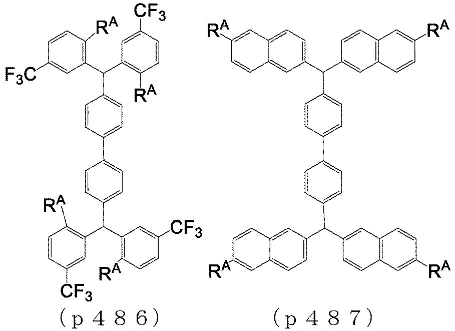

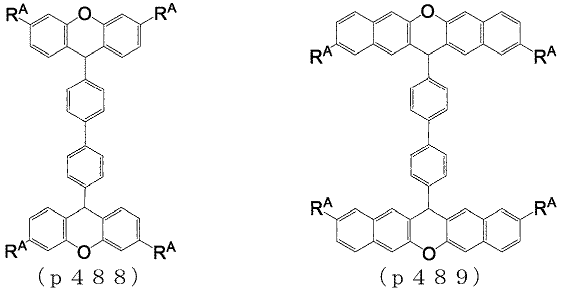

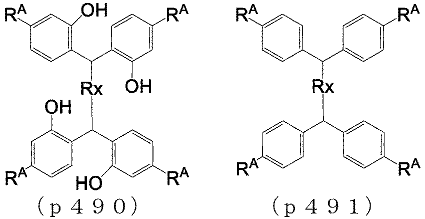

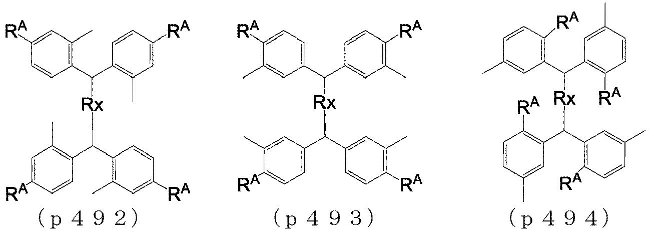

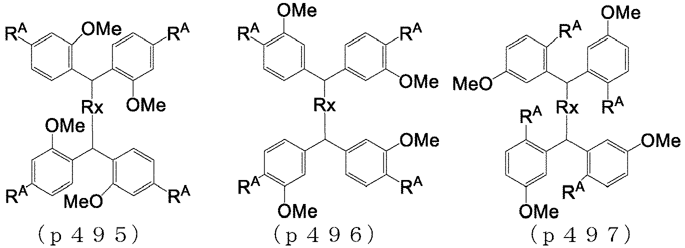

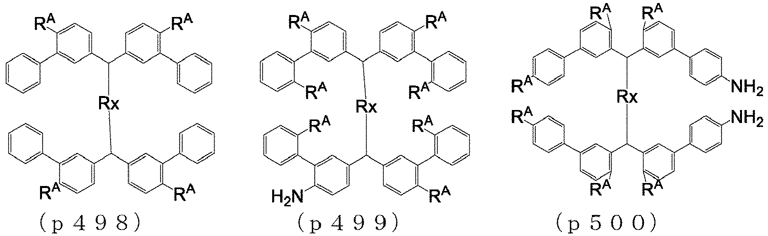

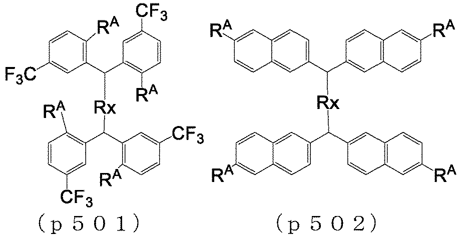

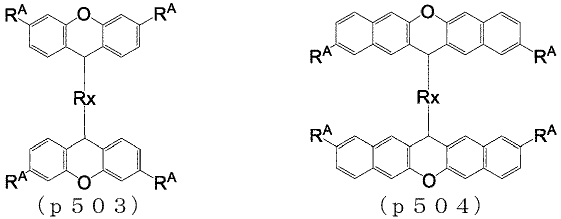

In the above formulas, examples of Rx include a divalent alkylene group obtained by removing one hydrogen atom from a monovalent alkyl group such as a methyl group, an ethyl group, a propyl group, a butyl group, a pentyl group, a hexyl group, a heptyl group, an octyl group, a nonyl group, a decyl group, an undecyl group, a dodecyl group and a triacontyl group, and a divalent group obtained by removing one hydrogen atom from a monovalent group having a alicyclic structure such as cyclopropyl, cyclobutyl, cyclopentyl, cyclohexyl, cycloheptyl, cyclooctyl, cyclononyl, cyclodecyl, cycloundecyl, cyclododecyl, cyclotriacontyl, norbornyl and adamantyl.

From the viewpoint of even further excellent structure forming ability, it is preferable that the compound represented by the formula (1) be any of compounds represented by the following formulas (MIBiA-1 to 34) and (MABiA-1 to 34).

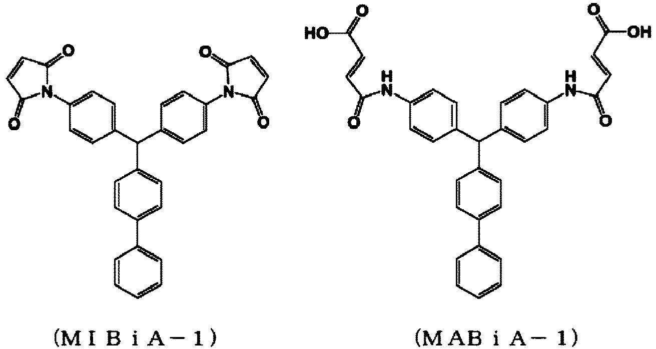

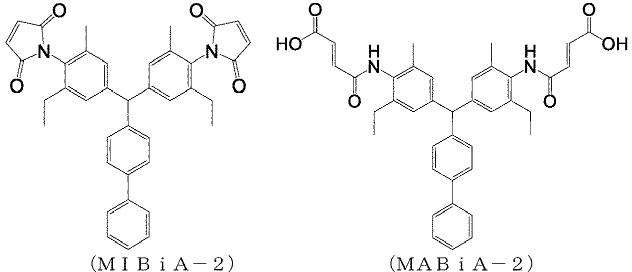

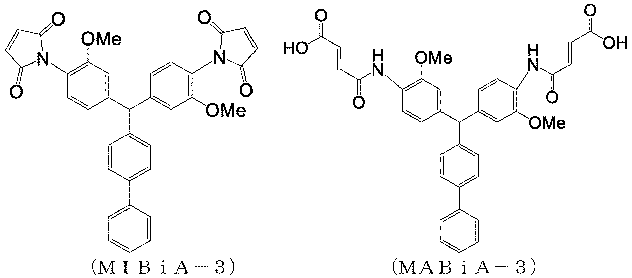

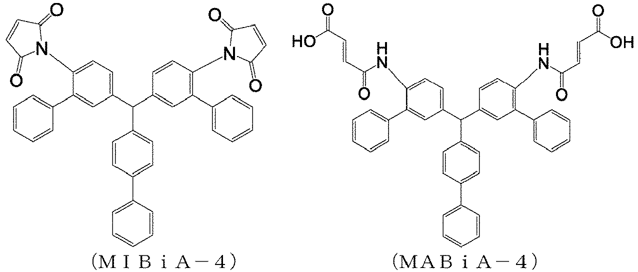

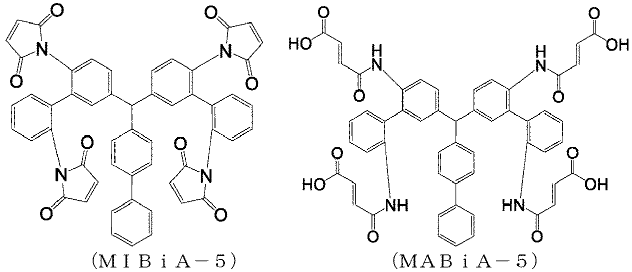

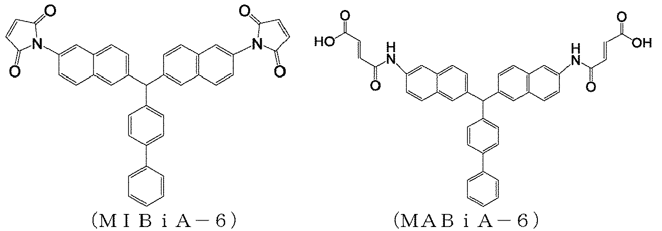

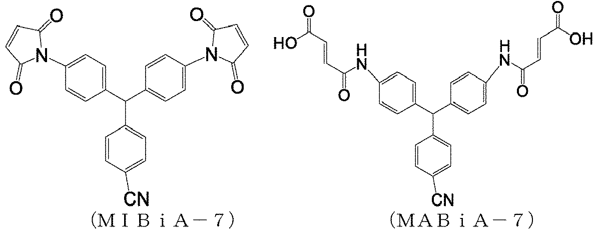

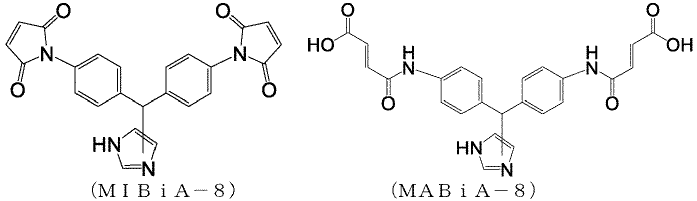

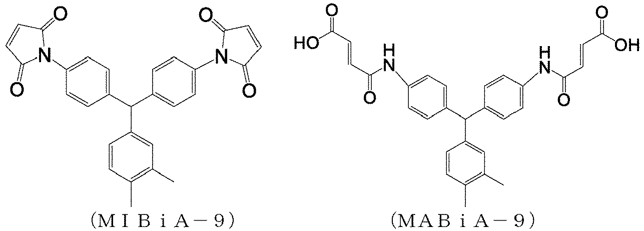

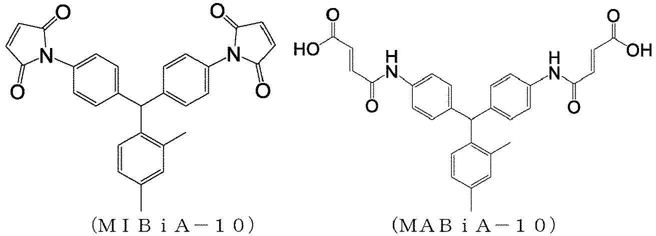

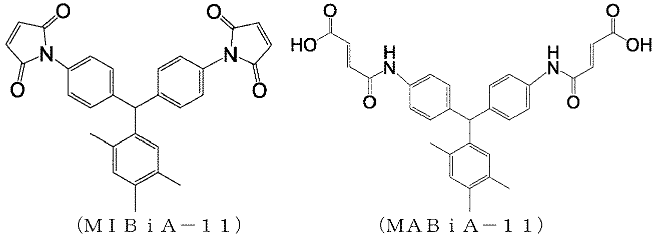

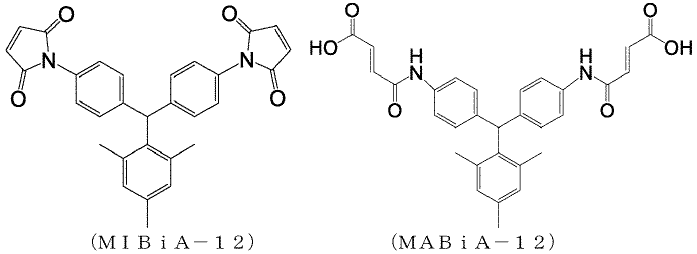

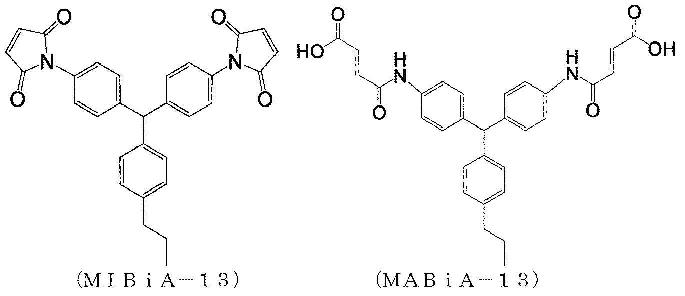

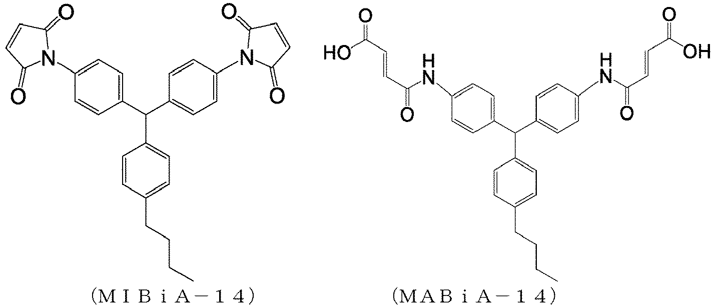

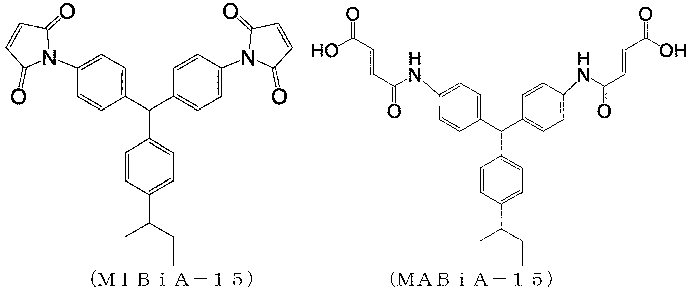

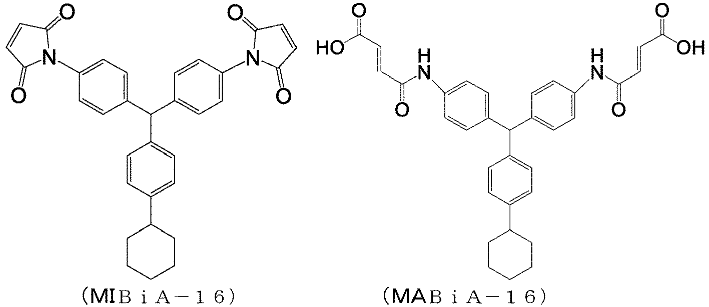

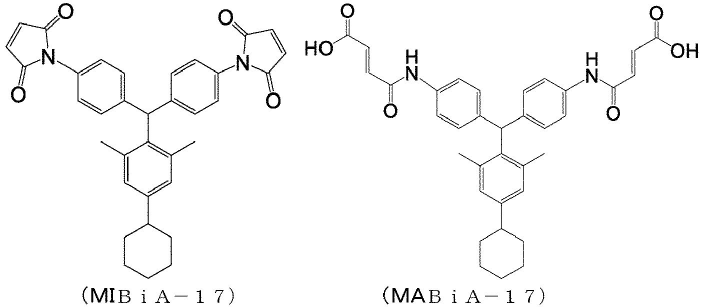

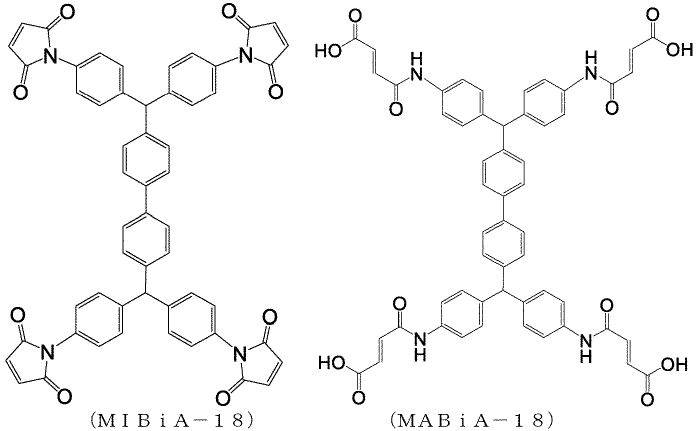

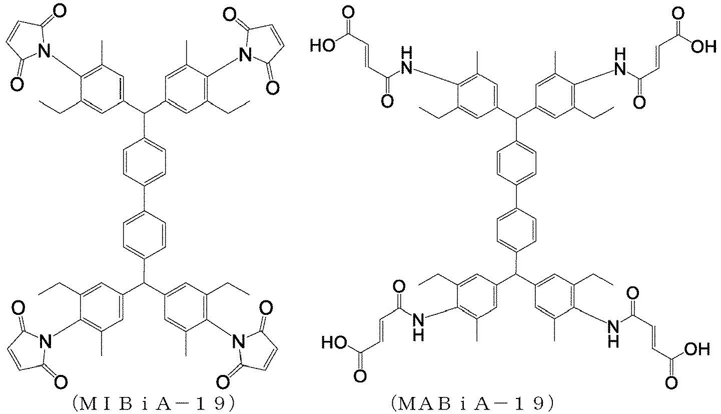

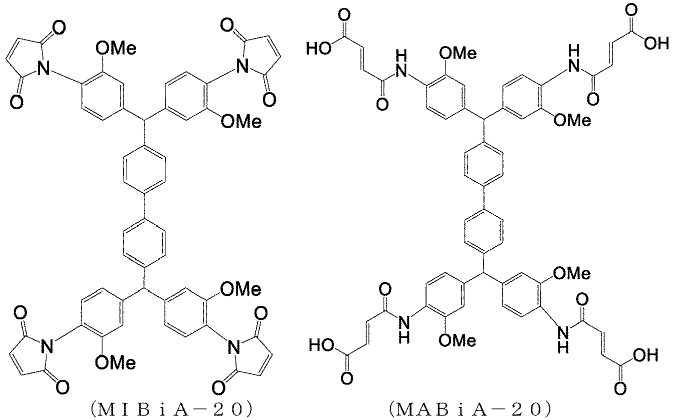

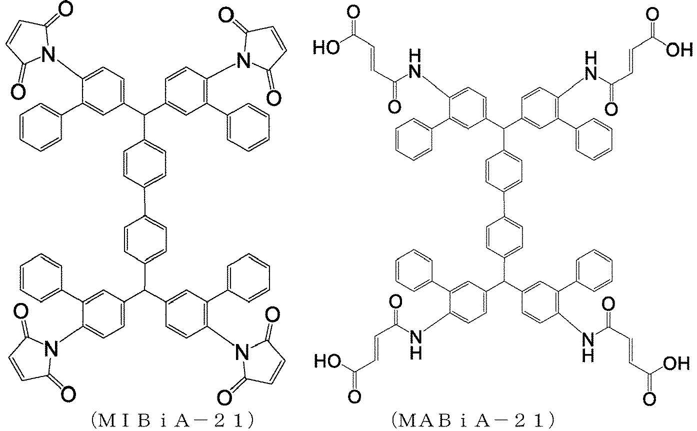

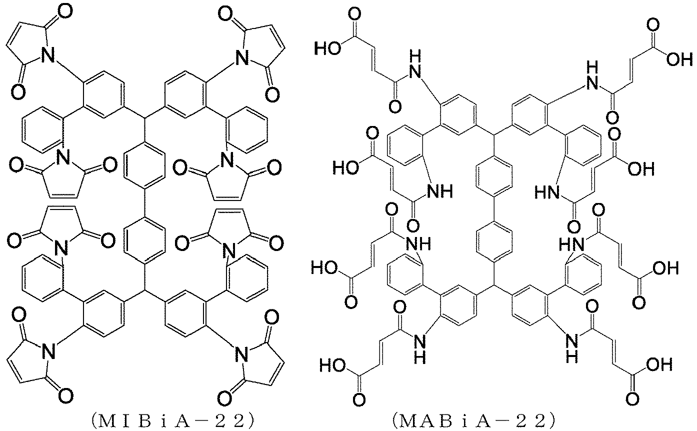

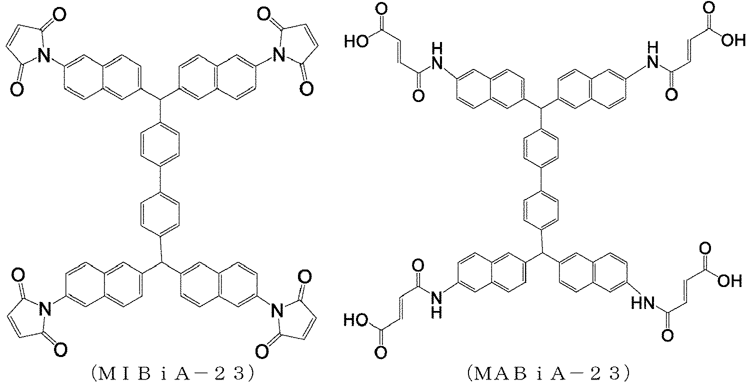

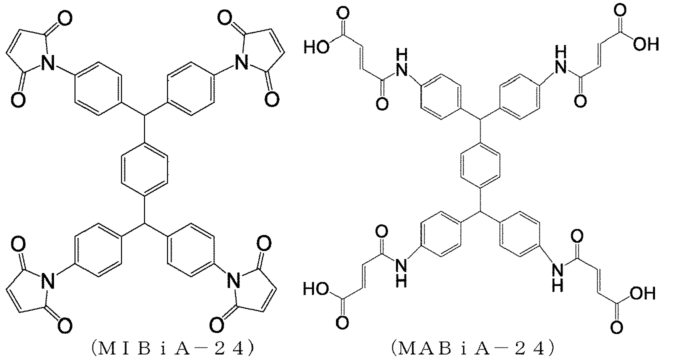

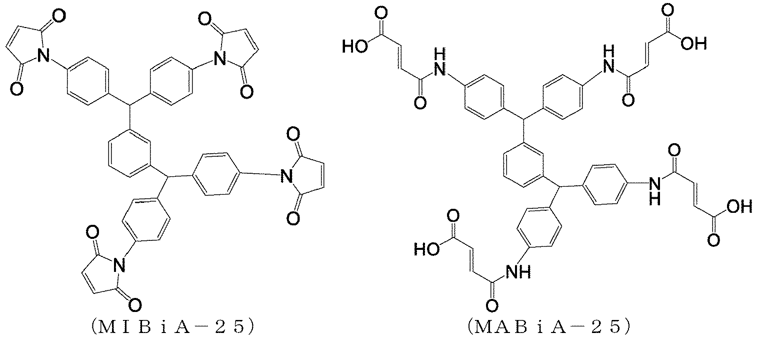

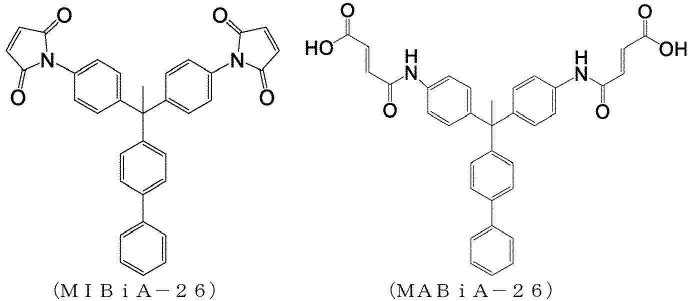

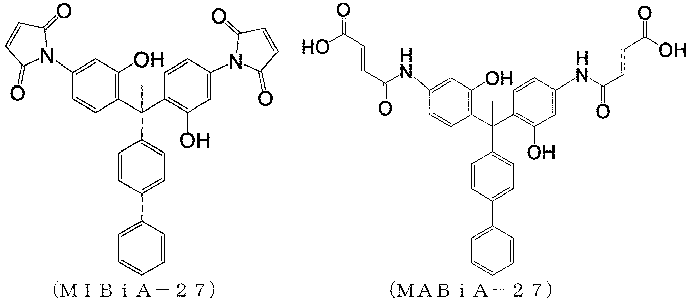

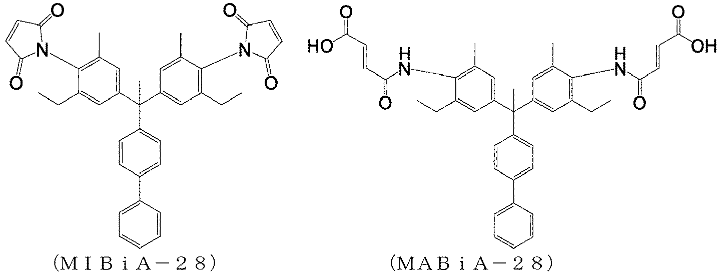

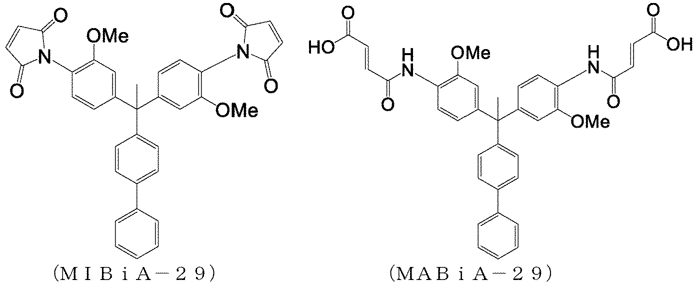

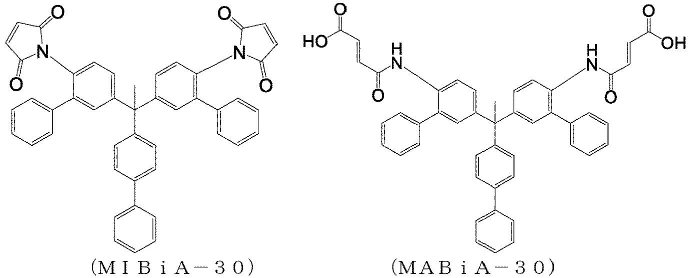

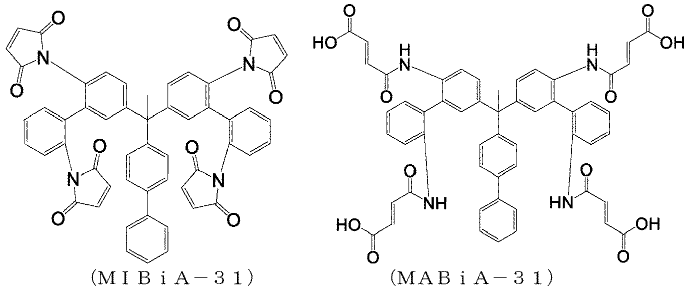

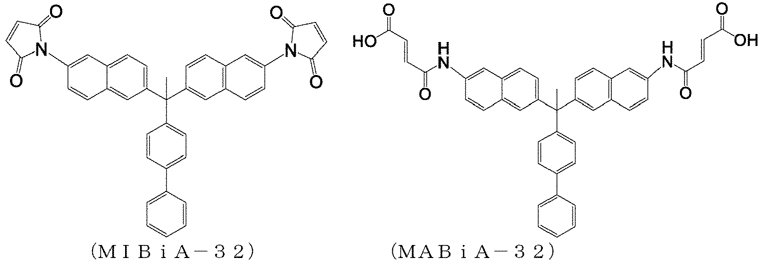

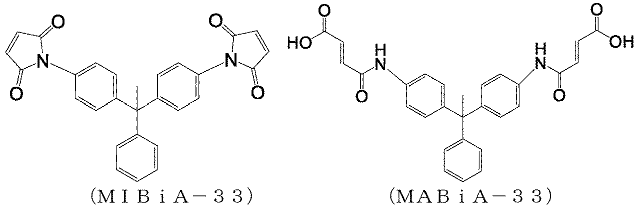

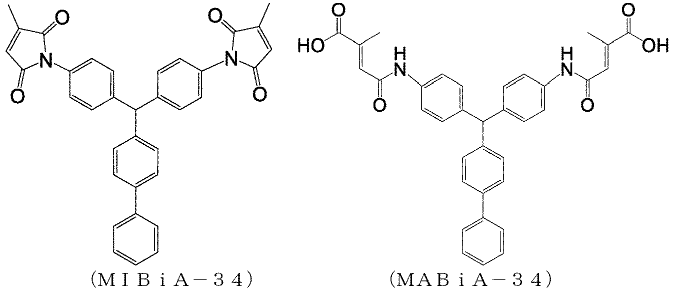

### [Compound represented by formula (1) ((poly)imide compound or (poly)amic acid)]

A method for producing a compound represented by any of the formula (0), the formula (1), the formula (2) and the formula (3) of the present embodiment includes subjecting an aniline and an aldehyde and/or ketone as reaction raw materials to a polycondensation reaction in the presence of an acid catalyst (polycondensation step). For more detailed polycondensation reaction methods, reference can be made to, for example, the methods described in Tetrahedron Letters; Vol. 46 (2005); p. 1119-1122 or the like. The polycondensation step may be carried out at normal pressure, for example.

### [Polycondensation step]

Hereinafter, the polycondensation step will be described.

### (1. Aniline)

Examples of the aniline include an aniline represented by the following formula (0-a), and it is preferably an aniline represented by the following formula (1-b).

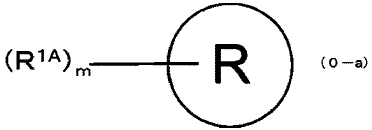

In the above formula, R^{1A}, m and R are as defined in R^{1A}, m and R in the formula (0), respectively.

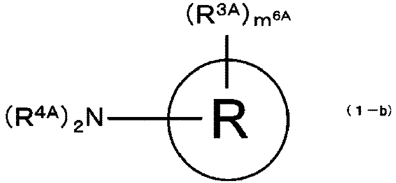

In the above formula, each R^{4A} is independently any of a hydrogen atom, a linear or branched, or cyclic alkyl group having 1 to 30 carbon atoms and optionally having a substituent, an aryl group having 6 to 30 carbon atoms and optionally having a substituent, and a crosslinkable group having 2 to 30 carbon atoms and optionally having a substituent, and when R^{4A} is any of the alkyl group, the aryl group and the crosslinkable group, at least one bond selected from the group consisting of an ether bond, a ketone bond and an ester bond may be contained; and R^{3A}, m^{6A} and R are as defined in R^{3A}, m^{6A} and R in the formula (1), respectively. Examples of the alkyl group include the alkyl groups exemplified as the alkyl group in (1-1-1a. R^{X1}). Examples of the aryl group include the aryl groups exemplified as the aryl group in (1-1-1a. R^{X1}). Examples of the crosslinkable group include the crosslinkable groups exemplified as the crosslinkable group in (1-1-1a. R^{X1}).

Examples of the aniline include an aromatic amine optionally having a substituent and/or a derivative thereof. Specific examples of the aniline include aniline, toluidine, trimethylaniline, anisidine, hydroxyphenylamine, phenylaniline, biphenyldiamine, (trifluoromethyl)aniline, aminophenol, naphthylamine, dimethylaminobenzene and acetanilide. The site of the substituent is not particularly limited and may be any of position 2, position 3, position 4, position 5 and position 6, and the number of the substituent may be one or may be multiple. These anilines can be used alone as one kind or can be used in combination of two or more kinds. Among them, it is preferable to use any of aniline, dimethylaminobenzene and aminophenol from the viewpoint of the stable supply of raw materials.

### (2. Aldehyde)

Examples of the aldehyde include a compound represented by the following formula (0-b), and it is preferably a compound represented by the following formula (3-a).

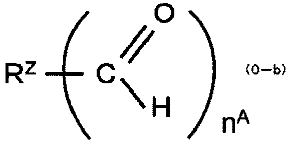

In the above formula, R^{z} and n^{A} are as defined in R^{z} and n^{A} in the formula (0), respectively.

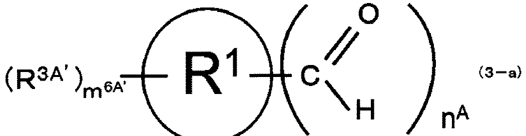

In the above formula, R^{3A'} is any of a linear or branched, or cyclic alkyl group having 1 to 30 carbon atoms and optionally having a substituent, an amino group optionally having a substituent, an aryl group having 6 to 30 carbon atoms and optionally having a substituent, and a crosslinkable group having 2 to 30 carbon atoms and optionally having a substituent; each R¹ independently represents any of a benzene ring, a naphthalene ring and an anthracene ring; and n^{A} is as defined in n^{A} in the formula (1). Examples of the alkyl group include the alkyl groups exemplified as the alkyl group in (1-1-1a. R^{X1}). Examples of the aryl group include the aryl groups exemplified as the aryl group in (1-1-1a. R^{X1}). Examples of the crosslinkable group include the crosslinkable groups exemplified as the crosslinkable group in (1-1-1a. R^{X1}).

Specific examples of the formaldehyde include formaldehyde, trioxane, paraformaldehyde, benzaldehyde, acetaldehyde, propylaldehyde, phenylacetaldehyde, phenylpropylaldehyde, hydroxybenzaldehyde, chlorobenzaldehyde, nitrobenzaldehyde, methylbenzaldehyde, dimethylbenzaldehyde, ethylbenzaldehyde, propylbenzaldehyde, butylbenzaldehyde, fluorobenzaldehyde, biphenylaldehyde, naphthaldehyde, anthracenecarbaldehyde, phenanthrenecarbaldehyde, pyrenecarbaldehyde and furfural. These formaldehydes can be used alone as one kind or can be used in combination of two or more kinds. Among them, it is preferable to use at least one kind selected from the group consisting of benzaldehyde, phenylacetaldehyde, phenylpropylaldehyde, hydroxybenzaldehyde, chlorobenzaldehyde, nitrobenzaldehyde, methylbenzaldehyde, ethylbenzaldehyde, butylbenzaldehyde, cyclohexylbenzaldehyde, biphenylaldehyde, naphthaldehyde, anthracenecarbaldehyde, phenanthrenecarbaldehyde, pyrenecarbaldehyde and furfural from the viewpoint of being capable of providing a (poly)imide compound or (poly)amic acid to be obtained with further higher heat resistance, and it is preferable to use at least one kind selected from the group consisting of benzaldehyde, hydroxybenzaldehyde, chlorobenzaldehyde, nitrobenzaldehyde, methylbenzaldehyde, ethylbenzaldehyde, butylbenzaldehyde, cyclohexylbenzaldehyde, biphenylaldehyde, naphthaldehyde, anthracenecarbaldehyde, phenanthrenecarbaldehyde, pyrenecarbaldehyde and furfural from the viewpoint of being capable of providing a (poly)imide compound or (poly)amic acid to be obtained with further higher etching resistance. It is also preferable to use an aldehyde having an aromatic ring from the viewpoint of being capable of providing a (poly)imide compound or (poly)amic acid to be obtained with further higher heat resistance and refractive index.

### (3. Ketone)

Examples of the ketone include a ketone represented by the following formula (0-C).

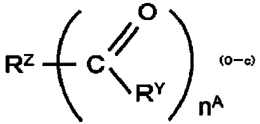

In the above formula, R^{Y}, R^{Z} and n^{A} are as defined in R^{Y}, R^{Z} and n^{A} in the formula (2), respectively.

The ketone may be a ketone represented by the following formula (cyl-c), (cy2-c), (cy3-c) or (cy4-c).

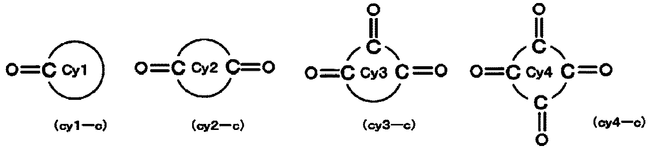

In the above formulas, cy1, cy2, cy3 and cy4 are as defined in cy1, cy2, cy3 and cy4 in the above formulas (cy1), (cy2), (cy3) and (cy4), respectively.

Specific examples of the ketone include acetone, methyl ethyl ketone, cyclobutanone, cyclopentanone, cyclohexanone, norbornanone, tricyclohexanone, tricyclodecanone, adamantanone, fluorenone, benzofluorenone, acenaphthenequinone, acenaphthenone, anthraquinone, acetophenone, diacetylbenzene, triacetylbenzene, acetonaphthone, diphenylcarbonylnaphthalene, phenylcarbonylbiphenyl, diphenylcarbonylbiphenyl, benzophenone, diphenylcarbonylbenzene, triphenylcarbonylbenzene, benzonaphthone, diphenylcarbonylnaphthalene, phenylcarbonylbiphenyl and diphenylcarbonylbiphenyl. These ketones can be used alone as one kind or can be used in combination of two or more kinds. Among them, it is preferable to use at least one kind selected from the group consisting of cyclopentanone, cyclohexanone, norbornanone, tricyclohexanone, tricyclodecanone, adamantanone, fluorenone, benzofluorenone, acenaphthenequinone, acenaphthenone, anthraquinone, acetophenone, diacetylbenzene, triacetylbenzene, acetonaphthone, diphenylcarbonylnaphthalene, phenylcarbonylbiphenyl, diphenylcarbonylbiphenyl, benzophenone, diphenylcarbonylbenzene, triphenylcarbonylbenzene, benzonaphthone, diphenylcarbonylnaphthalene, phenylcarbonylbiphenyl and diphenylcarbonylbiphenyl from the viewpoint of being capable of providing a (poly)imide compound or (poly)amic acid to be obtained with further higher heat resistance, and it is preferable to use at least one kind selected from the group consisting of acetophenone, diacetylbenzene, triacetylbenzene, acetonaphthone, diphenylcarbonylnaphthalene, phenylcarbonylbiphenyl, diphenylcarbonylbiphenyl, benzophenone, diphenylcarbonylbenzene, triphenylcarbonylbenzene, benzonaphthone, diphenylcarbonylnaphthalene, phenylcarbonylbiphenyl and diphenylcarbonylbiphenyl from the viewpoint of being capable of providing a (poly)imide compound or (poly)amic acid to be obtained with further higher etching resistance. It is also preferable to use a ketone having an aromatic ring from the viewpoint of being capable of providing a (poly)imide compound or (poly)amic acid to be obtained with further higher heat resistance and refractive index.

### (4. Acid Catalyst)

For the acid catalyst, those publicly known can be used, and examples thereof include an inorganic acid and an organic acid. Examples of the inorganic acid include hydrochloric acid, sulfuric acid, phosphoric acid, hydrobromic acid and hydrofluoric acid. Examples of the organic acid include oxalic acid, malonic acid, succinic acid, adipic acid, sebacic acid, citric acid, fumaric acid, maleic acid, formic acid, p-toluenesulfonic acid, methanesulfonic acid, trifluoroacetic acid, dichloroacetic acid, trichloroacetic acid, trifluoromethanesulfonic acid, benzenesulfonic acid, naphthalenesulfonic acid and naphthalenedisulfonic acid. The acid catalyst may be a Lewis acid such as zinc chloride, aluminum chloride, iron chloride and boron trifluoride, or a solid acid such as tungstosilicic acid, phosphotungstic acid, silico molybdic acid and phosphomolybdic acid. Among them, an organic acid and a solid acid are preferable from the viewpoint of production (from the viewpoint of easy availability and handleability), and hydrochloric acid or sulfuric acid is more preferable. These acid catalysts can be used alone as one kind or can be used in combination of two or more kinds. The amount of the acid catalyst used can be arbitrarily set according to, for example, the kind of the raw materials used and the catalyst used and moreover the reaction conditions, and it may be about 0.01 to 100 parts by mass based on 100 parts by mass of the entire reaction raw materials.

### (5. Reaction Solvent)

During the polycondensation step, reaction raw materials may be subjected to reaction in a solvent. The solvent may be any solvent as long as it allows sufficient progress of reaction between the aniline and the aldehyde and/or ketone, which are used as the reaction raw materials. Examples of the solvent include water, methanol, ethanol, propanol, butanol, tetrahydrofuran, dioxane, ethylene glycol dimethyl ether, ethylene glycol diethyl ether and a mixed solvent thereof. These solvents can be used alone as one kind or can be used in combination of two or more kinds.

The amount of the solvent used can be arbitrarily set according to, for example, the kind of the raw materials used and the catalyst used and moreover the reaction conditions, and it may be about 0 to 2000 parts by mass based on 100 parts by mass of the entire reaction raw materials.

The reaction temperature during the polycondensation reaction can be arbitrarily selected according to the reactivity of reaction raw materials, but it may be normally within the range of about 10 to 200°C. In order to obtain the (poly)amine compound of the present embodiment, a higher reaction temperature is more preferable. Specifically, the range of 60 to 200°C is preferable.

In the polycondensation step, the aniline, the aldehyde and/or ketone, and the catalyst may be charged in one portion to allow reaction, or the aniline, the aldehyde and/or ketone may be sequentially added dropwise in the presence of the catalyst to allow reaction. After the polycondensation reaction terminates, the obtained compound may be isolated by a publicly known method. For example, by elevating the temperature of the reaction vessel to 130 to 230°C in order to remove unreacted raw materials and catalyst present in the system, and by removing volatile portions at about 1 to 50 mmHg, the (poly)amine compound can be obtained.

It is preferable to add the aniline in an amount of 1.0 mole or more and to add the acid catalyst in the range of 0.001 to 1 mole, based on 1.0 mole of the aldehyde and/or ketone. In addition, it is preferable to conduct the reaction at normal pressure at a reaction temperature of 50 to 150°C and for a reaction time of about 20 minutes to 100 hours.

After the reaction terminates, the (poly)amine compounds may be isolated by a publicly known method. In the isolation method, for example, the reaction solution is first concentrated, and pure water is added to precipitate the reaction product. Next, the precipitated reaction product is cooled to room temperature, and then separated by filtration. The solid matter obtained by the separation is further filtered and dried. The dried reaction product is separated and purified by column chromatography into the product and a byproduct. Furthermore, the reaction product separated and purified is subjected to solvent distillation, followed by filtration and drying, thereby obtaining the (poly)amine compound of the present embodiment, which is the target compound.

With respect to a method for producing the (poly)maleamic acid or (poly)citraconamic acid of the present embodiment, they can be produced by further subjecting the (poly)amine compound obtained in the polycondensation step to a reaction with maleic anhydride or citraconic anhydride in an organic solvent.

Examples of the organic solvent may include N,N-dimethylformamide, N,N-dimethylacetamide, N,N-dimethylmethoxyacetamide, N,N-diethylmethoxyacetamide, N-methyl-2-pyrrolidone, N-methylcaprolactam, 1,2-dimethoxyethane, diethylene glycol dimethyl ether, diethylene glycol ethyl methyl ether, diethylene glycol diethyl ether, tetrahydrofuran, 1,3-dioxane, 1,4-dioxane, pyridine, picoline, dimethyl sulfoxide, dimethyl sulfone, tetramethylurea, ethyl acetate, methyl acetate, 2-butanone, toluene, xylene and mesitylene. These organic solvents can be used alone or in mixture of two or more kinds. The concentration of the reaction raw material is usually 2 to 50 wt%, and is preferably 5 to 30 wt%, and the reaction temperature is usually 60°C or less, and is preferably 50°C or less. The reaction pressure is not particularly limited, and the reaction can usually be performed at normal pressure. In addition, the reaction time is usually 0.5 to 24 hours. Through such a reaction, the (poly)amic acid of the present invention can be obtained.

With respect to a method for producing the (poly)maleimide compound or (poly)citraconimide compound of the present embodiment, they can be produced by subjecting the (poly)maleamic acid or (poly)citraconamic acid to ring closure with dehydration and imidation. As a method for the imidation, a publicly known reaction can be utilized, without limitations, such as a method in which a dehydrating agent such as acetic anhydride and a catalyst are added (for example, Japanese Patent Application Laid-Open No. 4-261411) or a method in which the (poly)maleamic acid or (poly)citraconamic acid is subjected to cyclodehydration and imidation by heating it in an aromatic hydrocarbon based solvent such as toluene or xylene in the presence of an acid catalyst (for example, Japanese Patent Application Laid-Open No. 7-118230).

Examples of the solvent for the imidation reaction in which a dehydrating agent is added include a fatty acid ester such as butyl formate, isobutyl formate, t-butyl formate, ethyl acetate, propyl acetate, isopropyl acetate, butyl acetate, isobutyl acetate, t-butyl acetate, methyl propionate and ethyl propionate; an ethylene glycol monoalkyl ether such as methylcellosolve and ethylcellosolve; an ether such as tetrahydrofuran, dioxane, dipropyl ether, diethylene glycol dimethyl ether, diethylene glycol ethyl methyl ether and diethylene glycol diethyl ether. However, as long as it is a solvent in which the bismaleamic acid of the present invention is dissolved, examples of the solvent are not limited to the above. Also, these reaction solvents can be used alone or in mixture of two or more kinds.

A carboxylic anhydride used as the dehydrating agent is practically limited to acetic anhydride for economic reasons, but there is no particular need for limitations. Also, as the catalyst used for the ring closure reaction with dehydration, a carbonate, a bicarbonate, a sulfate, a nitrate, a phosphate, an acetate, a formate or a higher fatty acid salt of an alkali metal (preferably sodium, potassium or lithium), or a salt or halide of a transition metal such as nickel, cobalt or manganese is used, and potassium acetate is preferable. The total amount of the dehydrating agent and the catalyst to be added is preferably 0.1 to 4.0 moles based on 1 mole of the amic acid group. Furthermore, if necessary, a tertiary amine or a pyridine derivative can be arbitrarily added in the ring closure reaction with dehydration for the purpose of accelerating the reaction. Examples thereof include triethylamine, tripropylamine, tributylamine, triphenylamine, pyridine, methylpyridine and 2,6-dimethylpyridine. Also, the amount of the reaction promoting agent added is about 0.1 to 2.0 moles based on 1 mole of the amic acid group. The reaction temperature is 0 to 60°C, and is preferably 10 to 40°C, and the reaction is performed by allowing the reaction raw materials to react for 0.5 to 100 hours.

Examples of the solvent for the imidation reaction in which the (poly)maleamic acid or (poly)citraconamic acid is heated in the presence of the acid catalyst include an aromatic hydrocarbon based solvent such as benzene, toluene, xylene and ethylbenzene, and if necessary, an aprotic polar solvent such as N,N-dimethylformamide, N,N-dimethylacetamide, N-methyl-2-pyrrolidone and dimethyl sulfoxide may be added. The amount of the aprotic polar solvent used in the reaction solvent is 0.05 to 20% by weight, and is preferably 1 to 30% by weight, and a bismaleimide is obtained by conducting cyclodehydration while azeotropically removing the water produced at a temperature range of 100 to 200°C.

Examples of the acid catalyst used in the cyclodehydration reaction include phosphoric acid, polyphosphoric acid, sulfuric acid and p-toluenesulfonic acid. The amount of these acid catalysts used is in the range of 0.001 to 0.1 moles, preferably 0.005 to 0.05 moles based on 1 mole of the amic acid group.

In addition, either method for carrying out the imidation reaction after isolating the (poly)maleamic acid or (poly)citraconic acid, which is obtained by allowing the above (poly)amine to react with maleic anhydride or citraconic anhydride, or method for carrying out the imidation reaction as is without isolating the bismaleamic acid can be used.

### [Resin]

A resin of the present embodiment comprises a monomer unit derived from the compound of the present embodiment ((poly)maleimide compound, (poly)citraconimide compound, (poly)maleamic acid or (poly)citraconamic acid). The resin of the present embodiment has excellent structure forming ability (for example, film forming ability), heat resistance and solubility due to the fact that it comprises the monomer unit of the present embodiment.

The resin of the present embodiment may be composed of the monomer unit of the present embodiment alone, may comprise another monomer unit that is copolymerizable with the compound ((poly)imide compound or (poly)amic acid), or may comprise a unit derived from a crosslinking compound.

Examples of the monomer unit that constitutes the resin of the present embodiment include the following formula (4). It is preferable that X be O (oxygen) from the viewpoint of even further improvement in solubility in an organic solvent.

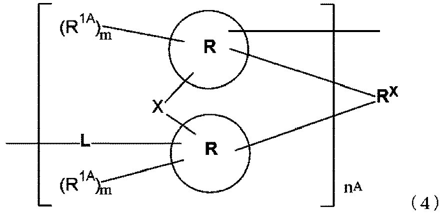

In the above formula (4), L is a linear or branched linking group having 1 to 30 carbon atoms, or a single bond. R^{X}, R^{1A}, X, R, m and n^{A} are as defined above.

When L is a linking group, examples of the linking group include a group (residue) derived from a compound that is capable of oligomerizing or polymerizing the (poly)imide compound or (poly)amic acid of the present embodiment. The group derived from a compound that is capable of oligomerization or polymerization will be mentioned later.

### [Monomer unit represented by formula (4)]

From the viewpoint of further improvement in solubility and heat resistance, the monomer unit that constitutes the resin, represented by the above formula (4), is preferably a monomer unit represented by the following formula (4a-1), and is more preferably a monomer unit represented by the following formula (4b-1).

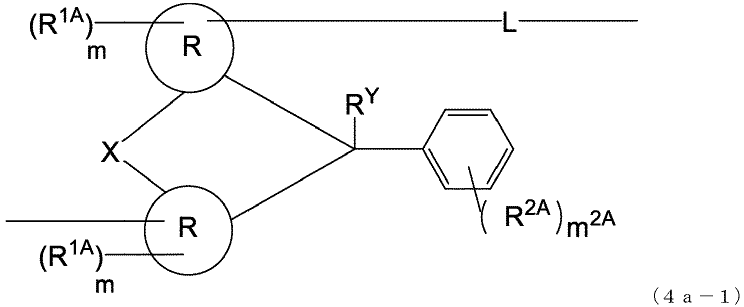

In the above formula (4a-1), X, R, R^{1A}, m, R^{Y} and L are as defined above; R^{2A} represents any of a hydrogen atom, a linear or branched, or cyclic alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, and a halogen atom (for example, a fluorine atom, a chlorine atom and a bromine atom). In addition, m^{2A} represents an integer of 1 to 5.

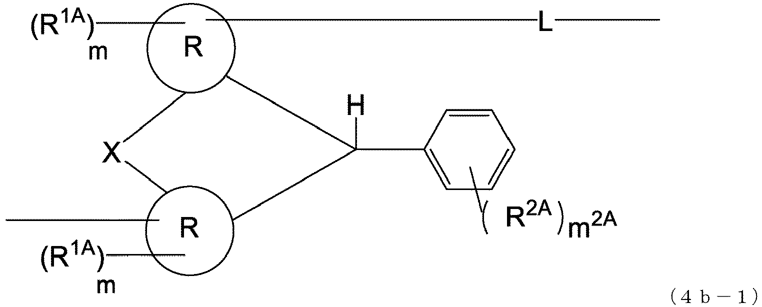

In the above formula (4b-1), X, R, R^{1A}, R^{2A}, m and m^{2A} are as defined above.

From the viewpoint of further improvement in solubility, the monomer unit that constitutes the resin, represented by the above formula (4), is preferably a monomer unit represented by the following formula (4a-2), and is more preferably a monomer unit represented by the following formula (4b-2).

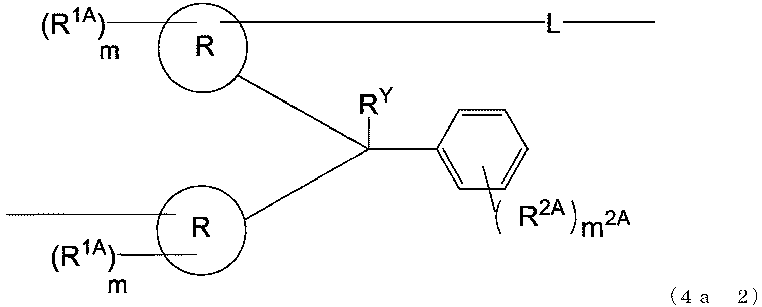

In the above formula (4a-2), R, R^{1A}, m and R^{Y} are as defined above; R^{2A} represents any of a hydrogen atom, a linear or branched, or cyclic alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, and a halogen atom (for example, a fluorine atom, a chlorine atom and a bromine atom). In addition, m^{2A} represents an integer of 1 to 5.

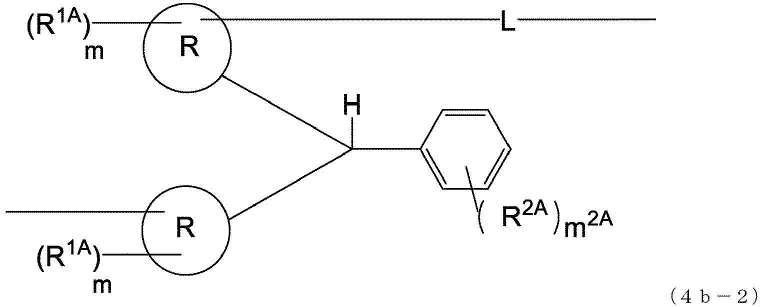

In the above formula (4b-2), X, R, R^{1A}, R^{2A}, m and m^{2A} are as defined above.

### [Monomer unit represented by formula (4a-3)]

It is preferable that the monomer unit represented by the above formula (4a-2) be a monomer unit represented by the following formula (4a-3) from the viewpoint of further improvement in solubility.

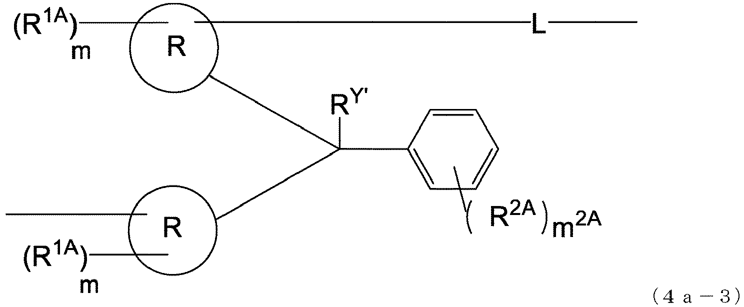

In the above formula (4a-3), R, R^{1A}, R^{2A}, m and m^{2A} are as defined above; and R^{Y'} is R^{Y} except for a hydrogen atom.

### [Monomer unit represented by formula (4b-3)]

It is preferable that the monomer unit represented by the above formula (4b-2) be a monomer unit represented by the following formula (4b-3) from the viewpoint of further improvement in flowability upon film formation.

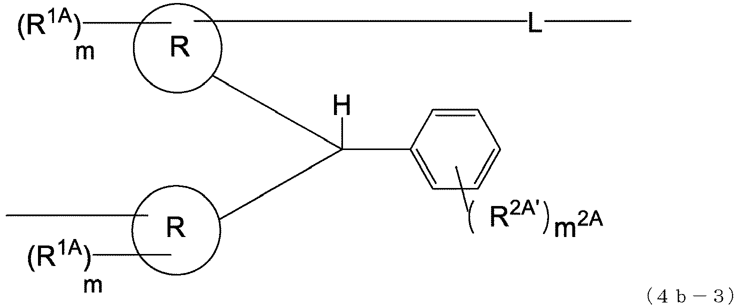

In the above formula (4b-3), R, R^{1A}, m and m^{2A} are as defined above; and R^{2A'} represents a linear or branched, or cyclic alkyl group having 5 or more carbon atoms and optionally having a substituent.

### [Method for producing resin of the present embodiment]

The resin of the present embodiment is obtained by allowing the compound of the present embodiment to react with a crosslinking compound as needed.

The crosslinking compound is not particularly limited as long as it is a compound that is capable of oligomerizing or polymerizing the compound of the present embodiment ((poly)imide compound or (poly)amic acid). Specific examples thereof include an aldehyde, a ketone, a carboxylic acid, a carboxylic acid anhydride, a carboxylic acid halide, a halogen containing compound, an isocyanate and an unsaturated hydrocarbon group containing compound. When L is a linking group in the formula (4), that linking group corresponds to a group derived from these compounds.

Specific examples of the resin of the present embodiment include a resin that has been made novolac by, for example, a condensation reaction between the compound of the present embodiment ((poly)imide compound or (poly)amic acid) and an aldehyde and/or ketone, which is a crosslinking compound.

Here, examples of the aldehyde for making a resin novolac include formaldehyde, trioxane, paraformaldehyde, benzaldehyde, acetaldehyde, propylaldehyde, phenylacetaldehyde, phenylpropylaldehyde, hydroxybenzaldehyde, chlorobenzaldehyde, nitrobenzaldehyde, methylbenzaldehyde, ethylbenzaldehyde, butylbenzaldehyde, biphenylaldehyde, naphthaldehyde, anthracenecarbaldehyde, phenanthrenecarbaldehyde, pyrenecarbaldehyde and furfural. Examples of the ketone for making a resin novolac include the ketones exemplified as the reaction raw material. Among them, a formaldehyde is preferable. These aldehydes and/or ketones can be used alone as one kind or can be used in combination of two or more kinds. The amount of the above aldehyde and/or ketone used is not particularly limited, but it is preferably 0.2 to 5 moles and more preferably 0.5 to 2 moles based on 1 mole of the (poly)amine compound of the present embodiment.

Also, a catalyst can be used in the condensation reaction between the compound of the present embodiment ((poly)imide compound or (poly)amic acid) and the aldehyde and/or ketone. Examples of the acid catalyst used herein include the acid catalysts exemplified in the section of the condensation step.

Also, the amount of the acid catalyst used can be arbitrarily set according to, for example, the kind of the raw materials used and the catalyst used and moreover the reaction conditions. The amount of the acid catalyst used is preferably 0.01 to 100 parts by mass based on 100 parts by mass of the reaction raw materials.

The aldehyde and/or ketone is not necessarily needed in the case of subjecting the compound of the present embodiment ((poly)imide compound or (poly)amic acid) to a copolymerization reaction with a compound having a nonconjugated double bond, such as indene, hydroxyindene, benzofuran, hydroxyanthracene, acenaphthylene, biphenyl, bisphenol, trisphenol, dicyclopentadiene, tetrahydroindene, 4-vinylcyclohexene, norbornadiene, 5-vinylnorborn-2-ene, α-pinene, β-pinene and limonene.

Also, a reaction solvent can be used in the condensation reaction between the compound of the present embodiment ((poly)imide compound or (poly)amic acid) and the aldehyde and/or ketone. Examples of the reaction solvent may include the reaction solvents exemplified in the section of the polycondensation step.

In addition, the amount of the solvent used, reaction temperature, reaction time, isolation method after the reaction and the like can also be exemplified by the amount of the solvent used, reaction temperature, reaction time and isolation method after the reaction, all of which are exemplified in the section of the polycondensation step.

The resin according to the present embodiment may be a homopolymer of the compound of the present embodiment ((poly)imide compound or (poly)amic acid), that is, a polymer composed of the formula (4) alone, but it may also be a copolymer between the (poly)imide compound or (poly)amic acid and a copolymerizable compound (for example, a phenol). Here, examples of the copolymerizable phenol include phenol, cresol, dimethylphenol, trimethylphenol, butylphenol, phenylphenol, diphenylphenol, naphthylphenol, resorcinol, methylresorcinol, catechol, butylcatechol, methoxyphenol, methoxyphenol, propylphenol, pyrogallol and thymol.

The resin of the present embodiment may be copolymerized with a polymerizable monomer other than the above-described further phenol. Examples of the copolymerizable monomer include naphthol, methylnaphthol, methoxynaphthol, dihydroxynaphthalene, indene, hydroxyindene, benzofuran, hydroxyanthracene, acenaphthylene, biphenyl, bisphenol, trisphenol, dicyclopentadiene, tetrahydroindene, 4-vinylcyclohexene, norbornadiene, vinylnorbornene, pinene and limonene. The resin of the present embodiment may be a binary or higher (for example, binary to quaternary) copolymer of the compound of the present embodiment ((poly)imide compound or (poly)amic acid) and the above-described phenol. It may be a binary or higher (for example, binary to quaternary) copolymer of the (poly)imide compound or (poly)amic acid of the present embodiment and the above-described copolymerizable monomer. It may be a ternary or higher (for example, ternary to quaternary) copolymer of the (poly)imide compound or (poly)amic acid of the present embodiment, the above-described phenol, and the above-described copolymerizable monomer.

The weight average molecular weight of the resin of the present embodiment is not particularly limited, but it is preferably 500 to 300,000 and is more preferably 750 to 200,000 in terms of polystyrene. The resin of the present embodiment preferably has a dispersibility (weight average molecular weight Mw/number average molecular weight Mn) within the range of 1.2 to 7 from the viewpoint of enhancing crosslinking efficiency while suppressing volatile components during baking.

The resin of the present embodiment preferably has high solubility in a solvent from the viewpoint of easier application to a wet process, etc. More specifically, in the case of using 1-methoxy-2-propanol (PGME) and/or propylene glycol monomethyl ether acetate (PGMEA) as a solvent, the (poly)imide compound or (poly)amic acid and/or the resin preferably has a solubility of 10% by mass or more in the solvent. Here, the solubility in PGME and/or PGMEA is defined as "mass of the resin / (mass of the resin + mass of the solvent) × 100 (% by mass)". For example, when 10 g of the resin is dissolved in 90 g of PGMEA, the solubility of the resin in PGMEA is "10% by mass or more"; and when 10 g of the resin is not dissolved in 90 g of PGMEA, the solubility is "less than 10% by mass".

### [Composition]

A composition of the present embodiment will be described. The composition contains one kind or two or more kinds of the above-described compound ((poly)imide compound or (poly)amic acid) and/or resin of the present embodiment. To the composition, a publicly known epoxy resin, oxetane resin, compound having a polymerizable unsaturated group, cyanate ester compound, benzoxazine resin, photopolymerization initiator and/or thermal polymerization initiator, photosensitizer, curing agent and the like can also be added.

As the epoxy resin, those publicly known can be generally used. Examples thereof include, for example, a bisphenol A-based epoxy resin, a bisphenol F-based epoxy resin, a biphenyl-based epoxy resin, a phenol novolac-based epoxy resin, a cresol novolac-based epoxy resin, a xylene novolac-based epoxy resin, triglycidyl isocyanurate, an alicyclic epoxy resin, a dicyclopentadiene novolac-based epoxy resin, a biphenyl novolac-based epoxy resin, and epoxy resins having a PPE skeleton described in Japanese Patent Application Laid-Open No. 2003-155340 and Japanese Patent Application Laid-Open No. 2003-212990. These epoxy resins are used as one kind, or in mixture of two or more kinds.

As the oxetane resin, those publicly known can be generally used. Examples thereof include oxetane, an alkyloxetane such as 2-methyloxetane, 2,2-dimethyloxetane, 3-methyloxetane and 3,3-dimethyloxetane, 3-methyl-3-methoxymethyloxetane, 3,3'-di(trifluoromethyl)perfluorooxetane, 2-chloromethyloxetane, 3,3-bis(chloromethyl)oxetane, OXT-101 (trade name manufactured by Toagosei Co., Ltd.) and OXT-121 (trade name manufactured by Toagosei Co., Ltd.). These oxetane resins are used as one kind, or in mixture of two or more kinds.

When an epoxy resin and/or oxetane resin is used in the composition of the present embodiment, an epoxy resin curing agent and/or oxetane resin curing agent can be used. As the epoxy resin curing agent, those publicly known can be generally used, and examples thereof may include an imidazole derivative such as 2-methylimidazole, 2-ethyl-4-methylimidazole, 2-phenylimidazole, 1-cyanoethyl-2-phenylimidazole, 1-cyanoethyl-2-ethyl-4-methylimidazole, 2-phenyl-4,5-dihydroxymethylimidazole and 2-phenyl-4-methyl-5-hydroxymethylimidazole; an amine compound such as dicyandiamide, benzyldimethylamine and 4-methyl-N,N-dimethylbenzylamine; and a phosphine-based or phosphonium-based phosphorus compound. As the oxetane resin curing agent, a publicly known cationic polymerization initiator can be used. Examples thereof include commercially available products such as SAN-AID SI-60L, SAN-AID SI-80L and SAN-AID SI-100L (manufactured by SANSHIN CHEMICAL INDUSTRY CO., LTD.), CI-2064 (manufactured by NIPPON SODA CO., LTD.), IRGACURE 261 (manufactured by Ciba Specialty Chemicals), Adeka Optomer SP-170 and Adeka Optomer SP-150 (manufactured by Asahi Denka Kogyo K.K.), and Cyracure UVI-6990 (manufactured by UCC). The cationic polymerization initiator can also be used as the epoxy resin curing agent. These curing agents are used as one kind, or in combination of two or more kinds.

As the compound having a polymerizable unsaturated group, those publicly known can be generally used. Examples thereof include a vinyl compound such as ethylene, propylene, styrene and vinyl compounds having a PPE skeleton described in Japanese Patent Application Laid-Open No. 2004-59644, a (meth)acrylate of a monohydric or polyhydric alcohol such as methyl (meth)acrylate, 2-hydroxyethyl (meth)acrylate, 2-hydroxypropyl (meth)acrylate, polypropylene glycol di(meth)acrylate, trimethylolpropane di(meth)acrylate, trimethylolpropane tri(meth)acrylate, pentaerythritol tetra(meth)acrylate and dipentaerythritol hexa(meth)acrylate, an epoxy (meth)acrylate such as a bisphenol A-based epoxy (meth)acrylate, a bisphenol F-based epoxy (meth)acrylate, epoxy (meth)acrylates having a PPE skeleton described in Japanese Patent Application Laid-Open No. 2003-183350 and Japanese Patent Application Laid-Open No. 2003-238653, (meth)acrylates having a PPE skeleton described in Japanese Patent Application Laid-Open No. 2003-252983 and Japanese Patent Application Laid-Open No. 2003-252833, a benzocyclobutene resin, a triallyl isocyanurate (TAIC) and a triallyl cyanurate (TAC). These compounds having an unsaturated group are used as one kind, or in mixture of two or more kinds.

As the cyanate ester compound, those publicly known can be generally used. Examples thereof include a bisphenol A dicyanate ester, a bisphenol F dicyanate ester, a bisphenol M dicyanate ester, a bisphenol P dicyanate ester, a bisphenol E dicyanate ester, a phenol novolac-based cyanate ester, a cresol novolac-based cyanate ester, a dicyclopentadiene novolac-based cyanate ester, a tetramethyl bisphenol F dicyanate ester, a biphenol dicyanate ester, and cyanate esters described in Japanese Patent Application Laid-Open No. 2006-328286. These cyanate ester compounds can be used as one kind, or in mixture of two or more kinds.

Upon curing the cyanate ester compound, a publicly known curing catalyst can be used. Examples thereof include a metal salt such as zinc octylate, zinc naphthenate, cobalt naphthenate, copper naphthenate and iron acetylacetonate, and a compound having an active hydroxy group such as a phenol, an alcohol and an amine.

As the photopolymerization initiator, those publicly known can be generally used. Examples thereof include an α-diketone such as benzyl and diacetyl, an acyloin ether such as benzoyl ethyl ether and benzoin isopropyl ether, a thioxanthone such as thioxanthone, 2,4-diethylthioxanthone and 2-isopropylthioxanthone, a benzophenone such as benzophenone and 4,4'-bis(dimethylamino)benzophenone, an acetophenone such as acetophenone, 2,2'-dimethoxy-2-phenylacetophenone and β-methoxyacetophenone, and an aminoacetophenone such as 2-methyl-1-[4-(methylthio)phenyl]-2-morpholinopropan-1-one and 2-benzyl-2-dimethylamino-1-(-4-morpholinophenyl)-butanone-1. These photopolymerization initiators are used as one kind, or in combination of two or more kinds.

Furthermore, these photopolymerization initiators can be used in combination with one kind or two or more kinds of publicly known photosensitizers. Examples of the photosensitizer may include ethyl N,N-dimethylaminobenzoate, isoamyl N,N-dimethylaminobenzoate, triethanolamine and triethylamine.

As the thermal polymerization initiator, those publicly known can be generally used. Examples thereof include a peroxide such as benzoyl peroxide, parachlorobenzoyl peroxide, 2,4-dichlorobenzoyl peroxide, lauroyl peroxide, dicumyl peroxide, acetyl peroxide, methyl ethyl ketone peroxide, cyclohexanone peroxide, bis(1-hydroxycyclohexyl peroxide), 2,5-dimethylhexane-2,5-dihydroxy peroxide, t-butyl perbenzoate, 2,5-dimethyl-2,5-di(t-butylperoxy)hexane, 2,5-dimethyl-2,5-(t-butylperoxy)hexyne-3, 2,5-dimethylhexyl-2,5-di(peroxybenzoate), cumene hydroperoxide, t-butyl hydroperoxide, t-butyl peroxybenzoate, t-butyl peroxyacetate, t-butyl peroxyoctate, t-butyl peroxyisobutyrate, dibenzyl peroxide, di-t-butyl peroxyphthalate, diisopropyl peroxycarbonate and di-2-ethylhexyl peroxycarbonate, and an azo compound such as azobisisobutyronitrile. These thermal polymerization initiators are used as one kind, or in combination of two or more kinds.

The amount of the polymerization initiator to be compounded is 0.01 to 10 parts by weight, and is particularly preferably 0.1 to 5 parts by weight based on 100 parts by weight of the resin composition. Also, a polymerization promoting agent, a retarding agent, a variety of pigments, a filler may be added, if necessary.

In addition, in order to adjust the cure degree, a publicly known polymerization inhibitor including a quinone such as hydroquinone, methylhydroquinone, t-butylhydroquinone, p-benzoquinone, chloranil and trimethylquinone, an aromatic diol, a copper salt and the like can be compounded. These polymerization inhibitors can be used alone, or in mixture of two or more kinds.

As the curing agent, an amine-based compound having at least two or more amino groups is used. The amino group is Michael-added to the double bond that the imide ring of the bismaleimide compound has, and the polymerization reaction proceeds. Examples of such an amine-based compound include an aromatic diamine such as diaminodiphenylmethane, diaminodiphenylsulfone, phenylenediamine and xylenediamine, and a halogenated derivative thereof.

Furthermore, upon producing the composition of the present embodiment, a publicly known additive agent such as a coupling agent, a thermoplastic resin, an inorganic filler, a coloring pigment, a defoaming agent, a surface conditioning agent, a flame retardant, an ultraviolet absorber, an antioxidant and a flow adjusting agent may be added, as necessary.

Examples of the coupling agent include a silane-based coupling agent such as vinyltrichlorosilane, vinyltriethoxysilane, vinyltrimethoxysilane, γ-methacryloxypropyltrimethoxysilane, β-(3,4-epoxycyclohexyl)ethyltrimethoxysilane, γ-glycidoxypropylmethyldiethoxysilane, N-β-(aminoethyl)-γ-aminopropylmethylmethoxysilane, γ-aminopropyltriethoxysilane, N-phenyl-γ-aminopropyltrimethoxysilane, γ-mercaptopropyltrimethoxysilane and γ-chloropropyltrimethoxysilane, a titanate-based coupling agent, an aluminum-based coupling agent, a zircoaluminate-based coupling agent, a silicone-based coupling agent and a fluorine-based coupling agent. These coupling agents can be used alone, or in mixture of two or more kinds.

Examples of the thermoplastic resin include a polyamide imide, a polyimide, a polybutadiene, a polyethylene, a polystyrene, a polycarbonate, a phenoxy resin, a polyisoprene, a polyester, a polyvinyl butyral and a polybutadiene.

Examples of the inorganic filler include a silica such as natural silica, fused silica and amorphous silica, a metal hydrate such as white carbon, titanium white, aerosil, alumina, talc, natural mica, synthetic mica, kaolin, clay, aluminum hydroxide, heat treated product of aluminum hydroxide (heat treated aluminum hydroxide to reduce a part of crystal water), boehmite and magnesium hydroxide, barium sulfate, E-glass, A-glass, NE-glass, C-glass, L-glass, D-glass, S-glass and M-glass G20.

As the flame retardant, those publicly known can be used. Examples thereof include a halogenated-based flame retardant such as a brominated epoxy resin, a brominated polycarbonate, a brominated polystyrene, a brominated styrene, a brominated maleimide, a brominated phthalimide, tetrabromobisphenol A, pentabenzyl (meth)acrylate, pentabromotoluene, tribromophenol, hexabromobenzene, decabromodiphenyl ether, bis-1,2-pentabromophenylethane, a chlorinated polystyrene and a chlorinated paraffin; a phosphorus-based flame retardant such as red phosphorus, tricresyl phosphate, triphenyl phosphate, cresyl diphenyl phosphate, trixylenyl phosphate, trialkyl phosphate, dialkyl phosphate, tris(chloroethyl) phosphate, phosphazene, 1,3-phenylene bis(2,6-dixylenylphosphate) and 10-(2,5-dihydroxyphenyl)-10H-9-oxa-10-phosphaphenanthrene-10-oxide; an inorganic-based flame retardant such as aluminum hydroxide, magnesium hydroxide, boehmite, partial boehmite, zinc borate and antimony trioxide; and a silicon-based flame retardant such as a silicone rubber and a silicone resin. These flame retardants may be used alone or in combination of two or more kinds.

As the antioxidant, those publicly known can be used. Examples thereof include a phenol-based antioxidant such as 2,6-di-t-butyl-4-methylphenol, 2,2'-methylenebis(6-t-butyl-4-methylphenol), N,N'-hexane-1,6-diylbis[3-(3,5-dit-butyl-4-hydroxyphenyl)propionamide] and 2,6-di-t-butyl-4-(4,6-bis(octylthio)-1,3,5-triazin-2-ylaminophenol, an amine-based antioxidant, and a sulfur-based antioxidant. These antioxidants may be used alone or in combination of two or more kinds.

For example, the composition of the present embodiment can be used as a prepreg obtained by dissolving the composition in a solvent, impregnating glass cloth, aramid nonwoven fabric, liquid crystal polyester nonwoven fabric, carbon fiber or the like with the solution, and removing the solvent through drying, or as a curable film obtained by applying the composition onto a base material such as polycarbonate film, polyethylene terephthalate film, ethylene tetrafluoroethylene copolymer film, polyimide film, copper foil, aluminum foil, glass plate and SUS plate. An applied film can also be obtained by dissolving the composition in a solvent and applying the solution onto a base material through spin coating while removing the solvent through drying.

The composition thus obtained is useful in a variety of applications such as an insulating material for printed wiring boards, a resin for resists, a semiconductor packaging material, a resin for encapsulating semiconductors, an adhesive for printed wiring boards, a buildup laminate material, a resin for fiber-reinforced plastics, a resin for encapsulation of liquid crystal display panels, a resin for color filters of liquid crystals, a paint, a variety of coating agents and an adhesive.

### [Cured product]

A cured product of the present embodiment is obtained by curing the composition of the present embodiment obtained through the aforementioned method according to a publicly known method, such as a curing method with electron beam, ultraviolet light and heat. When curing is carried out using ultraviolet light, as the light source for ultraviolet light, a low pressure mercury lamp, a medium pressure mercury lamp, a high pressure mercury lamp, an ultrahigh pressure mercury lamp, a xenon lamp, a metal halide lamp or the like can be used. In the case of curing by heat, the cured product is obtained by filling a metal mold or the like with the curable resin composition of the present invention at a temperature range in a molten state, and then elevating the temperature to a predetermined polymerization temperature or higher to advance the crosslinking reaction. The curing temperature is preferably 100 to 500°C and the curing time is preferably 0.01 to 5 hours.

### [Film forming material for lithography]

A film forming material for lithography in the present embodiment contains the above-described compound ((poly)imide compound or (poly)amic acid) and/or resin.

The film forming material for lithography of the present embodiment is applicable to a wet process. In addition, the film forming material for lithography of the present embodiment has an aromatic structure and also has a rigid maleimide skeleton, and therefore, when it is baked at a high temperature, its maleimide group, citraconimide group, maleamic acid group or citraconamic acid group undergoes a crosslinking reaction, thereby expressing high heat resistance even on its own. As a result, deterioration of the film upon baking at a high temperature is suppressed and an underlayer film excellent in etching resistance to oxygen plasma etching and the like can be formed. Furthermore, even though the film forming material for lithography of the present embodiment has an aromatic structure, its solubility in an organic solvent is high and its solubility in a safe solvent is high. Furthermore, an underlayer film for lithography composed of the composition for film formation for lithography of the present embodiment, which will be mentioned later, is not only excellent in embedding properties to a substrate having difference in level and film flatness, thereby having a good stability of the product quality, but also excellent in adhesiveness to a resist layer or a resist intermediate layer film material, and thus, an excellent resist pattern can be obtained.

### <Crosslinking agent>

The film forming material for lithography of the present embodiment may comprise a crosslinking agent, if required, in addition to the above-described compound and/or resin of the present embodiment from the viewpoint of lowering the curing temperature, suppressing intermixing and the like.

The crosslinking agent is not particularly limited as long as it undergoes a crosslinking reaction with the maleimide group, citraconimide group, maleamic acid group or citraconamic acid group of the present embodiment, and any of publicly known crosslinking agents can be applied. Specific examples of the crosslinking agent include, but not particularly limited to, a phenol compound, an epoxy compound, a cyanate compound, an amino compound, a benzoxazine compound, an acrylate compound, a melamine compound, a guanamine compound, a glycoluril compound, a urea compound, an isocyanate compound and an azide compound. These crosslinking agents can be used alone as one kind or can be used in combination of two or more kinds. Among them, a benzoxazine compound, an epoxy compound or a cyanate compound is preferable, and a benzoxazine compound is more preferable from the viewpoint of improvement in etching resistance.

In a crosslinking reaction with a maleimide group, citraconimide group, maleamic acid group or citraconamic acid group, for example, an active group these crosslinking agents have (a phenolic hydroxy group, an epoxy group, a cyanate group, an amino group, or a phenolic hydroxy group formed by ring opening of the alicyclic site of benzoxazine) undergoes an addition reaction with a carbon-carbon double bond that constitutes a maleimide group, citraconimide group, maleamic acid group or citraconamic acid group to form crosslinkage. Besides, two carbon-carbon double bonds that the compound of the present embodiment has are polymerized to form crosslinkage.

As the above phenol compound, a publicly known compound can be used. Examples of the phenol include phenol as well as an alkylphenol such as a cresol and a xylenol, a polyhydric phenol such as hydroquinone, a polycyclic phenol such as a naphthol and a naphthalenediol, a bisphenol such as bisphenol A and bisphenol F, and a polyfunctional phenol compound such as phenol novolac and phenol aralkyl resins. Among the above, a phenol aralkyl resin is preferable from the viewpoint of heat resistance and solubility.

As the above epoxy compound, a publicly known compound can be used and it is selected from among compounds having two or more epoxy groups in one molecule. Examples of the epoxy compound include an epoxidation product of a dihydric phenol such as bisphenol A, bisphenol F, 3,3',5,5'-tetramethyl-bisphenol F, bisphenol S, fluorene bisphenol, 2,2'-biphenol, 3,3',5,5'-tetramethyl-4,4'-dihydroxybiphenol, resorcin and a naphthalenediol; an epoxidation product of a trihydric or higher phenol such as tris-(4-hydroxyphenyl)methane, 1,1,2,2-tetrakis(4-hydroxyphenyl)ethane, tris(2,3-epoxypropyl) isocyanurate, trimethylolmethane triglycidyl ether, trimethylolpropane triglycidyl ether, triethylolethane triglycidyl ether, phenol novolac and o-cresol novolac; an epoxidation product of a co-condensed resin of dicyclopentadiene and a phenol; an epoxidation product of a phenol aralkyl resin synthesized from a phenol and paraxylylene dichloride or the like; an epoxidation product of a biphenyl aralkyl-based phenolic resin synthesized from a phenol and bischloromethylbiphenyl or the like; and an epoxidation product of a naphthol aralkyl resin synthesized from a naphthol and paraxylylene dichloride or the like. These epoxy resins may be used alone or in combination of two or more kinds. Among the above, an epoxy resin that is in a solid state at normal temperature, such as an epoxy resin obtained from a phenol aralkyl resin or a biphenyl aralkyl resin, is preferable from the viewpoint of heat resistance and solubility.

The above cyanate compound is not particularly limited as long as the compound has two or more cyanate groups in one molecule, and a publicly known compound can be used. Examples of the cyanate compound include those described in International Publication No. WO 2011/108524, and above all, preferable are those having a structure where hydroxy groups of a compound having two or more hydroxy groups in one molecule are replaced with cyanate groups. Also, the cyanate compound preferably has an aromatic group, and a structure where a cyanate group is directly bonded to an aromatic group can be suitably used. Examples of such a cyanate compound include a cyanate compound having a structure where hydroxy groups of bisphenol A, bisphenol F, bisphenol M, bisphenol P, bisphenol E, a phenol novolac resin, a cresol novolac resin, a dicyclopentadiene novolac resin, tetramethylbisphenol F, a bisphenol A novolac resin, a brominated bisphenol A, a brominated phenol novolac resin, a trifunctional phenol, a tetrafunctional phenol, a naphthalene-based phenol, a biphenyl-based phenol, a phenol aralkyl resin, a biphenyl aralkyl resin, a naphthol aralkyl resin, a dicyclopentadiene aralkyl resin, an alicyclic phenol, a phosphorus-containing phenol, or the like are replaced with cyanate groups. These cyanate compounds may be used alone or in arbitrary combination of two or more kinds. Also, the above cyanate compound may be in any form of a monomer, an oligomer and a resin.

Examples of the above amino compound include m-phenylenediamine, p-phenylenediamine, 4,4'-diaminodiphenylmethane, 4,4'-diaminodiphenylpropane, 4,4'-diaminodiphenyl ether, 3,4'-diaminodiphenyl ether, 3,3'-diaminodiphenyl ether, 4,4'-diaminodiphenylsulfone, 3,4'-diaminodiphenylsulfone, 3,3'-diaminodiphenylsulfone, 4,4'-diaminodiphenyl sulfide, 3,4'-diaminodiphenyl sulfide, 3,3'-diaminodiphenyl sulfide, 1,4-bis(4-aminophenoxy)benzene, 1,3-bis(4-aminophenoxy)benzene, 1,4-bis(3-aminophenoxy)benzene, 1,3-bis(3-aminophenoxy)benzene, bis[4-(4-aminophenoxy)phenyl]sulfone, 2,2-bis[4-(4-aminophenoxy)phenyl]propane, 2,2-bis[4-(3-aminophenoxy)phenyl]propane, 4,4'-bis(4-aminophenoxy)biphenyl, 4,4'-bis(3-aminophenoxy)biphenyl, bis[4-(4-aminophenoxy)phenyl] ether, bis[4-(3-aminophenoxy)phenyl] ether, 9,9-bis(4-aminophenyl)fluorene, 9,9-bis(4-amino-3-chlorophenyl)fluorene, 9,9-bis(4-amino-3-fluorophenyl)fluorene, O-tolidine, m-tolidine, 4,4'-diaminobenzanilide, 2,2'-bis(trifluoromethyl)-4,4'-diaminobiphenyl, 4-aminophenyl-4-aminobenzoate, and 2-(4-aminophenyl)-6-aminobenzoxazole. Among them, examples thereof include an aromatic amine such as 4,4'-diaminodiphenylmethane, 4,4'-diaminodiphenylpropane, 4,4'-diaminodiphenyl ether, 3,4'-diaminodiphenyl ether, 3,3'-diaminodiphenyl ether, 4,4'-diaminodiphenylsulfone, 3,3'-diaminodiphenylsulfone, 1,4-bis(4-aminophenoxy)benzene, 1,3-bis(4-aminophenoxy)benzene, 1,4-bis(3-aminophenoxy)benzene, 1,3-bis(3-aminophenoxy)benzene, bis[4-(4-aminophenoxy)phenyl]sulfone, 2,2-bis[4-(4-aminophenoxy)phenyl]propane, 2,2-bis[4-(3-aminophenoxy)phenyl]propane, 4,4'-bis(4-aminophenoxy)biphenyl, 4,4'-bis(3-aminophenoxy)biphenyl, bis[4-(4-aminophenoxy)phenyl] ether, and bis[4-(3-aminophenoxy)phenyl] ether; an alicyclic amine such as diaminocyclohexane, diaminodicyclohexylmethane, dimethyldiaminodicyclohexylmethane, tetramethyldiaminodicyclohexylmethane, diaminodicyclohexylpropane, diaminobicyclo[2.2.1]heptane, bis(aminomethyl)-bicyclo[2.2.1]heptane, 3(4),8(9)-bis(aminomethyl)tricyclo[5.2.1.02,6]decane, 1,3-bisaminomethylcyclohexane, and isophoronediamine; and an aliphatic amine such as ethylenediamine, hexamethylenediamine, octamethylenediamine, decamethylenediamine, diethylenetriamine, and triethylenetetramine.

The structure of oxazine of the above benzoxazine compound is not particularly limited, and examples thereof include a structure of oxazine having an aromatic group including a condensed polycyclic aromatic group, such as benzoxazine and naphthoxazine.

Examples of the benzoxazine compound include, for example, compounds represented by the following general formulas (a) to (f). Note that, in the general formulas described below, a bond displayed toward the center of a ring indicates a bond to any carbon that constitutes the ring and to which a substituent can be bonded.

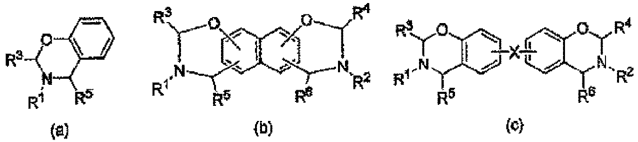

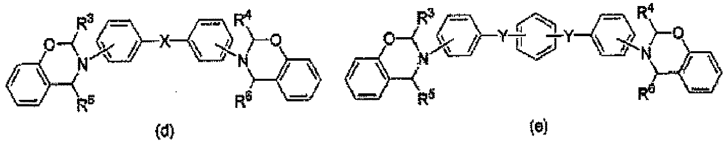

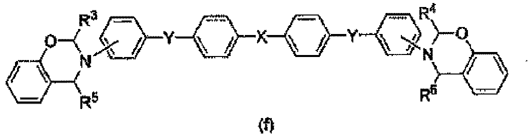

In the general formulas (a) to (c), R¹ and R² each independently represent an organic group having 1 to 30 carbon atoms. In addition, in the general formulas (a) to (f), R³ to R⁶ each independently represent hydrogen or a hydrocarbon group having 1 to 6 carbon atoms. Moreover, in the above general formulas (c), (d) and (f), X independently represents a single bond, -O-, -S-, -S-S-, -SO₂-, -CO-, -CONH-, -NHCO-, -C(CH₃)₂-, -C(CF₃)₂-, -(CH₂)m-, -O-(CH₂)m-O- or -S-(CH₂)m-S-. Here, m is an integer of 1 to 6. In addition, in the general formulas (e) and (f), Y independently represents a single bond, -O-, -S-, -CO-, -C(CH₃)₂-, -C(CF₃)₂- or an alkylene group having 1 to 3 carbon atoms.

Moreover, the benzoxazine compound includes an oligomer or polymer having an oxazine structure as a side chain, and an oligomer or polymer having a benzoxazine structure in the main chain.

The benzoxazine compound can be produced in a similar method as a method described in International Publication No. WO 2004/009708, Japanese Patent Application Laid-Open No. 11-12258 or Japanese Patent Application Laid-Open No. 2004-352670.

Specific examples of the melamine compounds include hexamethylolmelamine, hexamethoxymethylmelamine, a compound in which 1 to 6 methylol groups of hexamethylolmelamine are methoxymethylated or a mixture thereof, hexamethoxyethylmelamine, hexaacyloxymethylmelamine, and a compound in which 1 to 6 methylol groups of hexamethylolmelamine are acyloxymethylated or a mixture thereof.

Specific examples of the guanamine compounds include tetramethylolguanamine, tetramethoxymethylguanamine, a compound in which 1 to 4 methylol groups of tetramethylolguanamine are methoxymethylated or a mixture thereof, tetramethoxyethylguanamine, tetraacyloxyguanamine, and a compound in which 1 to 4 methylol groups of tetramethylolguanamine are acyloxymethylated or a mixture thereof.

Specific examples of the glycoluril compounds include tetramethylolglycoluril, tetramethoxyglycoluril, tetramethoxymethylglycoluril, a compound in which 1 to 4 methylol groups of tetramethylolglycoluril are methoxymethylated or a mixture thereof, and a compound in which 1 to 4 methylol groups of tetramethylolglycoluril are acyloxymethylated or a mixture thereof.

Specific examples of the urea compounds include tetramethylolurea, tetramethoxymethylurea, a compound in which 1 to 4 methylol groups of tetramethylolurea are methoxymethylated or a mixture thereof, and tetramethoxyethylurea.

In the present embodiment, a crosslinking agent having at least one allyl group may be used from the viewpoint of improvement in crosslinkability. Specific examples of the crosslinking agent having at least one allyl group include, but not limited to, an allylphenol such as 2,2-bis(3-allyl-4-hydroxyphenyl)propane, 1,1,1,3,3,3-hexafluoro-2,2-bis(3-allyl-4-hydroxyphenyl)propane, bis(3-allyl-4-hydroxyphenyl)sulfone, bis(3-allyl-4-hydroxyphenyl) sulfide, and bis(3-allyl-4-hydroxyphenyl) ether, an allyl cyanate such as 2,2-bis(3-allyl-4-cyanatophenyl)propane, 1,1,1,3,3,3-hexafluoro-2,2-bis(3-allyl-4-cyanatophenyl)propane, bis(3-allyl-4-cyanatophenyl)sulfone, bis(3-allyl-4-cyanatophenyl) sulfide, and bis(3-allyl-4-cyanatophenyl) ether, diallyl phthalate, diallyl isophthalate, diallyl terephthalate, triallyl isocyanurate, trimethylolpropane diallyl ether, and pentaerythritol allyl ether. These crosslinking agents may be alone, or may be a mixture of two or more kinds. Among the above, an allylphenol such as 2,2-bis(3-allyl-4-hydroxyphenyl)propane, 1,1,1,3,3,3-hexafluoro-2,2-bis(3-allyl-4-hydroxyphenyl)propane, bis(3-allyl-4-hydroxyphenyl)sulfone, bis(3-allyl-4-hydroxyphenyl) sulfide and bis(3-allyl-4-hydroxyphenyl) ether is preferable from the viewpoint of excellent compatibility with a maleimide compound, citraconimide compound, maleamic acid and/or citraconamic acid.

With the film forming material for lithography of the present embodiment, the film for lithography of the present embodiment can be formed by crosslinking and curing the compound and/or resin of the present embodiment alone, or after compounding with the above crosslinking agent, according to a publicly known method. Examples of the crosslinking method include approaches such as thermosetting and light curing.

The content ratio of the crosslinking agent is normally in the range of 0.1 to 10000 parts by mass based on 100 parts by mass of the total mass of the compound and resin of the present embodiment, preferably in the range of 0.1 to 1000 parts by mass from the viewpoint of heat resistance and solubility, more preferably in the range of 0.1 to 100 parts by mass, further preferably in the range of 1 to 50 parts by mass, and particularly preferably in the range of 1 to 30 parts by mass.

### <Crosslinking promoting agent>

In the film forming material for lithography of the present embodiment, if required, a crosslinking promoting agent for accelerating crosslinking and curing reaction can be used.

The crosslinking promoting agent is not particularly limited as long as it accelerates crosslinking or curing reaction, and examples thereof include an amine, an imidazole, an organic phosphine, and a Lewis acid. These crosslinking promoting agents can be used alone as one kind or can be used in combination of two or more kinds. Among them, an imidazole or an organic phosphine is preferable, and an imidazole is more preferable from the viewpoint of decrease in crosslinking temperature.

Examples of the crosslinking promoting agent include, but not limited to, a tertiary amine such as 1,8-diazabicyclo(5,4,0)undecene-7, triethylenediamine, benzyldimethylamine, triethanolamine, dimethylaminoethanol, and tris(dimethylaminomethyl)phenol; an imidazole such as 2-methylimidazole, 2-phenylimidazole, 2-ethyl-4-methylimidazole, 2-phenyl-4-methylimidazole, 2-heptadecylimidazole, and 2,4,5-triphenylimidazole; an organic phosphine such as tributylphosphine, methyldiphenylphosphine, triphenylphosphine, diphenylphosphine, and phenylphosphine; a tetra substituted phosphonium-tetra substituted borate such as tetraphenylphosphonium-tetraphenyl borate, tetraphenylphosphonium-ethyltriphenyl borate, and tetrabutylphosphonium-tetrabutyl borate; and a tetraphenylboron salt such as 2-ethyl-4-methylimidazole-tetraphenyl borate and N-methylmorpholine-tetraphenyl borate.

The amount of the crosslinking promoting agent to be compounded is usually preferably in the range of 0.1 to 10 parts by mass based on 100 parts by mass of the total mass of the compound and resin of the present embodiment, and is more preferably in the range of 0.1 to 5 parts by mass and still more preferably in the range of 0.1 to 3 parts by mass, from the viewpoint of easy control and cost efficiency.

### <Radical polymerization initiator>

The film forming material for lithography of the present embodiment can contain, if required, a radical polymerization initiator. The radical polymerization initiator may be a photopolymerization initiator that initiates radical polymerization by light, or may be a thermal polymerization initiator that initiates radical polymerization by heat.

The radical polymerization initiator is not particularly limited, and a radical polymerization initiator conventionally used can be arbitrarily adopted. Examples thereof include a ketone-based photopolymerization initiator such as 1-hydroxy cyclohexyl phenyl ketone, benzyl dimethyl ketal, 2-hydroxy-2-methyl-1-phenylpropan-1-one, 1-[4-(2-hydroxyethoxy)-phenyl]-2-hydroxy-2-methyl-1-propan-1-one, 2-hydroxy-1-{4-[4-(2-hydroxy-2-methyl-propionyl)-benzyl]phenyl}-2-methylpropan-1-one, 2,4,6-trimethylbenzoyl-diphenyl-phosphine oxide, and bis(2,4,6-trimethylbenzoyl)-phenylphosphine oxide, and an organic peroxide-based polymerization initiator such as methyl ethyl ketone peroxide, cyclohexanone peroxide, methylcyclohexanone peroxide, methyl acetoacetate peroxide, acetyl acetate peroxide, 1,1-bis(t-hexylperoxy)-3,3,5-trimethylcyclohexane, 1,1-bis(t-hexylperoxy)-cyclohexane, 1,1-bis(t-butylperoxy)-3,3,5-trimethylcyclohexane, 1,1-bis(t-butylperoxy)-2-methylcyclohexane, 1,1-bis(t-butylperoxy)-cyclohexane, 1,1-bis(t-butylperoxy)cyclododecane, 1,1-bis(t-butylperoxy)butane, 2,2-bis(4,4-di-t-butylperoxycyclohexyl)propane, p-menthane hydroperoxide, diisopropylbenzene hydroperoxide, 1,1,3,3-tetramethylbutyl hydroperoxide, cumene hydroperoxide, t-hexyl hydroperoxide, t-butyl hydroperoxide, α,α'-bis(t-butylperoxy)diisopropylbenzene, dicumyl peroxide, 2,5-dimethyl-2,5-bis(t-butylperoxy)hexane, t-butylcumyl peroxide, di-t-butyl peroxide, 2,5-dimethyl-2,5-bis(t-butylperoxy)hexyne-3, isobutyryl peroxide, 3,5,5-trimethylhexanoyl peroxide, octanoyl peroxide, lauroyl peroxide, stearoyl peroxide, succinic acid peroxide, m-toluoyl benzoyl peroxide, benzoyl peroxide, di-n-propyl peroxydicarbonate, diisopropyl peroxydicarbonate, bis(4-t-butylcyclohexyl) peroxydicarbonate, di-2-ethoxyethyl peroxydicarbonate, di-2-ethoxyhexyl peroxydicarbonate, di-3-methoxybutyl peroxydicarbonate, di-s-butyl peroxydicarbonate, di(3-methyl-3-methoxybutyl) peroxydicarbonate, α,α'-bis(neodecanoylperoxy)diisopropylbenzene, cumyl peroxyneodecanoate, 1,1,3,3-tetramethylbutyl peroxyneodecanoate, 1-cyclohexyl-1-methylethyl peroxyneodecanoate, t-hexyl peroxyneodecanoate, t-butyl peroxyneodecanoate, t-hexyl peroxypivalate, t-butyl peroxypivalate, 1,1,3,3-tetramethylbutyl peroxy-2-ethylhexanoate, 2,5-dimethyl-2,5-bis(2-ethylhexanoylperoxy)hexanoate, 1-cyclohexyl-1-methylethyl peroxy-2-ethylhexanoate, t-hexyl peroxy-2-ethylhexanoate, t-butyl peroxy-2-ethylhexanoate, t-hexyl peroxyisopropylmonocarbonate, t-butyl peroxyisobutyrate, t-butyl peroxymalate, t-butyl peroxy-3,5,5-trimethylhexanoate, t-butyl peroxylaurate, t-butyl peroxyisopropylmonocarbonate, t-butyl peroxy-2-ethylhexylmonocarbonate, t-butyl peroxyacetate, t-butyl peroxy-m-toluylbenzoate, t-butyl peroxybenzoate, bis(t-butylperoxy) isophthalate, 2,5-dimethyl-2,5-bis(m-toluylperoxy)hexane, t-hexyl peroxybenzoate, 2,5-dimethyl-2,5-bis(benzoylperoxy)hexane, t-butyl peroxyallylmonocarbonate, t-butyltrimethylsilyl peroxide, 3,3',4,4'-tetra(t-butylperoxycarbonyl)benzophenone, and 2,3-dimethyl-2,3-diphenylbutane.

Further examples thereof include an azo-based polymerization initiator such as 2-phenylazo-4-methoxy-2,4-dimethylvaleronitrile, 1-[(1-cyano-1-methylethyl)azo]formamide, 1,1'-azobis(cyclohexane-1-carbonitrile), 2,2'-azobis(2-methylbutyronitrile), 2,2'-azobisisobutyronitrile, 2,2'-azobis(2,4-dimethylvaleronitrile), 2,2'-azobis(2-methylpropionamidine) dihydrochloride, 2,2'-azobis(2-methyl-N-phenylpropionamidine) dihydrochloride, 2,2'-azobis[N-(4-chlorophenyl)-2-methylpropionamidine]dihydride chloride, 2,2'-azobis[N-(4-hydrophenyl)-2-methylpropionamidine] dihydrochloride, 2,2'-azobis[2-methyl-N-(phenylmethyl)propionamidine] dihydrochloride, 2,2'-azobis[2-methyl-N-(2-propenyl)propionamidine] dihydrochloride, 2,2'-azobis[N-(2-hydroxyethyl)-2-methylpropionamidine] dihydrochloride, 2,2'-azobis[2-(5-methyl-2-imidazolin-2-yl)propane] dihydrochloride, 2,2'-azobis[2-(2-imidazolin-2-yl)propane] dihydrochloride, 2,2'-azobis[2-(4,5,6,7-tetrahydro-1H-1,3-diazepin-2-yl)propane] dihydrochloride, 2,2'-azobis[2-(3,4,5,6-tetrahydropyrimidin-2-yl)propane] dihydrochloride, 2,2'-azobis[2-(5-hydroxy-3,4,5,6-tetrahydropyrimidin-2-yl)propane] dihydrochloride, 2,2'-azobis[2-[1-(2-hydroxyethyl)-2-imidazolin-2-yl]propane] dihydrochloride, 2,2'-azobis[2-(2-imidazolin-2-yl)propane], 2,2'-azobis[2-methyl-N-[1,1-bis(hydroxymethyl)-2-hydroxyethyl]propionamide], 2,2'-azobis[2-methyl-N-[1,1-bis(hydroxymethyl)ethyl]propionamide], 2,2'-azobis[2-methyl-N-(2-hydroxyethyl)propionamide], 2,2'-azobis(2-methylpropionamide), 2,2'-azobis(2,4,4-trimethylpentane), 2,2'-azobis(2-methylpropane), dimethyl-2,2-azobis(2-methylpropionate), 4,4'-azobis(4-cyanopentanoic acid), and 2,2'-azobis[2-(hydroxymethyl)propionitrile]. As the radical polymerization initiator according to the present embodiment, one kind thereof may be used alone, or two or more kinds may be used in combination. Alternatively, the radical polymerization initiator according to the present embodiment may be used in further combination with an additional publicly known polymerization initiator.

The content of the radical polymerization initiator can be a stoichiometrically necessary amount based on the total mass of the compound and resin of the present embodiment, and is preferably 0.05 to 25 parts by mass, and more preferably 0.1 to 10 parts by mass, based on 100 parts by mass of the total mass of the compound and resin of the present embodiment. When the content of the radical polymerization initiator is 0.05 parts by mass or more, there is a tendency that curing of the compound and/or resin of the present embodiment becomes sufficient. On the other hand, when the content of the radical polymerization initiator is 25 parts by mass or less, there is a tendency that the long term storage stability of the film forming material for lithography at room temperature becomes good.

### [Method for purifying film forming material for lithography]

The film forming material for lithography can be purified by washing with an acidic aqueous solution.

A method for purifying the film forming material for lithography of the present embodiment comprises, for example, the steps of:
obtaining an organic phase by dissolving the film forming material for lithography in a solvent; and
extracting impurities in the film forming material for lithography by bringing the organic phase into contact with an acidic aqueous solution (a first extraction step),
   and
the solvent used in the step of obtaining the organic phase contains a solvent that does not inadvertently mix with water.

In the first extraction step, metals contained in the organic phase containing the film forming material for lithography and the organic solvent are transferred to an aqueous phase, and then, the organic phase and the aqueous phase are separated.

According to the purification method of the present embodiment, the contents of various metals in the film forming material for lithography can be reduced remarkably.

The solvent that does not inadvertently mix with water is not particularly limited, but is preferably an organic solvent that is safely applicable to semiconductor manufacturing processes. Normally, the amount of the solvent used is approximately 1 to 100 times by mass relative to the compound used.

Specific examples of the organic solvent include those described in International Publication No. WO 2015/080240. Among them, toluene, 2-heptanone, cyclohexanone, cyclopentanone, methyl isobutyl ketone, propylene glycol monomethyl ether acetate, ethyl acetate, and the like are preferable, and cyclohexanone and propylene glycol monomethyl ether acetate are particularly preferable. These organic solvents can each be used alone, or can be used as a mixture of two or more kinds.

The acidic aqueous solution is appropriately selected from aqueous solutions in which generally known organic or inorganic compounds are dissolved in water. For example, examples thereof include those described in International Publication No. WO 2015/080240. These acidic aqueous solutions can each be used alone, or can also be used as a combination of two or more kinds. Examples of the acidic aqueous solution may include, for example, an aqueous mineral acid solution and an aqueous organic acid solution. Examples of the aqueous mineral acid solution may include, for example, an aqueous solution comprising one or more selected from the group consisting of hydrochloric acid, sulfuric acid, nitric acid and phosphoric acid. Examples of the aqueous organic acid solution may include, for example, an aqueous solution comprising one or more selected from the group consisting of acetic acid, propionic acid, oxalic acid, malonic acid, succinic acid, fumaric acid, maleic acid, tartaric acid, citric acid, methanesulfonic acid, phenolsulfonic acid, p-toluenesulfonic acid and trifluoroacetic acid. Moreover, as the acidic aqueous solution, aqueous solutions of sulfuric acid, nitric acid, and a carboxylic acid such as acetic acid, oxalic acid, tartaric acid and citric acid are preferable, aqueous solutions of sulfuric acid, oxalic acid, tartaric acid and citric acid are further preferable, and an aqueous solution of oxalic acid is particularly preferable. It is considered that polyvalent carboxylic acids such as oxalic acid, tartaric acid and citric acid coordinate with metal ions and provide a chelating effect, and thus are capable of removing more metals. Water used herein is preferably water, the metal content of which is small, such as ion exchanged water, according to the purpose of the present invention.

The pH of the acidic aqueous solution is not particularly limited, but when the acidity of the aqueous solution is too high, it may have a negative influence on the used compound or resin, which is not preferable. Normally, the pH range is about 0 to 5, and is more preferably about pH 0 to 3.

The amount of the acidic aqueous solution used is not particularly limited, but when the amount used is too small, it is required to increase the number of extraction treatments for removing metals, and on the other hand, when the amount of the aqueous solution used is too large, the entire fluid volume becomes large, which may cause operational problems. Normally, the amount of the aqueous solution used is 10 to 200 parts by mass and preferably 20 to 100 parts by mass relative to the solution of the film forming material for lithography.

By bringing the acidic aqueous solution into contact with a solution containing the film forming material for lithography and the organic solvent that does not inadvertently mix with water, metals can be extracted.

The temperature at which the above extraction treatment is carried out is generally in the range of 20 to 90°C, and preferably 30 to 80°C. The extraction operation is carried out, for example, by thoroughly mixing the acidic aqueous solution and a solution containing the film forming material for lithography and the organic solvent by stirring or the like and then leaving the obtained mixed solution to stand still. Thereby, metals contained in the solution containing the used compound and the organic solvent are transferred to the aqueous phase. Also, by this operation, the acidity of the solution is lowered, and the deterioration of the used compound can be suppressed.

After the extraction treatment, the mixed solution is separated into a solution phase containing the compound and the organic solvent and the aqueous phase, and the solution containing the organic solvent is recovered by decantation or the like. The time for leaving the mixed solution to stand still is not particularly limited, but when the time for leaving the mixed solution to stand still is too short, separation of the solution phase containing the organic solvent and the aqueous phase becomes poor, which is not preferable. Normally, the time for leaving the mixed solution to stand still is 1 minute or longer, more preferably 10 minutes or longer, and further preferably 30 minutes or longer. While the extraction treatment may be carried out once, it is effective to repeat mixing, leaving-to-stand-still, and separating operations multiple times.

When such an extraction treatment is carried out using the acidic aqueous solution, after carrying out the treatment, it is preferable to use the recovered organic phase that has been extracted from the aqueous solution and contains the organic solvent to carry out an extraction treatment with water (a second extraction step). The extraction operation is carried out by thoroughly mixing the organic phase and water by stirring or the like and then leaving the obtained mixed solution to stand still. The resultant solution is separated into a solution phase containing the compound and the organic solvent and an aqueous phase, and thus the solution phase is recovered by decantation or the like. Water used herein is preferably water, the metal content of which is small, such as ion exchanged water, according to the purpose of the present invention. While the extraction treatment may be carried out once, it is effective to repeat mixing, leaving-to-stand-still, and separating operations multiple times. The proportions of both used in the extraction treatment and temperature, time, and other conditions are not particularly limited, and may be the same as those of the previous contact treatment with the acidic aqueous solution.

Water that is unwantedly present in the thus-obtained solution containing the film forming material for lithography and the organic solvent can be easily removed by performing vacuum distillation or a like operation. Also, if required, the concentration of the compound can be regulated to be any concentration by adding an organic solvent.

A method for only obtaining the film forming material for lithography from the obtained solution containing the organic solvent can be carried out through a publicly known method such as reduced-pressure removal, separation by reprecipitation, and a combination thereof. Publicly known treatments such as concentration operation, filtration operation, centrifugation operation, and drying operation can be carried out if required.

### [Composition for film formation for lithography]

A composition for film formation for lithography of the present embodiment comprises the above film forming material for lithography and a solvent. The film for lithography is, for example, an underlayer film for lithography.

The composition for film formation for lithography of the present embodiment can form a desired cured film by applying it on a base material, subsequently heating it to evaporate the solvent if necessary, and then heating or photoirradiating it. A method for applying the composition for film formation for lithography of the present embodiment is arbitrary, and a method such as spin coating, dipping, flow coating, inkjet coating, spraying, bar coating, gravure coating, slit coating, roll coating, transfer printing, brush coating, blade coating and air knife coating can be employed appropriately.

The temperature at which the film is heated is not particularly limited according to the purpose of evaporating the solvent, and the heating can be carried out at, for example, 40 to 400°C. A method for heating is not particularly limited, and for example, the solvent may be evaporated under an appropriate atmosphere such as atmospheric air, an inert gas including nitrogen and vacuum by using a hot plate or an oven. For the heating temperature and heating time, it is only required to select conditions suitable for a processing step for an electronic device that is aimed at and to select heating conditions by which physical property values of the obtained film satisfy requirements of the electronic device. Conditions for photoirradiation are not particularly limited, either, and it is only required to employ appropriate irradiation energy and irradiation time depending on a film forming material for lithography to be used.

### <Solvent>

A solvent to be used in the composition for film formation for lithography of the present embodiment is not particularly limited as long as it can at least dissolve the compound and/or resin of the present embodiment, and any publicly known solvent can be used appropriately.

Specific examples of the solvent include those described in International Publication No. WO 2013/024779. These solvents can be used alone as one kind or used in combination of two or more kinds.

Among the above solvents, cyclohexanone, propylene glycol monomethyl ether, propylene glycol monomethyl ether acetate, ethyl lactate, methyl hydroxyisobutyrate or anisole is particularly preferable from the viewpoint of safety.

The content of the solvent is not particularly limited and is preferably 25 to 9,900 parts by mass, more preferably 400 to 7,900 parts by mass, and further preferably 900 to 4,900 parts by mass based on 100 parts by mass of the total mass of the compound and resin of the present embodiment in the material for film formation for lithography, from the viewpoint of solubility and film formation.

### <Acid generating agent>

The composition for film formation for lithography of the present embodiment may contain an acid generating agent, if required, from the viewpoint of, for example, further accelerating crosslinking reaction. An acid generating agent that generates an acid by thermal decomposition, an acid generating agent that generates an acid by light irradiation, and the like are known, any of which can be used.

Examples of the acid generating agent include, for example, those described in International Publication No. WO 2013/024779. Among them, in particular, an onium salt such as di-tertiary-butyl diphenyliodonium nonafluoromethanesulfonate, diphenyliodonium trifluoromethanesulfonate, (p-tert-butoxyphenyl)phenyliodonium trifluoromethanesulfonate, diphenyliodonium p-toluenesulfonate, (p-tert-butoxyphenyl)phenyliodonium p-toluenesulfonate, triphenylsulfonium trifluoromethanesulfonate, (p-tert-butoxyphenyl)diphenylsulfonium trifluoromethanesulfonate, tris(p-tert-butoxyphenyl) sulfonium trifluoromethanesulfonate, triphenylsulfonium p-toluenesulfonate, (p-tert-butoxyphenyl)diphenylsulfonium p-toluenesulfonate, tris(p-tert-butoxyphenyl)sulfonium p-toluenesulfonate, trinaphthylsulfonium trifluoromethanesulfonate, cyclohexylmethyl(2-oxocyclohexyl)sulfonium trifluoromethanesulfonate, (2-norbornyl)methyl(2-oxocyclohexyl) sulfonium trifluoromethanesulfonate and 1,2'-naphthylcarbonylmethyltetrahydrothiophenium triflate; a diazomethane derivative such as bis(benzenesulfonyl)diazomethane, bis(p-toluenesulfonyl)diazomethane, bis(cyclohexylsulfonyl)diazomethane, bis(n-butylsulfonyl)diazomethane, bis(isobutylsulfonyl)diazomethane, bis(sec-butylsulfonyl)diazomethane, bis(n-propylsulfonyl)diazomethane, bis(isopropylsulfonyl)diazomethane and bis(tert-butylsulfonyl)diazomethane; a glyoxime derivative such as bis-(p-toluenesulfonyl)-α-dimethylglyoxime and bis-(n-butanesulfonyl)-α-dimethylglyoxime; a bissulfone derivative such as bisnaphthylsulfonylmethane; a sulfonic acid ester derivative of a N-hydroxyimide compound such as N-hydroxysuccinimide methanesulfonic acid ester, N-hydroxysuccinimide trifluoromethanesulfonic acid ester, N-hydroxysuccinimide 1-propanesulfonic acid ester, N-hydroxysuccinimide 2-propanesulfonic acid ester, N-hydroxysuccinimide 1-pentanesulfonic acid ester, N-hydroxysuccinimide p-toluenesulfonic acid ester, N-hydroxynaphthalimido methanesulfonic acid ester and N-hydroxynaphthalimido benzenesulfonic acid ester are preferably used.

The content of the acid generating agent in the composition for film formation for lithography of the present embodiment is not particularly limited, and is preferably 0 to 50 parts by mass and more preferably 0 to 40 parts by mass based on 100 parts by mass of the total mass of the compound and resin of the present embodiment in the film forming material for lithography. By the preferable range mentioned above, crosslinking reaction tends to be enhanced. Also, a mixing event with a resist layer tends to be prevented.

### [Basic compound]

The composition for underlayer film formation for lithography of the present embodiment may further contain a basic compound from the viewpoint of, for example, improving storage stability.

The above basic compound plays a role as a quencher against acids in order to prevent crosslinking reaction from proceeding due to a trace amount of an acid generated by the acid generating agent. Examples of such a basic compound include, but are not limited to, for example, a primary, secondary or tertiary aliphatic amine, an amine blend, an aromatic amine, a heterocyclic amine, a nitrogen-containing compound having a carboxy group, a nitrogen-containing compound having a sulfonyl group, a nitrogen-containing compound having a hydroxy group, a nitrogen-containing compound having a hydroxyphenyl group, an alcoholic nitrogen-containing compound, an amide derivative or an imide derivative, described in International Publication No. WO 2013-024779.

The content of the basic compound in the composition for film formation for lithography of the present embodiment is not particularly limited, and is preferably 0 to 2 parts by mass and more preferably 0 to 1 part by mass based on 100 parts by mass of the total mass of the compound and resin of the present embodiment in the film forming material for lithography. By the preferable range mentioned above, storage stability tends to be enhanced without excessively deteriorating crosslinking reaction.

The composition for film formation for lithography of the present embodiment may further contain a publicly known additive agent. Examples of the publicly known additive agent include, but are not limited to, an ultraviolet absorber, an antifoaming agent, a colorant, a pigment, a nonionic surfactant, an anionic surfactant and a cationic surfactant.

### [Method for forming underlayer film for lithography and pattern]

The underlayer film for lithography of the present embodiment is formed by using the composition for film formation for lithography of the present embodiment.

A pattern formation method of the present embodiment has the steps of: forming an underlayer film on a substrate using the composition for film formation for lithography of the present embodiment (step (A-1)); forming at least one photoresist layer on the underlayer film (step (A-2)); and after the step (A-2), irradiating a predetermined region of the photoresist layer with radiation for development (step (A-3)).

Furthermore, another pattern formation method of the present embodiment has the steps of: forming an underlayer film on a substrate using the composition for film formation for lithography of the present embodiment (step (B-1)); forming an intermediate layer film on the underlayer film using a resist intermediate layer film material containing a silicon atom (step (B-2)); forming at least one photoresist layer on the intermediate layer film (step (B-3)); after the step (B-3), irradiating a predetermined region of the photoresist layer with radiation for development, thereby forming a resist pattern (step (B-4)); and after the step (B-4), etching the intermediate layer film with the resist pattern as a mask, etching the underlayer film with the obtained intermediate layer film pattern as an etching mask, and etching the substrate with the obtained underlayer film pattern as an etching mask, thereby forming a pattern on the substrate (step (B-5)).

The underlayer film for lithography of the present embodiment is not particularly limited by its formation method as long as it is formed from the composition for film formation for lithography of the present embodiment. A publicly known approach can be applied thereto. The underlayer film can be formed by, for example, applying the composition for film formation for lithography of the present embodiment onto a substrate by a publicly known coating method or printing method such as spin coating or screen printing, and then removing an organic solvent by volatilization or the like.

It is preferable to perform baking in the formation of the underlayer film, for preventing a mixing event with an upper layer resist while accelerating crosslinking reaction. In this case, the baking temperature is not particularly limited and is preferably in the range of 80 to 450°C, and more preferably 200 to 400°C. Also, the baking time is not particularly limited and is preferably in the range of 10 to 300 seconds. The thickness of the underlayer film can be arbitrarily selected according to required performances and is not particularly limited, but is preferably 30 to 20,000 nm, more preferably 50 to 15,000 nm, and further preferably 50 to 1000 nm.

After preparing the underlayer film on the substrate, in the case of a two-layer process, it is preferable to prepare a silicon-containing resist layer or a usual single-layer resist composed of hydrocarbon thereon, and in the case of a three-layer process, it is preferable to prepare a silicon-containing intermediate layer thereon and further prepare a single-layer resist layer not containing silicon thereon. In this case, for a photoresist material for forming this resist layer, a publicly known material can be used.

For the silicon-containing resist material for a two-layer process, a silicon atom-containing polymer such as a polysilsesquioxane derivative or a vinylsilane derivative is used as a base polymer, and a positive type photoresist material further containing an organic solvent, an acid generating agent, and if required, a basic compound or the like is preferably used, from the viewpoint of oxygen gas etching resistance. Herein, a publicly known polymer that is used in this kind of resist material can be used as the silicon atom-containing polymer.

A polysilsesquioxane-based intermediate layer is preferably used as the silicon-containing intermediate layer for a three-layer process. By imparting functions as an antireflection film to the intermediate layer, there is a tendency that reflection can be effectively suppressed. For example, use of a material containing a large amount of an aromatic group and having high substrate etching resistance as the underlayer film in a process for exposure at 193 nm tends to increase a k value and enhance substrate reflection. However, the intermediate layer suppresses the reflection so that the substrate reflection can be 0.5% or less. The intermediate layer having such an antireflection effect is not limited, and a polysilsesquioxane that crosslinks by an acid or heat in which a light absorbing group having a phenyl group or a silicon-silicon bond is introduced is preferably used for exposure at 193 nm.

Alternatively, an intermediate layer formed by chemical vapour deposition (CVD) may be used. The intermediate layer highly functional as an antireflection film prepared by CVD is not limited, and, for example, a SiON film is known. In general, the formation of an intermediate layer by a wet process such as spin coating or screen printing is more convenient and more advantageous in cost than CVD. The upper layer resist for a three-layer process may be positive type or negative type, and the same as a single-layer resist generally used can be used.

The underlayer film of the present embodiment can also be used as an antireflection film for usual single-layer resists or an underlying material for suppression of pattern collapse. The underlayer film of the present embodiment is excellent in etching resistance for an underlying process and can be expected to also function as a hard mask for an underlying process.

In the case of forming a resist layer from the above photoresist material, a wet process such as spin coating or screen printing is preferably used, as in the case of forming the above underlayer film. After coating with the resist material by spin coating or the like, prebaking is generally carried out. This prebaking is preferably carried out at 80 to 180°C in the range of 10 to 300 seconds. Then, exposure, post-exposure baking (PEB), and development can be carried out according to a conventional method to obtain a resist pattern. The thickness of the resist film is not particularly limited and is generally preferably 30 to 500 nm, and more preferably 50 to 400 nm.

The exposure light can be arbitrarily selected and used according to the photoresist material to be used. General examples thereof can include a high energy ray having a wavelength of 300 nm or less, specifically, excimer laser of 248 nm, 193 nm, or 157 nm, soft x-ray of 3 to 20 nm, electron beam, and X-ray.

In a resist pattern formed by the method mentioned above, pattern collapse is suppressed by the underlayer film of the present embodiment. Therefore, use of the underlayer film of the present embodiment can produce a finer pattern and can reduce an exposure amount necessary for obtaining the resist pattern.

Next, etching is performed with the obtained resist pattern as a mask. Gas etching is preferably used as the etching of the underlayer film in a two-layer process. As the gas etching, etching using oxygen gas is suitable. In addition to oxygen gas, an inert gas such as He or Ar, or CO, CO₂, NH₃, SO₂, N₂, NO₂, or H₂ gas may be added. Alternatively, the gas etching may be carried out only with CO, CO₂, NH₃, N₂, NO₂, or H₂ gas without the use of oxygen gas. Particularly, the latter gas is preferably used for side wall protection in order to prevent the undercut of pattern side walls.

On the other hand, gas etching is also preferably used as the etching of the intermediate layer in a three-layer process. The same gas etching as described in the two-layer process mentioned above is applicable. Particularly, it is preferable to process the intermediate layer in a three-layer process by using chlorofluorocarbon-based gas and using the resist pattern as a mask. Then, as mentioned above, for example, the underlayer film can be processed by oxygen gas etching with the intermediate layer pattern as a mask.

Herein, in the case of forming an inorganic hard mask intermediate layer film as the intermediate layer, a silicon oxide film, a silicon nitride film, or a silicon oxynitride film (SiON film) is formed by CVD, ALD, or the like. A method for forming the nitride film is not limited, and, for example, a method described in Japanese Patent Application Laid-Open No. 2002-334869 or International Publication No. WO 2004/066377 can be used. Although a photoresist film can be formed directly on such an intermediate layer film, an organic antireflection film (BARC) may be formed on the intermediate layer film by spin coating and a photoresist film may be formed thereon.

A polysilsesquioxane-based intermediate layer is preferably used as the intermediate layer. By imparting functions as an antireflection film to the resist intermediate layer film, there is a tendency that reflection can be effectively suppressed. A specific material for the polysilsesquioxane-based intermediate layer is not limited, and, for example, a material described in Japanese Patent Application Laid-Open No. 2007-226170 or Japanese Patent Application Laid-Open No. 2007-226204 can be used.

The subsequent etching of the substrate can also be performed by a conventional method. For example, the substrate made of SiO₂ or SiN can be etched mainly using chlorofluorocarbon-based gas, and the substrate made of p-Si, Al or W can be etched mainly using chlorine- or bromine-based gas. In the case of etching the substrate with chlorofluorocarbon-based gas, the silicon-containing resist of the two-layer resist process or the silicon-containing intermediate layer of the three-layer process is peeled at the same time with substrate processing. On the other hand, in the case of etching the substrate with chlorine- or bromine-based gas, the silicon-containing resist layer or the silicon-containing intermediate layer is separately peeled and in general, peeled by dry etching using chlorofluorocarbon-based gas after substrate processing.

A feature of the underlayer film of the present embodiment is that it is excellent in etching resistance of these substrates. The substrate can be arbitrarily selected from publicly known ones for use and is not particularly limited. Examples thereof include Si, a-Si, p-Si, SiO₂, SiN, SiON, W, TiN, and Al. The substrate may be a laminate having a film to be processed (substrate to be processed) on a base material (support). Examples of such a film to be processed include various low-k films such as Si, SiO₂, SiON, SiN, p-Si, α-Si, W, W-Si, Al, Cu, and Al-Si, and stopper films thereof. A material different from that for the base material (support) is generally used. The thickness of the substrate to be processed or the film to be processed is not particularly limited, and normally, it is preferably approximately 50 to 1,000,000 nm and more preferably 75 to 500,000 nm.

### Examples

Hereinafter, the present embodiment will be described with reference to Examples, but the present embodiment is not limited to Examples.

### [Molecular weight]

The molecular weight of a compound was measured by LC-MS analysis using Acquity UPLC/MALDI-Synapt HDMS manufactured by Waters Corp. The NMR measurement conditions are shown below.

### [Structure of compound]

The structure of a compound was confirmed by ¹H-NMR measurement using "Advance 600II spectrometer" manufactured by Bruker Corp. under the following conditions.

Frequency: 400 mhz
Solvent: d6-DMSO
Internal standard: TMS
Measurement temperature: 23°C

### (Synthesis Example 1) Synthesis of BiA-1

In a container (internal capacity: 100 mL) equipped with a stirrer, a condenser tube and a burette, 2.2 g (20 mmol) of aniline hydrochloride (a reagent manufactured by Sigma-Aldrich) was placed, 1.8 g (10 mmol) of 4-biphenylaldehyde (a reagent manufactured by Sigma-Aldrich) was added, and the contents were reacted by being stirred at a reaction temperature of 190°C for a reaction time of 6 hours. After adding 1000 mL of pure water to this reaction solution, the crude compound was filtered off and obtained. The obtained crude compound was purified by column chromatography to obtain 0.3 g of a (poly)amino compound (BiA-1) represented by the following formula (BiA-1). As a result of measuring the molecular weight of the obtained compound (BiA-1), it was 350. The following peaks were found by ¹H-NMR measurement carried out on the obtained compound (BiA-1), and the compound was confirmed to have a chemical structure of the following formula (BiA-1).
δ (ppm) 6.5-7.6 (17H, Ph-H), 5.2 (1H,C-H), 4.9 (4H, NH₂)

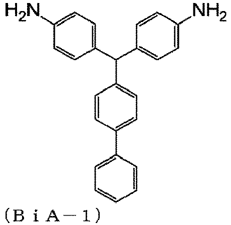

### (Synthesis Examples 2 to 17) Synthesis of BiA-2 to 17

(Poly)amine compounds represented by the following formulas (BiA-2) to (BiA-17) were obtained in the same manner as in Synthesis Example 1 except that the anilines and aldehydes described in the following Table 1 were used instead of m-aminophenol and 4-biphenylaldehyde in Synthesis Example 1.

**[Table 1]**

| Synthesis Example | Aniline | Aldehyde | Molecular weight |
|---|---|---|---|
| 2 | o-Methylethylaniline | 4-Biphenylaldehyde | 434 |
| 3 | o-Methoxyaniline | 4-Biphenylaldehyde | 410 |
| 4 | o-Phenylaniline | 4-Biphenylaldehyde | 502 |
| 5 | 2,2'-Diaminobiphenyl | 4-Biphenylaldehyde | 532 |
| 6 | Naphthylamine | 4-Biphenylaldehyde | 450 |
| 7 | Aniline | 4-Cyanobenzaldehyde | 299 |
| 8 | Aniline | Imidazole-4-carboxyaldehyde | 280 |
| 9 | Aniline | 3,4-Dimethylbenzaldehyde | 302 |
| 10 | Aniline | 2,4-Dimethylbenzaldehyde | 302 |
| 11 | Aniline | 3,4,6-Trimethylbenzaldehyde | 316 |
| 12 | Aniline | 2,4,6-Trimethylbenzaldehyde | 333 |
| 13 | Aniline | 4-Propylbenzaldehyde | 316 |
| 14 | Aniline | 4-Penthylbenzaldehyde | 330 |
| 15 | Aniline | 4-Cumylaldehyde | 330 |
| 16 | Aniline | 4-Cyclohexylbenzaldehyde | 350 |
| 17 | Aniline | 2,6-Dimethyl-4-cyclohexylbenzaldehyde | 384 |

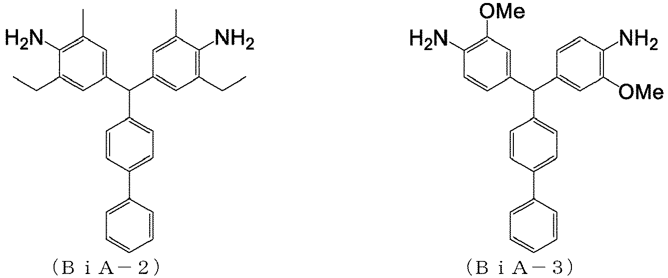

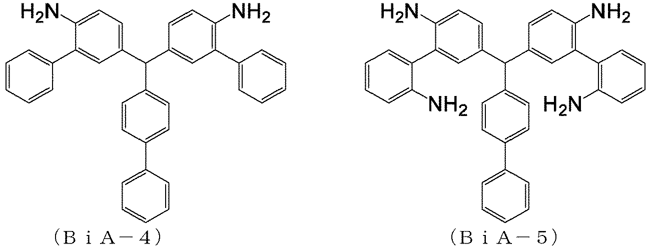

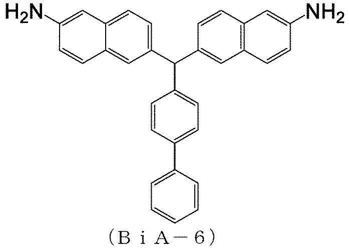

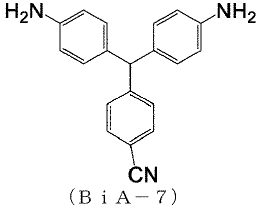

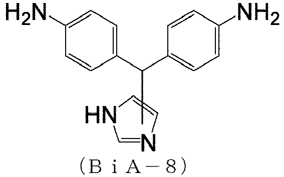

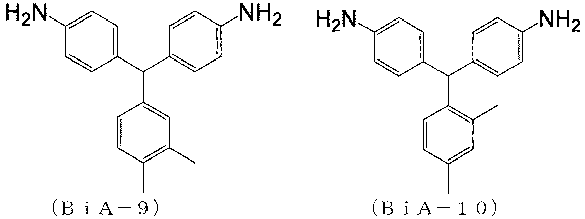

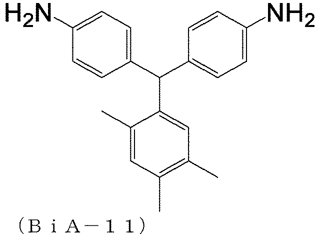

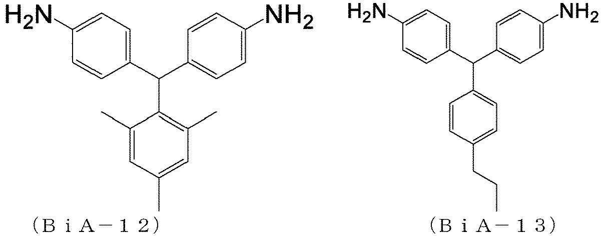

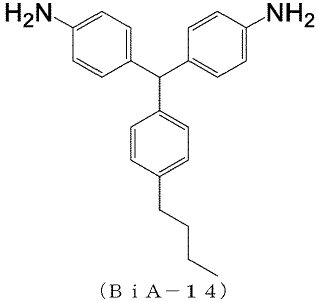

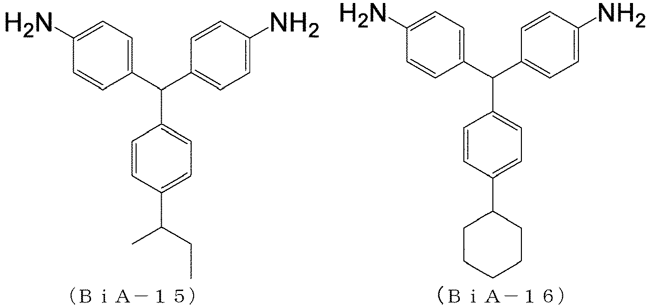

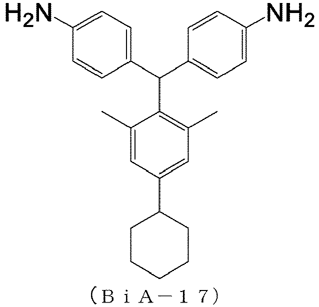

### (Synthesis Example 18) Synthesis of BiA-18

In a container (internal capacity: 100 mL) equipped with a stirrer, a condenser tube and a burette, 4.4 g (40 mmol) of aniline (a reagent manufactured by Sigma-Aldrich) and a solution of hydrogen chloride in 1,4-dioxane (20.0 ml) were placed, 1.8 g (10 mmol) of 4-biphenyldialdehyde (a reagent manufactured by Sigma-Aldrich) was added, and the contents were reacted by being stirred at a reaction temperature of 110°C for a reaction time of 8 hours. After adding 1000 mL of pure water to this reaction solution, the crude compound was filtered off and obtained. The obtained crude compound was purified by column chromatography to obtain 0.7 g of a (poly)amino compound (BiA-18) represented by the following formula (BiA-18). As a result of measuring the molecular weight of the obtained compound (BiA-18), it was 546. The following peaks were found by ¹H-NMR measurement carried out on the obtained compound (BiA-18), and the compound was confirmed to have a chemical structure of the following formula (BiA-18).
δ (ppm) 7.0-7.8 (24H, Ph-H), 6.6 (2H, C-H), 4.2 (8H, NH2)

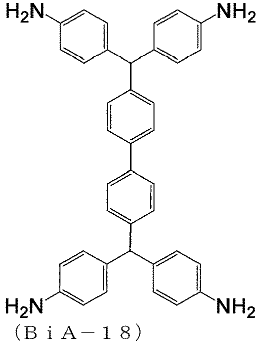

### (Synthesis Examples 19 to 25) Synthesis of BiA-19 to BiA-25

(Poly)amine compounds represented by the following formulas (BiA-19) to (BiA-25) were obtained in the same manner as in Synthesis Example 18 except that the anilines and aldehydes described in the following Table 2 were used instead of aniline and 4-biphenyldialdehyde in Synthesis Example 18.

**[Table 2]**

| Synthesis Example | Aniline | Aldehyde | Molecular weight |
|---|---|---|---|
| 19 | o-Methylethylaniline | 4,4'-Biphenyldialdehyde | 714 |
| 20 | o-Methoxyaniline | 4,4'-Biphenyldialdehyde | 666 |
| 21 | o-Phenylaniline | 4,4'-Biphenyldialdehyde | 850 |
| 22 | 2,2'-Diaminobiphenyl | 4,4'-Biphenyldialdehyde | 910 |
| 23 | Naphthylamine | 4,4'-Biphenyldialdehyde | 746 |
| 24 | Aniline | Terephthalaldehyde | 470 |
| 25 | Aniline | Isophthalaldehyde | 470 |

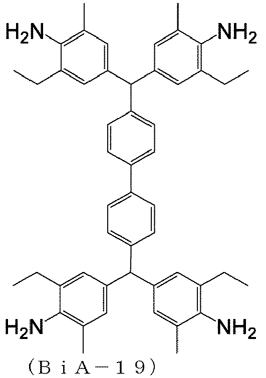

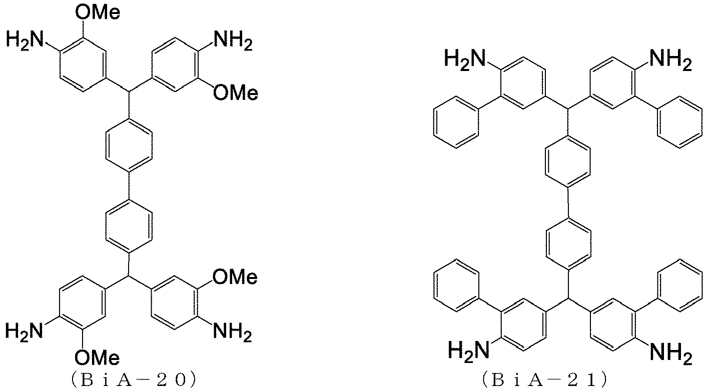

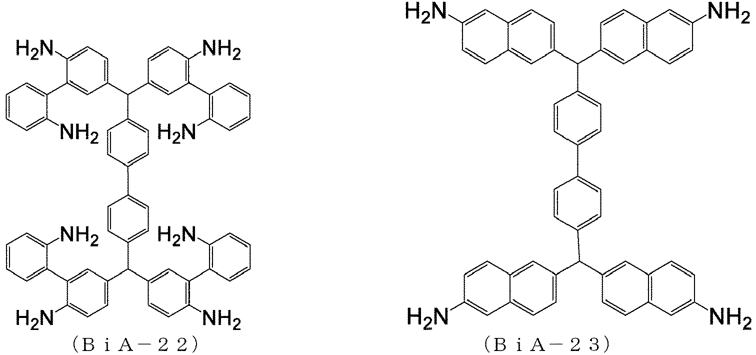

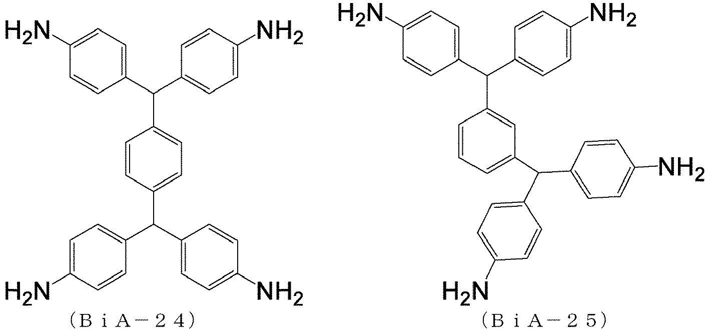

### (Synthesis Example 26) Synthesis of BiA-26

In a container (internal capacity: 100 mL) equipped with a stirrer, a condenser tube and a burette, 2.2 g (20 mmol) of aniline (a reagent manufactured by Sigma-Aldrich) and a solution of hydrogen chloride in 1,4-dioxane (20.0 ml) were placed, 1.3 g (10 mmol) of 4-acetylbiphenyl (a reagent manufactured by Sigma-Aldrich) was added, and the contents were reacted by being stirred at a reaction temperature of 80°C for a reaction time of 18 hours. After adding 1000 mL of pure water to this reaction solution, the crude compound was filtered off and obtained. The obtained crude compound was purified by column chromatography to obtain 0.9 g of a (poly)amino compound (BiA-26) represented by the following formula (BiA-26). As a result of measuring the molecular weight of the obtained compound (BiA-26), it was 364. The following peaks were found by ¹H-NMR measurement carried out on the obtained compound (BiA-26), and the compound was confirmed to have a chemical structure of the following formula (BiA-26).
δ (ppm) 7.0-7.8 (17H, Ph-H), 4.1 (4H, NH2), 2.3(3H,CH3)

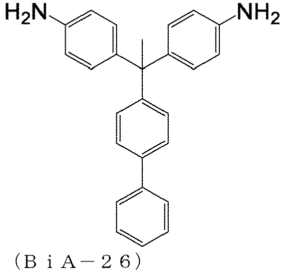

### (Synthesis Examples 27 to 33) Synthesis of BiA-27 to BiA-33

(Poly)amine compounds represented by the following formulas (BiA-27) to (BiA-33) were obtained in the same manner as in Synthesis Example 26 except that the anilines and ketones described in the following Table 3 were used instead of aniline and 4-acetylbiphenyl in Synthesis Example 26.

**[Table 3]**

| Synthesis Example | Aniline | Aldehyde | Molecular weight |
|---|---|---|---|
| 27 | m-Aminophenol | 4-Acetylbiphenyl | 396 |
| 28 | o-Methylethylaniline | 4-Acetylbiphenyl | 448 |
| 29 | o-Methoxyaniline | 4-Acetylbiphenyl | 424 |
| 30 | o-Phenylaniline | 4-Acetylbiphenyl | 516 |
| 31 | 2,2'-Diaminobiphenyl | 4-Acetylbiphenyl | 546 |
| 32 | Naphthylamine | 4-Acetylbiphenyl | 464 |
| 33 | Aniline | 4-Acetophenone | 288 |

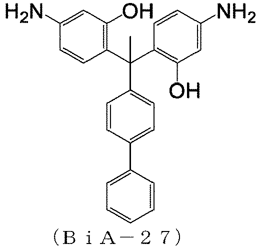

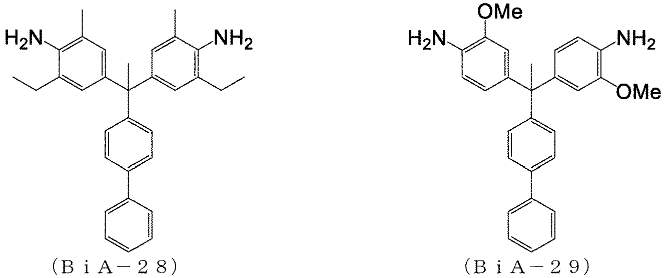

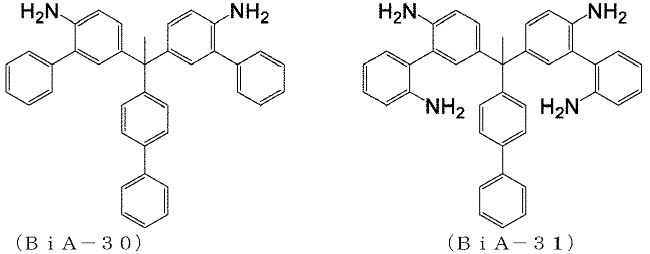

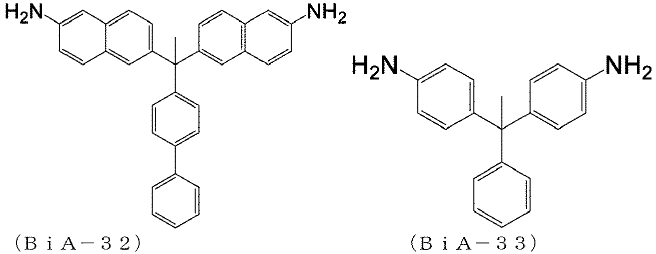

### (Example 1-1) Synthesis of MABiA-1

In a container (internal capacity: 100 mL) equipped with a stirrer, a condenser tube and a burette, 1.0 g of BiA-1 and 10 ml of acetone were placed, 0.62 g (6.3 mmol) of maleic anhydride (a product manufactured by Mitsubishi Gas Chemical Company, Inc.) was added, and the contents were reacted by being stirred at room temperature for 2 hours. The precipitates were filtered off to obtain the crude compound. The obtained crude compound was purified by column chromatography to obtain 0.3 g of a (poly)maleamic acid (MABiA-1) represented by the following formula (MABiA-1). As a result of measuring the molecular weight of the obtained compound (MABiA-1), it was 546. The following peaks were found by ¹H-NMR measurement carried out on the obtained compound (MABiA-1), and the compound was confirmed to have a chemical structure of the following formula (MABiA-1).
δ (ppm) 13.2 (2H, -COO-H), 10.4 (2H, N-H), 7.1-7.6 (17H,Ph-H), 6.3-6.5 (4H, -CH=CH-), 5.6 (1H, C-H)

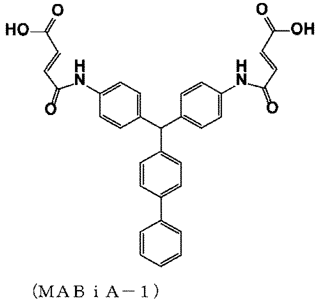

### (Examples 2-1 to 34-1) Synthesis of MABiA-2 to MABiA-34

(Poly)amic acids represented by the following formulas (MABiA-2) to (MABiA-34) were obtained in the same manner as in Example 1-1 except that the (poly)amine compounds and acid anhydrides described in the following Table 4 were used instead of BiA-1 and maleic anhydride in Example 1-1.

**[Table 4]**

| Example | (Poly)amine compound | Acid anhydride | Produced (poly)amic acid | Molecular weight |
|---|---|---|---|---|
| 2-1 | BiA-2 | Maleic anhydride | MABiA-2 | 630 |
| 3-1 | BiA-3 | Maleic anhydride | MABiA-3 | 606 |
| 4-1 | BiA-4 | Maleic anhydride | MABiA-4 | 698 |
| 5-1 | BiA-5 | Maleic anhydride | MABiA-5 | 924 |
| 6-1 | BiA-6 | Maleic anhydride | MABiA-6 | 646 |
| 7-1 | BiA-7 | Maleic anhydride | MABiA-7 | 495 |
| 8-1 | BiA-8 | Maleic anhydride | MABiA-8 | 460 |
| 9-1 | BiA-9 | Maleic anhydride | MABiA-9 | 498 |
| 10-1 | BiA-10 | Maleic anhydride | MABiA-10 | 498 |
| 11-1 | BiA-11 | Maleic anhydride | MABiA-11 | 512 |
| 12-1 | BiA-12 | Maleic anhydride | MABiA-12 | 512 |
| 13-1 | BiA-13 | Maleic anhydride | MABiA-13 | 512 |
| 14-1 | BiA-14 | Maleic anhydride | MABiA-14 | 526 |
| 15-1 | BiA-15 | Maleic anhydride | MABiA-15 | 526 |
| 16-1 | BiA-16 | Maleic anhydride | MABiA-16 | 552 |
| 17-1 | BiA-17 | Maleic anhydride | MABiA-17 | 580 |
| 18-1 | BiA-18 | Maleic anhydride | MABiA-18 | 938 |
| 19-1 | BiA-19 | Maleic anhydride | MABiA-19 | 1106 |
| 20-1 | BiA-20 | Maleic anhydride | MABiA-20 | 1058 |
| 21-1 | BiA-21 | Maleic anhydride | MABiA-21 | 1242 |
| 22-1 | BiA-22 | Maleic anhydride | MABiA-22 | 1694 |
| 23-1 | BiA-23 | Maleic anhydride | MABiA-23 | 1138 |
| 24-1 | BiA-24 | Maleic anhydride | MABiA-24 | 862 |
| 25-1 | BiA-25 | Maleic anhydride | MABiA-25 | 862 |
| 26-1 | BiA-26 | Maleic anhydride | MABiA-26 | 560 |
| 27-1 | BiA-27 | Maleic anhydride | MABiA-27 | 592 |
| 28-1 | BiA-28 | Maleic anhydride | MABiA-28 | 644 |
| 29-1 | BiA-29 | Maleic anhydride | MABiA-29 | 620 |
| 30-1 | BiA-30 | Maleic anhydride | MABiA-30 | 712 |
| 31-1 | BiA-31 | Maleic anhydride | MABiA-31 | 938 |
| 32-1 | BiA-32 | Maleic anhydride | MABiA-32 | 660 |
| 33-1 | BiA-33 | Maleic anhydride | MABiA-33 | 484 |
| 34-1 | BiA-1 | Citraconic anhydride | MABiA-34 | 574 |

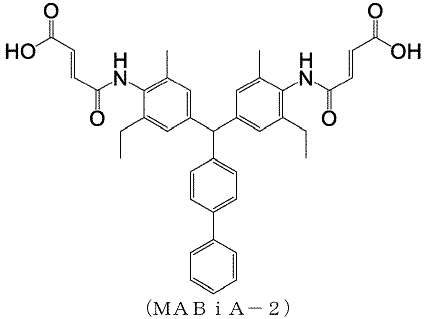

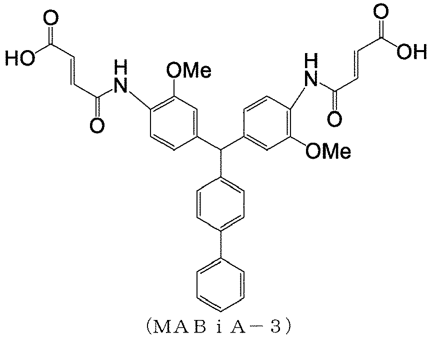

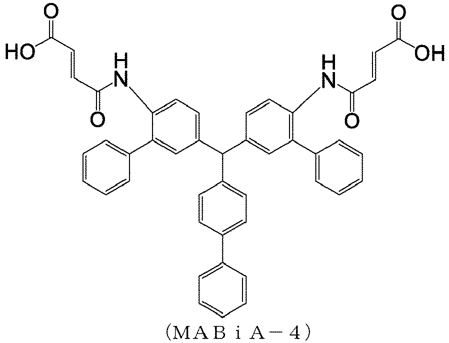

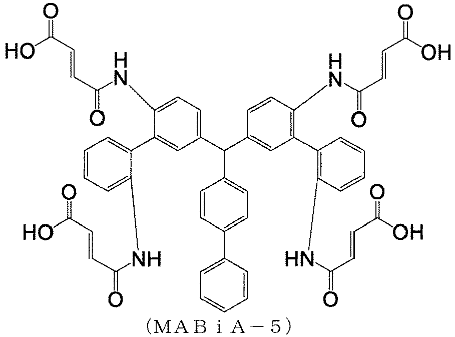

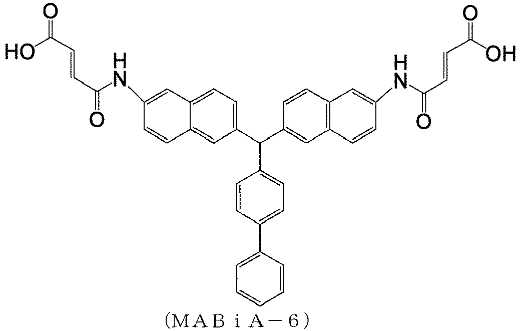

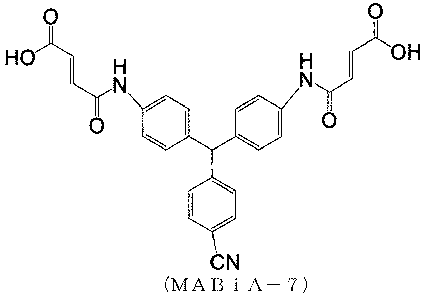

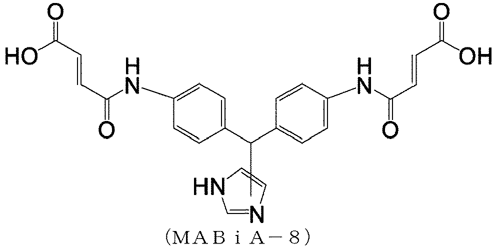

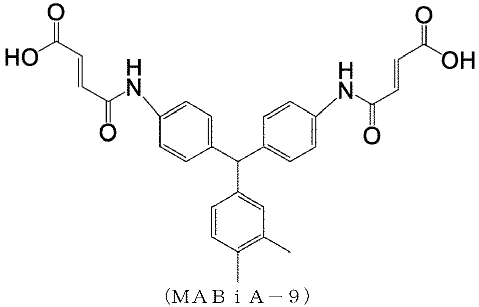

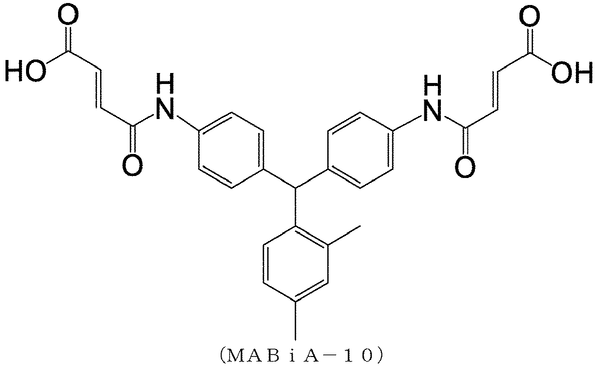

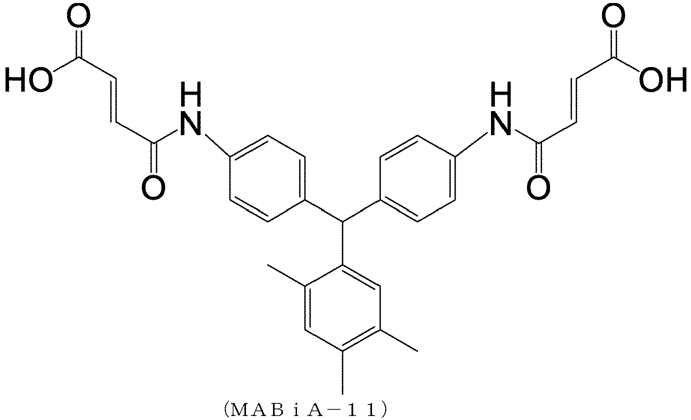

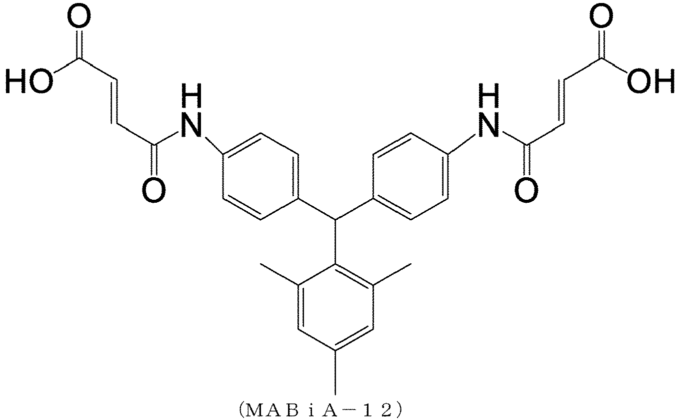

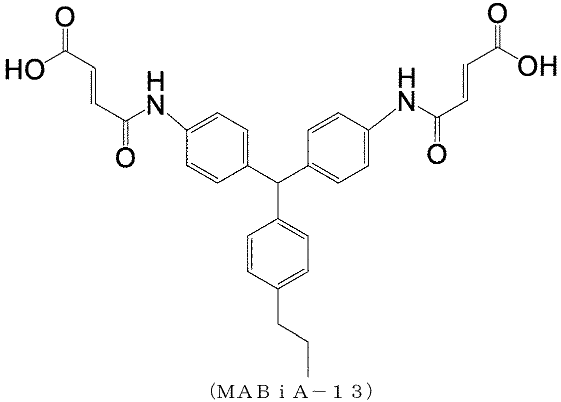

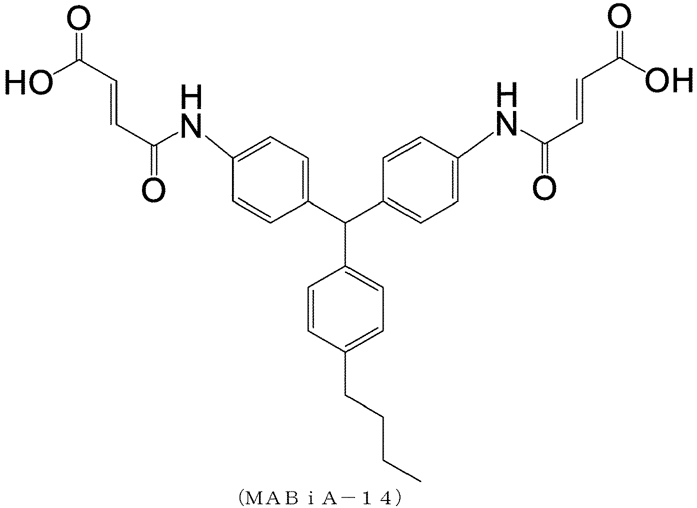

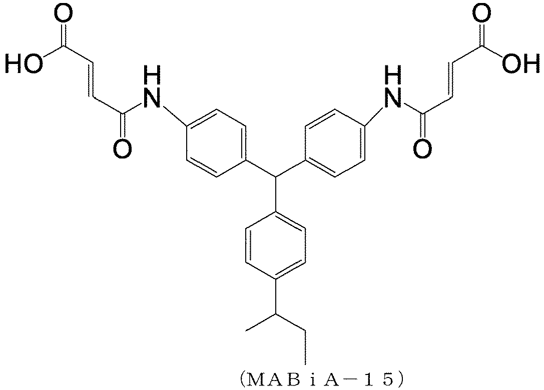

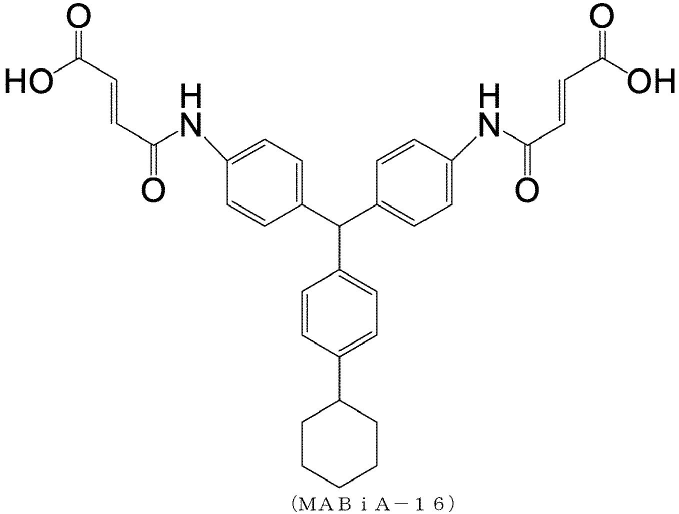

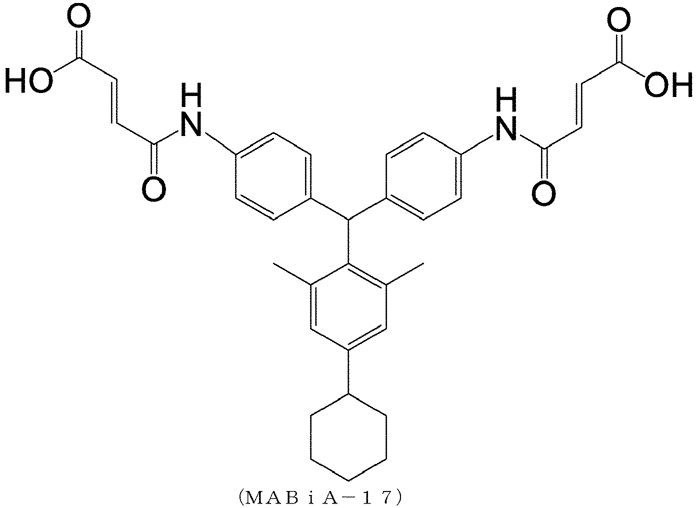

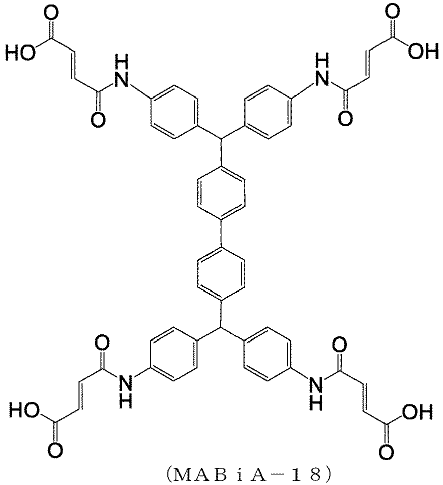

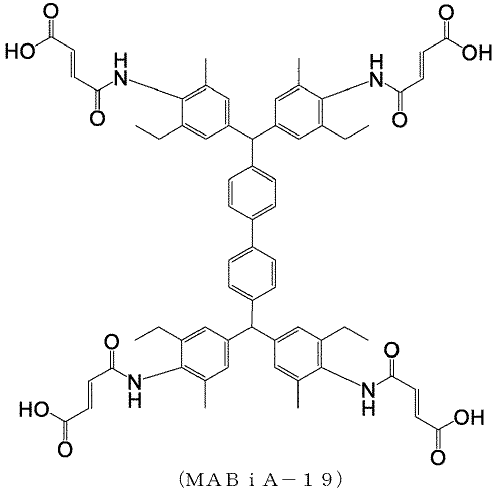

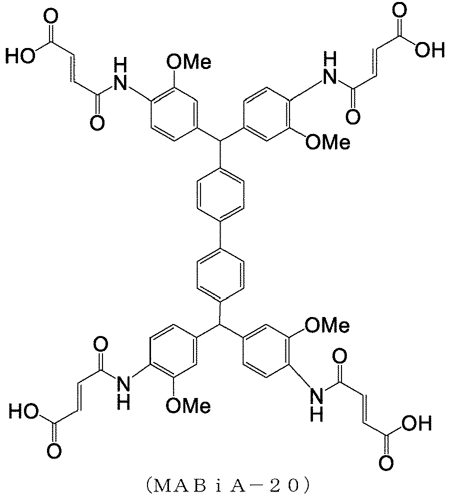

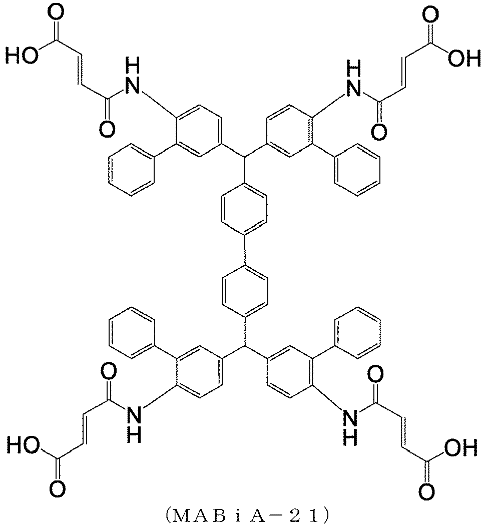

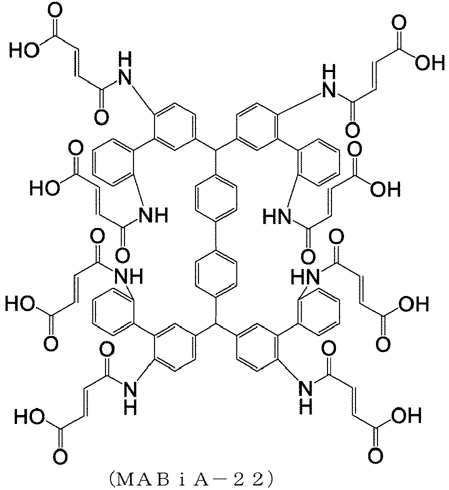

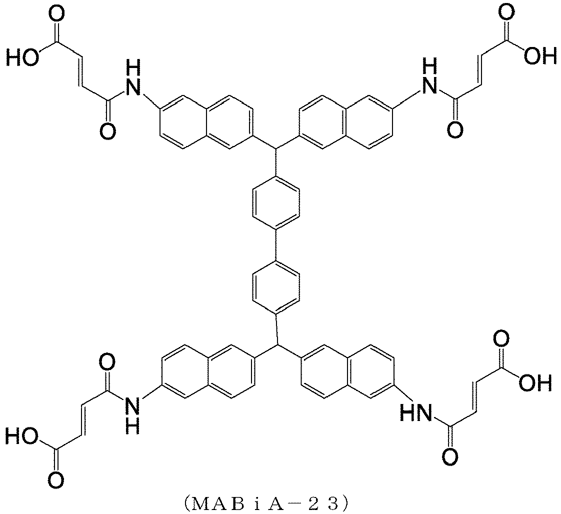

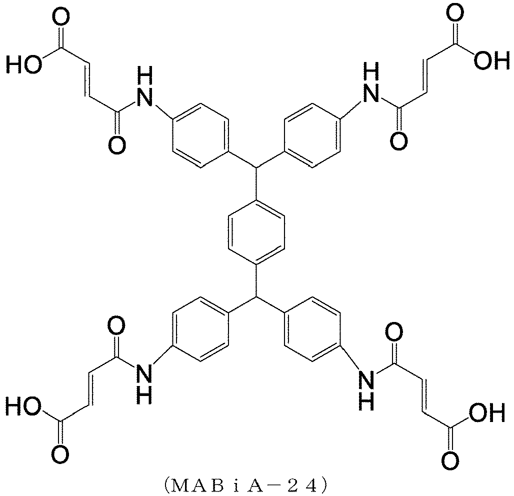

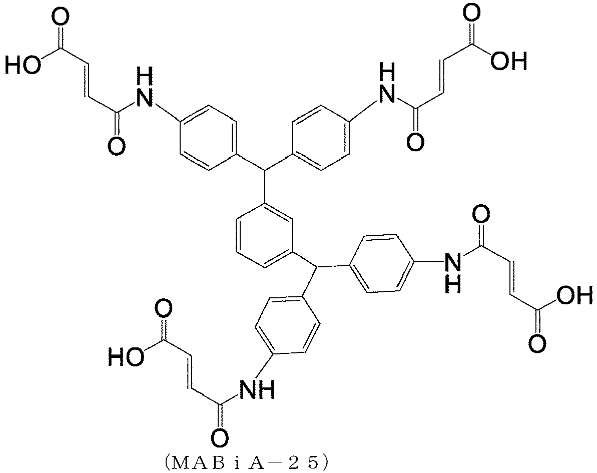

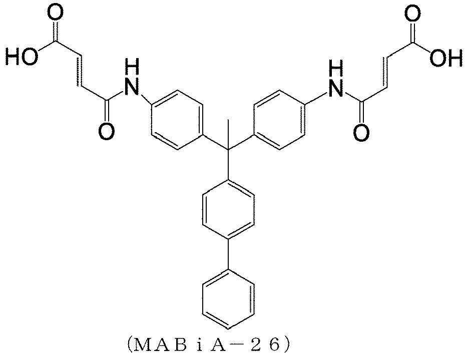

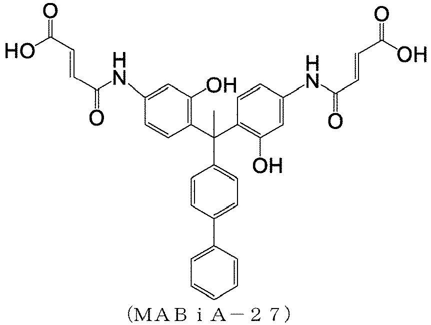

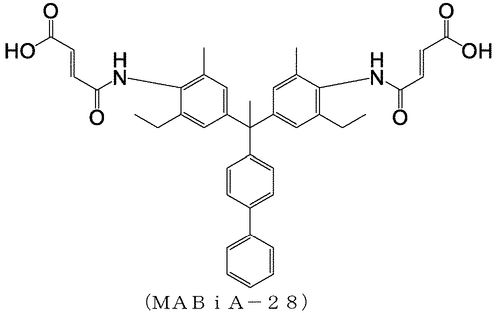

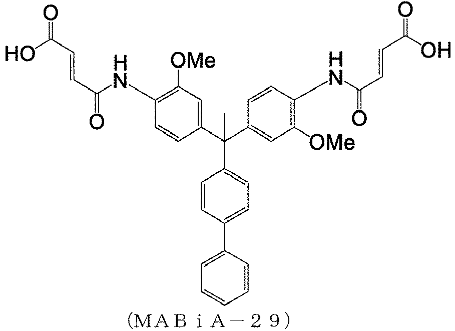

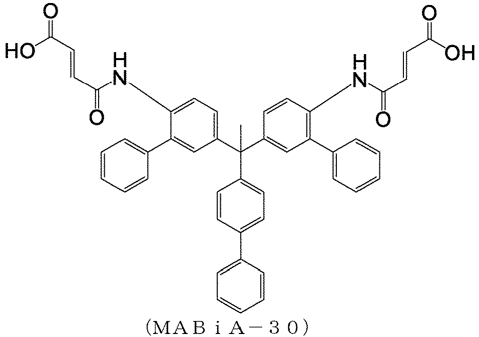

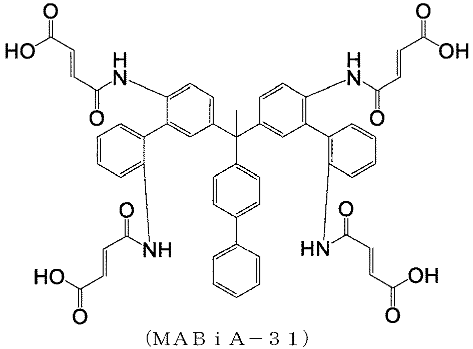

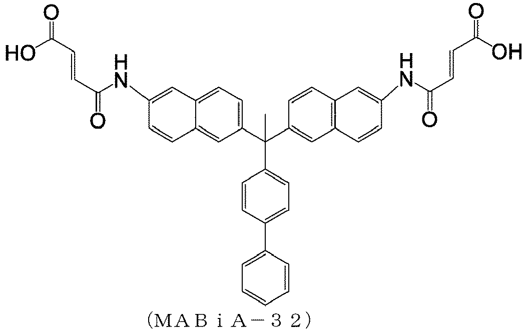

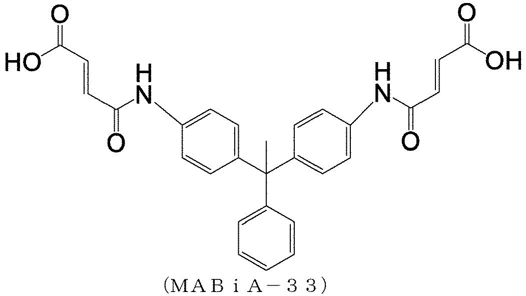

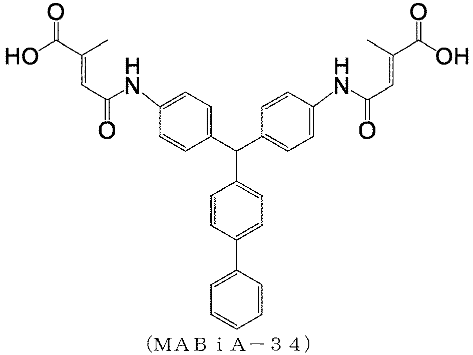

### (Example 1-2) Synthesis of MIBiA-1

In a container (internal capacity: 100 mL) equipped with a stirrer, a condenser tube and a burette, 1.87 g of MABiA-1 and 6.2 ml of NMP were placed and dissolved, 1.66 g of sodium acetate (anhydride) and 3.08 g of acetic anhydride were added, and the contents were reacted by being stirred at 50°C for 2 hours. After adding 1000 mL of pure water to this reaction solution, the crude compound was filtered off and obtained. The obtained crude compound was purified by column chromatography to obtain 0.3 g of a (poly)maleimide (MIBiA-1) represented by the following formula (MIBiA-1). As a result of measuring the molecular weight of the obtained compound (MIBiA-1), it was 510. The following peaks were found by ¹H-NMR measurement carried out on the obtained compound (MIBiA-1), and the compound was confirmed to have a chemical structure of the following formula (MIBiA-1).
δ (ppm) 7.2-7.7 (21H, Ph-H + maleimide skeleton methine H), 5.8 (1H, C-H)

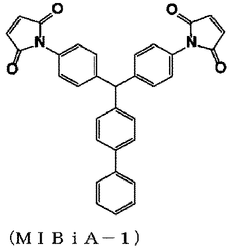

### (Examples 2-2 to 34-2) Synthesis of MIBiA-2 to MIBiA-34

(Poly)imides represented by the following formulas (MIBiA-2) to (MIBiA-34) were obtained in the same manner as in Example 2-1 except that the (poly)amic acids described in the following Table 5 were used instead of MABiA-1 in Example 1-2.

**[Table 5]**

| Example | (Poly)amic acid | Produced (poly)imide | Molecular weiqht |
|---|---|---|---|
| 2-2 | MABiA-2 | MIBiA-2 | 594 |
| 3-2 | MABiA-3 | MIBiA-3 | 570 |
| 4-2 | MABiA-4 | MIBiA-4 | 662 |
| 5-2 | MABiA-5 | MIBiA-5 | 852 |
| 6-2 | MABiA-6 | MIBiA-6 | 610 |
| 7-2 | MABiA-7 | MIBiA-7 | 459 |
| 8-2 | MABiA-8 | MIBiA-8 | 424 |
| 9-2 | MABiA-9 | MIBiA-9 | 462 |
| 10-2 | MABiA-10 | MIBiA-10 | 462 |
| 11-2 | MABiA-11 | MIBiA-11 | 476 |
| 12-2 | MABiA-12 | MIBiA-12 | 476 |
| 13-2 | MABiA-13 | MIBiA-13 | 476 |
| 14-2 | MABiA-14 | MIBiA-14 | 490 |
| 15-2 | MABiA-15 | MIBiA-15 | 490 |
| 16-2 | MABiA-16 | MIBiA-16 | 516 |
| 17-2 | MABiA-17 | MIBiA-17 | 544 |
| 18-2 | MABiA-18 | MIBiA-18 | 866 |
| 19-2 | MABiA-19 | MIBiA-19 | 1034 |
| 20-2 | MABiA-20 | MIBiA-20 | 986 |
| 21-2 | MABiA-21 | MIBiA-21 | 1170 |
| 22-2 | MABiA-22 | MIBiA-22 | 1550 |
| 23-2 | MABiA-23 | MIBiA-23 | 1066 |
| 24-2 | MABiA-24 | MIBiA-24 | 790 |
| 25-2 | MABiA-25 | MIBiA-25 | 790 |
| 26-2 | MABiA-26 | MIBiA-26 | 524 |
| 27-2 | MABiA-27 | MIBiA-27 | 556 |
| 28-2 | MABiA-28 | MIBiA-28 | 608 |
| 29-2 | MABiA-29 | MIBiA-29 | 584 |
| 30-2 | MABiA-30 | MIBiA-30 | 676 |
| 31-2 | MABiA-31 | MIBiA-31 | 866 |
| 32-2 | MABiA-32 | MIBiA-32 | 624 |
| 33-2 | MABiA-33 | MIBiA-33 | 448 |
| 34-2 | MABiA-34 | MIBiA-34 | 538 |

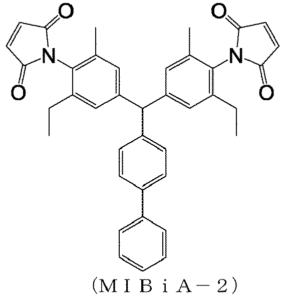

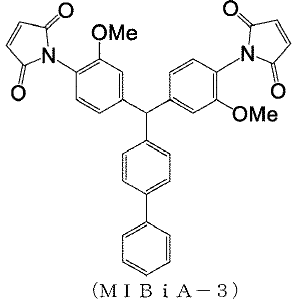

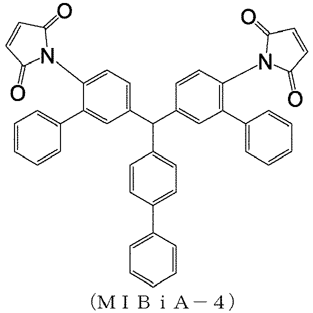

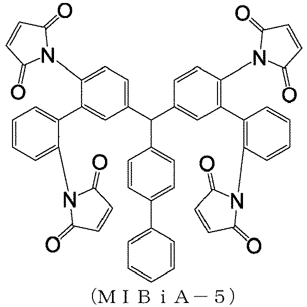

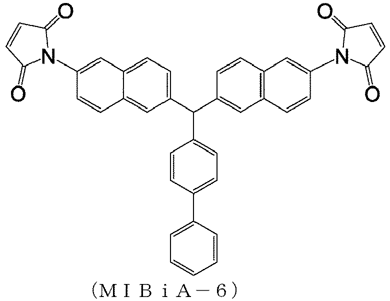

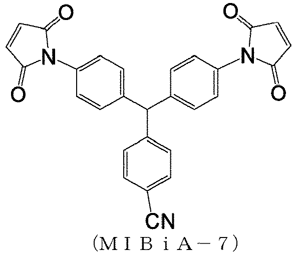

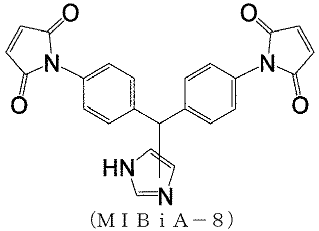

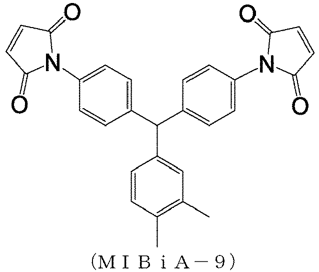

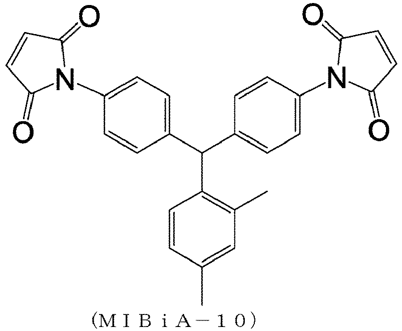

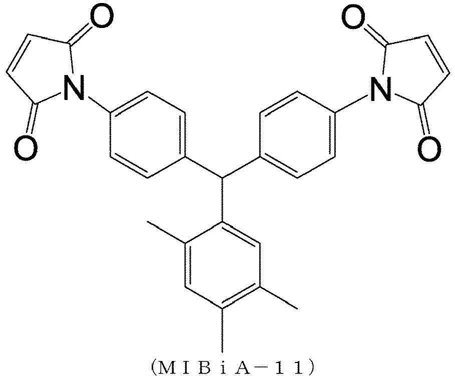

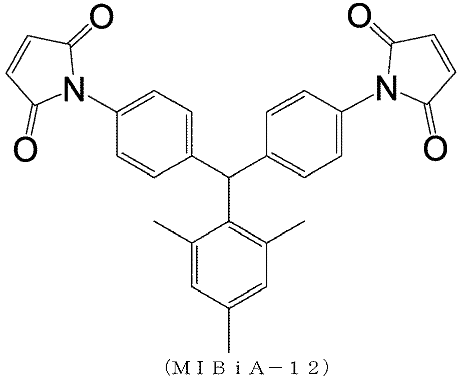

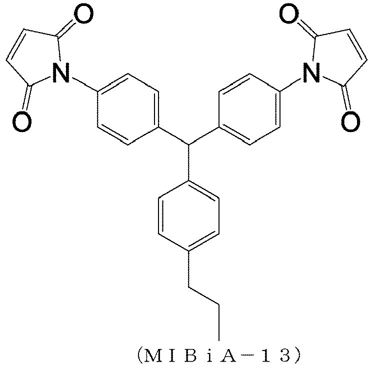

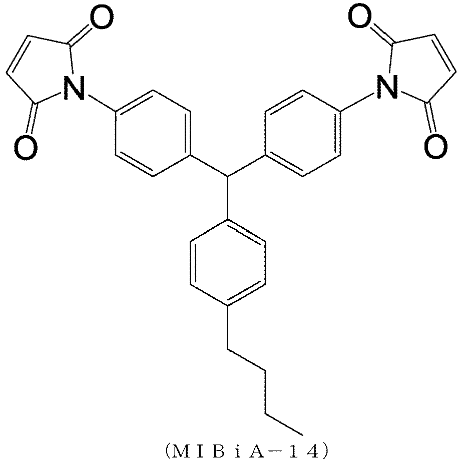

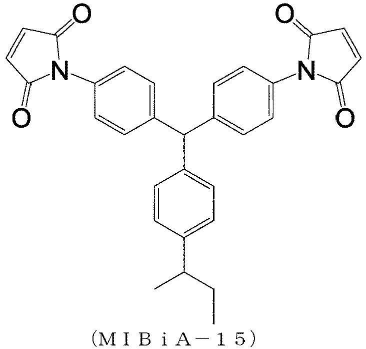

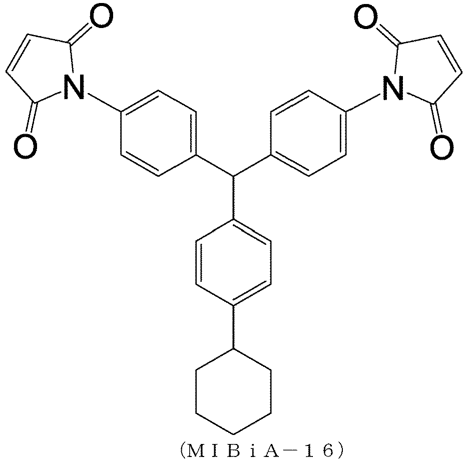

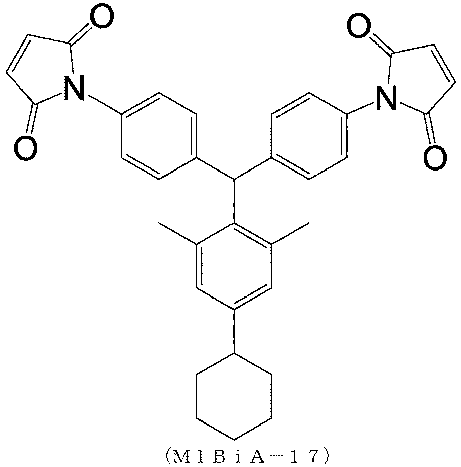

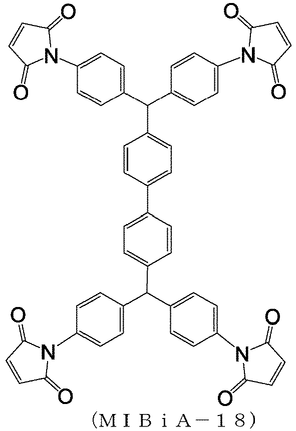

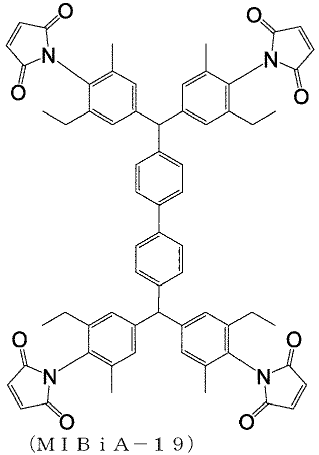

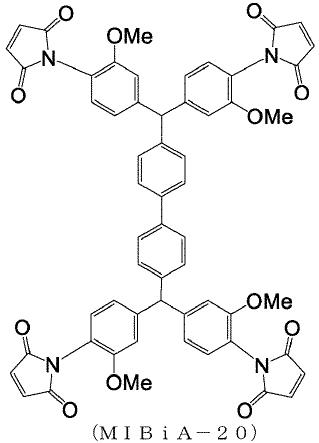

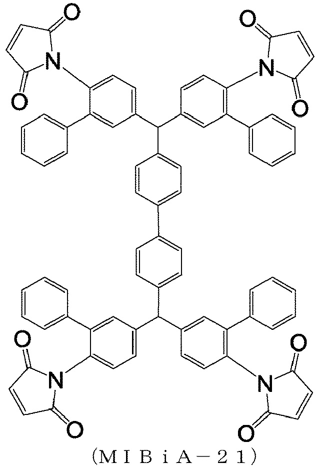

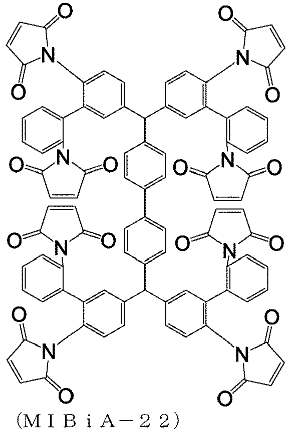

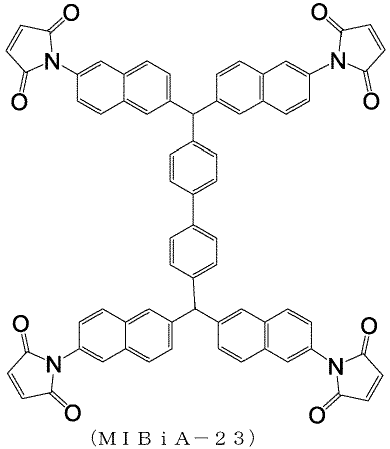

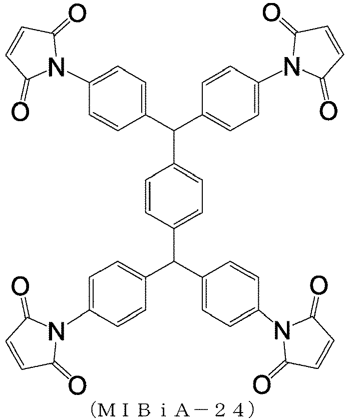

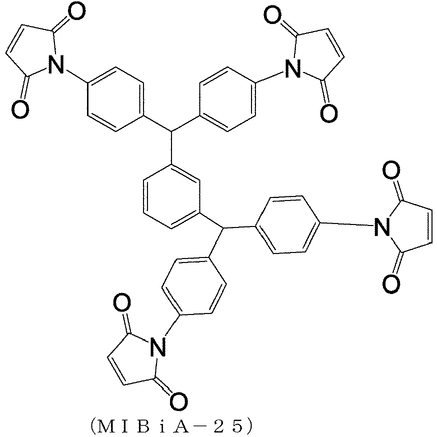

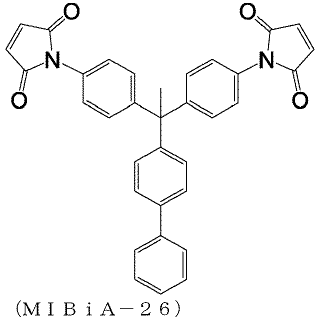

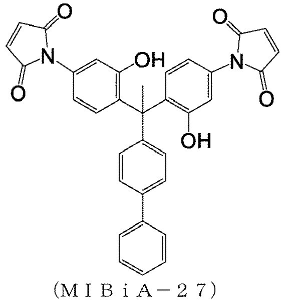

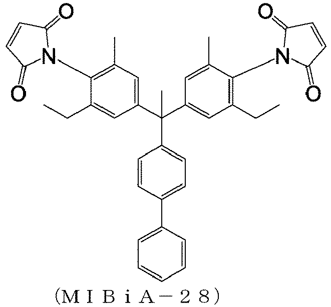

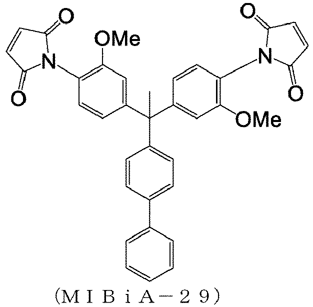

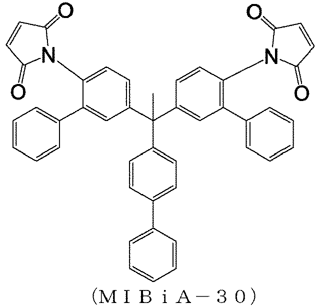

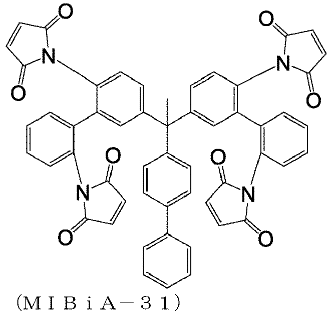

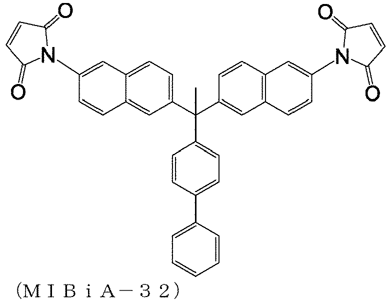

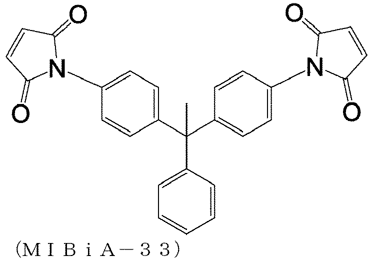

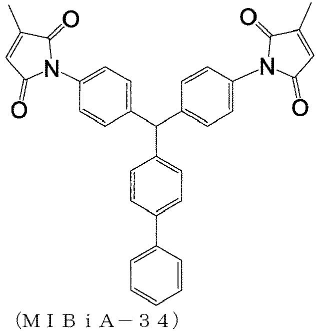

The solvent solubility of the compounds obtained in Examples 1-1 to 34-1 and 1-2 to 34-2, and of bis(4-maleimidophenyl)methane (BMI) (a product manufactured by K·I Chemical Industry Co., LTD.) as a Comparative Example 1 in ethyl acetate at room temperature was evaluated. When the total amount of each compound and ethyl acetate is 100 parts by mass, the case where each compound is soluble in an amount of 20 parts by mass or more is marked as ○, and the other case is marked as ×. The evaluation results are shown in Tables 6 and 7.

**[Table 6]**

| Example | Compound | Solvent solubility |
|---|---|---|
| Example 1-1 | MABiA-1 | ○ |
| Example 2-1 | MABiA-2 | ○ |
| Example 3-1 | MABiA-3 | ○ |
| Example 4-1 | MABiA-4 | ○ |
| Example 5-1 | MABiA-5 | ○ |
| Example 6-1 | MABiA-6 | ○ |
| Example 7-1 | MABiA-7 | ○ |
| Example 8-1 | MABiA-8 | ○ |
| Example 9-1 | MABiA-9 | ○ |
| Example 10-1 | MABiA-10 | ○ |
| Example 11-1 | MABiA-11 | ○ |
| Example 12-1 | MABiA-12 | ○ |
| Example 13-1 | MABiA-13 | ○ |
| Example 14-1 | MABiA-14 | ○ |
| Example 15-1 | MABiA-15 | ○ |
| Example 16-1 | MABiA-16 | ○ |
| Example 17-1 | MABiA-17 | ○ |
| Example 18-1 | MABiA-18 | ○ |
| Example 19-1 | MABiA-19 | ○ |
| Example 20-1 | MABiA-20 | ○ |
| Example 21-1 | MABiA-21 | ○ |
| Example 22-1 | MABiA-22 | ○ |
| Example 23-1 | MABiA-23 | ○ |
| Example 24-1 | MABiA-24 | ○ |
| Example 25-1 | MABiA-25 | ○ |
| Example 26-1 | MABiA-26 | ○ |
| Example 27-1 | MABiA-27 | ○ |
| Example 28-1 | MABiA-28 | ○ |
| Example 29-1 | MABiA-29 | ○ |
| Example 30-1 | MABiA-30 | ○ |
| Example 31-1 | MABiA-31 | ○ |
| Example 32-1 | MABiA-32 | ○ |
| Example 33-1 | MABiA-33 | ○ |
| Example 34-1 | MABiA-34 | ○ |
| Example 1-2 | MIBiA-1 | ○ |
| Example 2-2 | MIBiA-2 | ○ |
| Example 3-2 | MIBiA-3 | ○ |
| Example 4-2 | MIBiA-4 | ○ |
| Example 5-2 | MIBiA-5 | ○ |
| Example 6-2 | MIBiA-6 | ○ |
| Example 7-2 | MIBiA-7 | ○ |
| Example 8-2 | MIBiA-8 | ○ |
| Example 9-2 | MIBiA-9 | ○ |
| Example 10-2 | MIBiA-10 | ○ |

**[Table 7]**

| | | |
|---|---|---|
| Example 11-2 | MIBiA-11 | ○ |
| Example 12-2 | MIBiA-12 | ○ |
| Example 13-2 | MIBiA-13 | ○ |
| Example 14-2 | MIBiA-14 | ○ |
| Example 15-2 | MIBiA-15 | ○ |
| Example 16-2 | MIBiA-16 | ○ |
| Example 17-2 | MIBiA-17 | ○ |
| Example 18-2 | MIBiA-18 | ○ |
| Example 19-2 | MIBiA-19 | ○ |
| Example 20-2 | MIBiA-20 | ○ |
| Example 21-2 | MIBiA-21 | ○ |
| Example 22-2 | MIBiA-22 | ○ |
| Example 23-2 | MIBiA-23 | ○ |
| Example 24-2 | MIBiA-24 | ○ |
| Example 25-2 | MIBiA-25 | ○ |
| Example 26-2 | MIBiA-26 | ○ |
| Example 27-2 | MIBiA-27 | ○ |
| Example 28-2 | MIBiA-28 | ○ |
| Example 29-2 | MIBiA-29 | ○ |
| Example 30-2 | MIBiA-30 | ○ |
| Example 31-2 | MIBiA-31 | ○ |
| Example 32-2 | MIBiA-32 | ○ |
| Example 33-2 | MIBiA-33 | ○ |
| Example 34-2 | MIBiA-34 | ○ |
| Comparative Example 1 | BMI | × |

As can be understood from Tables 6 and 7, it was found that the (poly)imide compounds and (poly)amic acids of the present embodiment have excellent solubility in ethyl acetate, which is a general purpose solvent, compared to BMI.

### (Example 35) Synthesis of RMIBiA-1

To a container (internal capacity: 500 mL) equipped with a stirrer, a condenser tube and a burette, 43 g (329 mmol) of aniline hydrochloride and 20 g (109 mmol) of 4-biphenylaldehyde were added, and the contents were reacted by being stirred at a reaction temperature of 190°C for a reaction time of 6 hours in a nitrogen atmosphere. To this reaction solution, 48 mL of an aqueous 5M sodium hydroxide solution and 300 mL of water were added, and the precipitates were filtered off. Water was added to the precipitates for washing with water, filtered off, and then dried to obtain 26 g of an aniline resin (RMiA-1) represented by the following formula (RMiA-1).

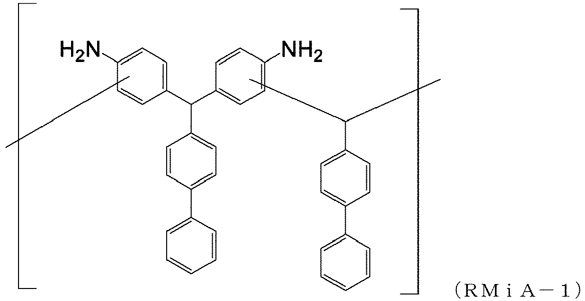

In a container (internal capacity: 100 mL) equipped with a stirrer, a condenser tube and a burette, 2 g of the resin (RMiA-1) obtained as described above was dissolved in 10 mL of γ-butyrolactone and added, 0.7 g of maleic anhydride was added in a nitrogen atmosphere, and the contents were reacted by being stirred at room temperature for 5 hours. Next, 0.2 mL of methanesulfonic acid was added, and the contents were reacted by being stirred at a reaction temperature of 120°C for a reaction time of 4 hours. This reaction solution was slowly added to 100 mL of water and 10 mL of saturated sodium bicarbonate was further added. The precipitates were filtered off and dried to obtain 2.6 g of a resin (RMIBiA-1) represented by the following formula (RMIBiA-1).

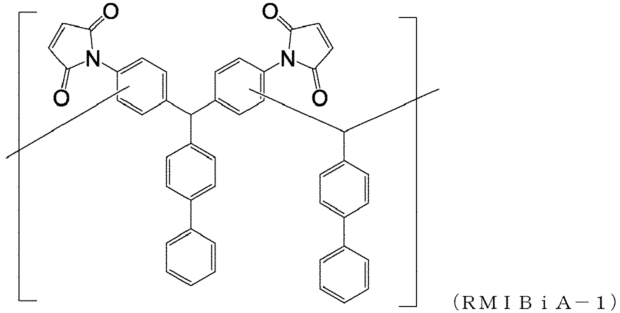

### <Production Example 1>

A four necked flask (internal capacity: 10 L) equipped with a Dimroth condenser tube, a thermometer, and a stirring blade and having a detachable bottom was prepared. To this four necked flask, 1.09 kg (7 mol) of 1,5-dimethylnaphthalene (manufactured by Mitsubishi Gas Chemical Co., Inc.), 2.1 kg (28 mol as formaldehyde) of 40% by mass of an aqueous formalin solution (manufactured by Mitsubishi Gas Chemical Co., Inc.), and 0.97 ml of 98% by mass of sulfuric acid (manufactured by Kanto Chemical Co., Inc.) were fed in the current of nitrogen, and the mixture was reacted for 7 hours while being refluxed at 100°C at normal pressure. Subsequently, 1.8 kg of ethylbenzene (manufactured by Wako Pure Chemical Industries, Ltd., a special grade reagent) was added as a diluting solvent to the reaction solution, and the mixture was left to stand still, followed by removal of an aqueous phase as a lower phase. Neutralization and washing with water were further carried out, and ethylbenzene and unreacted 1,5-dimethylnaphthalene were distilled off under reduced pressure to obtain 1.25 kg of a dimethylnaphthalene formaldehyde resin as a light brown solid.

The molecular weight of the obtained dimethylnaphthalene formaldehyde resin was as follows: number average molecular weight (Mn): 562, weight average molecular weight (Mw): 1168, and dispersity (Mw/Mn): 2.08.

Subsequently, a four necked flask (internal capacity: 0.5 L) equipped with a Dimroth condenser tube, a thermometer, and a stirring blade was prepared. To this four necked flask, 100 g (0.51 mol) of the dimethylnaphthalene formaldehyde resin obtained as described above and 0.05 g of p-toluenesulfonic acid were placed in the current of nitrogen, and the temperature was raised to 190°C at which the mixture was then heated for 2 hours, followed by stirring. Subsequently, 52.0 g (0.36 mol) of 1-naphthol was further added thereto, and the temperature was further raised to 220°C at which the mixture was allowed to react for 2 hours. After solvent dilution, neutralization and washing with water were carried out, and the solvent was removed under reduced pressure to obtain 126.1 g of a modified resin (CR-1) as a black brown solid.

The obtained resin (CR-1) had Mn: 885, Mw: 2220, and Mw/Mn: 4.17.

### <Examples 36 to 80 and Comparative Examples 2 to 3>

Each of the compositions for film formation for lithography of Examples 36 to 80 and Comparative Examples 2 to 3 was prepared using film forming materials for lithography obtained in the above Examples 1-2 to 34-2 and 35 and the resin obtained in the above Production Example 1 according to the compositions shown in Table 8. Then, a silicon substrate was spin coated with each of these compositions for film formation for lithography of Examples 36 to 80 and Comparative Examples 2 to 3, and then baked at 240°C for 60 seconds and further at 400°C for 120 seconds to prepare each underlayer film with a film thickness of 200 nm. From the difference in film thickness before and after the baking at 400°C, the decreasing rate of film thickness (%) was calculated to evaluate the film heat resistance of each underlayer film. Then, the etching resistance was evaluated under the conditions shown below.

In addition, the embedding properties to a substrate having difference in level and the flatness were evaluated under the conditions shown below.

When used, the following crosslinking agent and crosslinking promoting agent were employed.

Crosslinking agent: benzoxazine (BF-BXZ; manufactured by Konishi Chemical Ind. Co., Ltd.) represented by the following formula or a biphenyl aralkyl-based epoxy resin (NC-3000-L; manufactured by Nippon Kayaku Co., Ltd.) represented by the following formula

### Crosslinking promoting agent: 2,4,5-triphenylimidazole (TPIZ)

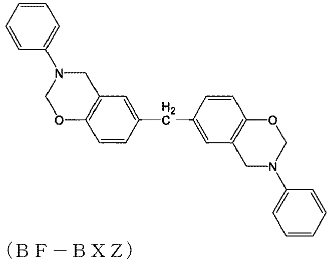

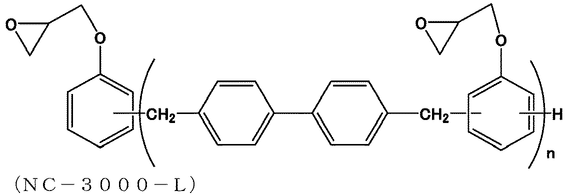

(In the above formula, n is an integer of 1 to 4.)

### [Evaluation of film heat resistance]

### <Evaluation criteria>

S: Decreasing rate of film thickness before and after baking at 400°C ≤ 10%
A: Decreasing rate of film thickness before and after baking at 400°C ≤ 15%
B: Decreasing rate of film thickness before and after baking at 400°C ≤ 20%
C: Decreasing rate of film thickness before and after baking at 400°C > 20%

### [Etching test]

Etching apparatus: RIE-10NR manufactured by Samco International, Inc.
Output: 50 W
Pressure: 4 Pa
Time: 2 min
Etching gas

### CF₄ gas flow rate:O₂ gas flow rate = 5:15 (sccm)

### [Evaluation of etching resistance]

The evaluation of etching resistance was carried out by the following procedures.

First, an underlayer film of novolac was prepared under the same conditions as Example 36 except that novolac (PSM 4357 manufactured by Gun Ei Chemical Industry Co., Ltd.) was used instead of the film forming material for lithography in Example 36 and the drying temperature was 110°C. Then, this underlayer film of novolac was subjected to the etching test mentioned above, and the etching rate was measured.

Next, underlayer films of Examples 36 to 80 and Comparative Examples 2 to 3 were subjected to the etching test in the same way as above, and the etching rate was measured.

Then, the etching resistance was evaluated according to the following evaluation criteria on the basis of the etching rate of the underlayer film of novolac. From a practical viewpoint, evaluation S described below is particularly preferable, and evaluation A and evaluation B are preferable.

### <Evaluation criteria>

S: The etching rate was less than -30% as compared with the underlayer film of novolac.
A: The etching rate was -30% or more to less than -20% as compared with the underlayer film of novolac.
B: The etching rate was -20% or more to less than -10% as compared with the underlayer film of novolac.
C: The etching rate was -10% or more and 0% or less as compared with the underlayer film of novolac.

### [Evaluation of embedding properties to substrate having difference in level]

The embedding properties to a substrate having difference in level were evaluated by the following procedures.

A SiO₂ substrate having a film thickness of 80 nm and a line and space pattern of 60 nm was coated with the composition for film formation for lithography, and baked at 240°C for 60 seconds to form a 90 nm underlayer film. The cross section of the obtained film was cut out and observed under an electron microscope to evaluate the embedding properties to a substrate having difference in level.

### <Evaluation criteria>

A: The underlayer film was embedded without defects in the asperities of the SiO₂ substrate having a line and space pattern of 60 nm.

C: The asperities of the SiO₂ substrate having a line and space pattern of 60 nm had defects which hindered the embedding of the underlayer film.

### [Evaluation of flatness]

Onto a SiO₂ substrate having difference in level on which trenches with a width of 100 nm, a pitch of 150 nm and a depth of 150 nm (aspect ratio: 1.5) and trenches with a width of 5 µm and a depth of 180 nm (open space) were mixedly present, each of the compositions for film formation for lithography was applied. Subsequently, it was calcined at 240°C for 120 seconds under the air atmosphere to form a resist underlayer film having a film thickness of 200 nm. The shape of this resist underlayer film was observed with a scanning electron microscope ("S-4800" from Hitachi High-Technologies Corporation), and the difference between the maximum value and the minimum value of the film thickness of the resist underlayer film on the trench or space (ΔFT) was measured.

### <Evaluation criteria>

S: ΔFT < 10 nm (best flatness)
A: 10 nm ≤ ΔFT < 20 nm (good flatness)
B: 20 nm ≤ ΔFT < 40 nm (partially good flatness)
C: 40 nm ≤ ΔFT (poor flatness)

**[Table 8]**

| | Imide or imide resin | Crosslinking agent | Crosslinking promoting agent | Solvent | Film heat resistance | Etching resistance | Embedding properties | Flatness |
|---|---|---|---|---|---|---|---|---|
| Example 36 | MIBiA-1 (10) | - | - | PGMEA (90) | A | A | A | A |
| Example 37 | MIBiA-2 (10) | - | - | PGMEA (90) | A | A | A | B |
| Example 38 | MIBiA-3 (10) | - | - | PGMEA (90) | A | A | A | B |
| Example 39 | MIBiA-4 (10) | - | - | PGMEA (90) | A | A | A | A |
| Example 40 | MIBiA-5 (10) | - | - | PGMEA (90) | A | A | A | A |
| Example 41 | MIBiA-6 (10) | - | - | PGMEA (90) | A | A | A | A |
| Example 42 | MIBiA-7 (10) | - | - | PGMEA (90) | A | A | A | A |
| Example 43 | MIBiA-8 (10) | - | - | PGMEA (90) | A | A | A | A |
| Example 44 | MIBiA-9 (10) | - | - | PGMEA (90) | A | A | A | A |
| Example 45 | MIBiA-10 (10) | - | - | PGMEA (90) | A | A | A | A |
| Example 46 | MIBiA-11 (10) | - | - | PGMEA (90) | A | A | A | A |
| Example 47 | MIBiA-12 (10) | - | - | PGMEA (90) | A | A | A | A |
| Example 48 | MIBiA-13 (10) | - | - | PGMEA (90) | A | A | A | A |
| Example 49 | MIBiA-14 (10) | - | - | PGMEA (90) | A | A | A | A |
| Example 50 | MIBiA-15 (10) | - | - | PGMEA (90) | A | A | A | A |
| Example 51 | MIBiA-16 (10) | - | - | PGMEA (90) | A | A | A | A |
| Example 52 | MIBiA-17 (10) | - | - | PGMEA (90) | A | A | A | A |
| Example 53 | MIBiA-18 (10) | - | - | PGMEA (90) | A | A | A | A |
| Example 54 | MIBiA-19 (10) | - | - | PGMEA (90) | A | A | A | A |
| Example 55 | MIBiA-20 (10) | - | - | PGMEA (90) | A | A | A | A |
| Example 56 | MIBiA-21 (10) | - | - | PGMEA (90) | A | A | A | A |
| Example 57 | MIBiA-22 (10) | - | - | PGMEA (90) | A | A | A | A |
| Example 58 | MIBiA-23 (10) | - | - | PGMEA (90) | A | A | A | A |
| Example 59 | MIBiA-24 (10) | - | - | PGMEA (90) | A | A | A | A |
| Example 60 | MIBiA-25 (10) | - | - | PGMEA (90) | A | A | A | A |
| Example 61 | MIBiA-26 (10) | - | - | PGMEA (90) | A | A | A | A |
| Example 62 | MIBiA-27 (10) | - | - | PGMEA (90) | A | A | A | A |
| Example 63 | MIBiA-28 (10) | - | - | PGMEA (90) | A | A | A | A |
| Example 64 | MIBiA-29 (10) | - | - | PGMEA (90) | A | A | A | A |
| Example 65 | MIBiA-30 (10) | - | - | PGMEA (90) | A | A | A | A |
| Example 66 | MIBiA-31 (10) | - | - | PGMEA (90) | A | A | A | A |
| Example 67 | MIBiA-32 (10) | - | - | PGMEA (90) | A | A | A | A |
| Example 68 | MIBiA-33 (10) | - | - | PGMEA (90) | A | A | A | A |
| Example 69 | MIBiA-34 (10) | - | - | PGMEA (90) | S | A | A | A |
| Example 70 | RMIBiA-1 (10) | - | - | PGMEA (90) | A | B | B | B |
| Example 71 | MIBiA-1 (10) | - | TPIZ (0.1) | PGMEA (90) | S | S | A | A |
| Example 72 | MIBiA-34 (10) | - | TPIZ (0.1) | PGMEA (90) | S | S | A | A |
| Example 73 | RMIBiA-1 (10) | - | TPIZ(0.1) | PGMEA (90) | S | A | A | A |
| Example 74 | MIBiA-1 (10) | BF-BXZ (2) | TPIZ(0.1) | PGMEA (90) | S | B | A | A |
| Example 75 | MIBiA-1 (10) | NC-3000-L (2) | TPIZ(0.1) | PGMEA (90) | S | B | A | A |
| Example 76 | MIBiA-34 (10) | BF-BXZ (2) | TPIZ (0.1) | PGMEA (90) | S | B | A | A |
| Example 77 | RMIBiA-1 (10) | BF-BXZ (2) | TPIZ(0.1) | PGMEA (90) | S | B | A | A |
| Example 78 | MIBiA-1 (10) | BF-BXZ (2) | - | PGMEA (90) | A | A | A | A |
| Example 79 | MIBiA-34 (10) | BF-BXZ (2) | - | PGMEA (90) | A | A | A | A |
| Example 80 | RMIBiA-1 (10) | BF-BXZ (2) | - | PGMEA (90) | A | B | A | A |
| Comparative Example 2 | CR-1 (10) | NC-3000-L (4) | TPIZ(0.1) | PGMEA (90) | C | C | C | C |
| Comparative Example 3 | CR-1 (10) | - | - | PGMEA (90) | C | C | C | C |

| | | | | | | | | |
|---|---|---|---|---|---|---|---|---|
| * Parts by mass of each component are shown in parentheses. | | | | | | | | |

### <Examples 81 to 84>

Each of the compositions for film formation for lithography of Examples 81 to 84 was prepared according to the compositions shown in Table 9. Then, a silicon substrate was spin coated with each of these compositions for film formation for lithography of Examples 81 to 84, and then baked at 110°C for 60 seconds to remove the solvent in the coated film. Subsequently, the film was cured using a high pressure mercury lamp with an accumulated light exposure of 600 mJ/cm² and an irradiation time of 20 seconds, and further baked at 400°C for 120 seconds to prepare each underlayer film with a film thickness of 200 nm. From the difference in film thickness before and after the baking at 400°C, the decreasing rate of film thickness (%) was calculated to evaluate the film heat resistance of each underlayer film. Then, the etching resistance, embedding properties to a substrate having difference in level and the flatness were evaluated under the conditions shown above.

When used, the following crosslinking agent and radical polymerization initiator were employed.

Crosslinking agent: benzoxazine (BF-BXZ; manufactured by Konishi Chemical Ind. Co., Ltd.) represented by the above formula

Radical polymerization initiator: IRGACURE 184 (manufactured by BASF SE) represented by the following formula

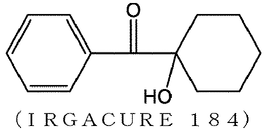

**[Table 9]**

| | Imide or imide resin | Crosslinking agent | Radical polymerization initiator | Solvent | Film heat resistance | Etching resistance | Embedding properties | Flatness |
|---|---|---|---|---|---|---|---|---|
| Example 81 | MIBiA-1 (10) | - | IRGACURE 184 (0.1) | PGMEA (90) | A | S | A | A |
| Example 82 | MIBiA-34 (10) | - | IRGACURE 184 (0.1) | PGMEA (90) | A | S | A | A |
| Example 83 | MIBiA-1 (10) | BF-BXZ(2) | IRGACURE 184 (0.1) | PGMEA (90) | S | A | A | A |
| Example 84 | MIBiA-34 (10) | BF-BXZ(2) | IRGACURE 184 (0.1) | PGMEA (90) | S | A | A | A |

| | | | | | | | | |
|---|---|---|---|---|---|---|---|---|
| * Parts by mass of each component are shown in parentheses. | | | | | | | | |

### <Examples 85 and 86 and Reference Example 1>

Each of the compositions for film formation for lithography of Examples 85 and 86 and Reference Example 1 was prepared according to the compositions shown in Table 10. Then, a silicon substrate was spin coated with each of these compositions for film formation for lithography of Examples 85 and 86 and Reference Example 1, and then baked at 110°C for 60 seconds to remove the solvent in the coated film. Subsequently, the film was cured using a high pressure mercury lamp with an accumulated light exposure of 600 mJ/cm² and an irradiation time of 20 seconds, and further baked at 400°C for 120 seconds to prepare each underlayer film with a film thickness of 200 nm. From the difference in film thickness before and after the baking at 400°C, the decreasing rate of film thickness (%) was calculated to evaluate the film heat resistance of each underlayer film. Then, the etching resistance, embedding properties to a substrate having difference in level and the flatness were evaluated under the conditions shown above.

As the base compound, a phenol compound (BisN-1) represented by the following formula, which is described in International Publication No. WO 2013/024779, was used.

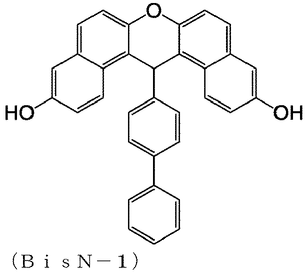

**[Table 10]**

| | Base compound | Bismaleimide and/or maleimide resin | Solvent | Film heat resistance | Etching resistance | Embedding properties | Flatness |
|---|---|---|---|---|---|---|---|
| Example 85 | BisN-1 (8) | MIBiA-1 (2) | PGMEA (90) | A | A | A | A |
| Example 86 | BisN-1 (8) | MIBiA-34 (2) | PGMEA (90) | A | A | A | A |
| Reference Example 1 | BisN-1 (10) | - | PGMEA (90) | B | A | A | A |

| | | | | | | | |
|---|---|---|---|---|---|---|---|
| * Parts by mass of each component are shown in parentheses. | | | | | | | |

### <Example 87>

A SiO₂ substrate with a film thickness of 300 nm was coated with the composition for film formation for lithography in Example 71, and baked at 240°C for 60 seconds and further at 400°C for 120 seconds to form an underlayer film with a film thickness of 70 nm. This underlayer film was coated with a resist solution for ArF and baked at 130°C for 60 seconds to form a photoresist layer with a film thickness of 140 nm. The resist solution for ArF used was prepared by compounding 5 parts by mass of a compound of the following formula (ArF-R), 1 part by mass of triphenylsulfonium nonafluoromethanesulfonate, 2 parts by mass of tributylamine, and 92 parts by mass of PGMEA.

Note that the compound of the following formula (ArF-R) was prepared as follows. 4.15 g of 2-methyl-2-methacryloyloxyadamantane, 3.00 g of methacryloyloxy-γ-butyrolactone, 2.08 g of 3-hydroxy-1-adamantyl methacrylate, and 0.38 g of azobisisobutyronitrile were dissolved in 80 mL of tetrahydrofuran to prepare a reaction solution. This reaction solution was polymerized for 22 hours with the reaction temperature kept at 63°C in a nitrogen atmosphere. Then, the reaction solution was added dropwise into 400 mL of n-hexane. The product resin thus obtained was solidified and purified, and the resulting white powder was filtered and dried overnight at 40°C under reduced pressure to obtain a compound represented by the following formula.

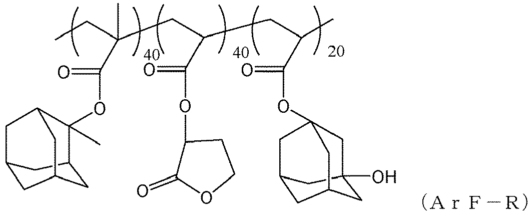

In the above formula (ArF-R), each of the numerical values 40, 40 and 20 represents the ratio of each constituent unit, and does not mean that the compound is a block copolymer.

Subsequently, the photoresist layer was exposed using an electron beam lithography system (manufactured by ELIONIX INC.; ELS-7500, 50 keV), baked (PEB) at 115°C for 90 seconds, and developed for 60 seconds in 2.38% by mass tetramethylammonium hydroxide (TMAH) aqueous solution to obtain a positive type resist pattern. The evaluation results are shown in Table 11.

### <Example 88>

A positive type resist pattern was obtained in the same way as Example 87 except that the composition for underlayer film formation for lithography in Example 72 was used instead of the composition for underlayer film formation for lithography in the above Example 71. The evaluation results are shown in Table 11.

### <Example 89>

A positive type resist pattern was obtained in the same way as Example 87 except that the composition for underlayer film formation for lithography in Example 73 was used instead of the composition for underlayer film formation for lithography in the above Example 71. The evaluation results are shown in Table 11.

### <Comparative Example 3>

The same operations as in Example 87 were performed except that no underlayer film was formed so that a photoresist layer was formed directly on a SiO₂ substrate to obtain a positive type resist pattern. The evaluation results are shown in Table 11.

### [Evaluation]

Concerning each of Examples 87 to 89 and Comparative Example 4, the shapes of the obtained 80 nm L/S (1:1) resist patterns were observed under an electron microscope (S-4800) manufactured by Hitachi, Ltd. The shapes of the resist patterns after development were evaluated as "goodness" when having good rectangularity without pattern collapse, and as "poorness" if this was not the case.

**[Table 11]**

| | Composition for film formation for lithography | Resist pattern shape after development |
|---|---|---|
| Example 87 | Composition described in Example 71 | Good |
| Example 88 | Composition described in Example 72 | Good |
| Example 89 | Composition described in Example 73 | Good |
| Comparative Example 4 | None | Poor |

As is evident from Table 11, in Examples 87 to 89 using the composition for film formation for lithography containing the maleimide compound or maleimide resin of the present embodiment, the resist pattern shapes after development were confirmed to have good rectangularity without pattern collapse, compared to Comparative Example 4. The difference in the resist pattern shapes after development indicated that the underlayer films of Examples 87 to 89 obtained from the compositions for film formation for lithography of Examples 71 to 73 have good adhesiveness to a resist material.

The present application is based on Japanese Patent Application No. 2018-034327 filed in the Japan Patent Office on February 28, 2018, the contents of which are incorporated herein by reference.

### Industrial Applicability

The compound of the present invention ((poly)imide compound or (poly)amic acid) and the resin containing units derived therefrom have, for example, excellent structure forming ability (film forming ability), heat resistance and solubility, and can be usefully used as a high heat resistant thermosetting resin.

## Claims

1. A compound represented by the following formula (0): 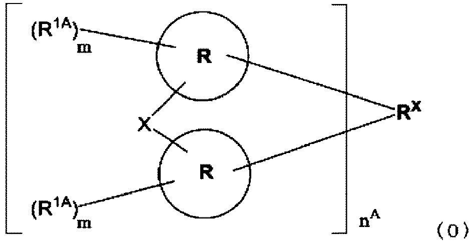 wherein
R^{X} represents a 2n^{A}-valent group having 1 to 70 carbon atoms or a single bond;
each R^{1A} independently represents any of an alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, an alkoxy group having 1 to 30 carbon atoms and optionally having a substituent, a maleamic acid group having 4 to 30 carbon atoms and optionally having a substituent, a maleimide group having 4 to 30 carbon atoms and optionally having a substituent, a halogen atom, a nitro group, an amino group having 0 to 30 carbon atoms and optionally having a substituent, a carboxyl group, a thiol group and a hydroxy group, wherein, when R^{1A} is any of the alkyl group, the aryl group, the crosslinkable group and the alkoxy group, at least one bond selected from the group consisting of an ether bond, a ketone bond and an ester bond is optionally contained, and at least one R^{1A} is any of a maleamic acid group having 4 to 30 carbon atoms and optionally having a substituent and a maleimide group having 4 to 30 carbon atoms and optionally having a substituent;
X represents an oxygen atom or a sulfur atom, or is optionally not present;
each R independently represents any of a benzene ring, a naphthalene ring and an anthracene ring;
each m is independently an integer of 0 to 9, wherein at least one m is an integer of 1 to 9; and
n^{A} is an integer of 1 to 4.

2. The compound according to claim 1, wherein the compound represented by the above formula (0) is a compound represented by the following formula (1): 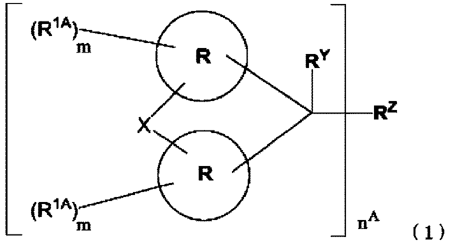 wherein
R^{Y} represents any of a hydrogen atom, a linear or branched, or cyclic alkyl group having 1 to 30 carbon atoms and optionally having a substituent, and an aryl group having 6 to 30 carbon atoms and optionally having a substituent;
R^{Z} represents an n^{A}-valent group having 1 to 60 carbon atoms or a single bond, and the total number of carbon atoms in R^{Y} and R^{Z} is 69 or less; and
X, R, n^{A}, R^{1A} and m are as defined above.

3. The compound according to claim 2, wherein the compound represented by the above formula (1) is a compound represented by the following formula (1a-1): 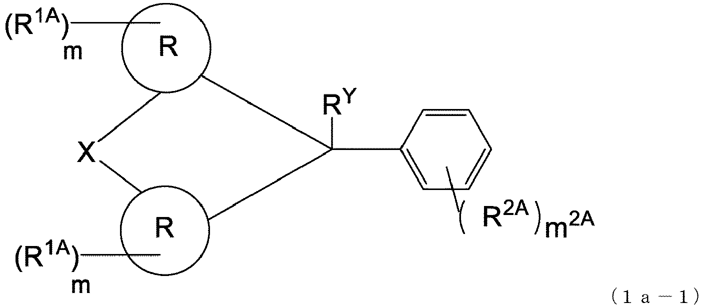 wherein, X, R, R^{1A}, m and R^{Y} are as defined above; R^{2A} represents any of a hydrogen atom, a linear or branched, or cyclic alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, and a halogen atom; and m^{2A} represents an integer of 1 to 5.

4. The compound according to claim 2, wherein the compound represented by the above formula (1) is a compound represented by the following formula (1b-1): 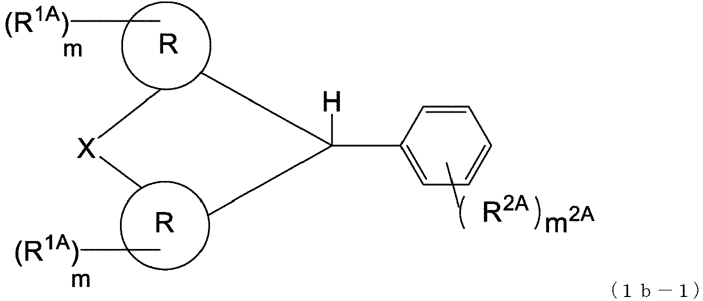 wherein, X, R, R^{1A} and m are as defined above; R^{2A} represents any of a hydrogen atom, a linear or branched, or cyclic alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, and a halogen atom; and m^{2A} represents an integer of 1 to 5.

5. The compound according to claim 2, wherein the compound represented by the above formula (1) is a compound represented by the following formula (1a-2): 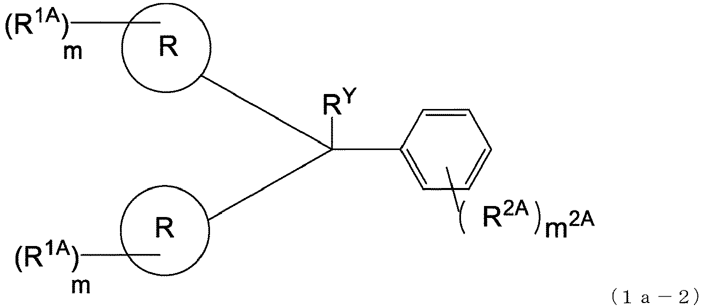 wherein, R, R^{1A}, m and R^{Y} are as defined above; R^{2A} represents any of a hydrogen atom, a linear or branched, or cyclic alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, and a halogen atom; and m^{2A} represents an integer of 1 to 5.

6. The compound according to claim 2, wherein the compound represented by the above formula (1) is a compound represented by the following formula (1b-2): 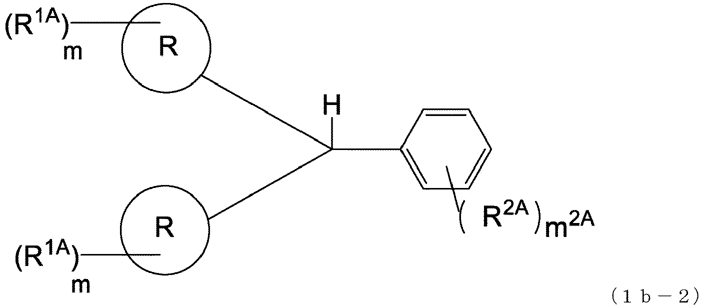 wherein, R, R^{1A} and m are as defined above; R^{2A} represents any of a hydrogen atom, a linear or branched, or cyclic alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, and a halogen atom; and m^{2A} represents an integer of 1 to 5.

7. The compound according to claim 5, wherein the compound represented by the above formula (1a-2) is a compound represented by the following formula (1a-3): 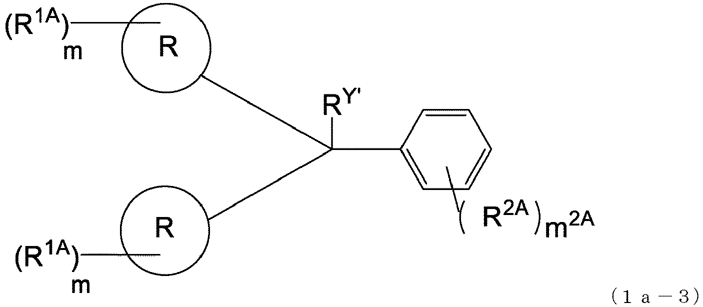 wherein, R, R^{1A}, R^{2A}, m and m^{2A} are as defined above; and R^{Y'} represents R^{Y} except for a hydrogen atom.

8. The compound according to claim 6, wherein the compound represented by the above formula (1b-2) is a compound represented by the following formula (1b-3): 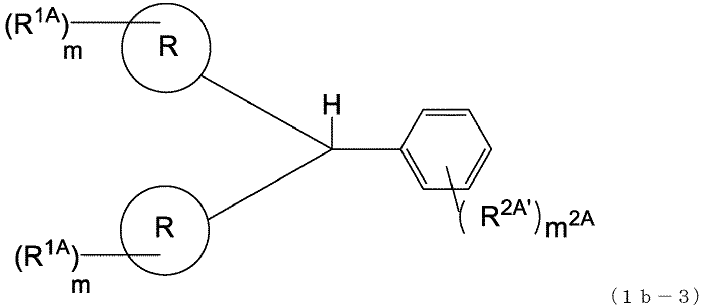 wherein, R, R^{1A}, m and m^{2A} are as defined above; and R^{2A'} represents a linear or branched, or cyclic alkyl group having 5 or more carbon atoms and optionally having a substituent.

9. The compound according to claim 2, wherein the compound represented by the above formula (1) is a compound represented by the following formula (2) or formula (2'): 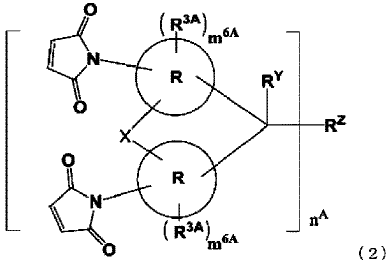 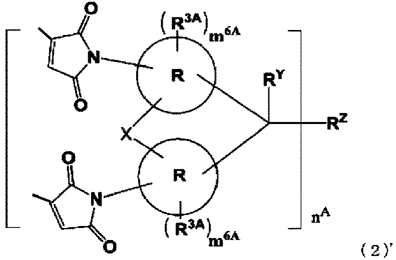 wherein
each R^{3A} independently represents any of a linear or branched, or cyclic alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, an alkoxy group having 1 to 30 carbon atoms and optionally having a substituent, and a halogen atom;
each m^{6A} is independently an integer of 0 to 5; and
R^{Y}, R^{Z}, X, R and n^{A} are as defined above.

10. The compound according to claim 2, wherein the compound represented by the above formula (1) is a compound represented by the following formula (3) or formula (3'): 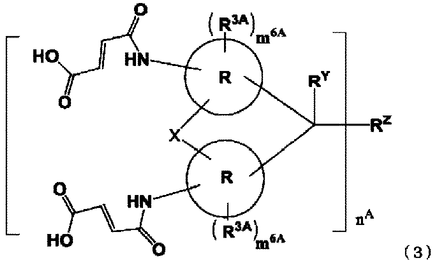 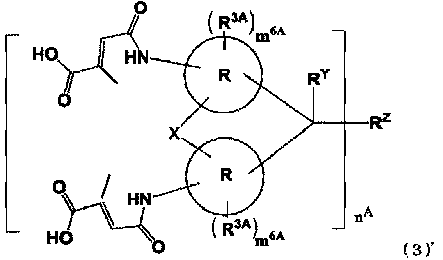 wherein
each R^{3A} is independently any of a linear or branched, or cyclic alkyl group having 1 to 30 carbon atoms and optionally having a substituent, an aryl group having 6 to 30 carbon atoms and optionally having a substituent, a crosslinkable group having 2 to 30 carbon atoms and optionally having a substituent, an alkoxy group having 1 to 30 carbon atoms and optionally having a substituent, and a halogen atom;
each m^{6A} is independently an integer of 0 to 5; and
R^{Y}, R^{Z}, X, R and n^{A} are as defined above.

11. The compound according to any one of claims 1 to 10, wherein X is an oxygen atom in the above formula (0), (1), (2), (2)', (3) or (3) ' .

12. The compound according to claim 2, wherein the compound represented by the above formula (1) is any of compounds represented by the following formulas (MIBiA-1) to (MIBiA-34) and (MABiA-1) to (MABiA-34):

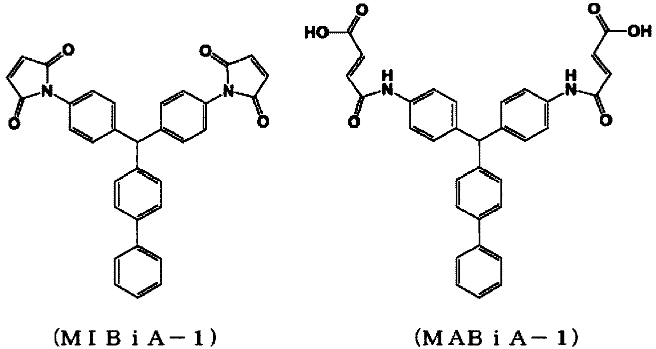

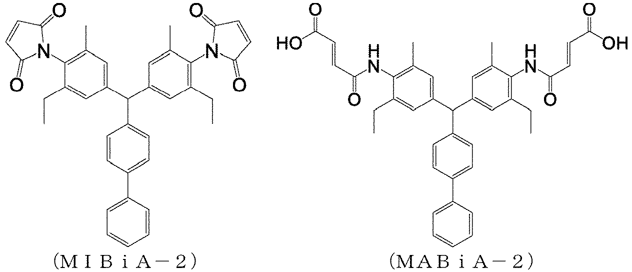

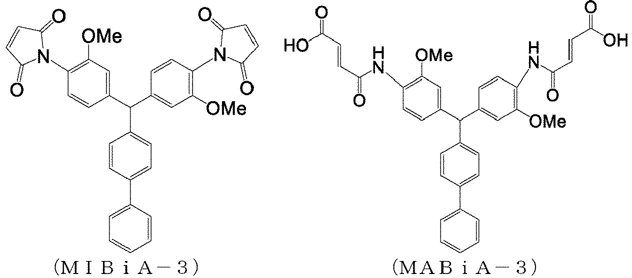

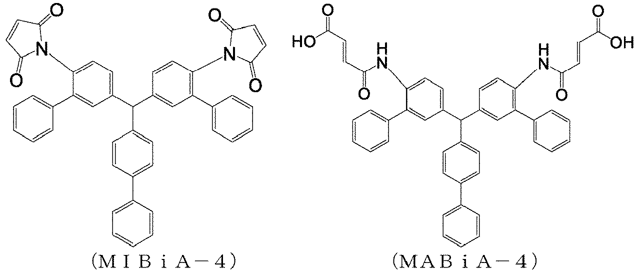

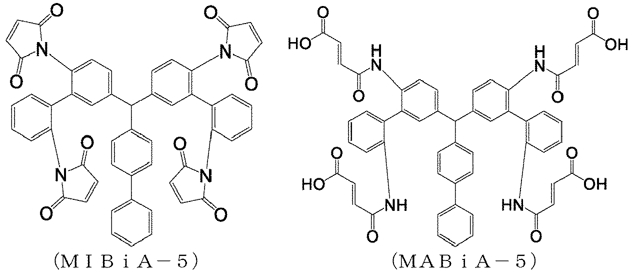

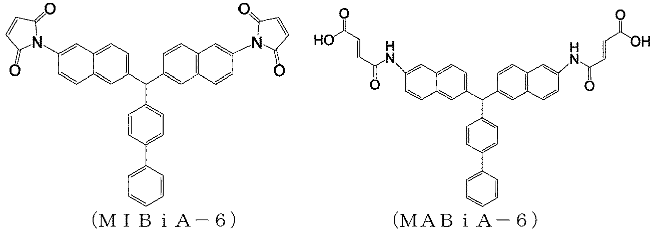

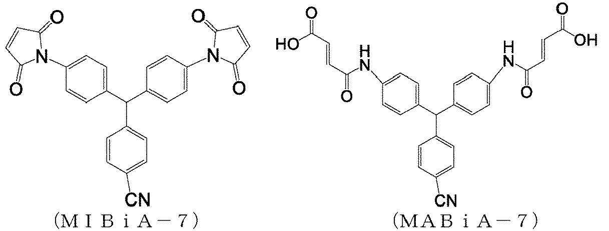

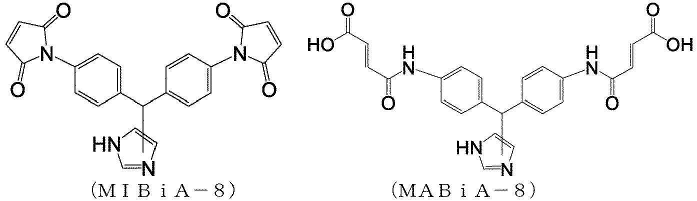

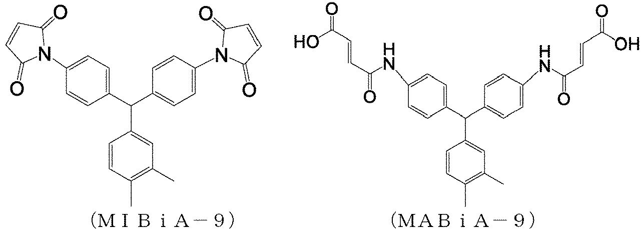

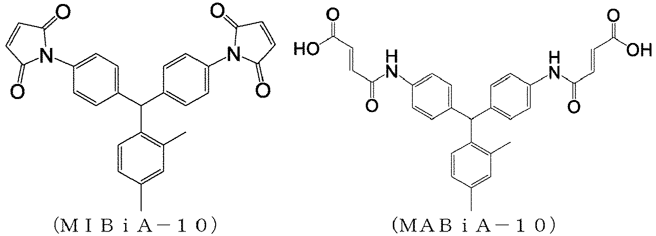

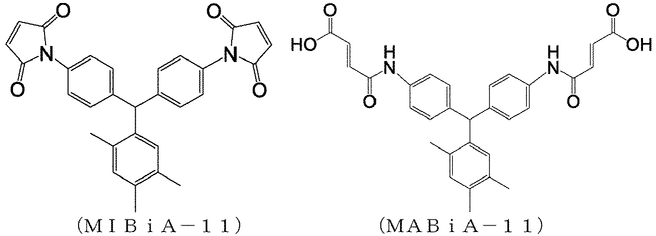

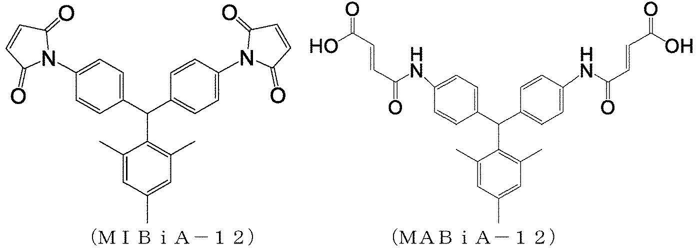

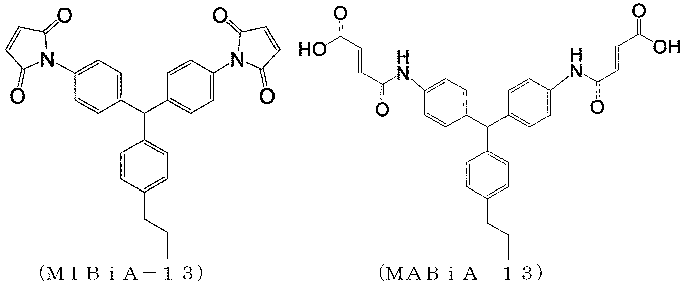

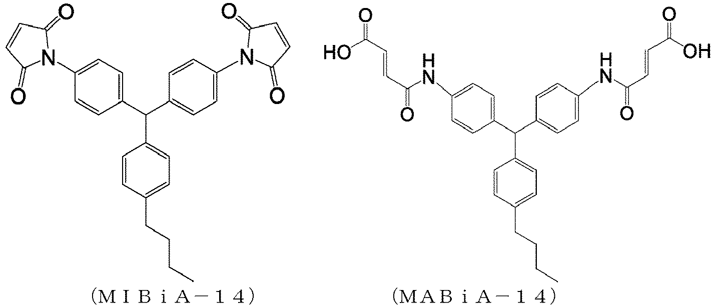

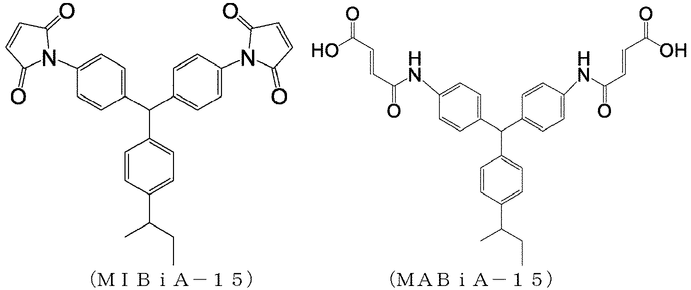

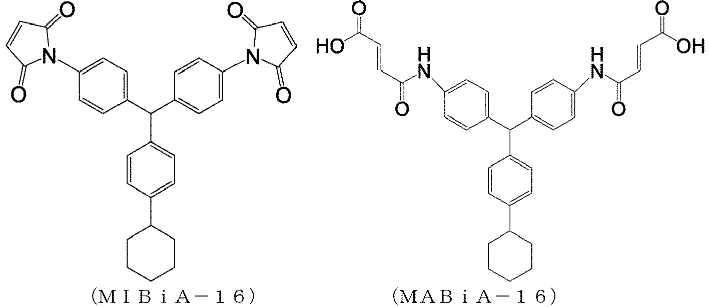

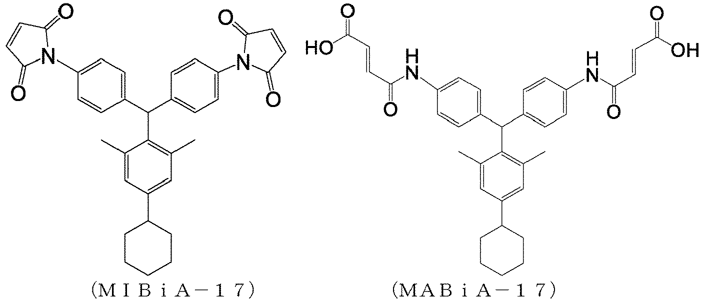

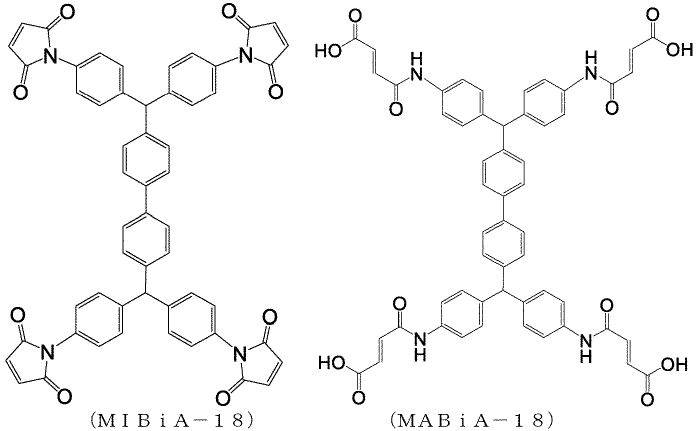

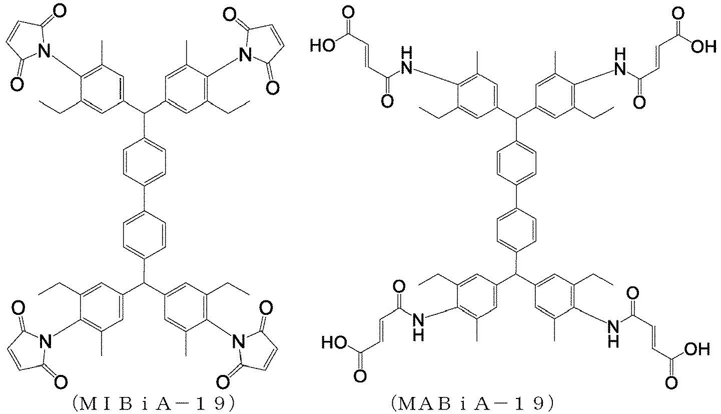

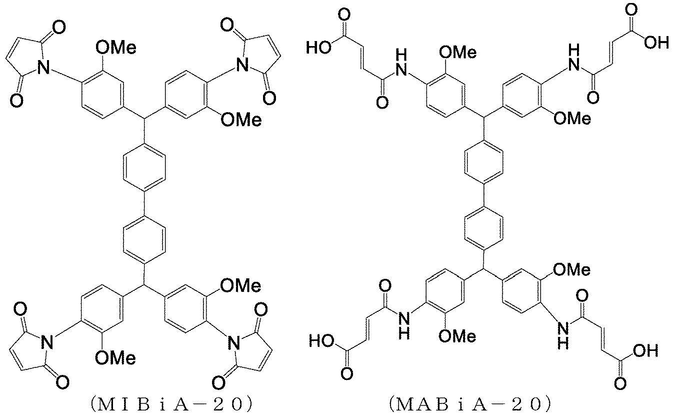

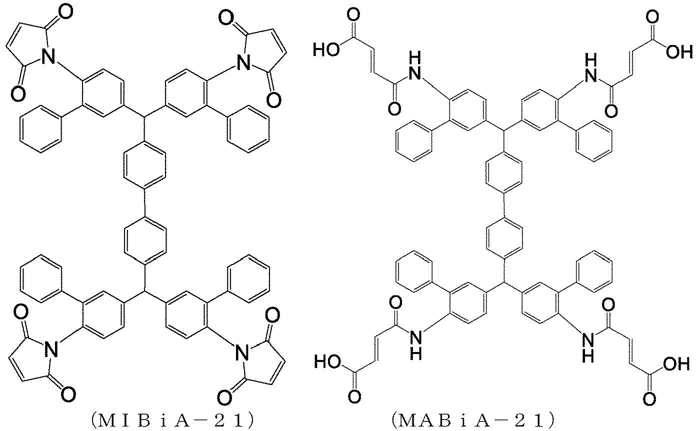

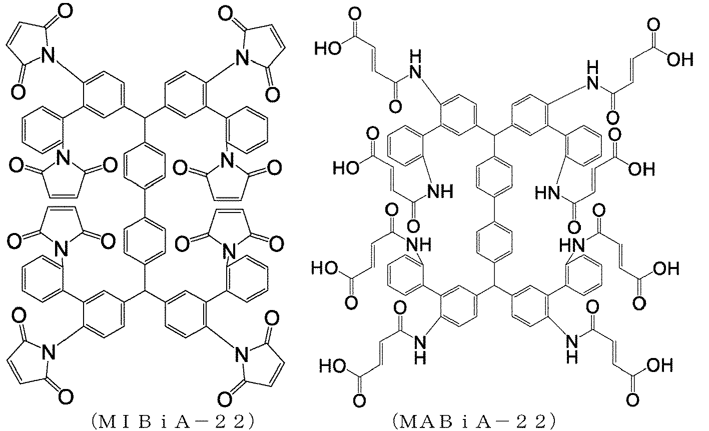

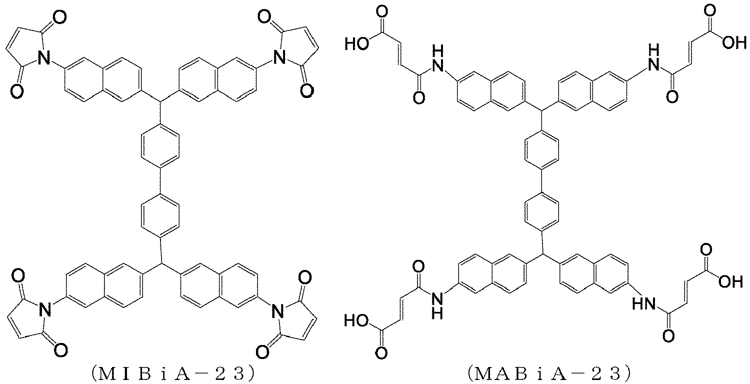

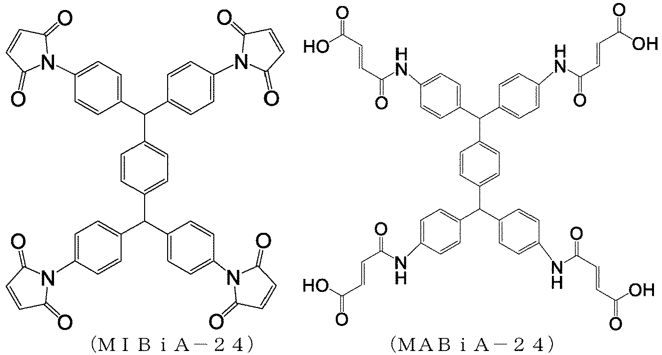

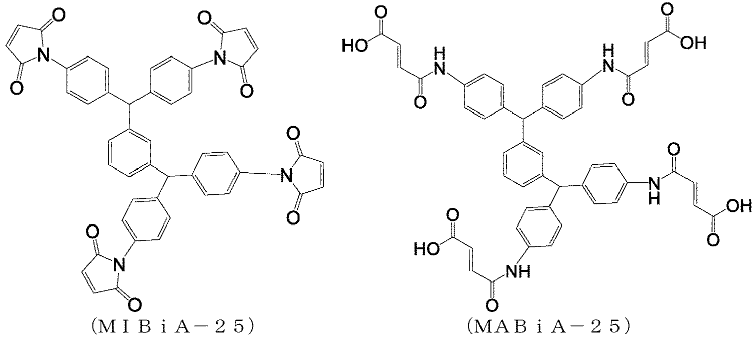

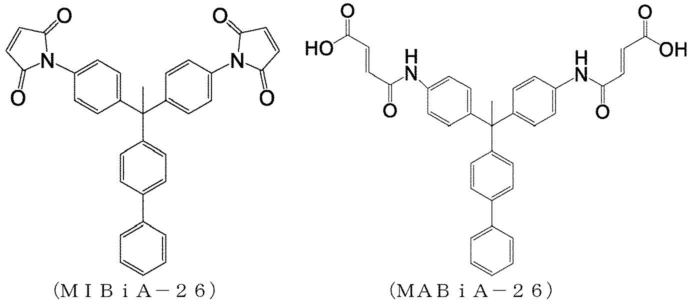

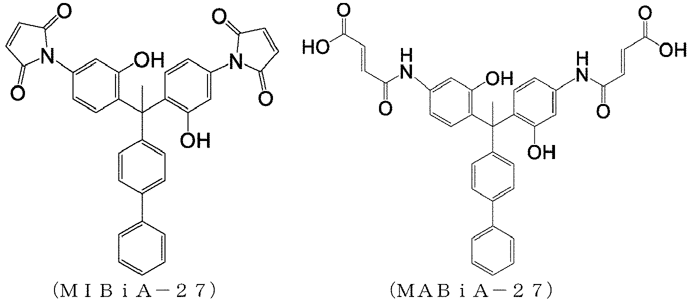

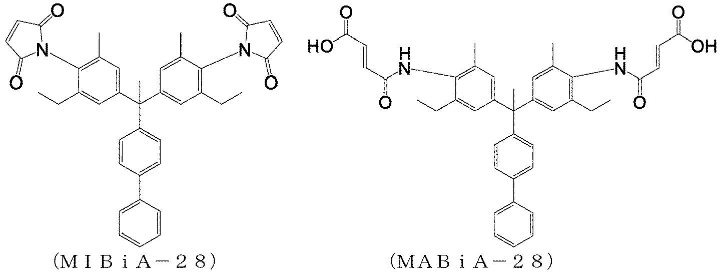

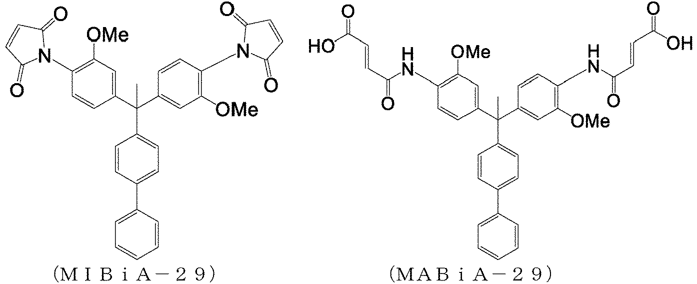

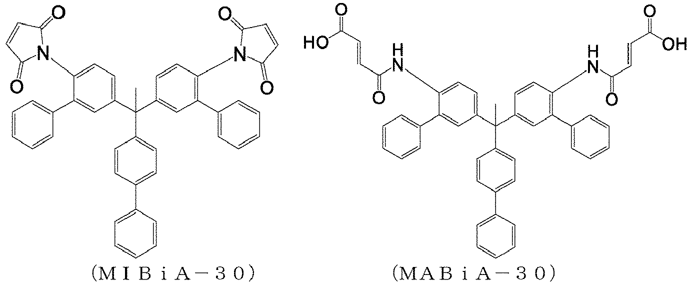

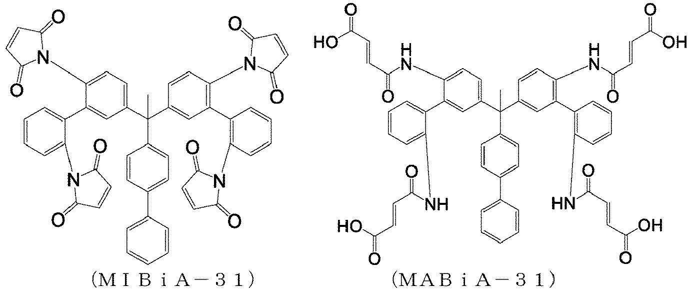

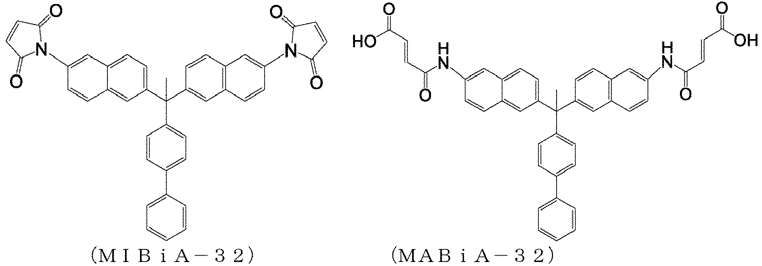

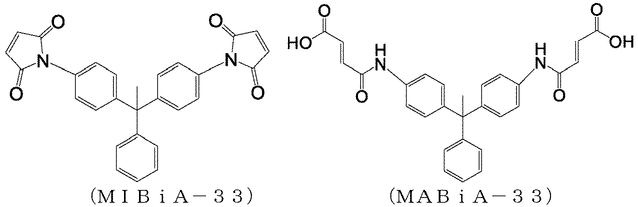

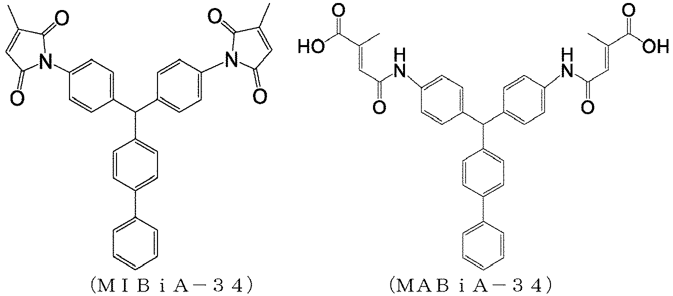

13. A resin comprising a monomer unit derived from the compound according to any one of claims 1 to 12.

14. The resin according to claim 13, wherein the monomer unit is a unit represented by the following formula (4): 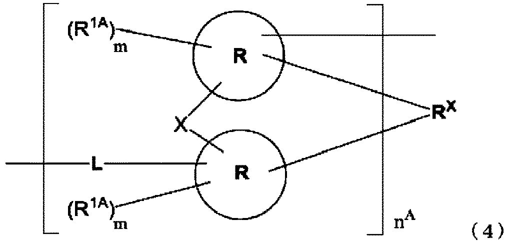 wherein
L represents a linear or branched linking group having 1 to 30 carbon atoms, or a single bond; and
R^{X}, R^{1A}, X, R, m and n^{A} are as defined above.

15. A composition comprising the compound or the resin according to any one of claims 1 to 14.

16. A cured product obtained by curing the composition according to claim 15.

17. A film forming material for lithography comprising the compound or the resin according to any one of claims 1 to 14.

18. The film forming material for lithography according to claim 17, further comprising a crosslinking agent.

19. The film forming material for lithography according to claim 17 or 18, further comprising a crosslinking promoting agent.

20. The film forming material for lithography according to any one of claims 17 to 19, further comprising a radical polymerization initiator.

21. A composition for film formation for lithography comprising the film forming material for lithography according to any one of claims 17 to 21 and a solvent.

22. The composition for film formation for lithography according to claim 21, further comprising an acid generating agent.

23. The composition for film formation for lithography according to claim 21 or 22, wherein the film for lithography is an underlayer film for lithography.

24. An underlayer film for lithography formed by using the composition for film formation for lithography according to claim 23.

25. A method for forming a resist pattern, comprising the steps of:
forming an underlayer film on a substrate by using the composition for film formation for lithography according to claim 23;
forming at least one photoresist layer on the underlayer film; and
irradiating a predetermined region of the photoresist layer with radiation for development.

26. A method for forming a circuit pattern, comprising the steps of:
forming an underlayer film on a substrate by using the composition for film formation for lithography according to claim 23;
forming an intermediate layer film on the underlayer film by using a resist intermediate layer film material containing a silicon atom;
forming at least one photoresist layer on the intermediate layer film;
irradiating a predetermined region of the photoresist layer with radiation for development, thereby forming a resist pattern;
etching the intermediate layer film with the resist pattern as a mask;
etching the underlayer film with the obtained intermediate layer film pattern as an etching mask; and
etching the substrate with the obtained underlayer film pattern as an etching mask, thereby forming a pattern on the substrate.

27. A method for purifying a film forming material for lithography, comprising the steps of:
obtaining an organic phase by dissolving the film forming material for lithography according to any one of claims 17 to 20 in a solvent; and
extracting impurities in the film forming material for lithography by bringing the organic phase into contact with an acidic aqueous solution (a first extraction step),
wherein
the solvent used in the step of obtaining the organic phase contains a solvent that does not inadvertently mix with water.
